(19) Europäisches Patentamt
European Patent Office
Office européen des brevets

(11) EP 3 903 830 A1

(12) EUROPEAN PATENT APPLICATION
published in accordance with Art. 153(4) EPC

(43) Date of publication:
**03.11.2021 Bulletin 2021/44**

(21) Application number: **19902173.4**

(22) Date of filing: **27.12.2019**

(51) Int Cl.:
***A61K 48/00*** (2006.01) ***C12N 15/113*** (2010.01)
***A61P 3/06*** (2006.01)

(86) International application number:
**PCT/CN2019/129016**

(87) International publication number:
**WO 2020/135673 (02.07.2020 Gazette 2020/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2018 CN 201811622633**

(71) Applicant: **Suzhou Ribo Life Science Co., Ltd.**
**Kunshan, Jiangsu 215300 (CN)**

(72) Inventors:
- **ZHANG, Hongyan**
**Kunshan, Jiangsu 215300 (CN)**
- **GAO, Shan**
**Kunshan, Jiangsu 215300 (CN)**
- **KANG, Daiwu**
**Kunshan, Jiangsu 215300 (CN)**

(74) Representative: **SSM Sandmair**
**Patentanwälte Rechtsanwalt**
**Partnerschaft mbB**
**Joseph-Wild-Straße 20**
**81829 München (DE)**

# (54) NUCLEIC ACID, COMPOSITION AND CONJUGATE CONTAINING NUCLEIC ACID, PREPARATION METHOD THEREFOR AND USE THEREOF

(57) Provided are an siRNA for inhibiting the expression of the apolipoprotein C3 gene, and a pharmaceutical composition and a conjugate containing the siRNA. Each nucleotide in the siRNA is, respectively and independently, a modified or unmodified nucleotide; the siRNA contains a sense strand and an anti-sense strand; the sense strand includes nucleotide sequence I; nucleotide sequence I has the same length as the nucleotide sequence as shown in SEQ ID NO: 1, and not more than three nucleotides are different; the anti-sense strand contains nucleotide sequence II; and nucleotide sequence II has the same length as the nucleotide sequence as shown in SEQ ID NO: 2, and not more than three nucleotides are different. The siRNA provided by the present disclosure and the pharmaceutical composition and the conjugate thereof can effectively treat and/or prevent dyslipidemia.

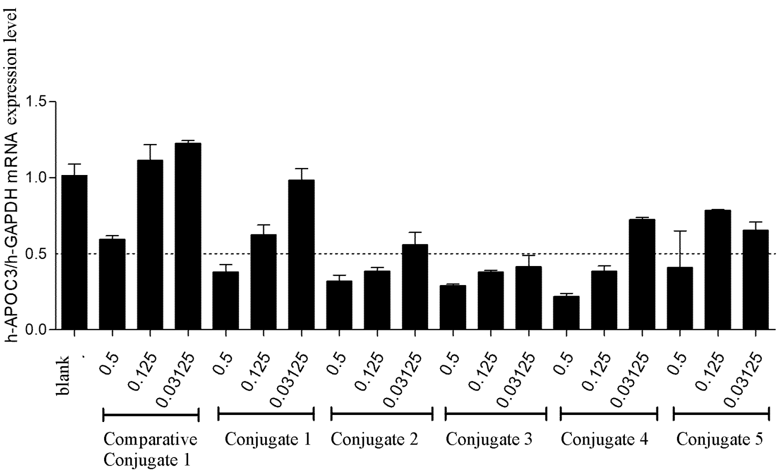


Figure 1

EP 3 903 830 A1

## Description

### TECHNICAL FIELD

The present disclosure relates to a nucleic acid capable of inhibiting the expression of Apolipoprotein C3 (APOC3) gene, a composition and a conjugate comprising the same. The present disclosure also relates to a preparation method and use of such nucleic acids, compositions and conjugates.

### BACKGROUND ART

Dyslipidemia (also known as hyperlipidemia), referring to a systemic disease in which abnormal metabolism and transportation of fat cause a higher plasma lipid content than the normal value, severely threatens the health of patients worldwide. The currently available medicines for treating dyslipidemia include mainly statins, cholesterol absorption inhibitors, resins, probucol, fibrates, niacins, and derivatives thereof.

Apolipoprotein C3 (APOC3) plays a crucial role in lipid metabolism. In the people carrying APOC3 mutant gene, the expression level of APOC3 in blood circulation reduces by 46%, and the triglyceride level in plasma reduces by 39% as compared with normal population. Meanwhile, the relatively low blood lipid level can reduce the risk of developing heart diseases in people carrying APOC3 mutant gene by 35.1% as compared with those not carrying said gene. Thus, silencing gene expression of APOC3 gene at genetic level to block APOC3 production is undoubtedly the most ideal treatment means. Based on the mechanism of RNA interference (RNAi), small interfering RNA (siRNA) could inhibit or block the expression of any target gene of interest in a sequence-specific manner, so as to achieve the purpose of treating diseases.

Suitable sequences and modification of siRNA and the delivery system thereof are two crucial technolgies in the development of siRNA medicines.

### SUMMARY OF THE INVENTION

In some embodiments, the present disclosure provides a siRNA conjugate having a structure as shown by Formula (308):

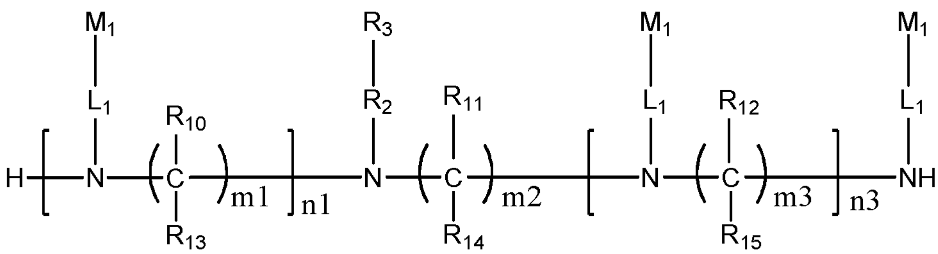

Formula (308),

wherein

n1 is an integer of 1-3, and n3 is an integer of 0-4;

m1, m2, and m3 independently of one another are an integer of 2-10;

$R_{10}$, $R_{11}$, $R_{12}$, $R_{13}$, $R_{14}$, and $R_{15}$ independently of one another are H, or selected from the group consisting of $C_1$-$C_{10}$ alkyl, $C_1$-$C_{10}$ haloalkyl, and $C_1$-$C_{10}$ alkoxy;

$R_3$ is a group having a structure as shown by Formula (A59):

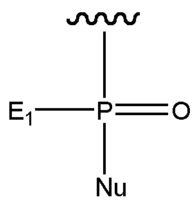

Formula (A59)

wherein,

$E_1$ is OH, SH or $BH_2$;
Nu is a siRNA;
the siRNA comprises a sense strand and an antisense strand; each nucleotide in the siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; the nucleotide sequence I and the nucleotide sequence II are the sequences selected from one of the following groups i)-v):

i) the nucleotide sequence I and the nucleotide sequence as shown by SEQ ID NO: 1 have an equal length and no more than 3 nucleotide differences, and the nucleotide sequence II and the nucleotide sequence as shown by SEQ ID NO: 2 have an equal length and no more than 3 nucleotide differences:

5'-UUAAAAGGGACAGUAUUC$Z_{a1}$-3' (SEQ ID NO: 1),
5'-$Z_{a2}$GAAUACUGUCCCUUUUAA-3' (SEQ ID NO: 2),

wherein, $Z_{a1}$ is A and $Z_{a2}$ is U, and
the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$, wherein $Z_{a4}$ is the first nucleotide at 5' terminal of the antisense strand; or

ii) the nucleotide sequence I and the nucleotide sequence as shown by SEQ ID NO: 13 have an equal length and no more than 3 nucleotide differences, and the nucleotide sequence II and the nucleotide sequence as shown by SEQ ID NO: 14 have an equal length and no more than 3 nucleotide differences:

5'-ACAGUAUUCUCAGUGCUC$Z_{b1}$-3' (SEQ ID NO: 13),
5'-$Z_{b2}$GAGCACUGAGAAUACUGU-3' (SEQ ID NO: 14),

wherein, $Z_{b1}$ is A and $Z_{b2}$ is U, and
the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$, wherein $Z_{b4}$ is the first nucleotide at 5' terminal of the antisense strand; or

iii) the nucleotide sequence I and the nucleotide sequence as shown by SEQ ID NO: 25 have an equal length and no more than 3 nucleotide differences, and the nucleotide sequence II and the nucleotide sequence as shown by SEQ ID NO: 26 have an equal length and no more than 3 nucleotide differences:

5'-UAUUCUCAGUGCUCUCCU$Z_{c1}$-3' (SEQ ID NO: 25),
5'-$Z_{c2}$AGGAGAGCACUGAGAAUA-3' (SEQ ID NO: 26),

wherein, $Z_{c1}$ is A and $Z_{c2}$ is U, and
the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$, wherein $Z_{c4}$ is the first nucleotide at 5' terminal of the antisense strand; or

iv) the nucleotide sequence I and the nucleotide sequence as shown by SEQ ID NO: 37 have an equal length and no more than 3 nucleotide differences, and the nucleotide sequence II and the nucleotide sequence as shown by SEQ ID NO: 38 have an equal length and no more than 3 nucleotide differences:

5'-AGUAUUCUCAGUGCUCUC$Z_{d1}$-3' (SEQ ID NO: 37),
5'-$Z_{d2}$GAGAGCACUGAGAAUACU-3' (SEQ ID NO: 38),

wherein, $Z_{d1}$ is A and $Z_{d2}$ is U, and
the nucleotide sequence I comprises a nucleotide $Z_{d3}$ at the position corresponding to $Z_{d1}$; the nucleotide sequence II comprises a nucleotide $Z_{d4}$ at the position corresponding to $Z_{d2}$, wherein $Z_{d4}$ is the first nucleotide at 5' terminal of the antisense strand; or

v) the nucleotide sequence I and the nucleotide sequence as shown by SEQ ID NO: 49 have an equal length and no more than 3 nucleotide differences, and the nucleotide sequence II and the nucleotide sequence as shown by SEQ ID NO: 50 have an equal length and no more than 3 nucleotide differences:

5'-GGACAGUAUUCUCAGUGC$Z_{e1}$-3' (SEQ ID NO: 49),
5'-$Z_{e2}$GCACUGAGAAUACUGUCC-3' (SEQ ID NO: 50),

wherein, $Z_{e1}$ is A and $Z_{e2}$ is U, and
the nucleotide sequence I comprises a nucleotide $Z_{e3}$ at the position corresponding to $Z_{e1}$; the nucleotide sequence II comprises a nucleotide $Z_{e4}$ at the position corresponding to $Z_{e2}$, wherein $Z_{e4}$ is the first nucleotide at 5' terminal of the antisense strand;

$R_2$ is a linear alkylene of 1 to 20 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -CON$H_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl,-$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl);
each $L_1$ is a linear alkylene of 1 to 70 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -CON$H_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl,-$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl);
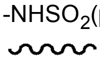 represents the site where a group is linked to the rest of the molecule; and $M_1$ represents a targeting group.

In some embodiments, the present disclosure provides a siRNA capable of inhibiting the expression of APOC3 gene, wherein the siRNA comprises a sense strand and an antisense strand; each nucleotide in the sense strand and the antisense strand is independently a fluoro modified nucleotide and a non-fluoro modified nucleotide; wherein the sense strand comprises a nucleotide sequence I and the antisense strand comprises a nucleotide sequence II; the nucleotide sequence I and the nucleotide sequence II are at least partly reverse complementary to form a double-stranded region; the fluoro modified nucleotides are located in the nucleotide sequence I and the nucleotide sequence II; and in the direction from 5' terminal to 3' terminal, the nucleotides at positions 7, 8 and 9 of the nucleotide sequence I in the sense strand are fluoro modified nucleotides, and the nucleotides at the rest of positions in the sense strand of the siRNA are non-fluoro modified nucleotides; in the direction from 5' terminal to 3' terminal, the nucleotides at positions 2, 6, 14, and 16 of the nucleotide sequence II in the antisense strand are fluoro modified nucleotides, and the nucleotides at the rest of positions in the antisense strand of the siRNA are non-fluoro modified nucleotides; and the nucleotide sequence I and the nucleotide sequence II are the sequences selected from one of the following groups i)-v):

i) the nucleotide sequence I and the nucleotide sequence as shown by SEQ ID NO: 1 have an equal length and no more than 3 nucleotide differences, and the nucleotide sequence II and the nucleotide sequence as shown by SEQ ID NO: 2 have an equal length and no more than 3 nucleotide differences:

5'-UUAAAAGGGACAGUAUUC$Z_{a1}$-3' (SEQ ID NO: 1),
5'-$Z_{a2}$GAAUACUGUCCCUUUUAA-3' (SEQ ID NO: 2),

wherein, $Z_{a1}$ is A and $Z_{a2}$ is U, and
the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$, wherein $Z_{a4}$ is the first nucleotide at 5' terminal of the antisense strand; or

ii) the nucleotide sequence I and the nucleotide sequence as shown by SEQ ID NO: 13 have an equal length and no more than 3 nucleotide differences, and the nucleotide sequence II and the nucleotide sequence as shown by SEQ ID NO: 14 have an equal length and no more than 3 nucleotide differences:

5'-ACAGUAUUCUCAGUGCUC$Z_{b1}$-3' (SEQ ID NO: 13),
5'-$Z_{b2}$GAGCACUGAGAAUACUGU-3' (SEQ ID NO: 14),

wherein, $Z_{b1}$ is A and $Z_{b2}$ is U, and
the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$, wherein $Z_{b4}$ is the first nucleotide at 5' terminal of the antisense strand; or

iii) the nucleotide sequence I and the nucleotide sequence as shown by SEQ ID NO: 25 have an equal length and no more than 3 nucleotide differences, and the nucleotide sequence II and the nucleotide sequence as shown by SEQ ID NO: 26 have an equal length and no more than 3 nucleotide differences:

5'-UAUUCUCAGUGCUCUCCU$Z_{c1}$-3' (SEQ ID NO: 25),
5'-$Z_{c2}$AGGAGAGCACUGAGAAUA-3' (SEQ ID NO: 26),

wherein, $Z_{c1}$ is A and $Z_{c2}$ is U, and
the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$, wherein $Z_{c4}$ is the first nucleotide at 5' terminal of the antisense strand; or

iv) the nucleotide sequence I and the nucleotide sequence as shown by SEQ ID NO: 37 have an equal length and no more than 3 nucleotide differences, and the nucleotide sequence II and the nucleotide sequence as shown by SEQ ID NO: 38 have an equal length and no more than 3 nucleotide differences:

5'-AGUAUUCUCAGUGCUCUC$Z_{d1}$-3' (SEQ ID NO: 37),
5'-$Z_{d2}$GAGAGCACUGAGAAUACU-3' (SEQ ID NO: 38),

wherein, $Z_{d1}$ is A and $Z_{d2}$ is U, and
the nucleotide sequence I comprises a nucleotide $Z_{d3}$ at the position corresponding to $Z_{d1}$; the nucleotide sequence II comprises a nucleotide $Z_{d4}$ at the position corresponding to $Z_{d2}$, wherein $Z_{d4}$ is the first nucleotide at 5' terminal of the antisense strand; or

v) the nucleotide sequence I and the nucleotide sequence as shown by SEQ ID NO: 49 have an equal length and no more than 3 nucleotide differences, and the nucleotide sequence II and the nucleotide sequence as shown by SEQ ID NO: 50 have an equal length and no more than 3 nucleotide differences:

5'-GGACAGUAUUCUCAGUGC$Z_{e1}$-3' (SEQ ID NO: 49),
5'-$Z_{e2}$GCACUGAGAAUACUGUCC-3' (SEQ ID NO: 50),

wherein, $Z_{e1}$ is A and $Z_{e2}$ is U, and
the nucleotide sequence I comprises a nucleotide $Z_{e3}$ at the position corresponding to $Z_{e1}$; the nucleotide sequence II comprises a nucleotide $Z_{e4}$ at the position corresponding to $Z_{e2}$, wherein $Z_{e4}$ is the first nucleotide at 5' terminal of the antisense strand.

In some embodiments, each non-fluoro modified nucleotide is independently selected from one of a nucleotide formed by substituting the 2' -hydroxy of the ribose group thereof with a non-fluoro group, and a nucleotide analogue.

In some embodiments, the nucleotide formed by substituting the 2' -hydroxy of the ribose group with a non-fluoro group is selected from the group consisting of 2' -alkoxy modified nucleotide, 2' -substituted alkoxy modified nucleotide, 2' -alkyl modified nucleotide, 2'-substituted alkyl modified nucleotide, 2' -amino modified nucleotide, 2' -substituted amino modified nucleotide and 2' -deoxy nucleotide; and the nucleotide analogue is selected from the group consisting of an isonucleotide, LNA, ENA, cET, UNA, and GNA.

In some embodiments, each non-fluoro modified nucleotide is a methoxy modified nucleotide.

In some embodiments, the present disclosure provides a pharmaceutical composition, comprising the above siRNA of the present disclosure, and a pharmaceutically acceptable carrier.

In some embodiments, the present disclosure provides use of the siRNA, and/or the pharmaceutical composition, and/or the siRNA conjugate of the present disclosure in the manufacture of a medicament for treating and/or preventing dyslipidemia caused by abnormal expression of the APOC3 gene.

In some embodiments, the present disclosure provides a method for treating and/or preventing dyslipidemia, comprising administering an effective amount of the siRNA, and/or the pharmaceutical composition, and/or the siRNA conjugate of the present disclosure to a subject suffering from dyslipidemia.

In some embodiments, the present disclosure provides a method for inhibiting the expression of APOC3 gene in hepatocytes, comprising contacting an effective amount of the siRNA, and/or the pharmaceutical composition, and/or the siRNA conjugate of the present disclosure with the hepatocytes.

In some embodiments, the present disclosure provides a kit, comprising the siRNA, and/or the pharmaceutical composition, and/or the siRNA conjugate of the present disclosure.

## BENEFICIAL EFFECTS

The siRNA, the composition comprising the siRNA, and the siRNA conjugate of the present disclosure could exhibit good stability, higher gene suppression activity, and/or significantly reduced blood lipid level.

In some embodiments, the siRNA conjuage of the present disclosure exhibits excellent property of suppressing APOC3 mRNA: inhibiting at least 82.0% of the expression of APOC3 mRNA in the liver of high-fat model mice at a dose of 1 mg/kg.

The experiments show that as compared with the conjugates formed from conjugation molecules as provided in the prior art, the siRNA conjugate of the present disclosure exhibits excellent capability of reducing blood lipid. Moreover, the siRNA conjugate of the present disclosure could continuously exhibit excellent effect of reducing blood lipid level over a period of up to 189 days under low administration dose and low administration frequency.

For example, after single administrations of 3 mg/kg, Conjugates 2, 4 and 5 maintain an inhibition rate of 70% to 90% against TG over a period of up to 77 days, substantially maintain an inhibition rate of 50% against total cholesterol (CHO) over a period of up to 77 days, and miantain an inhibition rate of about 50% or higher against TG over a period of up to 147 days; after single administrations of 3 mg/kg, the three conjugates exhibit an inhibition rate of up to about 80% against TG on day 7, and continuously exhibit an effect of reducing the TG content by no less than 50% over a period of up to 49 days; and on day 35 after single administration, the three conjugates in the 1 mg/kg dose group still show an effect of reducing the CHO content by at least about 50%. For Conjugate 1, in both of the dose groups of 3 mg/kg and 1 mg/kg, Conjugate 1 could significantly reduce the TG and CHO levels in the transgenic mice over a period of up to 112 days; and such reduction effects are significantly superior to that of Comparative Conjugate 2. In both of the dose groups of 3 mg/kg and 1 mg/kg, Conjugate 1 exhibits inhibition rates of 50% or higher against TG and CHO over a period of 56 days after single administration; and the inhibitory effects on TG are more significant: the two doses of Conjugate 1 continuously maintain the TG level of about 50% over a period of up to 112 days. As another example, Conjugate 3 could significantly reduce the TG and CHO levels in transgenic mice over a period of up to 98 days. On day 14 after single administration of 3 mg/kg, Conjugate 3 shows an inhibition rate of up to 93.6% against TG, and on day 7 after single administration shows an inhibition rate of up to 63.0% against CHO. Conjugates 4, 6 and 7 in the two doses could exhibit an effect of significantly reducing blood lipid level in human APOC3 transgenic mice, and in the 3 mg/kg dose group, continuously maintain an inhibition rate of 50% or higher against TG and an inhibition rate of 30% or higher against CHO over a period of 84 days after administration. It is noteworthy that at the doses of 3 mg/kg and 1 mg/kg, Conjugates 4, 6 and 7 continuously show higher inhibitory effects on TG than that of Comparative Conjugate 2; and the same tendency is also observed for the inhibitory effect on CHO.

As for Conjugates 8 and 9, the $ED_{50}$ values for inhibiting TG at different time points are measured. It can be seen that for human APOC3 transgenic mouse, even half a month after administration, a single subcutaneous injection of 0.16 mg/kg Conjugate 8 or 0.11 mg/kg Conjugate 9 could still realize the efficacy of reducing half of the serum TG content; and even one month after administration, a single subcutaneous injection of less than 1 mg/kg of the conjugates of the present disclosure could still realize the effect of reducing half of the serum TG content..

Therefore, the siRNA, the pharmaceutical composition and the siRNA conjugate of the present disclosure could inhibit the expression of APOC3 gene, effectively treat and/or prevent dyslipidemia caused by the overexpression of APOC3 gene, and thus show a promising prospect of application.

Additional features and advantages of the present disclosure will be detailedly illustrated in the following part "detailed description of the invention".

## BRIEF DESCRIPTION OF THE DRAWINGS

In order to more clearly illustrate the examples of the present invention and the technical solutions of the prior art, the following parts briefly describe the figures used in the examples and the prior art. Obviously, the figures in the following description represent only some examples of the present invention.

Figure 1 is a histogram showing the expression levels of APOC3 mRNA in untransfected Huh7 cells and in Huh7 cells transfected with different conjugates at different final concentrations.

Figures 2A-7D are diagrams showing the changes of total cholesterol (CHO) level and triglyceride (TG) level over time in serum of human APOC3 trangenic mice administered with normal saline and various conjugates at different doses.

Figure 8 is a scatterplot showing the expression levels of APOC3 mRNA in the *in vivo* liver tissues of human APOC3 trangenic mice administered with normal saline and Conjugate 4 at different doses.

## DETAILED DESCRIPTION OF THE INVENTION

The following is the detailed description of the specific embodiments of the present disclosure. It should be understood that the specific embodiments described herein are only used to illustrate and explain the present disclosure and are not intended to limit the present disclosure.

In the present disclosure, the sequence of APOC3 mRNA is the sequence as shown in Genbank Accession No. NM_000040.1. Further, unless otherwise specified, the term "target gene" used in the present disclosure refers to the gene expressing the above APOC3 mRNA; and the term "target mRNA" refers to the above APOC3 mRNA.

### Definitions

In the context of the present disclosure, unless otherwise specified, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents the two nucleotides adjacent to both sides of the letter s are linked by a thiophosphate linkage; PI represents that the nucleotide adjacent to the right side of P1 is a 5'-phosphate nucleotide or a 5'-phosphate analogue modified nucleotide; in some embodiments, PI represents the specific modifed nucleitide VP, Ps or P, wherein VP represents that the nucleotide adjacent to the right side of VP is a vinyl phosphate (5' -(E)-vinylphosphonate, E-VP) modified nucleotide; Ps represents that the nucleotide adjacent to the right side of Ps is a thiophosphate modified nucleotide; and P represents that the nucleotide adjacent to the right side of the letter P is a 5' -phosphate nucleotide.

In the context of the present disclosure, a "fluoro modified nucleotide" refers to a nucleotide formed by substituting 2'-hydroxy of the ribose group with a fluorine atom. A "non-fluoro modified nucleotide" refers to a nucleotide formed by substituting 2'-hydroxy of the ribose group with a non-fluoro group, or a nucleotide analogue. A "nucleotide analogue" refers to a group that can replace a nucleotide in a nucleic acid, while structurally differs from an adenine ribonucleotide, a guanine ribonucleotide, a cytosine ribonucleotide, a uracil ribonucleotide, or thymine deoxyribonucleotide, such as an isonucleotide, a bridged nucleotide (bridged nucleic acid, BNA) or an acyclic nucleotide. The "methoxy modified nucleotide" refers to a nucleotide formed by substituting 2'-hydroxy of the ribose group with a methoxy group.

In the context of the present disclosure, expressions "complementary" and "reverse complementary" can be interchangeably used, and have a well-known meaning in the art, namely, the bases in one strand are complementarily paired with those in the other strand in a double-stranded nucleic acid molecule. In DNAs, a purine base adenine (A) is always paired with a pyrimidine base thymine (T) (or a uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, the two strands are considered as being complementary with each other; and the sequence of a strand may be deduced from the sequence of its complementary strand. Correspondingly, a "mispairing" means that the bases at corresponding positions are not present in a manner of complementary pairing in a double-stranded nucleic acid.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely reverse complementary" means that there is no base mispairing between two nucleotide sequences.

In the context of the present disclosure, when a nucleotide sequence has a "nucleotide difference" from another nucleotide sequence, the bases of the nucleotides at the same position therebetween are changed. For example, if a

nucleotide base in the latter sequence is A and the nucleotide base at the same position in the former sequence is U, C, G, or T, the two nucleotide sequences are considered as having a nucleotide difference at this position. In some embodiments, if a nucleotide at a position is replaced with an abasic nucleotide or a nucleotide analogue, it is also considered that there is a nucleotide difference at the position.

In the context of the present disclosure, particularly in the description of the siRNA, the composition comprising the siRNA, or the method for preparing the siRNA conjugate of the present disclosure, unless otherwise specified, the "nucleoside monomer" refers to, according to the type and sequence of the nucleotides in the siRNA or siRNA conjugate to be prepared, unmodified or modified nucleoside phosphoramidite monomer (unmodified or modified RNA phosphoramidites; sometimes RNA phosphoramidites are referred to as nucleoside phosphoramidites) used in a phosphoramidite solid phase synthesis. The phosphoramidite solid phase synthesis is a well-known method for RNA synthesis by those skilled in the art. Nucleoside monomers used in the present disclosure are all commercially available.

In the context of the present disclosure, unless otherwise specified, "conjugation" means that two or more chemical moieties each having specific function are linked to each other via a covalent linkage. Correspondingly, a "conjugate" refers to a compound formed by covalent linkage of individual chemical moieties. Furthermore, a "siRNA conjugate" represents a compound formed by covalently attaching one or more chemical moieties each with specific functions to a siRNA. In the following text, the siRNA conjugate of the present disclosure is sometimes abbreviated as "conjugate". According to the context of the present disclosure, the siRNA conjugate should be understood as the generic term of siRNA conjugates, the generic term of siRNA conjugates of many specific chemical molecules, or each siRNA conjugate among the siRNA conjugates of many specific chemical molecules. In the context of the present disclosure, "conjugation molecules" should be interpreted as a class of compounds or specific compounds capable of being conjugated to a siRNA via reactions, thereby finally forming the siRNA conjugate of the present disclosure.

As used herein, a dash ("-") that is not present between two letters or symbols is used to indicate the position that is an attachment point for a substituent. For example, the dash on the far left side in the structure formula "$-C_1-C_{10}alkyl-NH_2$" means being attached through the $C_1-C_{10}$alkyl.

As used herein, "optional" or "optionally" means that the subsequently described event or circumstance may or may not occur, and that the description includes instances in which the event or circumstance occurs and instances in which it does not. For example, "optionally substituted alkyl" encompasses both "alkyl" and "substituted alkyl" as defined below. Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain alkyl having the indicated number of carbon atoms, usually from 1 to 20 carbon atoms, for example 1 to 10 carbon atoms, such as 1 to 8 or 1 to 6 carbon atoms. For example, $C_1-C_6$ alkyl encompasses both straight and branched chain alkyl of from 1 to 6 carbon atoms. When an alkyl residue having a specific number of carbon atoms is named, all branched and straight chain forms having that number of carbon atoms are intended to be encompassed; thus, for example, "butyl" is meant to encompass n-butyl, sec-butyl, isobutyl, and t-butyl; "propyl" includes n-propyl and isopropyl. Alkylene is a subset of alkyl, referring to the same residues as alkyl, but having two attachment points.

As used herein, "alkenyl" refers to an unsaturated branched or straight-chain alkyl group having at least one carbon-carbon double bond obtained by repectively removing one hydrogen molecule from two adjacent carbon atoms of the parent alkyl. The group may be in either the cis or trans configuration of the double bond(s). Typical alkenyl groups include, but are not limited to, ethenyl; propenyl, such as prop-1-en-1-yl, prop-1-en-2-yl, prop-2-en-1-yl (allyl), prop-2-en-2-yl; butenyl, such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms, and in other embodiments, from 2 to 10, 2 to 8, or 2 to 6 carbon atoms. Alkenylene is a subset of alkenyl, referring to the same residues as alkenyl, but having two attachment points.

As used herein, "alkynyl" refers to an unsaturated branched or straight-chain alkyl group having at least one carbon-carbon triple bond obtained by respectively removing two hydrogen molecules from two adjacent carbon atoms of the parent alkyl. Typical alkynyl groups include, but are not limited to, ethynyl; propynyl, such as prop-1-yn-1-yl, prop-2-yn-1-yl; butynyl, such as but-1-yn-1-yl, but-1-yn-3-yl, but-3-yn-1-yl; and the like. In certain embodiments, an alkynyl group has from 2 to 20 carbon atoms, and in other embodiments, from 2 to 10, 2 to 8, or 2 to 6 carbon atoms. Alkynylene is a subset of alkynyl, referring to the same residues as alkynyl, but having two attachment points.

As used herein, "alkoxy" refers to an alkyl group of the indicated number of carbon atoms attached through an oxygen bridge, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. Alkoxy group usually has from 1 to 10, 1 to 8, 1 to 6, or 1 to 4 carbon atoms attached through oxygen bridge.

As used herein, "aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon, including from 6 to 18 carbon atoms, wherein at least one ring in the ring

system is fully unsaturated, i.e., it contains a cyclic, delocalized (4n+2)π-electron system in accordance with the Hückel theory. Aryl groups include, but are not limited to, groups such as phenyl, fluorenyl, and naphthyl. Arylene is a subset of aryl, referring to the same residues as aryl, but having two attachment points.

As used herein, "cycloalkyl" refers to a non-aromatic carbon ring, usually having from 3 to 7 ring carbon atoms. The ring may be saturated or have one or more carbon-carbon double bonds. Examples of cycloalkyl groups include cyclopropyl, cyclobutyl, cyclopentyl, cyclopentenyl, cyclohexyl, and cyclohexenyl, as well as bridged and caged ring groups such as norbornane.

As used herein, "halo substituent" or "halo" refers to fluoro, chloro, bromo, and iodo, and the term "halogen" includes fluorine, chlorine, bromine, and iodine.

As used herein, "haloalkyl" refers to alkyl as defined above in which the specified number of carbon atoms are substituted with one or more (up to the maximum allowable number) halogen atoms. Examples of haloalkyl include, but are not limited to, trifluoromethyl, difluoromethyl, 2-fluoroethyl, and penta-fluoroethyl.

"Heterocyclyl" refers to a stable 3 to 18 membered non-aromatic ring radical that comprises 2 to 12 carbon atoms and 1 to 6 heteroatoms selected from nitrogen, oxygen or sulfur. Unless stated otherwise in the specification, the heterocyclyl is a monocyclic, bicyclic, tricyclic, or tetracyclic ring system, which may include fused or bridged ring system(s). The heteroatom(s) in the heterocyclyl may be optionally oxidized. One or more nitrogen atoms, if present, are optionally quaternized. The heterocyclyl is partially or fully saturated. The heterocyclyl may be attached to the rest of the molecule through any ring atom. Examples of such heterocyclyl include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heteroaryl" refers to a radical derived from a 3 to 18 membered aromatic ring radical that comprises 2 to 17 carbon atoms and 1 to 6 heteroatoms selected from nitrogen, oxygen or sulfur. As used herein, the heteroaryl may be a monocyclic, bicyclic, tricyclic or tetracyclic ring system, wherein at least one ring in the ring system is fully unsaturated, i.e., it contains a cyclic, delocalized (4n+2)π-electron system in accordance with the Hückel theory. Heteroaryl includes fused or bridged ring system(s). The heteroatom(s) in the heteroaryl is optionally oxidized. One or more nitrogen atoms, if present, are optionally quaternized. The heteroaryl is attached to the rest of the molecule through any ring atom. Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[*b*][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10 hexahydrocycloocta[d]pyrimidinyl, 5,6,7,8,9,10-hexahydrocyclohepta[d]pyridazinyl, 5,6,7,8,9,10- hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl.

Various hydroxyl protection groups may be used in the present disclosure. In general, protection groups render chemical functional groups inert to specific reaction conditions, and may be appended to and removed from such functional groups in a molecule without substantially damaging the remainder of the molecule. Representative hydroxyl protection groups are disclosed in Beaucage et al., Tetrahedron 1992, 48, 2223-2311, and also in Greene and Wuts, Protective Groups in Organic Synthesis, Chapter 2, 2d ed, John Wiley & Sons, New York, 1991, which are incorporated herein by reference in their entirety. In some embodiments, the protection group is stable under basic conditions but may be removed under acidic conditions. In some embodiments, non-exclusive examples of the hydroxyl protection groups that may be used herein include dimethoxytrityl (DMT), monomethoxytrityl, 9-phenylxanthen-9-yl (Pixyl) and 9-(p-methoxyphenyl)xanthen-9-yl (Mox). In some embodiments, non-exclusive examples of the hydroxyl protection groups that may be used herein comprises Tr (trityl), MMTr (4-methoxytrityl), DMTr (4,4'-dimethoxytrityl), and TMTr (4,4',4"-trimethoxytrityl).

The term "subject", as used herein, refers to any animal, e.g., a mammal or marsupial. The subject of the present disclosure includes, but are not limited to, human, non-human primate (e.g., rhesus or other types of macaques), mouse, pig, horse, donkey, cow, sheep, rat, and any kind of poultry.

As used herein, "treatment" or "treating", or "alleviating" or "ameliorating" can be used interchangeably herein. These terms refer to an approach for obtaining beneficial or desirable results, including but not limited to therapeutic benefit. By "therapeutic benefit" is meant eradication or amelioration of the underlying disorder being treated. Also, a therapeutic benefit is achieved by eradicating or ameliorating one or more physiological symptoms associated with the underlying disorder, thereby observaing amelioration in the subject, although the subject may still be afflicted with the underlying disorder.

As used herein, "prevention" and "preventing" can be used interchangeably. These terms refer to an approach for obtaining beneficial or desirable results, including but not limited to a prophylactic benefit. In order to obtain "prophylactic benefit", the conjugates or compositions may be administered to a subject at risk of developing a particular disease, or to a subject reporting one or more physiological symptoms of a disease, even a diagnosis of this disease has not been made.

The siRNA of the present disclosure comprises nucleotide groups as basic structural units. It is well known to those skilled in the art that the nucleotide group comprises a phosphate group, a ribose group and a base. Detailed illustrations of these groups are omitted herein.

## First siRNA

According to the present disclosure, the siRNA may be a first siRNA.

The first siRNA comprises a sense strand and an antisense strand; each nucleotide in the first siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; for the first siRNA, the nucleotide sequence I and the nucleotide sequence as shown by SEQ ID NO: 1 have an equal length and no more than 3 nucleotide differences, and the nucleotide sequence II and the nucleotide sequence as shown by SEQ ID NO: 2 have an equal length and no more than 3 nucleotide differences:

5'-UUAAAAGGGACAGUAUUC$Z_{a1}$-3' (SEQ ID NO: 1),
5'-$Z_{a2}$GAAUACUGUCCCUUUUAA-3' (SEQ ID NO: 2),

wherein, $Z_{a1}$ is A and $Z_{a2}$ is U, and
the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$, wherein $Z_{a4}$ is the first nucleotide at 5' terminal of the antisense strand;

In the context of the present disclosure, "corresponding position" refers to, the same position in the nucleotide sequence when counting from the same terminal of the nucleotide sequence. For example, for the first siRNA, the first nucleotide at 3' terminal of the nucleotide sequence I is a nucleotide at the position corresponding to the first nucleotide at 3' terminal of SEQ ID NO: 1. In some embodiments, the sense strand comprises only the nucleotide sequence I, and the antisense strand comprises only the nucleotide sequence II.

In some embodiments, the nucleotide sequence I and the nucleotide sequence as shown by SEQ ID NO: 1 have no more than 1 nucleotide difference, and/or the nucleotide sequence II and the nucleotide sequence as shown by SEQ ID NO: 2 have no more than 1 nucleotide difference.

In some embodiments, the nucleotide difference between the nucleotide sequence II and the nucleotide sequence as shown by SEQ ID NO: 2 includes a difference at the position $Z_{a4}$, wherein $Z_{a4}$ is selected from A, C or G. In some embodiments, the nucleotide difference is a difference at the position $Z_{a4}$, wherein $Z_{a4}$ is selected from A, C or G. In some embodiments, $Z_{a3}$ is a neucleotide complementary to $Z_{a4}$. These nucleotide differences will not significantly reduce the ability of the siRNA conjugates of inhibiting the target gene, and thus these siRNA conjugates comprising the nucleotide differences are within the protection scope of the present disclosure.

In some embodiments, the nucleotide sequence I and the nucleotide sequence II are basically reverse complementary, substantially reverse complementary, or completely reverse complementary to each other; the "basically reverse complementary" means that there is no more than 3 base mispairings between two nucleotide sequences; the "substantially reverse complementary" means that there is no more than 1 base mispairing between two nucleotide sequences; the "completely reverse complementary" means that there is no mispairing between two nucleotide sequences.

In some embodiments, the nucleotide sequence I is the nucleotide sequence as shown by SEQ ID NO: 3, and the nucleotide sequence II is the nucleotide sequence as shown by SEQ ID NO: 4:

5'-UUAAAAGGGACAGUAUUC$Z_{a3}$-3' (SEQ ID NO: 3),
5'-$Z_{a4}$GAAUACUGUCCCUUUUAA-3' (SEQ ID NO: 4),

wherein, $Z_{a4}$ is the first nucleotide at 5' terminal of the antisense strand, $Z_{a3}$ is selected from A, U, G, or C, and $Z_{a4}$ is a nucleotide complementary to $Z_{a3}$; in some embodiments, $Z_{a3}$ is U, and $Z_{a4}$ is A.

Moreover, the sense strand and the antisense strand have the same or different length, wherein the sense strand has a length of 19 to 23 nucleotides, and the antisense strand has a length of 20 to 26 nucleotides.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; and the nucleotide sequence III and the nucleotide sequence IV have an equal length.

In some embodiments, the nucleotide sequence III and the nucleotide sequence IV both have a length of 1 nucleotide, and the base of the nucleotide sequence III is C, and the base of the nucleotide sequence IV is G; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the bases of the nucleotide sequence III is GC, and the bases of the nucleotide sequence IV is GC; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the bases of the nucleotide sequence III is UGC, and the bases of the nucleotide sequence IV is GCA; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the bases of the nucleotide sequence III is UUGC, and the bases of the nucleotide sequence IV is GCAA; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the bases of the nucleotide sequence III is GC, and the bases of the nucleotide sequence IV is GC; in this case, the length ratio of the sense strand and the antisense strand thereof is 21/21.

In some embodiments, the nucleotide sequence III and the nucleotide sequence IV have an equal length and are completely reverse complementary. Hence, where the base(s) of nucleotide sequence III is(are) provided, the base(s) of nucleotide sequence IV is(are) also determined.

## Second siRNA

According to the present disclosure, the siRNA may be a second siRNA.

The second siRNA comprises a sense strand and an antisense strand; each nucleotide in the second siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein, the nucleotide sequence I and the nucleotide sequence as shown by SEQ ID NO: 13 have an equal length and no more than 3 nucleotide differences, and the nucleotide sequence II and the nucleotide sequence as shown by SEQ ID NO: 14 have an equal length and no more than 3 nucleotide differences:

5'-ACAGUAUUCUCAGUGCUC$Z_{b1}$-3' (SEQ ID NO: 13),
5'-$Z_{b2}$GAGCACUGAGAAUACUGU-3' (SEQ ID NO: 14),

wherein, $Z_{b1}$ is A and $Z_{b2}$ is U, and
the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$, wherein $Z_{b4}$ is the first nucleotide at 5' terminal of the antisense strand;

In some embodiments, the sense strand comprises only the nucleotide sequence I, and the antisense strand comprises only the nucleotide sequence II.

In some embodiments, the nucleotide sequence I and the nucleotide sequence as shown by SEQ ID NO: 13 have no more than 1 nucleotide difference, and/or the nucleotide sequence II and the nucleotide sequence as shown by SEQ ID NO: 14 have no more than 1 nucleotide difference.

In some embodiments, the nucleotide difference between the nucleotide sequence II and the nucleotide sequence as shown by SEQ ID NO: 14 includes a difference at the position $Z_{b4}$, wherein $Z_{b4}$ is selected from A, C or G. In some embodiments, the nucleotide difference is a difference at the position $Z_{b4}$, wherein $Z_{b4}$ is selected from A, C or G. In some embodiments, $Z_{b3}$ is a neucleotide complementary to $Z_{b4}$. These nucleotide differences will not significantly reduce the ability of the siRNA conjugates of inhibiting the target gene, and thus these siRNA conjugates comprising the nucleotide differences are within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is the nucleotide sequence as shown by SEQ ID NO: 15, and the nucleotide sequence II is the nucleotide sequence as shown by SEQ ID NO: 16:

5'-ACAGUAUUCUCAGUGCUC$Z_{b3}$-3' (SEQ ID NO: 15),
5'-$Z_{b4}$GAGCACUGAGAAUACUGU-3' (SEQ ID NO: 16),

wherein, $Z_{b4}$ is the first nucleotide at 5' terminal of the antisense strand, $Z_{b3}$ is selected from A, U, G, or C, and $Z_{b4}$ is a nucleotide complementary to $Z_{b3}$; in some embodiments, $Z_{b3}$ is U, and $Z_{b4}$ is A.

Moreover, the sense strand and the antisense strand have the same or different length, wherein the sense strand has a length of 19 to 23 nucleotides, and the antisense strand has a length of 20 to 26 nucleotides.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; and the nucleotide sequence III and the nucleotide sequence IV have an equal length.

In some embodiments, the nucleotide sequence III and the nucleotide sequence IV both have a length of 1 nucleotide, and the base of the nucleotide sequence III is G, and the base of the nucleotide sequence IV is C; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the bases of the nucleotide sequence III is GG, and the bases of the nucleotide sequence IV is CC; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the bases of the nucleotide sequence III is GGG, and the bases of the nucleotide sequence IV is CCC; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the bases of the nucleotide sequence III is AGGG, and the bases of the nucleotide sequence IV is CCCU; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the bases of the nucleotide sequence III is GG, and the bases of the nucleotide sequence IV is CC; in this case, the length ratio of the sense strand and the antisense strand thereof is 21/21.

In some embodiments, the nucleotide sequence III and the nucleotide sequence IV have an equal length and are completely reverse complementary. Hence, where the base(s) of nucleotide sequence III is(are) provided, the base(s) of nucleotide sequence IV is(are) also determined.

## Third siRNA

According to the present disclosure, the siRNA may be a third siRNA.

The third siRNA comprises a sense strand and an antisense strand; each nucleotide in the third siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein, the nucleotide sequence I and the nucleotide sequence as shown by SEQ ID NO: 25 have an equal length and no more than 3 nucleotide differences, and the nucleotide sequence II and the nucleotide sequence as shown by SEQ ID NO: 26 have an equal length and no more than 3 nucleotide differences:

5'-UAUUCUCAGUGCUCUCCU$Z_{c1}$-3' (SEQ ID NO: 25),
5'-$Z_{c2}$AGGAGAGCACUGAGAAUA-3' (SEQ ID NO: 26),

wherein, $Z_{c1}$ is A and $Z_{c2}$ is U, and
the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$, wherein
$Z_{c4}$ is the first nucleotide at 5' terminal of the antisense strand;

In some embodiments, the sense strand comprises only the nucleotide sequence I, and the antisense strand comprises only the nucleotide sequence II.

In some embodiments, the nucleotide sequence I and the nucleotide sequence as shown by SEQ ID NO: 25 have no more than 1 nucleotide difference, and/or the nucleotide sequence II and the nucleotide sequence as shown by SEQ ID NO: 26 have no more than 1 nucleotide difference.

In some embodiments, the nucleotide difference between the nucleotide sequence II and the nucleotide sequence as shown by SEQ ID NO: 26 includes a difference at the position $Z_{c4}$, wherein $Z_{c4}$ is selected from A, C or G. In some embodiments, the nucleotide difference is a difference at the position $Z_{c4}$, wherein $Z_{c4}$ is selected from A, C or G. In some embodiments, $Z_{c3}$ is a neucleotide complementary to $Z_{c4}$. These nucleotide differences will not significantly reduce the ability of the siRNA conjugates of inhibiting the target gene, and thus these siRNA conjugates comprising the nucleotide differences are within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is the nucleotide sequence as shown by SEQ ID NO: 27, and the nucleotide sequence II is the nucleotide sequence as shown by SEQ ID NO: 28:

5'-UAUUCUCAGUGCUCUCCU$Z_{c3}$-3'(SEQ ID NO: 27);
5'-$Z_{c4}$AGGAGAGCACUGAGAAUA-3'(SEQ ID NO: 28),

wherein, $Z_{c4}$ is the first nucleotide at 5' terminal of the antisense strand, $Z_{c3}$ is selected from A, U, G, or C, and $Z_{c4}$ is a nucleotide complementary to $Z_{c3}$; in some embodiments, $Z_{c3}$ is U, and $Z_{c4}$ is A.

Moreover, the sense strand and the antisense strand have the same or different length, wherein the sense strand has a length of 19 to 23 nucleotides, and the antisense strand has a length of 20 to 26 nucleotides.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; and the nucleotide sequence III and the nucleotide sequence IV have an equal length.

In some embodiments, the nucleotide sequence III and the nucleotide sequence IV both have a length of 1 nucleotide, and the base of the nucleotide sequence III is G, and the base of the nucleotide sequence IV is C; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the bases of the nucleotide sequence III is AG, and the bases of the nucleotide sequence IV is CU; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the bases of the nucleotide sequence III is CAG, and the bases of the nucleotide sequence IV is CUG; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the bases of the nucleotide sequence III is ACAG, and the bases of the nucleotide sequence IV is CUGU; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the bases of the nucleotide sequence III is AG, and the bases of the nucleotide sequence IV is CU; in this case, the length ratio of the sense strand and the antisense strand thereof is 21/21.

In some embodiments, the nucleotide sequence III and the nucleotide sequence IV have an equal length, and are completely reverse complementary. Hence, where the base(s) of nucleotide sequence III is(are) provided, the base(s) of nucleotide sequence IV is(are) also determined.

## Fourth siRNA

According to the present disclosure, the siRNA may be a fourth siRNA.

The fourth siRNA comprises a sense strand and an antisense strand; each nucleotide in the fourth siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein, the nucleotide sequence I and the nucleotide sequence as shown by SEQ ID NO: 37 have an equal length and no more than 3 nucleotide differences, and the nucleotide sequence II and the nucleotide sequence as shown by SEQ ID NO: 38 have an equal length and no more than 3 nucleotide differences:

5'-AGUAUUCUCAGUGCUCUC$Z_{d1}$-3' (SEQ ID NO: 37);
5'-$Z_{d2}$GAGAGCACUGAGAAUACU-3' (SEQ ID NO: 38),

wherein, $Z_{d1}$ is A and $Z_{d2}$ is U, and
the nucleotide sequence I comprises a nucleotide $Z_{d3}$ at the position corresponding to $Z_{d1}$, the nucleotide sequence II

comprises a nucleotide $Z_{d4}$ at the position corresponding to $Z_{d2}$, wherein $Z_{d4}$ is the first nucleotide at 5' terminal of the antisense strand.

In some embodiments, the sense strand comprises only the nucleotide sequence I, and the antisense strand comprises only the nucleotide sequence II.

In some embodiments, the nucleotide sequence I and the nucleotide sequence as shown by SEQ ID NO: 37 have no more than 1 nucleotide difference, and/or the nucleotide sequence II and the nucleotide sequence as shown by SEQ ID NO: 38 have no more than 1 nucleotide difference.

In some embodiments, the nucleotide difference between the nucleotide sequence II and the nucleotide sequence as shown by SEQ ID NO: 38 includes a difference at the position $Z_{d4}$, wherein $Z_{d4}$ is selected from A, C or G. In some embodiments, the nucleotide difference is a difference at the position $Z_{d4}$, wherein $Z_{d4}$ is selected from A, C or G. In some embodiments, $Z_{d3}$ is a neucleotide complementary to $Z_{d4}$. These nucleotide differences will not significantly reduce the ability of the siRNA conjugates of inhibiting the target gene, and thus these siRNA conjugates comprising the nucleotide differences are within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is the nucleotide sequence as shown by SEQ ID NO: 39, and the nucleotide sequence II is the nucleotide sequence as shown by SEQ ID NO: 40:

5'-AGUAUUCUCAGUGCUCUC$Z_{d3}$-3' (SEQ ID NO: 39);
5'-$Z_{d4}$GAGAGCACUGAGAAUACU-3' (SEQ ID NO: 40),

wherein, $Z_{d4}$ is the first nucleotide at 5' terminal of the antisense strand, $Z_{d3}$ is selected from A, U, G, or C, and $Z_{d4}$ is a nucleotide complementary to $Z_{d3}$; in some embodiments, $Z_{d3}$ is U, and $Z_{d4}$ is A.

Moreover, the sense strand and the antisense strand have the same or different length, wherein the sense strand has a length of 19 to 23 nucleotides, and the antisense strand has a length of 20 to 26 nucleotides.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; and the nucleotide sequence III and the nucleotide sequence IV have an equal length.

In some embodiments, the nucleotide sequence III and the nucleotide sequence IV both have a length of 1 nucleotide, and the base of the nucleotide sequence III is C, and the base of the nucleotide sequence IV is G; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the bases of the nucleotide sequence III is AC, and the bases of the nucleotide sequence IV is GU; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the bases of the nucleotide sequence III is GAC, and the bases of the nucleotide sequence IV is GUC; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the bases of the nucleotide sequence III is GGAC, and the bases of the nucleotide sequence IV is GUCC; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the bases of the nucleotide sequence III is AC, and the bases of the nucleotide sequence IV is GU; in this case, the length ratio of the sense strand and the antisense strand thereof is 21/21.

In some embodiments, the nucleotide sequence III and the nucleotide sequence IV have an equal length, and are completely reverse complementary. Hence, where the base(s) of nucleotide sequence III is(are) provided, the base(s) of nucleotide sequence IV is(are) also determined.

## Fifth siRNA

According to the present disclosure, the siRNA may be a fifth siRNA.

The fifth siRNA comprises a sense strand and an antisense strand; each nucleotide in the fifth siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein, the nucleotide sequence I and the nucleotide sequence as shown by SEQ ID NO: 49 have an equal length and no more than 3 nucleotide differences, and the nucleotide sequence II and the nucleotide sequence as shown by SEQ ID NO: 50 have an equal length and no more

than 3 nucleotide differences:

5'-GGACAGUAUUCUCAGUGC$Z_{e1}$-3' (SEQ ID NO: 49);
5'-$Z_{e2}$GCACUGAGAAUACUGUCC-3' (SEQ ID NO: 50),

wherein, $Z_{e1}$ is A and $Z_{e2}$ is U, and
the nucleotide sequence I comprises a nucleotide $Z_{e3}$ at the position corresponding to $Z_{e1}$, the nucleotide sequence II comprises a nucleotide $Z_{e4}$ at the position corresponding to $Z_{e2}$, wherein $Z_{e4}$ is the first nucleotide at 5' terminal of the antisense strand.

In some embodiments, the sense strand comprises only the nucleotide sequence I, and the antisense strand comprises only the nucleotide sequence II.

In some embodiments, the nucleotide sequence I and the nucleotide sequence as shown by SEQ ID NO: 49 have no more than 1 nucleotide difference, and/or the nucleotide sequence II and the nucleotide sequence as shown by SEQ ID NO: 50 have no more than 1 nucleotide difference.

In some embodiments, the nucleotide difference between the nucleotide sequence II and the nucleotide sequence as shown by SEQ ID NO: 50 includes a difference at the position $Z_{e4}$, wherein $Z_{e4}$ is selected from A, C or G. In some embodiments, the nucleotide difference is a difference at the position $Z_{e4}$, wherein $Z_{e4}$ is selected from A, C or G. In some embodiments, $Z_{e3}$ is a neucleotide complementary to $Z_{e4}$. These nucleotide differences will not significantly reduce the ability of the siRNA conjugates of inhibiting the target gene, and thus these siRNA conjugates comprising the nucleotide differences are within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is the nucleotide sequence as shown by SEQ ID NO: 51, and the nucleotide sequence II is the nucleotide sequence as shown by SEQ ID NO: 52:

5'-GGACAGUAUUCUCAGUGC$Z_{e3}$-3'(SEQ ID NO: 51);
5'-$Z_{e4}$GCACUGAGAAUACUGUCC-3'(SEQ ID NO: 52),

wherein, $Z_{e4}$ is the first nucleotide at 5' terminal of the antisense strand, $Z_{e3}$ is selected from A, U, G, or C, and $Z_{e4}$ is a nucleotide complementary to $Z_{e3}$; in some embodiments, $Z_{e3}$ is U, and $Z_{e4}$ is A.

Moreover, the sense strand and the antisense strand have the same or different length, wherein the sense strand has a length of 19 to 23 nucleotides, and the antisense strand has a length of 20 to 26 nucleotides.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; and the nucleotide sequence III and the nucleotide sequence IV have an equal length.

In some embodiments, the nucleotide sequence III and the nucleotide sequence IV both have a length of 1 nucleotide, and the base of the nucleotide sequence III is G, and the base of the nucleotide sequence IV is C; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the bases of the nucleotide sequence III is AG, and the bases of the nucleotide sequence IV is CU; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the bases of the nucleotide sequence III is AAG, and the bases of the nucleotide sequence IV is CUU; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the bases of the nucleotide sequence III is AAAG, and the bases of the nucleotide sequence IV is CUUU; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the bases of the nucleotide sequence III is AG, and the bases of the nucleotide sequence IV is CU; in this case, the length ratio of the sense strand and the antisense strand thereof is 21/21.

In some embodiments, the nucleotide sequence III and the nucleotide sequence IV have an equal length, and are completely reverse complementary. Hence, where the base(s) of nucleotide sequence III is(are) provided, the base(s) of nucleotide sequence IV is(are) also determined.

## Overhang terminal and modification of siRNA

The following description regarding the nucleotide sequence V, the nucleic acid sequence, or the nucleotide modification and the modified sequence of the siRNA is applicable to any one of the first siRNA to the fifth siRNA. Namely, unless stated otherwise, the following description of the siRNA should be regarded as the description of each of the first, second, third, fourth and fifth siRNAs. For example, if no particular siRNA is specifically indicated, "the siRNA further comprises a nucleotide sequence V" means that "the first siRNA, the second siRNA, the third siRNA, the fourth siRNA or the fifth siRNA further comprise a nucleotide sequence V".

In some embodiments, the siRNA further comprises a nucleotide sequence V. The nucleotide sequence V has a length of 1 to 3 nucleotides and is linked to 3' terminal of the antisense strand (i.e., linking to the terminal of the nucleotide sequence II or the nucleotide sequence IV), thereby forming a 3' overhang terminal of the antisense strand. In this case, the length ratio of the sense strand and the antisense strand of the siRNA may be 19/20, 19/21, 19/22, 20/21, 20/22, 20/23, 21/22, 21/23, 21/24, 22/23, 22/24, 22/25, 23/24, 23/25, or 23/26. In some embodiments, the nucleotide sequence V has a length of 2 nucleotides. Thus, the length ratio of the sense strand and the antisense strand of the siRNA of the present disclosure may be 19/21, 21/23 or 23/25.

Each nucleotide in the nucleotide sequence V may be any nucleotide. In order to facilitate the synthesis and to save synthesis cost, the nucleotide sequence V is 2 consecutive thymine deoxyribonucleotides (dTdT) or 2 consecutive uracil ribonucleotides (UU); in order to enhance the affinity between the antisense strand of the siRNA and the target mRNA, the nucleotide sequence V is complementary to the nucleotides at the corresponding positions of the target mRNA. Thus, in some embodiments, the length ratio of the sense strand and the antisense strand of the siRNA of the present disclosure is 19/21 or 21/23. In this case, the siRNA of the present disclosure exhibits better mRNA silencing activity.

The nucleotides at the corresponding positions of the target mRNA refer to the nucleotides or nucleotide sequence adjacent to 5' terminal of a segment of the nucleotide sequence of the target mRNA. This segment of the nucleotide sequence of the target mRNA refers to the segment of the nucleotide sequence which is substantially reverse complementary or completely reverse complementary with the nucleotide sequence II, or is substantially reverse complementary or completely reverse complementary with the nucleotide sequence consisted of the nucleotide sequence II and the nucleotide sequence IV.

In some embodiments, for the first siRNA, the sense strand of the siRNA comprises the nucleotide sequence as shown by SEQ ID NO: 5, and the antisense strand of the siRNA comprises the nucleotide sequence as shown by SEQ ID NO: 6:

5'-UUAAAAGGGACAGUAUUC$Z_{a3}$-3' (SEQ ID NO: 5);
5'-$Z_{a4}$GAAUACUGUCCCUUUUAAGC-3' (SEQ ID NO: 6);

Alternatively, the sense strand of the siRNA comprises the nucleotide sequence as shown by SEQ ID NO: 7, and the antisense strand of the siRNA comprises the nucleotide sequence as shown by SEQ ID NO: 8:

5'-GCUUAAAAGGGACAGUAUUC$Z_{a3}$-3' (SEQ ID NO: 7);
5'-$Z_{a4}$GAAUACUGUCCCUUUUAAGCAA-3' (SEQ ID NO: 8);

wherein, $Z_{a4}$ is the first nucleotide at 5' terminal of the antisense strand; $Z_{a3}$ is selected from A, U, G or C, and $Z_{a4}$ is a nucleotide complementary to $Z_{a3}$.

In some embodiments, for the second siRNA, the sense strand of the siRNA comprises the nucleotide sequence as shown by SEQ ID NO: 17, and the antisense strand of the siRNA comprises the nucleotide sequence as shown by SEQ ID NO: 18:

5'-ACAGUAUUCUCAGUGCUC$Z_{b3}$ -3'(SEQ ID NO: 17);
5'-$Z_{b4}$GAGCACUGAGAAUACUGUCC-3'(SEQ ID NO: 18);

Alternatively, the sense strand of the siRNA comprises the nucleotide sequence as shown by SEQ ID NO: 19, and the antisense strand of the siRNA comprises the nucleotide sequence as shown by SEQ ID NO: 20:

5'-GGACAGUAUUCUCAGUGCUC$Z_{b3}$ -3'(SEQ ID NO: 19);
5'-$Z_{b4}$GAGCACUGAGAAUACUGUCCCU-3'(SEQ ID NO: 20);

wherein, $Z_{b4}$ is the first nucleotide at 5' terminal of the antisense strand; $Z_{b3}$ is selected from A, U, G or C, and $Z_{b4}$ is a nucleotide complementary to $Z_{b3}$.

In some embodiments, for the third siRNA, the sense strand of the siRNA comprises the nucleotide sequence as shown by SEQ ID NO: 29, and the antisense strand of the siRNA comprises the nucleotide sequence as shown by SEQ ID NO: 30:

5'-UAUUCUCAGUGCUCUCCUZ$_{c3}$-3' (SEQ ID NO: 29);
5'-Z$_{c4}$AGGAGAGCACUGAGAAUACU -3' (SEQ ID NO: 30);

Alternatively, the sense strand of the siRNA comprises the nucleotide sequence as shown by SEQ ID NO: 31, and the antisense strand of the siRNA comprises the nucleotide sequence as shown by SEQ ID NO: 32:

5'-AGUAUUCUCAGUGCUCUCCUZ$_{c3}$ -3' (SEQ ID NO: 31);
5'-Z$_{c4}$AGGAGAGCACUGAGAAUACUGU-3' (SEQ ID NO: 32);

wherein, $Z_{c4}$ is the first nucleotide at 5' terminal of the antisense strand; $Z_{c3}$ is selected from A, U, G or C, and $Z_{c4}$ is a nucleotide complementary to $Z_{c3}$.

In some embodiments, for the fourth siRNA, the sense strand of the siRNA comprises the nucleotide sequence as shown by SEQ ID NO: 41, and the antisense strand of the siRNA comprises the nucleotide sequence as shown by SEQ ID NO: 42:

5'-AGUAUUCUCAGUGCUCUCZ$_{d3}$ -3' (SEQ ID NO: 41);
5'-Z$_{d4}$GAGAGCACUGAGAAUACUGU-3' (SEQ ID NO: 42);

Alternatively, the sense strand of the siRNA comprises the nucleotide sequence as shown by SEQ ID NO: 43, and the antisense strand of the siRNA comprises the nucleotide sequence as shown by SEQ ID NO: 44:

5'-ACAGUAUUCUCAGUGCUCUCZ$_{d3}$ -3'(SEQ ID NO: 43);
5'-Z$_{d4}$GAGAGCACUGAGAAUACUGUCC -3'(SEQ ID NO: 44);

wherein, $Z_{d4}$ is the first nucleotide at 5' terminal of the antisense strand; $Z_{d3}$ is selected from A, U, G or C, and $Z_{d4}$ is a nucleotide complementary to $Z_{d3}$.

In some embodiments, for the fifth siRNA, the sense strand of the siRNA comprises the nucleotide sequence as shown by SEQ ID NO: 53, and the antisense strand of the siRNA comprises the nucleotide sequence as shown by SEQ ID NO: 54:

5'-GGACAGUAUUCUCAGUGCZ$_{e3}$ -3' (SEQ ID NO: 53);
5'-Z$_{e4}$GCACUGAGAAUACUGUCCCU -3' (SEQ ID NO: 54);

Alternatively, the sense strand of the siRNA comprises the nucleotide sequence as shown by SEQ ID NO: 55, and the antisense strand of the siRNA comprises the nucleotide sequence as shown by SEQ ID NO: 56:

5'-AGGGACAGUAUUCUCAGUGCZ$_{e3}$ -3' (SEQ ID NO: 55);
5'-Z$_{e4}$GCACUGAGAAUACUGUCCCUUU -3' (SEQ ID NO: 56);

wherein, $Z_{e4}$ is the first nucleotide at 5' terminal of the antisense strand; $Z_{e3}$ is selected from A, U, G or C, and $Z_{e4}$ is a nucleotide complementary to $Z_{e3}$.

In some embodiments, the siRNA of the present disclosure is siAPa1, siAPa2, siAPb1, siAPb2, siAPc1, siAPc2, siAPd1, siAPd2, siAPe1, or siAPe2:

siAPa1

sense strand: 5'- UUAAAAGGGACAGUAUUCU -3' (SEQ ID NO: 9)
antisense strand: 5'- AGAAUACUGUCCCUUUUAAGC-3' (SEQ ID NO: 10)

siAPa2

sense strand: 5'- GCUUAAAAGGGACAGUAUUCU -3' (SEQ ID NO: 11)
antisense strand: 5'- AGAAUACUGUCCCUUUUAAGCAA -3' (SEQ ID NO: 12)

siAPb1

sense strand: 5'- ACAGUAUUCUCAGUGCUCU -3'(SEQ ID NO: 21)
antisense strand: 5'- AGAGCACUGAGAAUACUGUCC -3'(SEQ ID NO: 22)

siAPb2

sense strand: 5'- GGACAGUAUUCUCAGUGCUCU -3'(SEQ ID NO: 23)
antisense strand: 5'- AGAGCACUGAGAAUACUGUCCCU -3'(SEQ ID NO: 24)

siAPc1

sense strand: 5'- UAUUCUCAGUGCUCUCCUA -3'(SEQ ID NO: 33)
antisense strand: 5'- UAGGAGAGCACUGAGAAUACU-3'(SEQ ID NO: 34)

siAPc2

sense strand: 5'- AGUAUUCUCAGUGCUCUCCUA -3'(SEQ ID NO: 35)
antisense strand: 5'- UAGGAGAGCACUGAGAAUACUGU -3'(SEQ ID NO: 36)

siAPd1

sense strand: 5'- AGUAUUCUCAGUGCUCUCC -3'(SEQ ID NO: 45)
antisense strand: 5'- GGAGAGCACUGAGAAUACUGU -3'(SEQ ID NO: 46)

siAPd2

sense strand: 5'- ACAGUAUUCUCAGUGCUCUCC -3'(SEQ ID NO: 47)
antisense strand: 5'- GGAGAGCACUGAGAAUACUGUCC -3'(SEQ ID NO: 48)

siAPe1

sense strand: 5'- GGACAGUAUUCUCAGUGCU -3'(SEQ ID NO: 57)
antisense strand: 5'- AGCACUGAGAAUACUGUCCCU -3'(SEQ ID NO: 58)

siAPe2

sense strand: 5'- AGGGACAGUAUUCUCAGUGCU -3'(SEQ ID NO: 59)
antisense strand: 5'-AGCACUGAGAAUACUGUCCCUUU -3'(SEQ ID NO: 60).

In some embodiments, the siRNA has the nucleotide sequence (i.e., nucleic acid or base sequence) as shown by siAPa1, siAPa2, siAPb1, siAPb2, siAPc1, siAPc2, siAPd1, siAPd2, siAPe1, or siAPe2.

As mentioned above, in the siRNA of the present disclosure, each nucleotide is independently a modified or unmodified nucleotide. In some embodiments, the nucleotide in the siRNA of the present disclosure is an unmodified nucleotide; in some embodiments, in the siRNA of the present disclosure, some or all of the nucleotides are modified necleotides. These modifications on the nucleotide groups would not lead to significant decrease or loss of the functions for inhibiting the expression of APOC3 gene of the siRNA conjugate of the present disclosure.

In some embodiments, the siRNA of the present disclosure comprises at least 1 modified nucleotide. In the context of the present disclosure, the term "modified nucleotide" used refers to a nucleotide formed by substituting 2'-hydroxy of the ribose group with other groups, or nucleotide analogue, or a nucleotide of which the base is a modified base. The modified nucleotide would not lead to significant impairment or loss of the functions for inhibiting gene expression of the siRNA conjugate. For example, the modified nucleotides disclosed in Watts, J.K., G. F. Deleavey and M. J. Damha, Chemically Modified siRNA: tools and applications. Drug Discov Today, 2008.13(19-20): p.842-55 may be selected.

In some embodiments, at least one nucleotide in the sense strand or the antisense strand of the siRNA of the present disclosure is a modified nucleotide, and/or at least one phosphate group is a phosphate group with modified group(s). In other words, at least a portion of the phosphate and/or ribose groups in the phosphate-ribose backbone of at least one single strand in the sense strand and the antisense strand are phosphate groups with modified groups

and/or ribose groups with modified groups.

In some embodiments, all the nucleotides in the sense strand and/or the antisense strand are modified nucleotides. In some embodiments, each nucleotide in the sense strand and antisense strand of the siRNA of the present disclosure is independently a fluoro modified nucleotide or a non-fluoro modified nucleotide.

The inventors of the present disclosure surprisingly found that the siRNA of the present disclosure achieves high balance between plasma stability and gene silencing efficiency in animal experiments.

In some embodiments, the fluoro modified nucleotides are located in the nucleotide sequence I and the nucleotide sequence II; and in the direction from 5' terminal to 3' terminal, the nucleotides at positions 7, 8 and 9 of the nucleotide sequence I are fluoro modified nucleotides; and in the direction from 5'terminal to 3' terminal, the nucleotides at positions 2, 6, 14, and 16 of the nucleotide sequence II are fluoro modified nucleotides.

In some embodiments, the fluoro modified nucleotides are located in the nucleotide sequence I and the nucleotide sequence II; and the nucleotide sequence I comprises no more than 5 fluoro modified nucleotides; and in the direction from 5' terminal to 3' terminal, the nucleotides at positions 7, 8 and 9 of the nucleotide sequence I are fluoro modified nucleotides; the nucleotide sequence II comprises no more than 7 fluoro modified nucleotides; and the nucleotides at positions 2, 6, 14, and 16 of the nucleotide sequence II are fluoro modified nucleotides.

In some embodiments, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14, and 16 or at positions 2, 6, 8, 9, 14, and 16 of the nucleotide sequence II in the antisense strand are fluoro modified nucleotides, and the nucleotides at the other positions in the antisense strand are non-fluoro modified nucleotides.

In the context of the present disclosure, a "fluoro modified nucleotide" refers to a nucleotide formed by substituting 2'-hydroxy of the ribose group with a fluorine atom, which has a structure as shown by the following Formula (7). A "non-fluoro modified nucleotide" refers to a nucleotide formed by substituting 2'-hydroxy of the ribose group with a non-fluoro group, or a nucleotide analogue. In some embodiments, each non-fluoro modified nucleotide is independently selected from a nucleotide formed by substituting 2'-hydroxy of the ribose group thereof with a non-fluoro group, or a nucleotide analogue.

The nucleotides formed by replacing 2'-hydroxy of the ribose group with a non-fluoro group are well-known in the art, and can be one selected from the group consisting of 2'-alkoxy modified nucleotides, 2'-substituted alkoxy modified nucleotides, 2'-alkyl modified nucleotides, 2'-substituted alkyl modified nucleotides, 2'-amino modified nucleotides, 2'-substituted amino modified nucleotides, and 2'-deoxy nucleotides.

In some embodiments, the 2'-alkoxy modified nucleotide is a 2'-methoxy (2' -OMe) modified nucleotide, as shown by Formula (8). In some embodiments, the 2'-substituted alkoxy modified nucleotide is for example a 2'-O-methoxyethyl (2'-MOE) modified nucleotide, as shown by Formula (9). In some embodiments, the 2'-amino (2'-$NH_2$) modified nucleotide is as shown by Formula (10). In some embodiments, the 2'-deoxy nucleotide (DNA) is as shown by Formula (11).

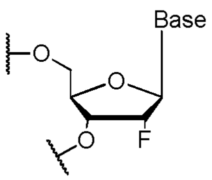
Formula (7)

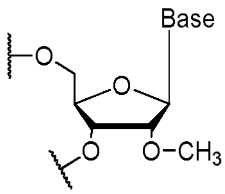
Formula (8)

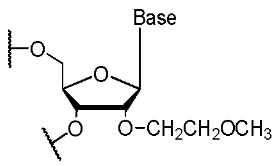
Formula (9)

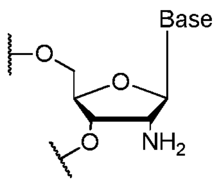
Formula (10)

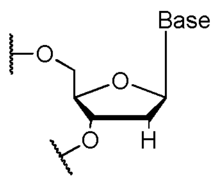
Formula (11)

A nucleotide analogue refers to a group that can replace a nucleotide in a nucleic acid, while structurally differs from an adenine ribonucleotide, a guanine ribonucleotide, a cytosine ribonucleotide, a uracil ribonucleotide, or thymine deoxyribonucleotide. In some embodiments, the nucleotide analogue may be an isonucleotide, a bridged nucleotide or an acyclic nucleotide.

A BNA nucleotide (bridged nucleic acid, BNA) is a nucleotide that is constrained or inaccessible. BNA can contain a 5-, 6- membered or a 7-membered ring bridged structure with a "fixed" C3'-endo sugar puckering. The bridge is typically incorporated at the 2'- and 4'-positions of the ribose to afford a 2', 4'-BNA nucleotide. In some embodiments, BNA may be LNA, ENA, cET BNA and so on, which are shown by Formulae (12), (13) and (14), respectively.

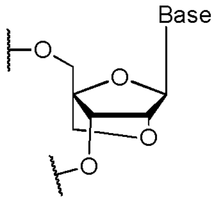 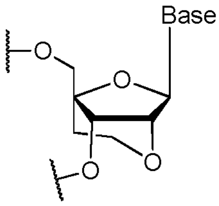 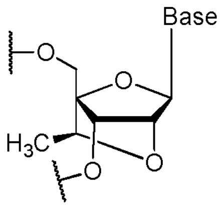

Formula (12) Formula (13) Formula (14)

An acyclic nucleotide refers to a class of nucleotides in which the sugar ring is opened. In some embodiments, the acrylic nucleotide is an unlocked nucleic acid (UNA) or a glycerol nucleic acid (GNA), which are as shown by Formulae (15) and (16), respectively.

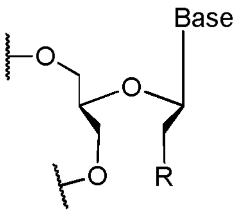 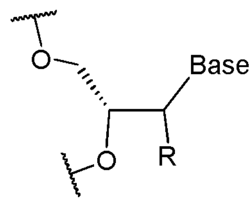

Formula (15) Formula (16)

In the above Formulae (15) and (16), R is selected from H, OH or alkoxy (O-alkyl).

An isonucleotide is a compound formed by changing the position of the base on the ribose ring in the nucleotide. In some embodiments, the isonucleotide is a compound formed by transposing the base from 1'-position to 2'-position or 3'-position on the ribose ring, as shown by Formula (17) or (18), respectively.

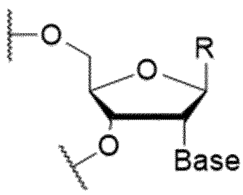 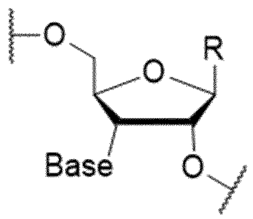

Formula (17) Formula (18)

In the above compounds of Formulae (17)-(18), "Base" represents a base of a nucleic acid, such as A, U, G, C, or T; R is selected from H, OH, F, or the above non-fluoro group.

In some embodiments, a nucleotide analogue is one selected from the group consisting of isonucleotide, LNA, ENA, cET, UNA, and GNA. In some embodiments, each non-fluoro modified nucleotide is a methoxy modified nucleotide. In the context of the present disclosure, the methoxy modified nucleotide refers to a nucleotide formed by substituting 2'-hydroxy of the ribose group with a methoxy group.

In the context of the disclosure, a "fluoro modified nucleotide", a "2'-fluoro modified nucleotide", a "nucleotide in which 2'-hydroxy of a ribose group is substituted with a fluorine atom", and a "nucleotide with 2'-fluororibosyl" have the same meaning, referring to a compound in which 2'-hydroxy of the nucleotide is substituted with a flurorin atom, which has a structure as shown by Formula (7). A "methoxy modified nucleotide", a "2'-methoxy modified nucleotide", a "nucleotide in which 2'-hydroxy of a ribose group is substituted with a methoxy" and a "nucleotide with 2'-methoxyribosyl" have the same meaning, referring to a compound in which 2'-hydroxy of the ribose group in the nucleotide is substituted with a methoxy, which has a structure as shown by Formula (8).

In some embodiments, the siRNA of the present disclosure is a siRNA with the following modifications: in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are methoxy modified nucleotides; the nucleotides at positions 2, 6, 14, and 16 or at positions 2, 6, 8, 9, 14, and 16 of the nucleotide sequence II in the antisense strand are fluoro modified nucleotides, and the nucleotides at the other

positions in the antisense strand are methoxy modified nucleotides.

In some embodiments, the siRNA of the present disclosure is a siRNA with the following modifications: in the direction from 5' terminal to 3' terminal, the nucleotides at positions 5, 7, 8, and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 8, 9, 14, and 16 of the nucleotide sequence II in the antisense strand of the siRNA are fluoro modified nucleotides, and the nucleotides at the other positions in the antisense strand of the siRNA are methoxy modified nucleotides;

Alternatively, in the direction from 5' terminal to 3' terminal, the nucleotides at positions 5, 7, 8, and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14, and 16 of the nucleotide sequence II in the antisense strand of the siRNA are fluoro modified nucleotides, and the nucleotides at the other positions in the antisense strand of the siRNA are methoxy modified nucleotides.

Alternatively, in the direction from 5' terminal to 3' terminal, the nucleotides at positions 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14, and 16 of the nucleotide sequence II in the antisense strand of the siRNA are fluoro modified nucleotides, and the nucleotides at the other positions in the antisense strand of the siRNA are methoxy modified nucleotides.

In some embodiments, the siRNA of the present disclosure is any one of siAPa1-M1, siAPa2-M1, siAPa1-M2, siAPa2-M2, siAPa1-M3, siAPa2-M3, siAPb1-M1, siAPb2-M1, siAPb1-M2, siAPb2-M2, siAPb1-M3, siAPb2-M3, siAPc1-M1, siAPc2-M1, siAPc1-M2, siAPc2-M2, siAPc1-M3, siAPc2-M3, siAPd1-M1, siAPd2-M1, siAPd1-M2, siAPd2-M2, siAPd1-M3, siAPd2-M3, siAPe1-M1, siAPe2-M1, siAPe1-M2, siAPe2-M2, siAPel-M3, or siAPe2-M3:

siAPa1-M1

sense strand: 5'-UmUmAmAmAfAmGfGfGfAmCmAmGmUmAmUmUmCmUm-3'(SEQ ID NO: 61)
antisense strand: 5'-AmGfAmAmUmAfCmUfGfUmCmCmCmUfUmUfUmAmAmGmCm-3' (SEQ ID NO: 62)

siAPa2-M1

sense strand: 5'-GmCmUmUmAmAmAfAmGfGfGfAmCmAmGmUmAmUmUmCmUm -3'(SEQ ID NO: 63)

antisense strand: 5'- AmGfAmAmUmAfCmUfGfUmCmCmCmUfUmUfUmAmAmGmCm AmAm-3' (SEQ ID NO: 64)

siAPa1-M2
sense strand: 5'-UmUmAmAmAfAmGfGfGfAmCmAmGmUmAmUmUmCmUm-3'(SEQID NO: 65)

antisense strand: 5'-AmGfAmAmUmAfCmUmGmUmCmCmCmUfUmUfUmAmAmGmCm -3' (SEQ ID NO: 66)

siAPa2-M2

sense strand: 5'-GmCmUmUmAmAmAfAmGfGfGfAmCmAmGmUmAmUmUmCmUm -3'(SEQ ID NO: 67)

antisense strand: 5'-AmGfAmAmUmAfCmUmGmUmCmCmCmUfUmUfUmAmAmGm CmAmAm -3' (SEQ ID NO: 68)

siAPa1-M3

sense strand: 5’-UmUmAmAmAmAmGfGfGfAmCmAmGmUmAmUmUmCmUm -3’(SEQ ID NO: 69)

antisense strand: 5'- AmGfAmAmUmAfCmUmGmUmCmCmCmUfUmUfUmAmAmGmCm -3' (SEQ ID NO: 70)

siAPa2-M3

sense strand: 5'-GmCmUmUmAmAmAmAmGfGfGfAmCmAmGmUmAmUmUmCmUm -3'(SEQ ID NO: 71)

antisense strand: 5'- AmGfAmAmUmAfCmUmGmUmCmCmCmUfUmUfUmAmAmGmCm AmAm -3' (SEQ ID NO: 72)

siAPb1-M1

sense strand: 5’-AmCmAmGmUfAmUfUfCfUmCmAmGmUmGmCmUmCmUm -3’(SEQ ID NO: 73)
antisense strand: 5’-AmGfAmGmCmAfCmUfGfAmGmAmAmUfAmCfUmGmUmCmCm-3’ (SEQ ID NO: 74)

siAPb2-M1

sense strand: 5'- GmGmAmCmAmGmUfAmUfUfCfUmCmAmGmUmGmCmUmCmUm -3'(SEQ ID NO: 75)

antisense strand: 5'- AmGfAmGmCmAfCmUfGfAmGmAmAmUfAmCfUmGmUmCmCm CmUm -3' (SEQ ID NO: 76)

siAPb1-M2
sense strand: 5’-AmCmAmGmUfAmUfUfCfUmCmAmGmUmGmCmUmCmUm-3’(SEQ ID NO: 77)

antisense strand: 5'- AmGfAmGmCmAfCmUmGmAmGmAmAmUfAmCfUmGmUmCmCm -3' (SEQ ID NO: 78)

siAPb2-M2

sense strand: 5'- GmGmAmCmAmGmUfAmUfUfCfUmCmAmGmUmGmCmUmCmUm -3'(SEQ ID NO: 79)

antisense strand: 5'- AmGfAmGmCmAfCmUmGmAmGmAmAmUfAmCfUmGmUmCmCm CmUm -3' (SEQ ID NO: 80)

siAPb1-M3
sense strand: 5’-AmCmAmGmUmAmUfUfCfUmCmAmGmUmGmCmUmCmUm-3’(SEQ ID NO: 81)

antisense strand: 5'- AmGfAmGmCmAfCmUmGmAmGmAmAmUfAmCfUmGmUmCmCm -3' (SEQ ID NO: 82)

siAPb2-M3

sense strand: 5'- GmGmAmCmAmGmUmAmUfUfCfUmCmAmGmUmGmCmUmCmUm -3'(SEQ ID NO: 83)

antisense strand: 5'- AmGfAmGmCmAfCmUmGmAmGmAmAmUfAmCfUmGmUmCmCm CmUm -3' (SEQ ID NO: 84)

siAPc1-M1

sense strand: 5'- UmAmUmUmCfUmCfAfGfUmGmCmUmCmUmCmCmUmAm -3'(SEQ ID NO: 85)
antisense strand: 5'- UmAfGmGmAmGfAmGfCfAmCmUmGmAfGmAfAmUmAmCmUm -3' (SEQ ID NO: 86)

siAPc2-M1

sense strand: 5'- AmGmUmAmUmUmCfUmCfAfGfUmGmCmUmCmUmCmCmUmAm -3'(SEQ ID NO: 87)

antisense strand: 5'- UmAfGmGmAmGfAmGfCfAmCmUmGmAfGmAfAmUmAmCm UmGmUm -3'(SEQ ID NO: 88)

siAPc1-M2
sense strand: 5'- UmAmUmUmCfUmCfAfGfUmGmCmUmCmUmCmCmUmAm -3'(SEQ ID NO: 89)

antisense strand: 5'- UmAfGmGmAmGfAmGmCmAmCmUmGmAfGmAfAmUmAmCmUm -3' (SEQ ID NO: 90)

siAPc2-M2

sense strand: 5'- AmGmUmAmUmUmCfUmCfAfGfUmGmCmUmCmUmCmCmUmAm -3'(SEQ ID NO: 91)

antisense strand: 5'- UmAfGmGmAmGfAmGmCmAmCmUmGmAfGmAfAmUmAmCmUm GmUm -3'(SEQ ID NO: 92)

siAPc1-M3
sense strand: 5'-UmAmUmUmCmUmCfAfGfUmGmCmUmCmUmCmCmUmAm-3'(SEQ ID NO: 93)

antisense strand: 5'- UmAfGmGmAmGfAmGmCmAmCmUmGmAfGmAfAmUmAmCmUm -3' (SEQ ID NO: 94)

siAPc2-M3

sense strand: 5'- AmGmUmAmUmUmCmUmCfAfGfUmGmCmUmCmUmCmCmUmAm -3'(SEQ ID NO: 95)

antisense strand: 5'- UmAfGmGmAmGfAmGmCmAmCmUmGmAfGmAfAmUmAmCmUm GmUm -3'(SEQ ID NO: 96)

siAPd1-M1 sense strand: 5'-AmGmUmAmUfUmCfUfCfAmGmUmGmCmUmCmUmCmCm-3'(SEQ ID NO: 97)

antisense strand: 5'- GmGfAmGmAmGfCmAfCfUmGmAmGmAfAmUfAmCmUmGm Um-3'(SEQ ID NO: 98)

siAPd2-M1

sense strand: 5'- AmCmAmGmUmAmUfUmCfUfCfAmGmUmGmCmUmCmUmCmCm - 3'(SEQ ID NO: 99)

antisense strand: 5'- GmGfAmGmAmGfCmAfCfUmGmAmGmAfAmUfAmCmUmGmUm CmCm -3'(SEQ ID NO: 100)

siAPd1-M2

sense strand: 5'-AmGmUmAmUfUmCfUfCfAmGmUmGmCmUmCmUmCmCm-3'(SEQ ID NO: 101)

sense strand: 5'- AmGmUmAmUfUmCfUfCfAmGmUmGmCmUmCmUmCmCm -3'(SEQ ID NO: 101)

antisense strand: 5'- GmGfAmGmAmGfCmAmCmUmGmAmGmAfAmUfAmCmUm GmUm-3'(SEQ ID NO: 102)

siAPd2-M2

sense strand: 5'- AmCmAmGmUmAmUfUmCfUfCfAmGmUmGmCmUmCmUmCmCm - 3'(SEQ ID NO: 103)

antisense strand: 5'- GmGfAmGmAmGfCmAmCmUmGmAmGmAfAmUfAmCmUmGm UmCmCm -3'(SEQ ID NO: 104)

siAPd1-M3

sense strand: 5'- AmGmUmAmUmUmCfUfCfAmGmUmGmCmUmCmUmCmCm -3'(SEQ ID NO: 105)

antisense strand: 5'- GmGfAmGmAmGfCmAmCmUmGmAmGmAfAmUfAmCmUmGmUm -3'(SEQ ID NO: 106)

siAPd2-M3

sense strand: 5'- AmCmAmGmUmAmUmUmCfUfCfAmGmUmGmCmUmCmUmCmCm - 3'(SEQ ID NO: 107)

antisense strand: 5'- GmGfAmGmAmGfCmAmCmUmGmAmGmAfAmUfAmCmUmGmUm CmCm -3'(SEQ ID NO: 108)

siAPe1-M1

sense strand: 5'- GmGmAmCmAfGmUfAfUfUmCmUmCmAmGmUmGmCmUm -3'(SEQ ID NO: 109)

antisense strand: 5'- AmGfCmAmCmUfGmAfGfAmAmUmAmCfUmGfUmCmCmCmUm -3'(SEQ ID NO: 110)

siAPe2-M1

sense strand: 5'- AmGmGmGmAmCmAfGmUfAfUfUmCmUmCmAmGmUmGmCmUm -3'(SEQ ID NO: 111)

antisense strand: 5'-AmGfCmAmCmUfGmAfGfAmAmUmAmCfUmGfUmCmCmCmUm UmUm -3'(SEQ ID NO: 112)

siAPe1-M2

sense strand: 5'- GmGmAmCmAfGmUfAfUfUmCmUmCmAmGmUmGmCmUm -3'(SEQ ID NO: 113)

antisense strand: 5'- AmGfCmAmCmUfGmAmGmAmAmUmAmCfUmGfUmCmCmCmUm -3'(SEQ ID NO: 114)

siAPe2-M2

sense strand: 5'- AmGmGmGmAmCmAfGmUfAfUfUmCmUmCmAmGmUmGmCmUm -

3'(SEQ ID NO: 115)

antisense strand: 5'-AmGfCmAmCmUfGmAmGmAmAmUmAmCfUmGfUmCmCm CmUmUmUm -3'(SEQ ID NO: 116)

siAPe1-M3

sense strand: 5'- GmGmAmCmAmGmUfAfUfUmCmUmCmAmGmUmGmCmUm -3'(SEQ ID NO: 117)

antisense strand: 5'- AmGfCmAmCmUfGmAmGmAmAmUmAmCfUmGfUmCmCmCmUm -3'(SEQ ID NO: 118)

siAPe2-M3

sense strand: 5'-AmGmGmGmAmCmAmGmUfAfUfUmCmUmCmAmGmUmGmCmUm -3'(SEQ ID NO: 119)

antisense strand: 5'-AmGfCmAmCmUfGmAmGmAmAmUmAmCfUmGfUmCmCmCmUm UmUm-3'(SEQ ID NO: 120)

The siRNAs with the above modifications not only have lower costs, but also cause that the ribonucleases in the blood cannot easily cleave the nucleic acid, thereby increasing the stability of the nucleic acid and rendering the nucleic acid to have stronger resistance against nuclease hydrolysis.

In some embodiments, at least a portion of the phosphate groups in the phosphate-ribose backbone of at least one single strand in the sense strand and the antisense strand of the siRNA of the present disclosure are phosphate groups with modified groups. In some embodiments, the phosphate group with modified group(s) is a phosphorothioate group formed by substituting at least one oxygen atom in a phosphodiester bond in a phosphate groups with a sulfur atom. In some embodiments, the phosphate group with modified group(s) is a phosphorothioate group having a structure as shown by Formula (1):

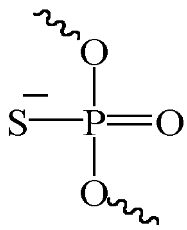

Formula (1).

This modification stabilizes the double-stranded structure of the siRNA, thereby maintaining high specificity and high affinity of base pairing.

In some embodiments, in the siRNA of the present disclosure, the phosphorothioate linkage is located in at least one of the group consisting of the following positions: the position between the first and the second nucleotides at either terminal of the sense or antisense strand, the position between the second and the third nucleotides at either terminal of the sense or antisense strand, or any combination thereof. In some embodiments, the phosphorothioate linkage is located in all the above positions except 5' terminal of the sense strand. In some embodiments, the phosphorothioate linkage is located in all the above positions except 3' terminal of the sense strand. In some embodiments, the phosphorothioate linkage is located in at least one of the following positions:

the position between the first and second nucleotides at 5' terminal of the sense strand;
the position between the second and third nucleotides at 5' terminal of the sense strand;
the position between the first and second nucleotides at 3' terminal of the sense strand;
the position between the second and third nucleotides at 3' terminal of the sense strand;
the position between the first and second nucleotides at 5' terminal of the antisense strand;
the position between the second and third nucleotides at 5' terminal of the antisense strand;
the position between the first and second nucleotides at 3' terminal of the antisense strand; and
the position between the second and third nucleotides at 3' terminal of the antisense strand.

In some embodiments, the siRNA of the present disclosure is siAPa1-M1S, siAPa2-M1S, siAPa1-M2S, siAPa2-M2S, siAPa1-M3S, siAPa2-M3S, siAPb1-M1S, siAPb2-M1S, siAPb1-M2S, siAPb2-M2S, siAPb1-M3S, siAPb2-M3S, siAPc1-M1S, siAPc2-M1S, siAPc1-M2S, siAPc2-M2S, siAPc1-M3S, siAPc2-M3S, siAPd1-M1S, siAPd2-M1S, siAPd1-M2S, siAPd2-M2S, siAPd1-M3S, siAPd2-M3S, siAPe1-MIS, siAPe2-MIS, siAPe1-M2S, siAPe2-M2S, siAPe1-M3S, and siAPe2-M3S.

siAPa1-M1S
sense strand: 5'- UmsUmsAmAmAfAmGfGfGfAmCmAmGmUmAmUmUmCmUm -3' (SEQ ID NO: 121)

antisense strand: 5'- AmsGfsAmAmUmAfCmUfGfUmCmCmCmUfUmUfUmAmAmsGmsCm -3' (SEQ ID NO: 122)

siAPa2-M1S

sense strand: 5'- GmsCmsUmUmAmAmAfAmGfGfGfAmCmAmGmUmAmUmUmCmUm -3' (SEQ ID NO: 123)

antisense strand: 5'- AmsGfsAmAmUmAfCmUfGfUmCmCmCmUfUmUfUmAmAmGmCmsAmsAm -3' (SEQ ID NO: 124)

siAPa1-M2S
sense strand: 5'- UmsUmsAmAmAfAmGfGfGfAmCmAmGmUmAmUmUmCmUm -3' (SEQ ID NO: 125)

antisense strand: 5'- AmsGfsAmAmUmAfCmUmGmUmCmCmCmUfUmUfUmAmAmsGmsCm -3' (SEQ ID NO: 126)

siAPa2-M2S

sense strand: 5'- GmsCmsUmUmAmAmAfAmGfGfGfAmCmAmGmUmAmUmUmCmUm -3' (SEQ ID NO: 127)

antisense strand: 5'- AmsGfsAmAmUmAfCmUmGmUmCmCmCmUfUmUfUmAmAmGmCmsAmsAm -3' (SEQ ID NO: 128)

siAPa1-M3S
sense strand: 5'-UmsUmsAmAmAmAmGfGfGfAmCmAmGmUmAmUmUmCmUm-3' (SEQ ID NO: 129)

antisense strand: 5'- AmsGfsAmAmUmAfCmUmGmUmCmCmCmUfUmUfUmAmAmsGmsCm -3' (SEQ ID NO: 130)

siAPa2-M3 S

sense strand: 5'- GmsCmsUmUmAmAmAmAmGfGfGfAmCmAmGmUmAmUmUmCmUm -3' (SEQ ID NO: 131)

antisense strand: 5'- AmsGfsAmAmUmAfCmUmGmUmCmCmCmUfUmUfUmAmAmGmCmsAmsAm -3' (SEQ ID NO: 132)

siAPb1-MIS
sense strand: 5'-AmsCmsAmGmUfAmUfUfCfUmCmAmGmUmGmCmUmCmUm-3' (SEQ ID NO: 133)

antisense strand: 5'- AmsGfsAmGmCmAfCmUfGfAmGmAmAmUfAmCfUmGmUmsCmsCm -3' (SEQ ID NO: 134)

siAPb2-M1S

sense strand: 5'- GmsGmsAmCmAmGmUfAmUfUfCfUmCmAmGmUmGmCmUmCmUm -3' (SEQ ID NO: 135)

antisense strand: 5'- AmsGfsAmGmCmAfCmUfGfAmGmAmAmUfAmCfUmGmUmCm CmsCmsUm -3' (SEQ ID NO: 136)

siAPb1-M2S
sense strand: 5'-AmsCmsAmGmUfAmUfUfCfUmCmAmGmUmGmCmUmCmUm-3' (SEQ ID NO: 137)

antisense strand: 5'- AmsGfsAmGmCmAfCmUmGmAmGmAmAmUfAmCfUmGmUms CmsCm -3' (SEQ ID NO: 138)

siAPb2-M2S
sense strand: 5'- GmsGmsAmCmAmGmUfAmUfUfCfUmCmAmGmUmGmCmUmCmUm -3' (SEQ ID NO: 139)

antisense strand: 5'- AmsGfsAmGmCmAfCmUmGmAmGmAmAmUfAmCfUmGmUmCm CmsCmsUm -3' (SEQ ID NO: 140)

siAPb1-M3S
sense strand: 5'-AmsCmsAmGmUmAmUfUfCfUmCmAmGmUmGmCmUmCmUm-3' (SEQ ID NO: 141)

antisense strand: 5'- AmsGfsAmGmCmAfCmUmGmAmGmAmAmUfAmCfUmGm UmsCmsCm -3' (SEQ ID NO: 142)

siAPb2-M3 S

sense strand: 5'- GmsGmsAmCmAmGmUmAmUfUfCfUmCmAmGmUmGmCmUmCmUm -3' (SEQ ID NO: 143)
antisense strand: 5'- AmsGfsAmGmCmAfCmUmGmAmGmAmAmUfAmCfUmGmUm CmCmsCmsUm -3' (SEQ ID NO: 144)

siAPc1-M1S
sense strand: 5'- UmsAmsUmUmCfUmCfAfGfUmGmCmUmCmUmCmCmUmAm -3' (SEQ ID NO: 145)

antisense strand: 5'- UmsAfsGmGmAmGfAmGfCfAmCmUmGmAfGmAfAmUmAms CmsUm -3' (SEQ ID NO: 146)

siAPc2-M1S
sense strand: 5'-AmsGmsUmAmUmUmCfUmCfAfGfUmGmCmUmCmUmCmCmUmAm-3' (SEQ ID NO: 147)

antisense strand: 5'- UmsAfsGmGmAmGfAmGfCfAmCmUmGmAfGmAfAmUmAmCm UmsGmsUm -3' (SEQ ID NO: 148)

siAPc1-M2S
sense strand: 5'- UmsAmsUmUmCfUmCfAfGfUmGmCmUmCmUmCmCmUmAm -3' (SEQ ID NO: 149)

antisense strand: 5'- UmsAfsGmGmAmGfAmGmCmAmCmUmGmAfGmAfAmUm AmsCmsUm -3' (SEQ ID NO: 150)

siAPc2-M2S
sense strand: 5'- AmsGmsUmAmUmUmCfUmCfAfGfUmGmCmUmCmUmCmCmUmAm -3' (SEQ ID NO: 151)

antisense strand: 5'- UmsAfsGmGmAmGfAmGmCmAmCmUmGmAfGmAfAmUmAmCm UmsGmsUm -3' (SEQ ID NO: 152)

siAPc1-M3S
sense strand: 5'-UmsAmsUmUmCmUmCfAfGfUmGmCmUmCmUmCmCmUmAm-3' (SEQ ID NO: 153)

antisense strand: 5'- UmsAfsGmGmAmGfAmGmCmAmCmUmGmAfGmAfAmUmAms CmsUm -3' (SEQ ID NO: 154)

siAPc2-M3 S

sense strand: 5'- AmsGmsUmAmUmUmCmUmCfAfGfUmGmCmUmCmUmCmCmUmAm -3' (SEQ ID NO: 155)

antisense strand: 5'- UmsAfsGmGmAmGfAmGmCmAmCmUmGmAfGmAfAmUmAmCm UmsGmsUm -3' (SEQ ID NO: 156)

siAPd1-M1S
sense strand: 5'-AmsGmsUmAmUfUmCfUfCfAmGmUmGmCmUmCmUmCmCm-3' (SEQ ID NO: 157)

antisense strand: 5'- GmsGfsAmGmAmGfCmAfCfUmGmAmGmAfAmUfAmCmUms GmsUm -3' (SEQ ID NO: 158)

siAPd2-M1S
sense strand: 5'-AmsCmsAmGmUmAmUfUmCfUfCfAmGmUmGmCmUmCmUmCmCm-3' (SEQ ID NO: 159)

antisense strand: 5'- GmsGfsAmGmAmGfCmAfCfUmGmAmGmAfAmUfAmCmUmGm UmsCmsCm -3' (SEQ ID NO: 160)

siAPd1-M2S
sense strand: 5'- AmsGmsUmAmUfUmCfUfCfAmGmUmGmCmUmCmUmCmCm -3' (SEQ ID NO: 161)

antisense strand: 5'- GmsGfsAmGmAmGfCmAmCmUmGmAmGmAfAmUfAmCmUms GmsUm-3' (SEQ ID NO: 162)

siAPd2-M2S
sense strand: 5'- AmsCmsAmGmUmAmUfUmCfUfCfAmGmUmGmCmUmCmUmCmCm -3' (SEQ ID NO: 163)

antisense strand: 5'- GmsGfsAmGmAmGfCmAmCmUmGmAmGmAfAmUfAmCmUmGm UmsCmsCm -3' (SEQ ID NO: 164)

siAPd1-M3S
sense strand: 5'-AmsGmsUmAmUmUmCfUfCfAmGmUmGmCmUmCmUmCmCm-3' (SEQ ID NO: 165)

antisense strand: 5'- GmsGfsAmGmAmGfCmAmCmUmGmAmGmAfAmUfAmCmUms GmsUm -3' (SEQ ID NO: 166)

siAPd2-M3 S

sense strand: 5'- AmsCmsAmGmUmAmUmUmCfUfCfAmGmUmGmCmUmCmUmCmCm -3’ (SEQ ID NO: 167)

antisense strand: 5'- GmsGfsAmGmAmGfCmAmCmUmGmAmGmAfAmUfAmCmUmGm UmsCmsCm -3’ (SEQ ID NO: 168)

siAPe1-M1S
sense strand: 5’-GmsGmsAmCmAfGmUfAfUfUmCmUmCmAmGmUmGmCmUm-3’ (SEQ ID NO: 169)

antisense strand: 5'- AmsGfsCmAmCmUfGmAfGfAmAmUmAmCfUmGfUmCmCmsCmsUm -3’ (SEQ ID NO: 170)

siAPe2-M1S
sense strand: 5’-AmsGmsGmGmAmCmAfGmUfAfUfUmCmUmCmAmGmUmGmCmUm-3’ (SEQ ID NO: 171);

antisense strand: 5'- AmsGfsCmAmCmUfGmAfGfAmAmUmAmCfUmGfUmCmCmCmUmsUmsUm -3’ (SEQ ID NO: 172)

siAPe1-M2S
sense strand: 5’-GmsGmsAmCmAfGmUfAfUfUmCmUmCmAmGmUmGmCmUm-3’ (SEQ ID NO: 173)

antisense strand: 5'- AmsGfsCmAmCmUfGmAmGmAmAmUmAmCfUmGfUmCmCms CmsUm -3’ (SEQ ID NO: 174)

siAPe2-M2S
sense strand: 5’-AmsGmsGmGmAmCmAfGmUfAfUfUmCmUmCmAmGmUmGmCmUm-3’ (SEQ ID NO: 175)

antisense strand: 5'-AmsGfsCmAmCmUfGmAmGmAmAmUmAmCfUmGfUmCmCm CmUmsUmsUm -3’ (SEQ ID NO: 176)

siAPe1-M3S
sense strand: 5’-GmsGmsAmCmAmGmUfAfUfUmCmUmCmAmGmUmGmCmUm-3’ (SEQ ID NO: 177)

antisense strand: 5'- AmsGfsCmAmCmUfGmAmGmAmAmUmAmCfUmGfUmCmCms CmsUm -3’ (SEQ ID NO: 178)

siAPe2-M3 S

sense strand: 5'- AmsGmsGmGmAmCmAmGmUfAfUfUmCmUmCmAmGmUmGmCmUm -3’ (SEQ ID NO: 179)

antisense strand: 5'-AmsGfsCmAmCmUfGmAmGmAmAmUmAmCfUmGfUmCmCm CmUmsUmsUm -3’ (SEQ ID NO: 180)

In some embodiments, the nucleotide at 5’-terminal in the antisense strand of the siRNA is a 5’-phosphate nucleotide or a 5’-phosphate analogue modified nucleotide.

Typical 5'-phosphate nucleotides or 5'-phosphate analogue modified nucleotides are well known to those skilled in the art. For example, the 5'-phosphate nucleotides may have the following structure:

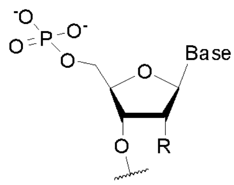

Formula (2)

As another example, Anastasia Khvorova and Jonathan K. Watts, The chemical evolution of oligonucleotide therapies of clinical utility. Nature Biotechnology, 2017, 35(3): 238-48 discloses the following four 5'-phosphate analogue modified nucleotides:

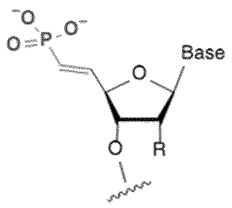

Formula (3)

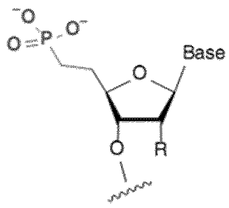

Formula (4)

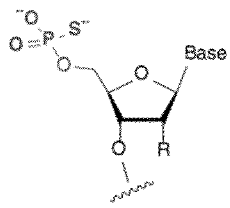

Formula (5)

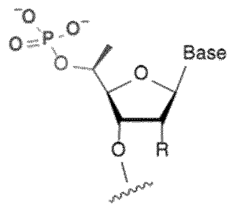

Formula (6)

wherein R is selected from H, OH, methoxy, and F;
"Base" represents a base selected from A, U, C, G, or T.

In some embodiments, the 5' -phosphate nucleotide is a nucleotide with 5'-phosphate modification as shown by Formula (2); the 5'-phosphate analogue modified nucleotide is a nucleotide with vinylphosphonate modification as shown by Formula (3), or a phosphorothioate modified nucleotide as shown by Formula (5).

In some embodiments, the siRNA of the present disclosure is any one of siAPa1-M1P1, siAPa2-M1P1, siAPa1-M2P1, siAPa2-M2P1, siAPa1-M3P1, siAPa2-M3P1, siAPa1-M1SP1, siAPa2-M1SP1, siAPa1-M2SP1, siAPa2-M2SP1, siAPa1-M3SP1, siAPa2-M3SP1, siAPa1U-M1P1, siAPa2U-M1P1, siAPa1U-M2P1, siAPa2U-M2P1, siAPa1U-M3P1, siAPa2U-M3P1, siAPa1U-M1SP1, siAPa2U-M1SP1, siAPa1U-M2SP1, siAPa2U-M2SP1, siAPa1U-M3SP1, siAPa2U-M3SP1, siAPb1-M1P1, siAPb2-M1P1, siAPb1-M2P1, siAPb2-M2P1, siAPb1-M3P1, siAPb2-M3P1, siAPb1-M1SP1, siAPb2-M1SP1, siAPb1-M2SP1, siAPb2-M2SP1, siAPb1-M3SP1, siAPb2-M3SP1, siAPb1U-M1P1, siAPb2U-M1P1, siAPb1U-M2P1, siAPb2U-M2P1, siAPb1U-M3P1, siAPb2U-M3P1, siAPb1U-M1SP1, siAPb2U-M1SP1, siAPb1U-M2SP1, siAPb2U-M2SP1, siAPb1U-M3SP1, siAPb2U-M3SP1, siAPc1-M1P1, siAPc2-M1P1, siAPc1-M2P1, siAPc2-M2P1, siAPc1-M3P1, siAPc2-M3P1, siAPc1-M1SP1, siAPc2-M1SP1, siAPc1-M2SP1, siAPc2-M2SP1, siAPc1-M3SP1, siAPc2-M3SP1, siAPd1-M1P1, siAPd2-M1P1, siAPd1-M2P1, siAPd2-M2P1, siAPd1-M3P1, siAPd2-M3P1, siAPd1-M1SP1, siAPd2-M1SP1, siAPd1-M2SP1, siAPd2-M2SP1, siAPd1-M3SP1, siAPd2-M3SP1, siAPd1U-M1P1, siAPd2U-M1P1, siAPd1U-M2P1, siAPd2U-M2P1, siAPd1U-M3P1, siAPd2U-M3P1, siAPd1U-M1SP1, siAPd2U-M1SP1, siAPd1U-M2SP1, siAPd2U-M2SP1, siAPd1U-M3SP1, siAPd2U-M3SP1, siAPe1-M1P1, siAPe2-M1P1, siAPe1-M2P1, siAPe2-M2P1, siAPe1-M3P1, siAPe2-M3P1, siAPe1-M1SP1, siAPe2-M1SP1, siAPe1-M2SP1, siAPe2-M2SP1, siAPe1-M3SP1, siAPe2-M3SP1, siAPe1U-M1P1, siAPe2U-M1P1, siAPe1U-M2P1, siAPe2U-M2P1, siAPe1U-M3P1, siAPe2U-M3P1, siAPe1U-M1SP1, siAPe2U-M1SP1, siAPe1U-M2SP1, siAPe2U-M2SP1, siAPe1U-M3SP1, and siAPe2U-M3SP1.

siAPa1-M1P1
sense strand: 5'-UmUmAmAmAfAmGfGfGfAmCmAmGmUmAmUmUmCmUm-3'(SEQ ID NO: 181)

antisense strand: 5'-P1-AmGfAmAmUmAfCmUfGfUmCmCmCmUfUmUfUmAmAmGmCm -3' (SEQ ID NO: 182)

siAPa2-M1P1

sense strand: 5'- GmCmUmUmAmAmAfAmGfGfGfAmCmAmGmUmAmUmUmCmUm -3'(SEQ ID NO: 183)

antisense strand: 5'-P1-AmGfAmAmUmAfCmUfGfUmCmCmCmUfUmUfUmAmAmGm CmAmAm -3' (SEQ ID NO: 184)

siAPa1-M2P1 sense strand: 5'-UmUmAmAmAfAmGfGfGfAmCmAmGmUmAmUmUmCmUm-3'(SEQ ID NO: 185)

antisense strand: 5'-P1-AmGfAmAmUmAfCmUmGmUmCmCmCmUfUmUfUmAmAGmCm -3' (SEQ ID NO: 186)

siAPa2-M2P1

sense strand: 5'- GmCmUmUmAmAmAfAmGfGfGfAmCmAmGmUmAmUmUmCmUm -3'(SEQ ID NO: 187)

antisense strand: 5'-P1-AmGfAmAmUmAfCmUmGmUmCmCmCmUfUmUfUmAmAm GmCmAmAm -3' (SEQ ID NO: 188)

siAPa1-M3P1
sense strand: 5'-UmUmAmAmAmAmGfGfGfAmCmAmGmUmAmUmUmCmUm-3'(SEQ ID NO: 189)

antisense strand: 5'-P1-AmGfAmAmUmAfCmUmGmUmCmCmCmUfUmUfUmAmAm GmCm -3' (SEQ ID NO: 190)

siAPa2-M3P1

sense strand: 5'- GmCmUmUmAmAmAmAmGfGfGfAmCmAmGmUmAmUmUmCmUm -3'(SEQ ID NO: 191)

antisense strand: 5'-P1-AmGfAmAmUmAfCmUmGmUmCmCmCmUfUmUfUmAmAmGm CmAmAm -3' (SEQ ID NO: 192)

siAPa1-M1SP1
sense strand: 5'- UmsUmsAmAmAfAmGfGfGfAmCmAmGmUmAmUmUmCmUm -3'(SEQ ID NO: 193)

antisense strand: 5'-P1-AmsGfsAmAmUmAfCmUfGfUmCmCmCmUfUmUfUmAmAms GmsCm -3' (SEQ ID NO: 194)

siAPa2-M1SP1

sense strand: 5'- GmsCmsUmUmAmAmAfAmGfGfGfAmCmAmGmUmAmUmUmCmUm -3'(SEQ ID NO: 195)

antisense strand: 5'-P1-AmsGfsAmAmUmAfCmUfGfUmCmCmCmUfUmUfUmAmAmGm CmsAmsAm -3' (SEQ ID NO: 196)

siAPa1-M2SP1
sense strand: 5'- UmsUmsAmAmAfAmGfGfGfAmCmAmGmUmAmUmUmCmUm -3'(SEQ ID NO: 197)

antisense strand: 5'-P1-AmsGfsAmAmUmAfCmUmGmUmCmCmCmUfUmUfUmAmAms GmsCm -3' (SEQ ID NO: 198)

siAPa2-M2SP1

sense strand: 5'- GmsCmsUmUmAmAmAfAmGfGfGfAmCmAmGmUmAmUmUmCmUm -3'(SEQ ID NO: 199)

antisense strand: 5'-P1-AmsGfsAmAmUmAfCmUmGmUmCmCmCmUfUmUfUmAmAmGm CmsAmsAm -3' (SEQ ID NO: 200)

siAPa1-M3SP1
sense strand: 5'-UmsUmsAmAmAmAmGfGfGfAmCmAmGmUmAmUmUmCmUm-3'(SEQ ID NO: 201)

antisense strand: 5'- P1-AmsGfsAmAmUmAfCmUmGmUmCmCmCmUfUmUfUmAmAms GmsCm -3' (SEQ ID NO: 202)

siAPa2-M3SP1

sense strand: 5'- GmsCmsUmUmAmAmAmAmGfGfGfAmCmAmGmUmAmUmUmCmUm -3'(SEQ ID NO: 203)

antisense strand: 5'-P1-AmsGfsAmAmUmAfCmUmGmUmCmCmCmUfUmUfUmAmAmGm CmsAmsAm-3' (SEQ ID NO: 204)

siAPa1U-M1P1 sense strand: 5'-UmUmAmAmAfAmGfGfGfAmCmAmGmUmAmUmUmCmAm-3'(SEQ ID NO: 327)

antisense strand: 5'-P1-UmGfAmAmUmAfCmUfGfUmCmCmCmUfUmUfUmAmAmGmCm -3' (SEQ ID NO: 328)

siAPa2U-M1P1

sense strand: 5'- GmCmUmUmAmAmAfAmGfGfGfAmCmAmGmUmAmUmUmCmAm -3'(SEQ ID NO: 329)

antisense strand: 5'-P1-UmGfAmAmUmAfCmUfGfUmCmCmCmUfUmUfUmAmAm GmCmAmAm -3' (SEQ ID NO: 330)

siAPa1U-M2P1
sense strand: 5'-UmUmAmAmAfAmGfGfGfAmCmAmGmUmAmUmUmCmAm-3'(SEQ ID NO: 331)

antisense strand: 5'-P1-UmGfAmAmUmAfCmUmGmUmCmCmCmUfUmUfUmAmAm GmCm -3' (SEQ ID NO: 332)

siAPa2U-M2P1

sense strand: 5'- GmCmUmUmAmAmAfAmGfGfGfAmCmAmGmUmAmUmUmCmAm - 3'(SEQ ID NO: 333)

antisense strand: 5'-P1-UmGfAmAmUmAfCmUmGmUmCmCmCmUfUmUfUmAmAmGm CmAmAm -3' (SEQ ID NO: 334)

siAPa1U-M3P1
sense strand: 5'-UmUmAmAmAmAmGfGfGfAmCmAmGmUmAmUmUmCmAm-3'(SEQ ID NO: 335)

antisense strand: 5'-P1-UmGfAmAmUmAfCmUmGmUmCmCmCmUfUmUfUmAmAm GmCm -3' (SEQ ID NO: 336)

siAPa2U-M3P1

sense strand: 5'- GmCmUmUmAmAmAmAmGfGfGfAmCmAmGmUmAmUmUmCmAm - 3'(SEQ ID NO: 337)

antisense strand: 5'-P1-UmGfAmAmUmAfCmUmGmUmCmCmCmUfUmUfUmAmAmGm CmAmAm -3' (SEQ ID NO: 338)

siAPa1U-M1SP1
sense strand: 5'-UmsUmsAmAmAfAmGfGfGfAmCmAmGmUmAmUmUmCmAm-3'(SEQ ID NO: 339)

antisense strand: 5'-P1-UmsGfsAmAmUmAfCmUfGfUmCmCmCmUfUmUfUmAmAms GmsCm -3' (SEQ ID NO: 340)

siAPa2U-M1SP1

sense strand: 5'- GmsCmsUmUmAmAmAfAmGfGfGfAmCmAmGmUmAmUmUmCmAm - 3'(SEQ ID NO: 341)

antisense strand: 5'-P1-UmsGfsAmAmUmAfCmUfGfUmCmCmCmUfUmUfUmAmAmGm CmsAmsAm -3' (SEQ ID NO: 342)

siAPa1U-M2SP1
sense strand: 5'-UmsUmsAmAmAfAmGfGfGfAmCmAmGmUmAmUmUmCmAm-3'(SEQ ID NO: 343)

antisense strand: 5'-P1-UmsGfsAmAmUmAfCmUmGmUmCmCmCmUfUmUfUmAmAms GmsCm -3' (SEQ ID NO: 344)

siAPa2U-M2SP1

sense strand: 5'- GmsCmsUmUmAmAmAfAmGfGfGfAmCmAmGmUmAmUmUmCmAm - 3'(SEQ ID NO: 345)

antisense strand: 5'-P1-UmsGfsAmAmUmAfCmUmGmUmCmCmCmUfUmUfUmAmAmGm CmsAmsAm -3' (SEQ ID NO: 346)

siAPa1U-M3SP1
sense strand: 5'-UmsUmsAmAmAmAmGfGfGfAmCmAmGmUmAmUmUmCmAm-3'(SEQ ID NO: 347)

antisense strand: 5'- P1-UmsGfsAmAmUmAfCmUmGmUmCmCmCmUfUmUfUmAmAms GmsCm -3' (SEQ ID NO: 348)

siAPa2U-M3SP1

sense strand: 5'- GmsCmsUmUmAmAmAmAmGfGfGfAmCmAmGmUmAmUmUmCmAm -3'(SEQ ID NO: 349)

antisense strand: 5'-P1-UmsGfsAmAmUmAfCmUmGmUmCmCmCmUfUmUfUmAmAmGm CmsAmsAm -3' (SEQ ID NO: 350)

siAPb1-M1P1
sense strand: 5'-AmCmAmGmUfAmUfUfCfUmCmAmGmUmGmCmUmCmUm-3'(SEQ ID NO: 205)

antisense strand: 5'-P1-AmGfAmGmCmAfCmUfGfAmGmAmAmUfAmCfUmGmUmCmCm -3' (SEQ ID NO: 206)

siAPb2-M1P1

sense strand: 5'- GmGmAmCmAmGmUfAmUfUfCfUmCmAmGmUmGmCmUmCmUm -3'(SEQ ID NO: 207)

antisense strand: 5'-P1-AmGfAmGmCmAfCmUfGfAmGmAmAmUfAmCfUmGmUmCm CmCmUm -3' (SEQ ID NO: 208)

siAPb1-M2P1
sense strand: 5'-AmCmAmGmUfAmUfUfCfUmCmAmGmUmGmCmUmCmUm-3'(SEQ ID NO: 209)

antisense strand: 5'-P1-AmGfAmGmCmAfCmUmGmAmGmAmAmUfAmCfUmGm UmCmCm -3' (SEQ ID NO: 210)

siAPb2-M2P1

sense strand: 5'- GmGmAmCmAmGmUfAmUfUfCfUmCmAmGmUmGmCmUmCmUm -3'(SEQ ID NO: 211)

antisense strand: 5'-P1-AmGfAmGmCmAfCmUmGmAmGmAmAmUfAmCfUmGmUm CmCmCmUm -3' (SEQ ID NO: 212)

siAPb1-M3P1
sense strand: 5'-AmCmAmGmUmAmUfUfCfUmCmAmGmUmGmCmUmCmUm-3'(SEQ ID NO: 213)

antisense strand: 5'- P1-AmGfAmGmCmAfCmUmGmAmGmAmAmUfAmCfUmGmUm CmCm -3' (SEQ ID NO: 214)

siAPb2-M3P1

sense strand: 5'- GmGmAmCmAmGmUmAmUfUfCfUmCmAmGmUmGmCmUmCmUm -3'(SEQ ID NO: 215)

antisense strand: 5'-P1-AmGfAmGmCmAfCmUmGmAmGmAmAmUfAmCfUmGmUmCm CmCmUm -3' (SEQ ID NO: 216)

siAPb1-M1SP1
sense strand: 5'-AmsCmsAmGmUfAmUfUfCfUmCmAmGmUmGmCmUmCmUm-3'(SEQ ID NO: 217)

antisense strand: 5'-P1-AmsGfsAmGmCmAfCmUfGfAmGmAmAmUfAmCfUmGmUms CmsCm -3' (SEQ ID NO: 218)

siAPb2-M1SP1

sense strand: 5'- GmsGmsAmCmAmGmUfAmUfUfCfUmCmAmGmUmGmCmUmCmUm -3'(SEQ ID NO: 219)

antisense strand: 5'-P1-AmsGfsAmGmCmAfCmUfGfAmGmAmAmUfAmCfUmGmUm CmCmsCmsUm -3' (SEQ ID NO: 220)

siAPb1-M2SP1
sense strand: 5'-AmsCmsAmGmUfAmUfUfCfUmCmAmGmUmGmCmUmCmUm-3'(SEQ ID NO: 221)

antisense strand: 5'-P1-AmsGfsAmGmCmAfCmUmGmAmGmAmAmUfAmCfUmGm UmsCmsCm -3' (SEQ ID NO: 222)

siAPb2-M2SP1

sense strand: 5'- GmsGmsAmCmAmGmUfAmUfUfCfUmCmAmGmUmGmCmUmCmUm -3'(SEQ ID NO: 223)

antisense strand: 5'-P1-AmsGfsAmGmCmAfCmUmGmAmGmAmAmUfAmCfUmGmUm CmCmsCmsUm -3' (SEQ ID NO: 224)

siAPb1-M3SP1
sense strand: 5'- AmsCmsAmGmUmAmUfUfCfUmCmAmGmUmGmCmUmCmUm -3'(SEQ ID NO: 225)

antisense strand: 5'-P1-AmsGfsAmGmCmAfCmUmGmAmGmAmAmUfAmCfUmGmUms CmsCm -3' (SEQ ID NO: 226)

siAPb2-M3SP1
sense strand: 5'-GmsGmsAmCmAmGmUmAmUfUfCfUmCmAmGmUmGmCmUmCmUm-3'(SEQ ID NO: 227)

antisense strand: 5'-P1-AmsGfsAmGmCmAfCmUmGmAmGmAmAmUfAmCfUmGmUm CmCmsCmsUm -3' (SEQ ID NO: 228)

siAPb1U-M1P1

sense strand: 5'-AmCmAmGmUfAmUfUfCfUmCmAmGmUmGmCmUmCmAm-3'(SEQ ID NO: 351)

antisense strand: 5'-P1-UmGfAmGmCmAfCmUfGfAmGmAmAmUfAmCfUmGmUmCmCm -3' (SEQ ID NO: 352)

siAPb2U-M1P1

sense strand: 5'- GmGmAmCmAmGmUfAmUfUfCfUmCmAmGmUmGmCmUmCmAm - 3'(SEQ ID NO: 353)

antisense strand: 5'-P1-UmGfAmGmCmAfCmUfGfAmGmAmAmUfAmCfUmGmUmCmCm CmUm -3' (SEQ ID NO: 354)

siAPb1U-M2P1

sense strand: 5'-AmCmAmGmUfAmUfUfCfUmCmAmGmUmGmCmUmCmAm-3'(SEQ ID NO: 355)

antisense strand: 5'-P1-UmGfAmGmCmAfCmUmGmAmGmAmAmUfAmCfUmGmUm CmCm -3' (SEQ ID NO: 356)

siAPb2U-M2P1

sense strand: 5'- GmGmAmCmAmGmUfAmUfUfCfUmCmAmGmUmGmCmUmCmAm - 3'(SEQ ID NO: 357)

antisense strand: 5'-P1-UmGfAmGmCmAfCmUmGmAmGmAmAmUfAmCfUmGmUm CmCmCmUm -3' (SEQ ID NO: 358)

siAPb1U-M3P1

sense strand: 5'- AmCmAmGmUmAmUfUfCfUmCmAmGmUmGmCmUmCmAm -3'(SEQ ID NO: 359)

antisense strand: 5'- P1-UmGfAmGmCmAfCmUmGmAmGmAmAmUfAmCfUmGmUm CmCm -3' (SEQ ID NO: 360)

siAPb2U-M3P1

sense strand: 5'- GmGmAmCmAmGmUmAmUfUfCfUmCmAmGmUmGmCmUmCmAm - 3'(SEQ ID NO: 361)

antisense strand: 5'-P1-UmGfAmGmCmAfCmUmGmAmGmAmAmUfAmCfUmGmUmCm CmCmUm -3' (SEQ ID NO: 362)

siAPb1U-M1SP1

sense strand: 5'- AmsCmsAmGmUfAmUfUfCfUmCmAmGmUmGmCmUmCmAm -3'(SEQ ID NO: 363)

antisense strand: 5'-P1-UmsGfsAmGmCmAfCmUfGfAmGmAmAmUfAmCfUmGmUms CmsCm -3' (SEQ ID NO: 364)

siAPb2U-M1SP1

sense strand: 5'- GmsGmsAmCmAmGmUfAmUfUfCfUmCmAmGmUmGmCmUmCmAm -3'(SEQ ID NO: 365)

antisense strand: 5'-P1-UmsGfsAmGmCmAfCmUfGfAmGmAmAmUfAmCfUmGm UmCmCmsCmsUm -3' (SEQ ID NO: 366)

siAPb1U-M2SP1

sense strand: 5'- AmsCmsAmGmUfAmUfUfCfUmCmAmGmUmGmCmUmCmAm -3'(SEQ ID NO: 367)

antisense strand: 5'-P1-UmsGfsAmGmCmAfCmUmGmAmGmAmAmUfAmCfUmGmUms CmsCm -3' (SEQ ID NO: 368)

siAPb2U-M2SP1

sense strand: 5'- GmsGmsAmCmAmGmUfAmUfUfCfUmCmAmGmUmGmCmUmCmAm -3'(SEQ ID NO: 369)

antisense strand: 5'-P1-UmsGfsAmGmCmAfCmUmGmAmGmAmAmUfAmCfUmGmUmCm CmsCmsUm -3' (SEQ ID NO: 370)

siAPb1U-M3SP1

sense strand: 5'-AmsCmsAmGmUmAmUfUfCfUmCmAmGmUmGmCmUmCmAm -3'(SEQ ID NO: 371)

antisense strand: 5'-P1-UmsGfsAmGmCmAfCmUmGmAmGmAmAmUfAmCfUmGmUms CmsCm -3' (SEQ ID NO: 372)

siAPb2U-M3SP1

sense strand: 5'- GmsGmsAmCmAmGmUmAmUfUfCfUmCmAmGmUmGmCmUmCmAm -3'(SEQ ID NO: 373)

antisense strand: 5'-P1-UmsGfsAmGmCmAfCmUmGmAmGmAmAmUfAmCfUmGmUmCm CmsCmsUm-3' (SEQ ID NO: 374)

siAPc1-M1P1

sense strand: 5'- UmAmUmUmCfUmCfAfGfUmGmCmUmCmUmCmCmUmAm -3'(SEQ ID NO: 229)

antisense strand: 5'-P1-UmAfGmGmAmGfAmGfCfAmCmUmGmAfGmAfAmUmAmCmUm -3' (SEQ ID NO: 230)

siAPc2-M1P1

sense strand: 5'- AmGmUmAmUmUmCfUmCfAfGfUmGmCmUmCmUmCmCmUmAm -3'(SEQ ID NO: 231)

antisense strand: 5'-P1-UmAfGmGmAmGfAmGfCfAmCmUmGmAfGmAfAmUmAmCm UmGmUm -3'(SEQ ID NO: 232)

siAPc1-M2P1
sense strand: 5'- UmAmUmUmCfUmCfAfGfUmGmCmUmCmUmCmCmUmAm -3'(SEQ ID NO: 233)

antisense strand: 5'-P1-UmAfGmGmAmGfAmGmCmAmCmUmGmAfGmAfAmUmAm CmUm -3' (SEQ ID NO: 234)

siAPc2-M2P1

sense strand: 5'- AmGmUmAmUmUmCfUmCfAfGfUmGmCmUmCmUmCmCmUmAm -3'(SEQ ID NO: 235)

antisense strand: 5'-P1-UmAfGmGmAmGfAmGmCmAmCmUmGmAfGmAfAmUmAmCm UmGmUm -3'(SEQ ID NO: 236)

siAPc1-M3P1
sense strand: 5'-UmAmUmUmCmUmCfAfGfUmGmCmUmCmUmCmCmUmAm-3'(SEQ ID NO: 237)

antisense strand: 5'-P1-UmAfGmGmAmGfAmGmCmAmCmUmGmAfGmAfAmUmAmCm Um -3' (SEQ ID NO: 238)

siAPc2-M3P1

sense strand: 5'- AmGmUmAmUmUmCmUmCfAfGfUmGmCmUmCmUmCmCmUmAm -3'(SEQ ID NO: 239)

antisense strand: 5'-P1-UmAfGmGmAmGfAmGmCmAmCmUmGmAfGmAfAmUmAmCm UmGmUm -3'(SEQ ID NO: 240)

siAPc1-M1SP1
sense strand: 5'-UmsAmsUmUmCfUmCfAfGfUmGmCmUmCmUmCmCmUmAm-3'(SEQ ID NO: 241)

antisense strand: 5'-P1-UmsAfsGmGmAmGfAmGfCfAmCmUmGmAfGmAfAmUmAms CmsUm -3' (SEQ ID NO: 242)

siAPc2-M1SP1

sense strand: 5'- AmsGmsUmAmUmUmCfUmCfAfGfUmGmCmUmCmUmCmCmUmAm -3'(SEQ ID NO: 243)

antisense strand: 5'-P1-UmsAfsGmGmAmGfAmGfCfAmCmUmGmAfGmAfAmUmAmCm UmsGmsUm -3'(SEQ ID NO: 244)

siAPc1-M2SP1
sense strand: 5'-UmsAmsUmUmCfUmCfAfGfUmGmCmUmCmUmCmCmUmAm-3'(SEQ ID NO: 245)

antisense strand: 5'-P1-UmsAfsGmGmAmGfAmGmCmAmCmUmGmAfGmAfAmUmAms CmsUm -3' (SEQ ID NO: 246)

siAPc2-M2SP1

sense strand: 5'- AmsGmsUmAmUmUmCfUmCfAfGfUmGmCmUmCmUmCmCmUmAm -3'(SEQ ID NO: 247)

antisense strand: 5'-P1-UmsAfsGmGmAmGfAmGmCmAmCmUmGmAfGmAfAmUmAm CmUmsGmsUm -3'(SEQ ID NO: 248)

siAPc1-M3SP1
sense strand: 5'-UmsAmsUmUmCmUmCfAfGfUmGmCmUmCmUmCmCmUmAm-3'(SEQ ID NO: 249)

antisense strand: 5'-P1-UmsAfsGmGmAmGfAmGmCmAmCmUmGmAfGmAfAmUmAm sCmsUm -3' (SEQ ID NO: 250)

siAPc2-M3SP1

sense strand: 5'- AmsGmsUmAmUmUmCmUmCfAfGfUmGmCmUmCmUmCmCmUmAm -3'(SEQ ID NO: 251)

antisense strand: 5'- P1-UmsAfsGmGmAmGfAmGmCmAmCmUmGmAfGmAfAmUmAm CmUmsGmsUm -3'(SEQ ID NO: 252)

siAPd1-M1P1
sense strand: 5'-AmGmUmAmUfUmCfUfCfAmGmUmGmCmUmCmUmCmCm-3'(SEQ ID NO: 253)

antisense strand: 5'-P1-GmGfAmGmAmGfCmAfCfUmGmAmGmAfAmUfAmCmUmGmUm -3'(SEQ ID NO: 254)

siAPd2-M1P1

sense strand: 5'- AmCmAmGmUmAmUfUmCfUfCfAmGmUmGmCmUmCmUmCmCm -3'(SEQ ID NO: 255)

antisense strand: 5'-P1-GmGfAmGmAmGfCmAfCfUmGmAmGmAfAmUfAmCmUmGm UmCmCm -3'(SEQ ID NO: 256)

siAPd1-M2P1
sense strand: 5'-AmGmUmAmUfUmCfUfCfAmGmUmGmCmUmCmUmCmCm-3'(SEQ ID NO: 257)

antisense strand: 5'-P1-GmGfAmGmAmGfCmAmCmUmGmAmGmAfAmUfAmCm UmGmUm -3'(SEQ ID NO: 258)

siAPd2-M2P1

sense strand: 5'- AmCmAmGmUmAmUfUmCfUfCfAmGmUmGmCmUmCmUmCmCm -3'(SEQ ID NO: 259)

antisense strand: 5'-P1- GmGfAmGmAmGfCmAmCmUmGmAmGmAfAmUfAmCmUm GmUmCmCm -3'(SEQ ID NO: 260)

siAPd1-M3P1
sense strand: 5'-AmGmUmAmUmUmCfUfCfAmGmUmGmCmUmCmUmCmCm-3'(SEQ ID NO: 261)

antisense strand: 5'-P1- GmGfAmGmAmGfCmAmCmUmGmAmGmAfAmUfAmCmUm GmUm -3'(SEQ ID NO: 262)

siAPd2-M3P1

sense strand: 5'- AmCmAmGmUmAmUmUmCfUfCfAmGmUmGmCmUmCmUmCmCm -3'(SEQ ID NO: 263)

antisense strand: 5'- P1-GmGfAmGmAmGfCmAmCmUmGmAmGmAfAmUfAmCmUm GmUmCmCm -3'(SEQ ID NO: 264)

siAPd1-M1SP1
sense strand: 5'- AmsGmsUmAmUfUmCfUfCfAmGmUmGmCmUmCmUmCmCm -3'(SEQ ID NO: 265)

antisense strand: 5'-P1-GmsGfsAmGmAmGfCmAfCfUmGmAmGmAfAmUfAmCmUms GmsUm -3'(SEQ ID NO: 266)

siAPd2-M1SP1

sense strand: 5'- AmsCmsAmGmUmAmUfUmCfUfCfAmGmUmGmCmUmCmUmCmCm -3'(SEQ ID NO: 267)

antisense strand: 5'-P1-GmsGfsAmGmAmGfCmAfCfUmGmAmGmAfAmUfAmCmUm GmUmsCmsCm -3'(SEQ ID NO: 268)

siAPd1-M2SP1
sense strand: 5'-AmsGmsUmAmUfUmCfUfCfAmGmUmGmCmUmCmUmCmCm-3'(SEQ ID NO: 269)

antisense strand: 5'-P1-GmsGfsAmGmAmGfCmAmCmUmGmAmGmAfAmUfAmCmUm sGmsUm-3'(SEQ ID NO: 270)

siAPd2-M2SP1

sense strand: 5'- AmsCmsAmGmUmAmUfUmCfUfCfAmGmUmGmCmUmCmUmsCmsCm -3'(SEQ ID NO: 271)

antisense strand: 5'-P1-GmsGfsAmGmAmGfCmAmCmUmGmAmGmAfAmUfAmCmUmGmUmsCmsCm -3'(SEQ ID NO: 272)

siAPd1-M3SP1

sense strand: 5'- AmsCmsAmGmUmAmUmUmCfUfCfAmGmUmGmCmUmCmUmCmCm -3'(SEQ ID NO: 273)

antisense strand: 5'-P1-GmsGfsAmGmAmGfCmAmCmUmGmAmGmAfAmUfAmCmUmsGmsUm -3'(SEQ ID NO: 274)

siAPd2-M3SP1

sense strand: 5'- AmsCmsAmGmUmAmUmUmCfUfCfAmGmUmGmCmUmCmUmCmCm -3'(SEQ ID NO: 275)

antisense strand: 5'-P1-GmsGfsAmGmAmGfCmAmCmUmGmAmGmAfAmUfAmCmUmGmUmsCmsCm -3'(SEQ ID NO: 276)

siAPd1U-M1P1

sense strand: 5'-AmGmUmAmUfUmCfUfCfAmGmUmGmCmUmCmUmCmAm-3'(SEQ ID NO: 375)

antisense strand: 5'- P1-UmGfAmGmAmGfCmAfCfUmGmAmGmAfAmUfAmCmUmGmUm -3'(SEQ ID NO: 376)

siAPd2U-M1P1

sense strand: 5'- AmCmAmGmUmAmUfUmCfUfCfAmGmUmGmCmUmCmUmCmAm -3'(SEQ ID NO: 377)

antisense strand: 5'- P1-UmGfAmGmAmGfCmAfCfUmGmAmGmAfAmUfAmCmUmGmUmCmCm -3'(SEQ ID NO: 378)

siAPd1U-M2P1

sense strand: 5'-AmGmUmAmUfUmCfUfCfAmGmUmGmCmUmCmUmCmAm-3'(SEQ ID NO: 379);

antisense strand: 5'- P1-UmGfAmGmAmGfCmAmCmUmGmAmGmAfAmUfAmCmUmGmUm -3'(SEQ ID NO: 380)

siAPd2U-M2P1

sense strand: 5'- AmCmAmGmUmAmUfUmCfUfCfAmGmUmGmCmUmCmUmCmAm -3'(SEQ ID NO: 381)

antisense strand: 5'- P1-UmGfAmGmAmGfCmAmCmUmGmAmGmAfAmUfAmCmUmGmUmCmCm -3'(SEQ ID NO: 382)

siAPd1U-M3P1

sense strand: 5'-AmGmUmAmUmUmCfUfCfAmGmUmGmCmUmCmUmCmAm-3'(SEQ ID NO: 383)

antisense strand: 5'- P1-UmGfAmGmAmGfCmAmCmUmGmAmGmAfAmUfAmCmUm GmUm -3'(SEQ ID NO: 384)

siAPd2U-M3P1

sense strand: 5'- AmCmAmGmUmAmUmUmCfUfCfAmGmUmGmCmUmCmUmCmAm -3'(SEQ ID NO: 385)

antisense strand: 5'- P1-UmGfAmGmAmGfCmAmCmUmGmAmGmAfAmUfAmCmUmGm UmCmCm -3'(SEQ ID NO: 386)

siAPd1U-M1SP1

sense strand: 5'- AmsGmsUmAmUfUmCfUfCfAmGmUmGmCmUmCmUmCmAm -3'(SEQ ID NO: 387)

antisense strand: 5'- P1-UmsGfsAmGmAmGfCmAfCfUmGmAmGmAfAmUfAmCmUms GmsUm -3'(SEQ ID NO: 388)

siAPd2U-M1SP1

sense strand: 5'- AmsCmsAmGmUmAmUfUmCfUfCfAmGmUmGmCmUmCmUmCmAm -3'(SEQ ID NO: 389)

antisense strand: 5'- P1-UmsGfsAmGmAmGfCmAfCfUmGmAmGmAfAmUfAmCmUmGm UmsCmsCm -3'(SEQ ID NO: 390)

siAPd1U-M2SP1

sense strand: 5'- AmsGmsUmAmUfUmCfUfCfAmGmUmGmCmUmCmUmCmAm -3'(SEQ ID NO: 391)

antisense strand: 5'- P1-UmsGfsAmGmAmGfCmAmCmUmGmAmGmAfAmUfAmCmUms GmsUm -3'(SEQ ID NO: 392)

siAPd2U-M2SP1

sense strand: 5'- AmsCmsAmGmUmAmUfUmCfUfCfAmGmUmGmCmUmCmUmCmAm -3'(SEQ ID NO: 393)

antisense strand: 5'- P1-UmsGfsAmGmAmGfCmAmCmUmGmAmGmAfAmUfAmCmUm GmUmsCmsCm -3'(SEQ ID NO: 394)

siAPd1U-M3SP1

sense strand: 5'- AmsGmsUmAmUmUmCfUfCfAmGmUmGmCmUmCmUmCmAm -3'(SEQ ID NO: 395)

antisense strand: 5'- P1-UmsGfsAmGmAmGfCmAmCmUmGmAmGmAfAmUfAmCmUms GmsUm -3'(SEQ ID NO: 396)

siAPd2U-M3SP1

sense strand: 5'- AmsCmsAmGmUmAmUmUmCfUfCfAmGmUmGmCmUmCmUmCmAm -3'(SEQ ID NO: 397)

antisense strand: 5'- P1-UmsGfsAmGmAmGfCmAmCmUmGmAmGmAfAmUfAmCmUm GmUmsCmsCm -3'(SEQ ID NO: 398)

siAPe1-M1P1
sense strand: 5'- GmGmAmCmAfGmUfAfUfUmCmUmCmAmGmUmGmCmUm -3'(SEQ ID NO: 277)

antisense strand: 5'-P1- AmGfCmAmCmUfGmAfGfAmAmUmAmCfUmGfUmCmCmCmUm -3'(SEQ ID NO: 278)

siAPe2-M1P1

sense strand: 5'- AmGmGmGmAmCmAfGmUfAfUfUmCmUmCmAmGmUmGmCmUm -3'(SEQ ID NO: 279)

antisense strand: 5'-P1-AmGfCmAmCmUfGmAfGfAmAmUmAmCfUmGfUmCmCmCm UmUmUm -3'(SEQ ID NO: 280)

siAPe1-M2P1
sense strand: 5'- GmGmAmCmAfGmUfAfUfUmCmUmCmAmGmUmGmCmUm -3'(SEQ ID NO: 281)

antisense strand: 5'-P1-AmGfCmAmCmUfGmAmGmAmAmUmAmCfUmGfUmCmCm CmUm -3'(SEQ ID NO: 282)

siAPe2-M2P1

sense strand: 5'- AmGmGmGmAmCmAfGmUfAfUfUmCmUmCmAmGmUmGmCmUm -3'(SEQ ID NO: 283)

antisense strand: 5'-P1-AmGfCmAmCmUfGmAmGmAmAmUmAmCfUmGfUmCmCm CmUmUmUm -3'(SEQ ID NO: 284)

siAPe1-M3P1
sense strand: 5'- GmGmAmCmAmGmUfAfUfUmCmUmCmAmGmUmGmCmUm -3'(SEQ ID NO: 285)

antisense strand: 5'-P1- AmGfCmAmCmUfGmAmGmAmAmUmAmCfUmGfUmCmCm CmUm -3'(SEQ ID NO: 286)

siAPe2-M3P1

sense strand: 5'- AmGmGmGmAmCmAmGmUfAfUfUmCmUmCmAmGmUmGmCmUm -3'(SEQ ID NO: 287);

antisense strand: 5'-P1-AmGfCmAmCmUfGmAmGmAmAmUmAmCfUmGfUmCmCmCm UmUmUm -3'(SEQ ID NO: 288)

siAPe1-M1SP1
sense strand: 5'- GmsGmsAmCmAfGmUfAfUfUmCmUmCmAmGmUmGmCmUm -3'(SEQ ID NO: 289)

antisense strand: 5'-P1-AmsGfsCmAmCmUfGmAfGfAmAmUmAmCfUmGfUmCmCms CmsUm -3'(SEQ ID NO: 290)

siAPe2-M1SP1

sense strand: 5'- AmsGmsGmGmAmCmAfGmUfAfUfUmCmUmCmAmGmUmGmCmUm -3'(SEQ ID NO: 291)

antisense strand: 5'-P1-AmsGfsCmAmCmUfGmAfGfAmAmUmAmCfUmGfUmCmCmCm UmsUmsUm -3'(SEQ ID NO: 292)

siAPe1-M2SP1
sense strand: 5'- GmsGmsAmCmAfGmUfAfUfUmCmUmCmAmGmUmGmCmUm -3'(SEQ ID NO: 293)

antisense strand: 5'-P1-AmsGfsCmAmCmUfGmAmGmAmAmUmAmCfUmGfUmCmCms CmsUm -3'(SEQ ID NO: 294)

siAPe2-M2SP1

sense strand: 5'- AmsGmsGmGmAmCmAfGmUfAfUfUmCmUmCmAmGmUmGmCmUm -3'(SEQ ID NO: 295);

antisense strand: 5'-P1-AmsGfsCmAmCmUfGmAmGmAmAmUmAmCfUmGfUmCmCmCm UmsUmsUm -3'(SEQ ID NO: 296)

siAPe1-M3SP1
sense strand: 5'- GmsGmsAmCmAmGmUfAfUfUmCmUmCmAmGmUmGmCmUm -3'(SEQ ID NO: 297);

antisense strand: 5'-P1-AmsGfsCmAmCmUfGmAmGmAmAmUmAmCfUmGfUmCmCms CmsUm -3'(SEQ ID NO: 298)

siAPe2-M3SP1

sense strand: 5'- AmsGmsGmGmAmCmAmGmUfAfUfUmCmUmCmAmGmUmGmCmUm -3'(SEQ ID NO: 299)

antisense strand: 5'-P1-AmsGfsCmAmCmUfGmAmGmAmAmUmAmCfUmGfUmCmCmCm UmsUmsUm -3'(SEQ ID NO: 300)

siAPe1U-M1P1
sense strand: 5'- GmGmAmCmAfGmUfAfUfUmCmUmCmAmGmUmGmCmAm -3'(SEQ ID NO: 399)

antisense strand: 5'- P1-UmGfCmAmCmUfGmAfGfAmAmUmAmCfUmGfUmCmCmCmUm -3'(SEQ ID NO: 400)

siAPe2U-M1P1

sense strand: 5'- AmGmGmGmAmCmAfGmUfAfUfUmCmUmCmAmGmUmGmCmAm -3'(SEQ ID NO: 401)

antisense strand: 5'- P1-UmGfCmAmCmUfGmAfGfAmAmUmAmCfUmGfUmCmCmCm UmUmUm -3'(SEQ ID NO: 402)

siAPe1U-M2P1
sense strand: 5'- GmGmAmCmAfGmUfAfUfUmCmUmCmAmGmUmGmCmAm -3'(SEQ ID NO: 403)

antisense strand: 5'- P1-UmGfCmAmCmUfGmAmGmAmAmUmAmCfUmGfUmCmCm CmUm -3'(SEQ ID NO: 404)

siAPe2U-M2P1

sense strand: 5'- AmGmGmGmAmCmAfGmUfAfUfUmCmUmCmAmGmUmGmCmAm -3'(SEQ ID NO: 405)

antisense strand: 5'- P1-UmGfCmAmCmUfGmAmGmAmAmUmAmCfUmGfUmCmCmCm UmUmUm -3'(SEQ ID NO: 406)

siAPe1U-M3P1
sense strand: 5'- GmGmAmCmAmGmUfAfUfUmCmUmCmAmGmUmGmCmAm -3'(SEQ ID NO: 407)

antisense strand: 5'- P1-UmGfCmAmCmUfGmAmGmAmAmUmAmCfUmGfUmCmCm CmUm -3'(SEQ ID NO: 408)

siAPe2U-M3P1

sense strand: 5'- AmGmGmGmAmCmAmGmUfAfUfUmCmUmCmAmGmUmGmCmAm -3'(SEQ ID NO: 409);

antisense strand: 5'- P1-UmGfCmAmCmUfGmAmGmAmAmUmAmCfUmGfUmCmCmCm UmUmUm -3'(SEQ ID NO: 410)

siAPe1U-M1SP1
sense strand: 5'- GmsGmsAmCmAfGmUfAfUfUmCmUmCmAmGmUmGmCmAm -3'(SEQ ID NO: 411)

antisense strand: 5'- P1-UmsGfsCmAmCmUfGmAfGfAmAmUmAmCfUmGfUmCmCms CmsUm -3'(SEQ ID NO: 412)

siAPe2U-M1SP1

sense strand: 5'- AmsGmsGmGmAmCmAfGmUfAfUfUmCmUmCmAmGmUmGmCmAm -3'(SEQ ID NO: 413)

antisense strand: 5'- P1-UmsGfsCmAmCmUfGmAfGfAmAmUmAmCfUmGfUmCmCmCm UmsUmsUm -3'(SEQ ID NO: 414)

siAPe1U-M2SP1
sense strand: 5'- GmsGmsAmCmAfGmUfAfUfUmCmUmCmAmGmUmGmCmAm -3'(SEQ ID NO: 415)

antisense strand: 5'- P1-UmsGfsCmAmCmUfGmAmGmAmAmUmAmCfUmGfUmCmCms CmsUm -3'(SEQ ID NO: 416)

siAPe2U-M2SP1

sense strand: 5'- AmsGmsGmGmAmCmAfGmUfAfUfUmCmUmCmAmGmUmGmCmAm -3'(SEQ ID NO: 417)

antisense strand: 5'- P1-UmsGfsCmAmCmUfGmAmGmAmAmUmAmCfUmGfUmCmCm CmUmsUmsUm -3'(SEQ ID NO: 418)

siAPe1U-M3SP1
sense strand: 5'- GmsGmsAmCmAmGmUfAfUfUmCmUmCmAmGmUmGmCmAm -3'(SEQ ID NO: 419)

antisense strand: 5'- P1-UmsGfsCmAmCmUfGmAmGmAmAmUmAmCfUmGfUmCmCms CmsUm -3'(SEQ ID NO: 420)

siAPe2U-M3SP1

sense strand: 5'- AmsGmsGmGmAmCmAmGmUfAfUfUmCmUmCmAmGmUmGmCmAm -3'(SEQ ID NO: 421)

antisense strand: 5'- P1-UmsGfsCmAmCmUfGmAmGmAmAmUmAmCfUmGfUmCmCm CmUmsUmsUm -3'(SEQ ID NO: 422)

In some embodiments, the siRNA may be one selected from siAPa1UM3SVP, siAPe1UM3SVP, siAPb1UM3SVP, siAPd1UM3SVP, siAPc1M3SVP, siAPd1UM3SP, siAPd1UM3SPs, siAPa1M3SP, siAPe1M3SP, siAPb1M3SP, and siAPc1M3SP, as shown in Table 7.

In the above siRNAs of the present disclosure, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; P1 represents that the nucleotide adjacent to the right side of PI is a 5'-phosphate nucleotide or a 5'-phosphate analog modified nucleotide. In some embodiments, PI represents the specific modifed nucleotide VP, Ps or P, wherein VP represents that the nucleotide adjacent to the right side of VP is a vinyl phosphate (5' -(E)-vinylphosphonate, E-VP) modified nucleotide; Ps represents that the nucleotide adjacent to the right side of Ps is a thiophosphate modified nucleotide; and P represents that the nucleotide adjacent to the right side of the letter P is a 5'-phosphate nucleotide.

The inventors of the present disclosure surprisingly found that the siRNAs of the present disclosure have significantly enhanced plasma and lysosomal stability, while maintaining high gene-suppressing activity.

The siRNAs of the present disclosure can be obtained by conventional methods for preparing siRNAs in the art, e.g., solid phase synthesis method and liquid phase synthesis method. Commercial customization services have

already been available for solid phase synthesis. A modified nucleotide group can be introduced into the siRNA of the present disclosure by using a nucleotide monomer having the corresponding modification. The method for preparing a nucleotide monomer having the corresponding modification and the method for introducing a modified nucleotide group into a siRNA are also well known to those skilled in the art.

## Pharmaceutical Composition

The present disclosure provides a pharmaceutical composition, comprising the above siRNA as an active ingredient and a pharmaceutically acceptable carrier.

The pharmaceutically acceptable carrier may be a conventional carrier in the field of siRNA administration, for example, but not limited to, one or more of magnetic nanoparticles (such as $Fe_3O_4$ and $Fe_2O_3$-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, in the pharmaceutical composition, there are no special requirements for the contents of the siRNA and the pharmaceutically acceptable carrier. In some embodiments, the weight ratio of the siRNA to the pharmaceutically acceptable carrier is 1: (1-500), and in some embodiments, the weight ratio is 1: (1-50).

In some embodiments, the pharmaceutical composition may also contain other pharmaceutically acceptable excipients, which may be one or more of various conventional formulations or compounds in the art. For example, said other pharmaceutically acceptable excipients may comprise at least one of a pH buffer, a protection agent and an osmotic pressure regulator.

The pH buffer may be a tris(hydroxymethyl) aminomethane hydrochloride buffer solution with a pH of 7.5-8.5, and/or a phosphate buffer solution with a pH of 5.5-8.5, such as a phosphate buffer solution with a pH of 5.5-8.5.

The protection agent may be at least one of inositol, sorbitol, sucrose, trehalose, mannose, maltose, lactose, and glucose. The content of the protection agent may be from 0.01 wt % to 30 wt % based on the total weight of the pharmaceutical composition.

The osmotic pressure regulator may be sodium chloride and/or potassium chloride. The content of the osmotic pressure regulator renders the osmotic pressure of the pharmaceutical composition to be 200-700 milliosmol/kg. Depending on the desired osmotic pressure, those skilled in the art can readily determine the content of the osmotic pressure regulator.

In some embodiments, the pharmaceutical composition may be a liquid formulation, for example, an injection solution; or a lyophilized powder for injection, which will be mixed with a liquid excipient to form a liquid formulation upon administration. The liquid formulation may be administered by, but not limited to, subcutaneous, intramuscular or intravenous injection, and also may be administered to, but not limited to, lung by spray, or other organs (such as liver) via lung by spray. In some embodiments, the pharmaceutical composition is administered by intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a PEGylated lipid. Therein, the organic amine, the helper lipid and the PEGylated lipid may be respectively one or more selected from the amine-containing transfection compounds or the pharmaceutically acceptable salts or derivatives thereof, the helper lipids and the PEGylated lipids as described in CN103380113A, which is incorporated herein by reference in its entirety.

In some embodiments, the organic amine may be a compound as shown by Formula (201) as described in CN103380113A or a pharmaceutically acceptable salt thereof:

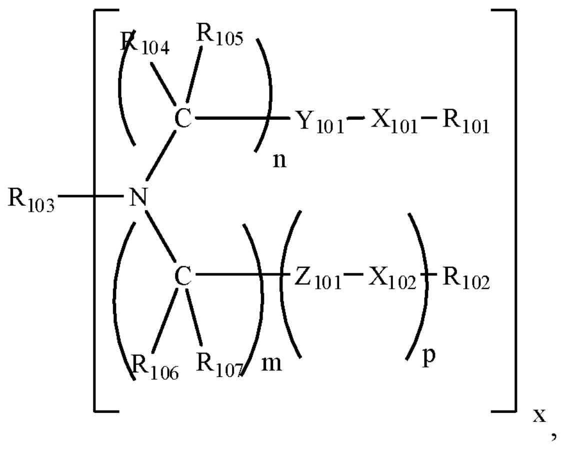

Formula (201)

wherein,

$X_{101}$ and $X_{102}$ independently of one another are selected from O, S, N-A and C-A, wherein A is hydrogen or a $C_1$-$C_{20}$ hydrocarbon chain;

$Y_{101}$ and $Z_{101}$ independently of one another are selected from C=O, C=S, S=O, CH-OH and $SO_2$;

$R_{101}$, $R_{102}$, $R_{103}$, $R_{104}$, $R_{105}$, $R_{106}$ and $R_{107}$ independently of one another are selected from hydrogen; a cyclic or an acyclic, substituted or unsubstituted, branched or linear aliphatic group; a cyclic or an acyclic, substituted or unsubstituted, branched or linear heteroaliphatic group; a substituted or unsubstituted, branched or linear acyl group; a substituted or unsubstituted, branched or linear aryl group; and a substituted or unsubstituted, branched or linear heteroaryl group;

x is an integer of 1-10;

n is an integer of 1-3, m is an integer of 0-20, p is 0 or 1, wherein if m=p=0, then $R_{102}$ is hydrogen; and

if at least one of n and m is 2, then $R_{103}$ and nitrogen in Formula (201) form a structure as shown by Formula (202) or (203):

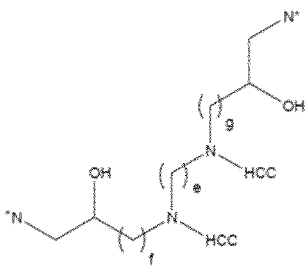

Formula (202)

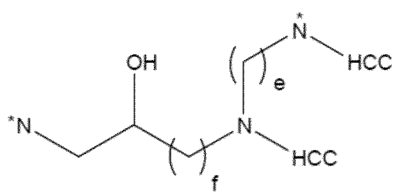

Formula (203)

wherein g, e and f independently of one another are an integer of 1-6; "HCC" represents a hydrocarbon chain, and each *N represents a nitrogen atom shown in Formula (201).

In some embodiments, $R_{103}$ is a polyamine. In other embodiments, $R_{103}$ is a ketal. In some embodiments, $R_{101}$ and $R_{102}$ in the Formula (201) independently of one another are any substituted or unsubstituted, branched or linear alkyl or alkenyl, wherein the alkyl or alkenyl has 3-20 carbon atoms (such as 8-18 carbon atoms) and 0-4 double bonds (such as 0-2 double bonds).

In some embodiments, if n and m independently of one another are 1 or 3, $R_{103}$ is any of the following Formulae (204)-(213):

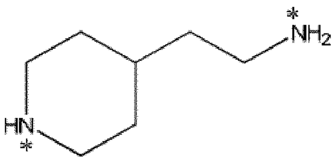,

Formula (204)

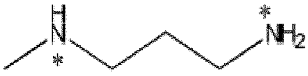,

Formula (205)

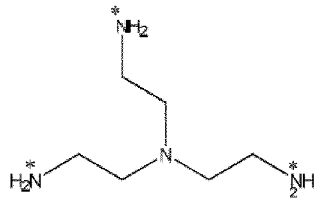,

Formula (206)

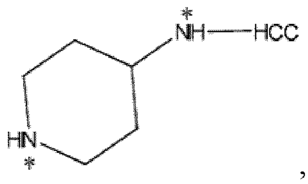,

Formula (207)

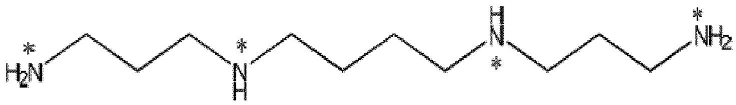,

Formula (208)

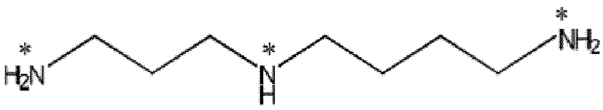,

Formula (209)

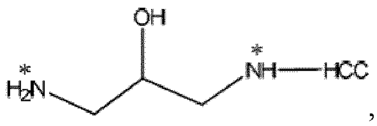,

Formula (210)

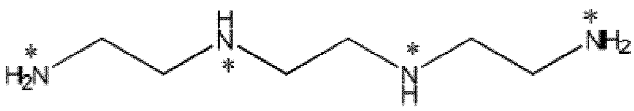,

Formula (211)

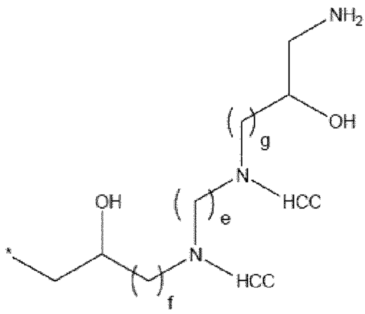, and

Formula (212)

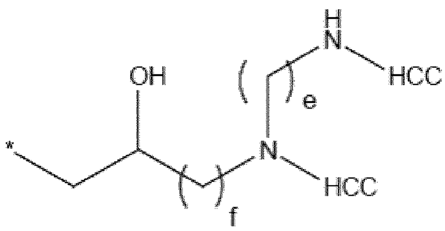

Formula (213)

wherein, in Formulae (204)-(213), g, e and f independently of one another are an integer of 1-6, each "HCC" represents a hydrocarbon chain, and each * represents a potential attachment point of $R_{103}$ to the nitrogen atom in Formula (201), wherein each H at any * position can be replaced to achieve the attachment to the nitrogen atom in Formula (201).

The compound as shown by Formula (201) may be prepared as described in CN103380113A.

In some embodiments, the organic amine is an organic amine as shown by Formula (214) and/or an organic amine as shown by Formula (215):

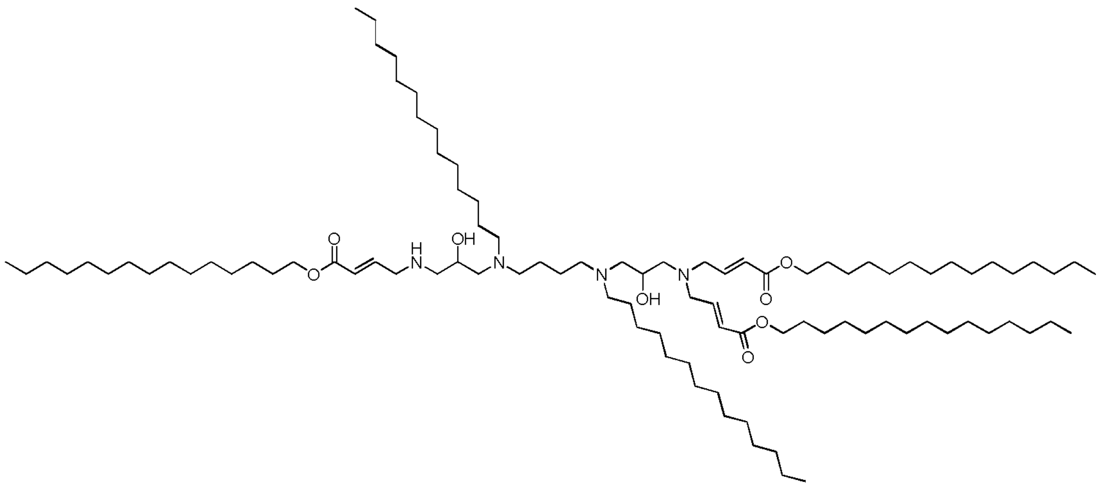

,

Formula (214)

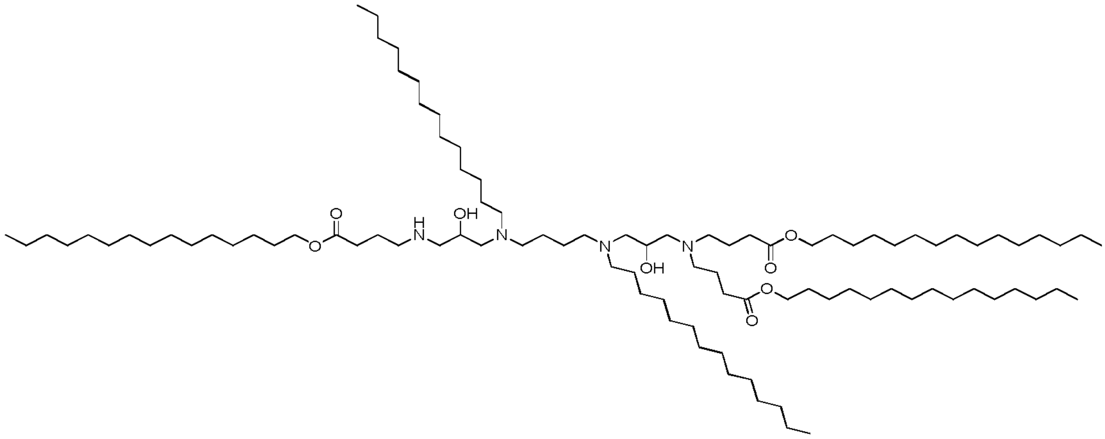

,

Formula (215)

The helper lipid is cholesterol, cholesterol analogs and/or cholesterol derivatives.

The PEGylated lipid is 1,2-dipalmitoyl-sn-glycero-3-phosphatidylethanolamine-N-[methoxy(polyethylene glycol)]-2000.

In some embodiments, the molar ratio among the organic amine, the helper lipid, and the PEGylated lipid in the pharmaceutical composition is (19.7-80): (19.7-80): (0.3-50), for example, the molar ratio may be (50-70): (20-40): (3-20).

In some embodiments, the pharmaceutical composition particles formed by the siRNA of the present disclosure and the above amine-containing transfection reagents have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm; for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and the above amine-containing transfection reagents, the weight ratio (weight/weight ratio) of the siRNA to total lipids, e.g., the organic amines, the helper lipids and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the siRNA of the present disclosure to total lipids is about 1:5, 1:6, 1:7, 1:8, 1:9, 1:10, 1:11, 1:12, 1:13, 1:14, 1:15, 1:16, 1:17, or 1:18.

In some embodiments, the pharmaceutical composition may be marketed with each component being separate, and used in the form of a liquid formulation. In some embodiments, the pharmaceutical composition formed by the siRNA of the present disclosure and the above pharmaceutically acceptable carrier may be prepared by various known processes, except for replacing the existing siRNA with the siRNA of the present disclosure. In some specific embodiments, the pharmaceutical composition may be prepared according to the following process:

The organic amines, helper lipids and PEGylated lipids are suspended in alcohol at a molar ratio as described above and mixed homogeneously to yield a lipid solution; the alcohol is used in an amount such that the resultant lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, PEG 200, PEG 300, and PEG 400, such as ethanol.

The siRNA of the present disclosure is dissolved in a buffered salt solution to produce an aqueous solution of the siRNA. The buffered salt solution has a concentration of 0.05 to 0.5 M, such as 0.1 to 0.2 M. The pH of the buffered salt solution is adjusted to 4.0 to 5.5, such as 5.0 to 5.2. The buffered salt solution is used in an amount such that the siRNA is present at a concentration of less than 0.6 mg/ml, such as 0.2 to 0.4 mg/mL. The buffered salt may be one or more selected from the group consisting of soluble acetate and soluble citrate, such as sodium acetate and/or potassium acetate.

The lipid solution and the aqueous solution of the siRNA are mixed. The product obtained by mixing is incubated at a temperature of 40 to 60°C for at least 2 minutes (e.g., 5 to 30 minutes) to produce an incubated lipid formulation. The volume ratio of the lipid solution to the aqueous solution of the siRNA is 1: (2-5).

The incubated lipid formulation is concentrated or diluted, purified to remove impurities, and then sterilized to afford the pharmaceutical composition of the present disclosure, which has the following physicochemical parameters: a pH of 6.5 to 8, an encapsulation percentage of more than 80%, a particle size of 40 to 200 nm, a polydispersity index of less than 0.30, and an osmotic pressure of 250 to 400 mOsm/kg. For example, the physicochemical parameters may be as follows: a pH of 7.2 to 7.6, an encapsulation percentage of more than 90%, a particle size of 60 to 100 nm, a polydispersity index of less than 0.20, and an osmotic pressure of 300 to 400 mOsm/kg.

Therein, the concentration or dilution step may be performed before, after or simultaneously with removal of the impurities. The method for removing impurities may be any of various existing methods, for example, ultrafiltration with 100 kDa hollow fiber column and PBS at pH 7.4 as ultrafiltration exchange solution using tangential flow system. The method for sterilization may be any of various existing methods, such as filtration sterilization on a 0.22 μm filter.

## siRNA conjugate

The present disclosure provides a siRNA conjugate comprising the above siRNA and a conjugation group conjugatively linked to the siRNA.

Generally speaking, the conjugation group comprises at least one pharmaceutically acceptable targeting group and an optional linker. Moreover, the siRNA, the linker and the targeting group are sequentialy linked. In some embodiments, the nubmer of the targeting groups is 1 to 6. In some embodiments, the number of traget groups is 2 to 4. The siRNA molecule may be noncovalently or covalently conjugated to the conjugation group, for example the siRNA molecule may be covalently conjugated to the conjugation group. The conjugation site between the siRNA and the conjugation group can be at 3' terminal or 5' terminal of the sense strand of the siRNA, or at 5' terminal of the antisense strand of the siRNA, and can be within the internal sequence of the siRNA. In some embodiments, the conjugation site between the siRNA and the conjugation group is at 3' terminal of the sense strand of the siRNA.

In some embodiments, the conjugation group may be linked to the phosphate group, the 2'-hydroxy or the base of a nucleotide. The conjugation group may also be linked to the 3'-hydroxy group when the nucleotides are linked via a 2'-5'-phosphodiester bond. When the conjugation group is linked to a terminal of the siRNA, the conjugation group is typically linked to the phosphate group of a nucleotide; when the conjugation group is linked to an internal sequence of the siRNA, the conjugation group is typically linked to a ribose ring or a base. For variou linking modes, reference may be made to: Muthiah Manoharan et.al. siRNA conjugates carrying sequentially assembled trivalent N-acetylgalactosamine linked through nucleosides elicit robust gene silencing in vivo in hepatocytes. ACS Chemical biology, 2015, 10(5): 1181-7.

In some embodiments, the siRNA and the conjugation group can be linked by an acid-labile or reducible chemical bond, and these chemical bonds can be degraded under the acidic environment of cell endosomes, thereby making the siRNA be in free state. For non-degradable conjugation modes, the conjugation group can be linked to the sense strand of the siRNA, thereby minimizing the effect of conjugation on the activity of the siRNA.

In some embodiments, the pharmaceutically acceptable targeting group may be a conventional ligand in the field of siRNA administration, for example, various ligands as described in WO2009082607A2, which is incorporated herein by reference in its entirety.

In some embodiments, the pharmaceutically acceptable targeting group may be one or more selected from the ligands fromed by the following targeting molecules or derivatives thereof: lipophilic molecules, such as cholesterol, bile acids, vitamins (such as vitamin E), lipid molecules with different chain lengths; polymers, such as polyethylene glycol; polypeptides, such as cell-penetrating peptide; aptamers; antibodies; quantum dots; saccharides, such as lactose, polylactose, mannose, galactose, N-acetylgalactosamine (GalNAc); folate; or receptor ligands expressed in hepatic parenchymal cells, such as asialoglycoprotein, asialo-sugar residue, lipoproteins (such as high density lipoprotein, low density lipoprotein and the like), glucagon, neurotransmitters (such as adrenaline), growth factors, transferrin and the like.

In some embodiments, each ligand is independently selected from a ligand capable of binding to a cell surface receptor. In some embodiments, at least one ligand is a ligand capable of binding to a surface receptor of a hepatocyte. In some embodiments, at least one ligand is a ligand capable of binding to a surface receptor of a mammalian hepatocyte. In some embodiments, at least one ligand is a ligand capable of binding to a surface receptor of a human hepatocyte. In some embodiments, at least one ligand is a ligand capable of binding to an asialoglycoprotein receptor (ASGPR) on the surface of hepatocytes. The types of these ligands are well-known to those skilled in the art and they typically serve the function of binding to specific receptor on the surface of the target cell, thereby mediating delivery of the siRNA linked to the ligand into the target cell.

In some embodiments, the pharmaceutically acceptable targeting group may be any ligand that binds to the asialoglycoprotein receptors on the surface of mammalian hepatocytes. In some embodiments, each ligand is independently an asialoglycoprotein, such as asialoorosomucoid (ASOR) or asialofetuin (ASF). In some embodiments, the ligand is a saccharide or its derivatives.

In some embodiments, at least one ligand is a saccharide. In some embodiments, each ligand is a saccharide. In some embodiments, at least one ligand is a monosaccharide, polysaccharide, modified monosaccharide, modified polysaccharide, or saccharide derivative. In some embodiments, at least one ligand may be a monosaccharide, disaccharide or trisaccharide. In some embodiments, at least one ligand is a modified saccharide. In some embodiments, each ligand is a modified saccharide. In some embodiments, each ligand is independently selected from a polysaccharide, modified polysaccharide, monosaccharide, modified monosaccharide, polysaccharide derivative, and monosaccharide derivative. In some embodiments, each ligand or at least one ligand may be independently selected from the group consisting of glucose and its derivatives, mannose and its derivatives, galactose and its derivatives, xylose and its derivatives, ribose and its derivatives, fucose and its derivatives, lactose and its derivatives, maltose and its derivatives, arabinose and its derivatives, fructose and its derivatives, and sialic acid.

In some embodiments, each ligand may be independently selected from the group consisting of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, L-4-thioribose. Other options of the ligand may be found, for example, in the disclosure of CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecules may be be mono-, bi-, tri-, or tetra-valent. It should be understood that the terms mono-, bi-, tri-, or tetra-valent described herein respectively mean that the molar ratio of the siRNA molecule to the galactose or N-acetylgalactosamine molecule in the siRNA conjugate is 1:1, 1:2, 1:3 or 1:4, wherein the siRNA conjugate is formed from the siRNA molecule and the conjugation group containing galactose or N-acetylgalactosamine molecule as the targeting group. In some embodiments, the pharmaceutically acceptable targeting group is N-acetylgalactosamine. In some embodiments, when the double-stranded oligonucleotide of the present disclosure is conjugated to a conjugation group comprising N-acetylgalactosamine, the N-acetylgalactosamine molecule is trivalent or tetravalent. In some embodiments, when the siRNA of the present disclosure is conjugated to a conjugation group containing N-acetylgalactosamine, the N-acetylgalactosamine molecule is trivalent.

The targeting group can be linked to the siRNA molecule via an appropriate linker, and the appropriate linker can be selected by those skilled in the art according to the specific type of the targeting group. The types of these linkers and targeting groups and the linking modes with the siRNA may be found in the disclosure of WO2015006740A2, which is incorporated herein by reference in its entirety.

In some embodiments, when the targeting group is N-acetylgalactosamine, a suitable linker may have the following structure as shown by Formula (301):

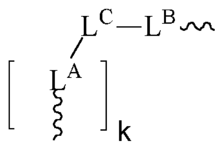

Formula (301)

wherein,

k is an integer of 1-3;

$L^A$ is an amide bond-comprising chain moiety that has a structure as shown by Formula (302), and two terminals of each $L^A$ are respectively linked to the targeting group and the $L^C$ moiety through an ether bond:

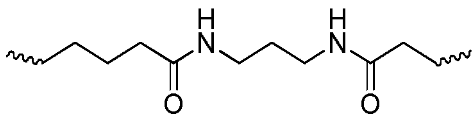

;

Formula (302)

$L^B$ is a N-acylpyrrolidine-comprising chain moiety that has a structure as shown by Formula (303), wherein the chain moity has a carbonyl group at one terminal and is linked to the $L^C$ moiety through an amide bond, and has an oxy group at the other terminal and is linked to the siRNA via a phosphoester bond:

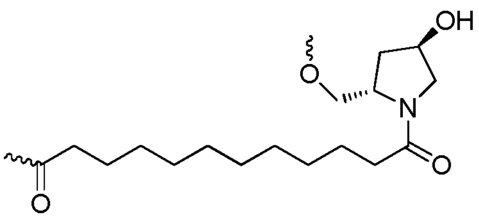

;

Formula (303)

$L^C$ is a bivalent to tetravalent linking group based on hydroxymethyl aminomethane, dihydroxymethyl aminomethane or trihydroxymethyl aminomethane, and $L^C$ is linked to $L^A$ moieties through an ether bond via an oxygen atom, and is linked to $L^B$ moiety through an amide bond via a nitrogen atom.

In some embodiments, when n=3 and $L^C$ is a tetravalent linking group based on trihydroxymethyl aminomethane, the siRNA conjugate formed by linking N-acetylgalactosamine molecules with a siRNA molecule via -$(L^A)_3$-trihydroxymethyl aminomethane-$L^B$- as a linker has a structure as shown by Formula (304):

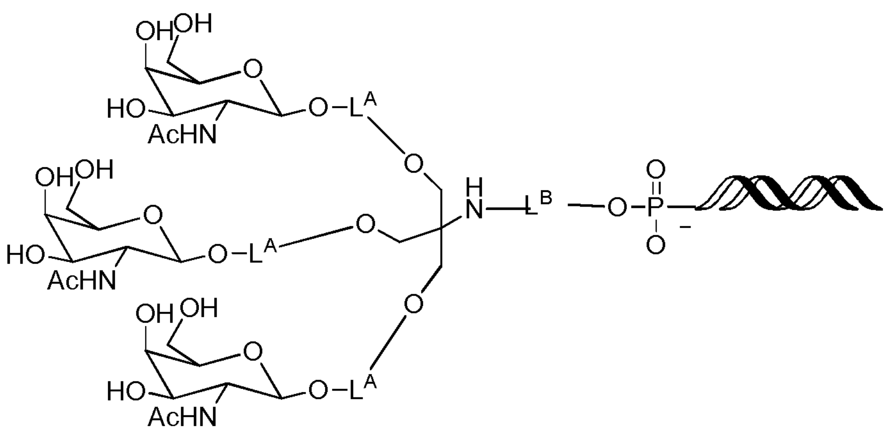

,

Formula (304)

wherein the double helix structure represents a siRNA.

Likewise, the conjugation site between the siRNA and the conjugation group can be at 3'-terminal or 5'-terminal of the sense strand of the siRNA, or at 5'-terminal of the antisense strand, or within the internal sequence of the siRNA.

In some embodiments, the 3'-terminal of the sense strand of the siRNA of the present disclosure is covalently conjugated to three N-acetylgalactosamine molecules via a linker - $(L^A)_3$-trihydroxymethyl aminomethane-$L^B$-, to afford a siRNA conjugate in which the molar ratio of the siRNA molecule to the GalNAc molecule is 1:3 (hereinafter also referred to as $(GalNAc)_3$-siRNA), and this conjugate has a structure as shown by Formula (305):

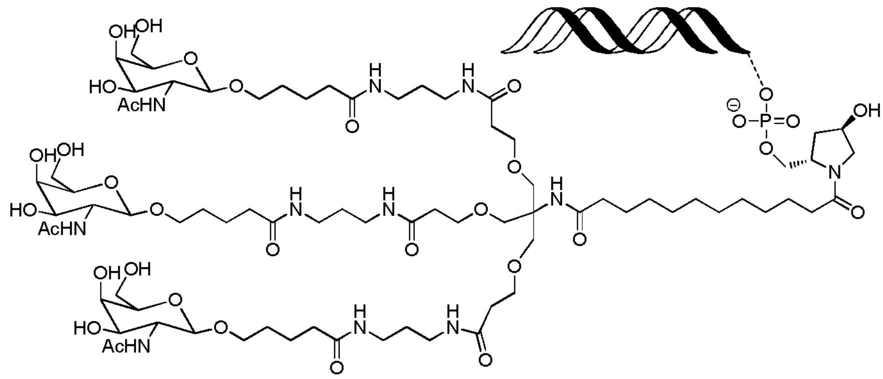

,

Formula (305)

wherein the double helix structure represents a siRNA; and the linker is linked to 3'-terminal of the sense strand of the siRNA.

In some embodiments, when the targeting group is N-acetylgalactosamine, a suitable linker may have a structure as shown by Formula (306):

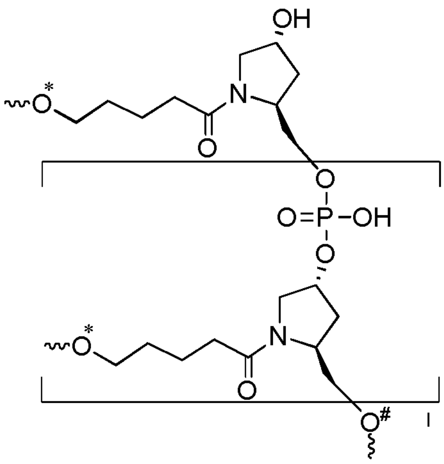

,

Formula (306)

wherein,

1 is an integer of 0 - 3;

* represents a site on the linker linked to the targeting group via an ether bond; and

# represents a site on the linker linked to the siRNA via a phosphoester bond.

In some specific embodiments, when 1=2, the siRNA conjugate has a structure as shown by Formula (307):

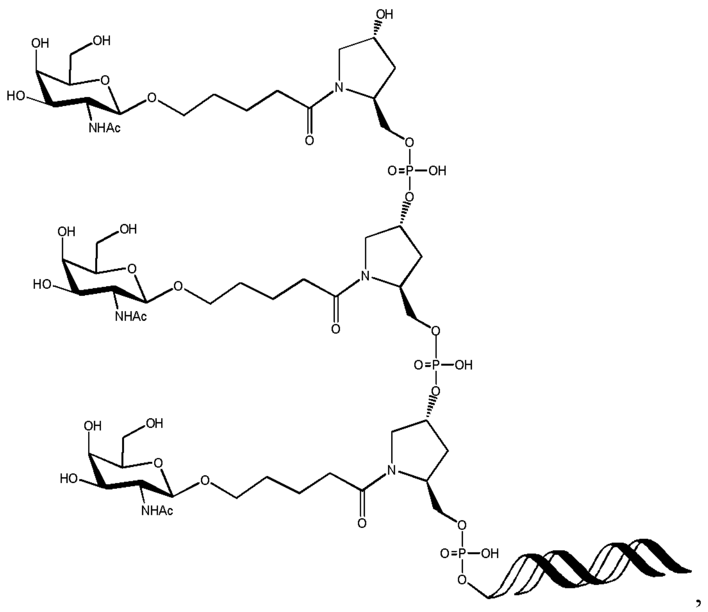

Formula (307)

wherein, the double helix structure denotes a siRNA; and the linker is linked to 3'-terminal of the sense strand of the siRNA.

The above conjugates can be synthesized according to the method described in detail in the prior art. For example, WO2015006740 A2 describes in detail the preparation methods of various conjugates. The siRNA conjugate of the present disclosure may be obtained by the methods well-known to those skilled in the art. For example, WO2014025805A1 describes the preparation method of the conjugate having the structure as shown by Formula (305). Rajeev et al., ChemBioChem 2015, 16, 903-908 describes the preparation method of the conjugate having the structure as shown by Formula (307).

In some embodiments, the siRNA conjugate has a structure as shown by Formula (308):

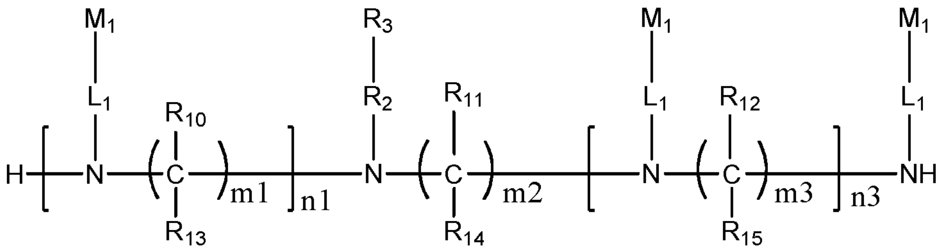

Formula (308)

wherein,

- n1 is an integer of 1-3, and n3 is an integer of 0-4;
- m1, m2, and m3 independently of one another are an integer of 2-10;
- $R_{10}$, $R_{11}$, $R_{12}$, $R_{13}$, $R_{14}$, and $R_{15}$ independently of one another are H, or selected from the group consisting of $C_1$-$C_{10}$ alkyl, $C_1$-$C_{10}$ haloalkyl, and $C_1$-$C_{10}$ alkoxy,
- $R_3$ is a group having a structure as shown by Formula (A59):

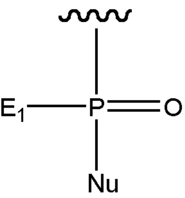

Formula (A59)

wherein $E_1$ is OH, SH or $BH_2$; and Nu is the siRNA of the present disclosure; $R_2$ is a linear alkylene of 1 to 20 carbon atoms in length, wherein one or more carbon atoms are optionally replaced with any one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $R_2$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -NH($C_1$-$C_{10}$ alkylphenyl), -N($C_1$-$C_{10}$ alkyl)($C_1$-$C_{10}$ alkylphenyl), cyano, nitro, -$CO_2H$, -C(O)$OC_1$-$C_{10}$ alkyl, -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl); each $L_1$ is a linear alkylene of 1 to 70 carbon atoms in length, wherein one or more carbon atoms are optionally replaced with any one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -NH($C_1$-$C_{10}$ alkylphenyl), -N($C_1$-$C_{10}$ alkyl)($C_1$-$C_{10}$ alkylphenyl), cyano, nitro, -$CO_2H$, -C(O)$OC_1$-$C_{10}$ alkyl, -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2NH$($C_1$-$C_{10}$ alkyl), -$SO_2NH$(phenyl), -$NHSO_2$($C_1$-$C_{10}$ alkyl), -$NHSO_2$(phenyl), and -$NHSO_2$($C_1$-$C_{10}$ haloalkyl).

In some embodiments, $L_1$ may be selected from the group consisting of the groups of Formulae (A1)-(A26) and any combination thereof, wherein the structures and definitions of A1-A26 are as follows:

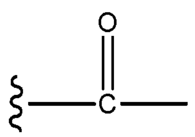, 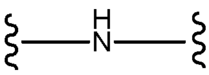, 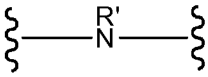, 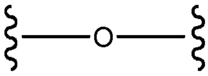,

(A1) (A2) (A3) (A4)

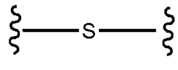, 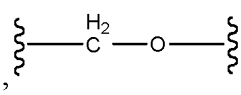, 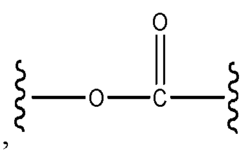, 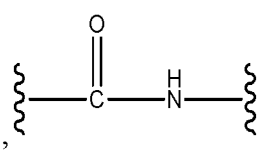,

(A5) (A6) (A7) (A8)

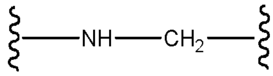, 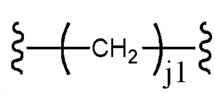, 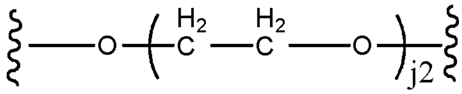,

(A9) (A10) (A11)

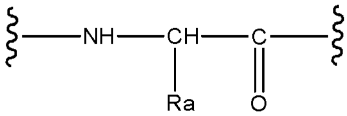, 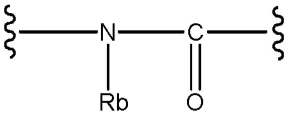, 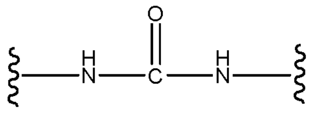,

(A12) (A13) (A14)

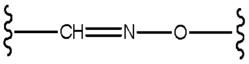, 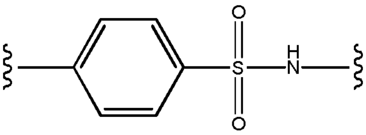, 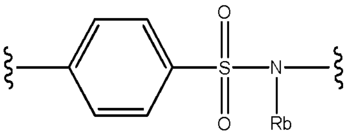,

(A15) (A16) (A17)

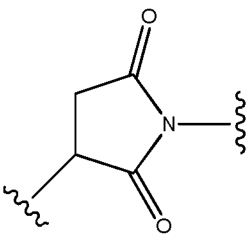, 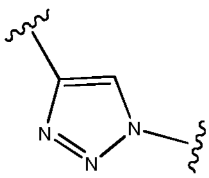, 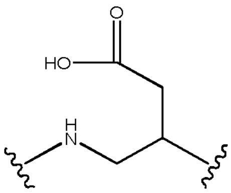, 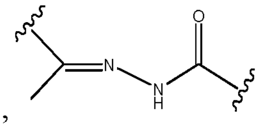,

(A18) (A19) (A20) (A21)

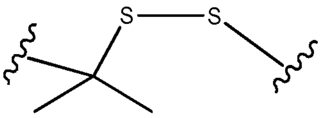, 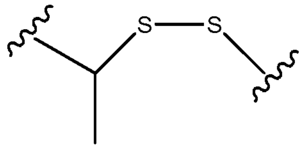, 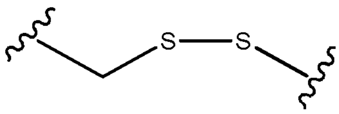,

(A22) (A23) (A24)

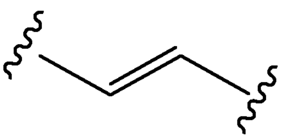 and 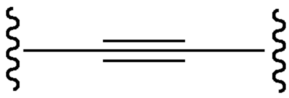;

(A25) (A26)

wherein j1 is an integer of 1-20;

j2 is an integer of 1-20;
R' is a $C_1$-$C_{10}$ alkyl;
Ra is selected from the group consisting of the groups of Formulae (A27)-(A45) or any combination thereof:

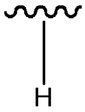,
(A27)

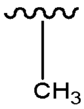,
(A28)

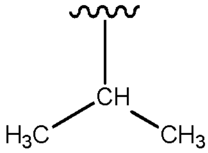,
(A29)

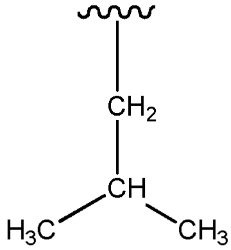,
(A30)

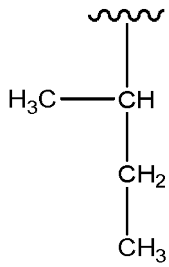,
(A31)

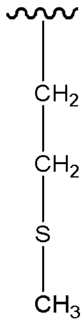,
(A32)

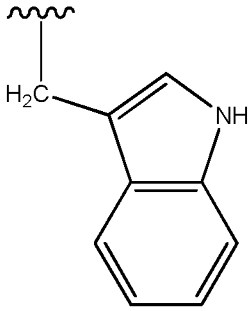,
(A33)

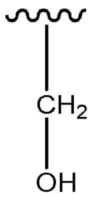,
(A34)

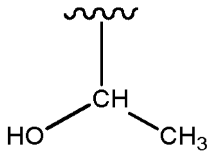,
(A35)

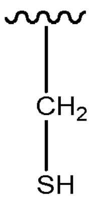,
(A36)

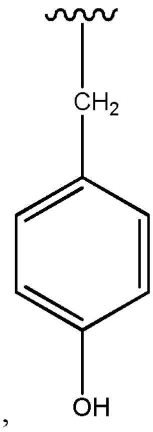,
(A37)

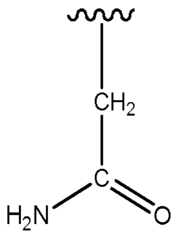,
(A38)

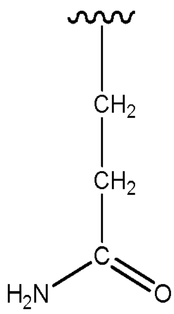,
(A39)

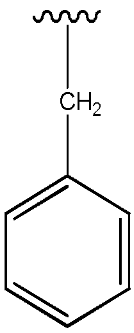,
(A40)

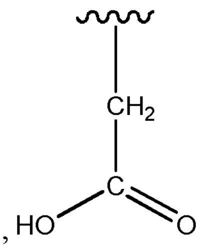,
(A41)

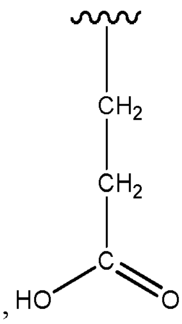,
(A42)

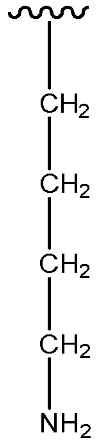, 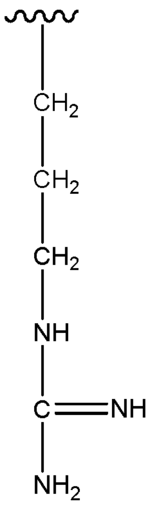 , and 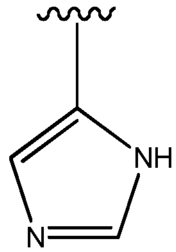 ;

(A43) (A44) (A45)

Rb is a $C_1$-$C_{10}$ alkyl; and

 represents the site where a group is linked to the rest of the molecule.

Those skilled in the art would understand that, though $L_1$ is defined as a linear alkyl for convenience, but it may not be a linear group or be named differently, such as an amine or alkenyl produced by the above replacement and/or substitution. For the purpose of the present disclosure, the length of $L_1$ is the number of the atoms in the chain linking the two attachment points. For this purpose, a ring obtained by replacing a carbon atom in the linear alkylene, such as a heterocyclylene or heteroarylene, is counted as one atom.

$M_1$ represents a targeting group, of which the definitions and options are the same as those of the above targeting groups. In some embodiments, each $M_1$ is independently one selected from the ligands that have affinity to the asialoglycoprotein receptor on the surface of mammalian hepatocytes.

When $M_1$ is a ligand that has affinity to the asialoglycoprotein receptor on the surface of mammalian hepatocyte, in some embodiments, n1 may be an integer of 1-3, and n3 may be an integer of 0-4 to ensure that the number of the $M_1$ targeting group in the conjugate may be at least 2. In some embodiments, $n1+n3 \geq 2$, such that the number of the $M_1$ targeting group is at least 3, thereby rendering the $M_1$ targeting group to more easily bind to the asialoglycoprotein receptor on the surface of hepatocytes, which may facilitates the endocytosis of the conjugate into cells. Experiments have shown that when the number of the $M_1$ ligand is greater than 3, the ease of the binding between the $M_1$ ligand and the asialoglycoprotein receptor on the surface of hepatocytes is not significantly increased. Therefore, in view of various aspects such as synthesis convenience, structure/process costs and delivery efficiency, in some embodiments, n1 is an integer of 1-2, n3 is an integer of 0-1, and n1+n3 =2-3.

In some embodiments, when m1, m2, and m3 independently of one another are an integer selected from 2-10, the steric positions among many $M_1$ ligands may be suitable for the binding between the $M_1$ ligands and the asialoglycoprotein receptor on the surface of hepatocytes. In order to make the conjugate of the present disclosure have simpler structure, easier synthesis and/or reduced cost, in some embodiments, m1, m2 and m3 independently of one another are an integer of 2-5; in some embodiments, m1 = m2 = m3.

Those skilled in the art would understand that when $R_{10}$, $R_{11}$, $R_{12}$, $R_{13}$, $R_{14}$, and $R_{15}$ independently of one another is one selected from H, $C_1$-$C_{10}$ alkyl, $C_1$-$C_{10}$ haloalkyl, and $C_1$-$C_{10}$ alkoxy, they would not change the properties of the conjugate of the present disclosure and could all achieve the purpose of the present disclosure. In some embodiments, $R_{10}$, $R_{11}$, $R_{12}$, $R_{13}$, $R_{14}$, and $R_{15}$ independently of one another are selected from H, methyl and ethyl. In some embodiments, $R_{10}$, $R_{11}$, $R_{12}$, $R_{13}$, $R_{14}$, and $R_{15}$ are H.

$R_3$ is a group having the structure as shown by Formula A59, wherein $E_1$ is OH, SH or $BH_2$, and considering the easy availability of the starting materials, in some embodiments, $E_1$ is OH or SH.

$R_2$ is selected to achieve the linkage between the group as shown by Formula A59 and the N atom on a nitrogenous backbone. In the context of the present disclosure, a "nitrogenous backbone" refers to a chain structure in which the N atom are coadjacently linked to the carbon atoms to which $R_{10}$, $R_{11}$, $R_{12}$, $R_{13}$, $R_{14}$, and $R_{15}$ are attached. Therefore, $R_2$ may be any linking group capable of linking the group as shown by Formula (A59) to the N atom on the nitrogenous backbone by suitable means. In some embodiments, in the case where the siRNA conjugate as shown by Formula (308) is prepared by a solid phase synthesis process, $R_2$ group needs to have both a site linking to the N atom

on the nitrogenous backbone and a site linking to the P atom in $R_3$. In some embodiments, in $R_2$, the site linking to the N atom on the nitrogenous backbone forms an amide bond with the N atom, and the site linking to the P atom in $R_3$ forms a phosphoester bond with the P atom. In some embodiments, $R_2$ may be B5, B6, B5', orB6':

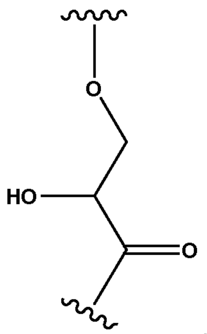

,

(B5)

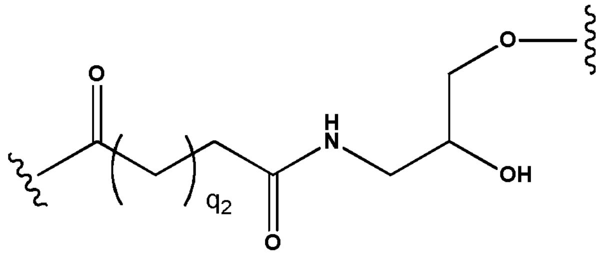

,

(B6)

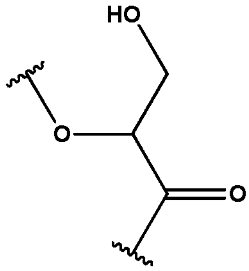

, or

(B5')

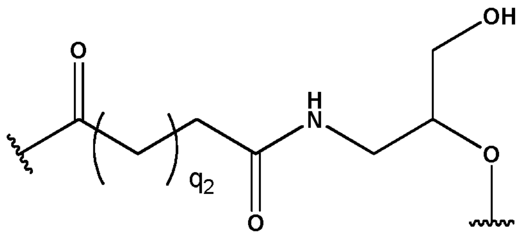

;

(B6')

wherein  represents the site where the group is linked via a covalent bond;

$q_2$ is an integer of 1-10; in some embodiments, $q_2$ is an integer of 1-5.

$L_1$ is used to link the $M_1$ targeting group to the N atom on the nitrogenous backbone, thereby providing liver targeting function for the siRNA conjugate as shown by Formula (308). In some embodiments, $L_1$ is selected from the linkage combinations of one or more of Formulae (A1)-(A26). In some embodiments, $L_1$ is selected from the linkage combinations of one or more of Formulae (A1), (A4), (A5), (A6), (A8), (A10), (A11), and (A13). In some embodiments, $L_1$ is selected from the linkage combinations of at least two of Formulae (A1), (A4), (A8), (A10), and (A11). In some embodiments, $L_1$ is selected from the linkage combinations of at least two of Formulae (A1), (A8) and (A10).

In some embodiments, $L_1$ may have a length of 3 to 25, 3 to 20, 4 to 15 or 5 to 12 atoms. In some embodiments, $L_1$ has a length of 3, 4, 5, 6, 7, 8, 9, 10, 11, 12, 13, 14, 15, 16, 17, 18, 19, 20, 21, 22, 23, 24, 25, 30, 35, 40, 45, 50, 55, or 60 atoms.

In some embodiments, j 1 is an integer of 2-10, and in some embodiments, j 1 is an integer of 3-5. In some embodiments, j2 is an integer of 2-10, and in some embodiments, j2 is an integer of 3-5. R' is a $C_1$-$C_4$ alkyl, and in some embodiments, R' is one of methyl, ethyl and isopropyl. Ra is one of Formulae (A27), (A28), (A29), (A30), and (A31), and in some embodiments, Ra is Formula (A27) or (A28). Rb is a $C_1$-$C_5$ alkyl, and in some embodiments, is one of methyl, ethyl, isopropyl, and butyl. In some embodiments, j1, j2, R', Ra, and Rb in Formulae (A1)-(A26) are respectively selected to achieve the linkage between the $M_1$ targeting groups and the N atom on the nitrogenous backbone, and to make the steric position among the $M_1$ targeting groups more suitable for binding between the $M_1$ targeting groups and the asialoglycoprotein receptor on the surface of hepatocytes.

In some embodiments, the siRNA conjugate has a structure as shown by Formula (403), (404), (405), (406), (407), (408), (409), (410), (411), (412), (413), (414), (415), (416), (417), (418), (419), (420), (421) or (422):

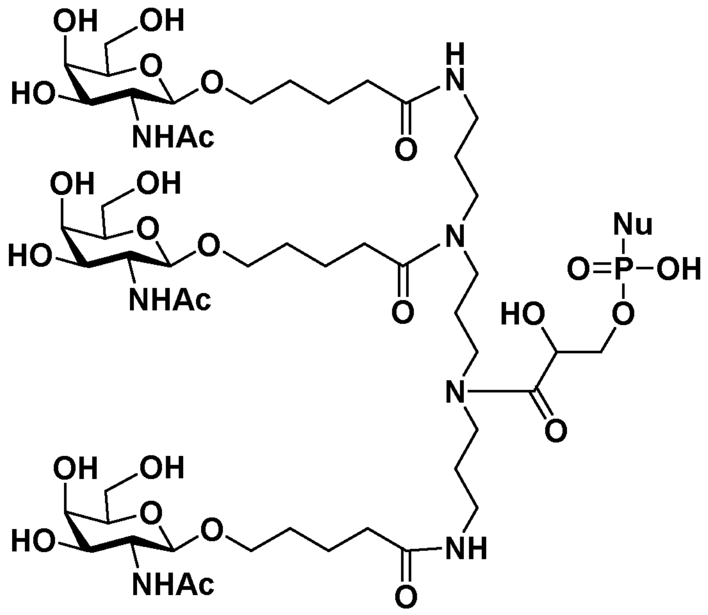

Formula (403)

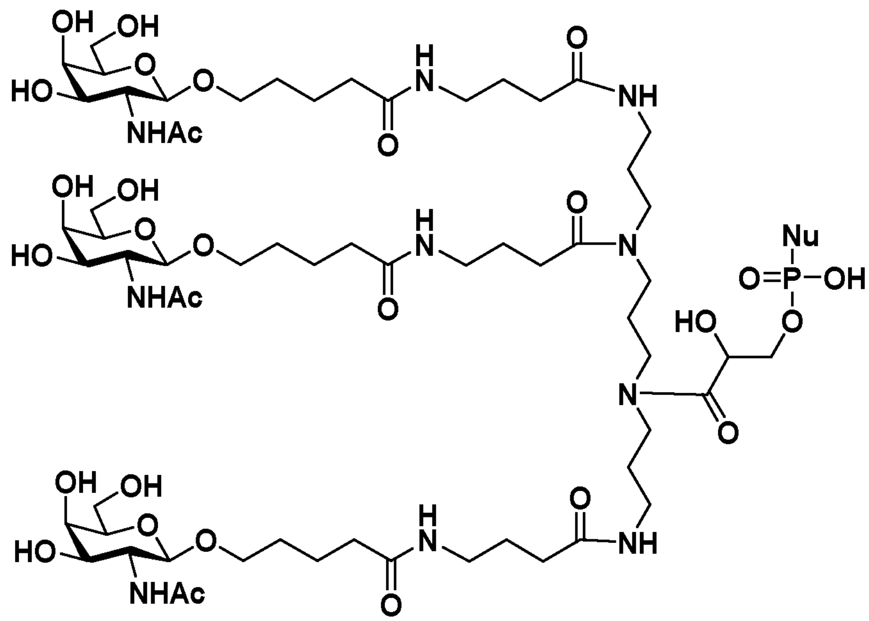

Formula (404)

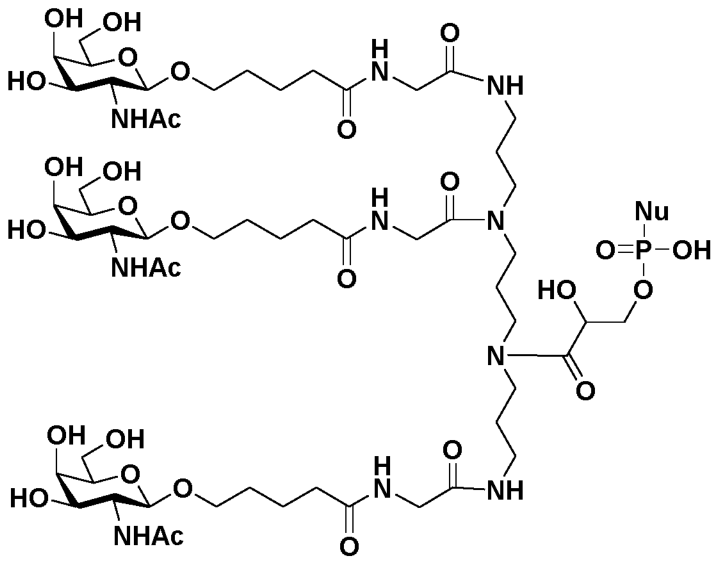

Formula (405)

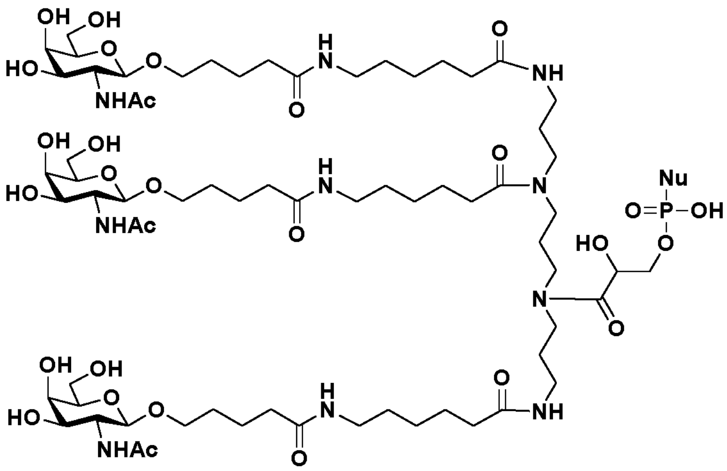

Formula (406)

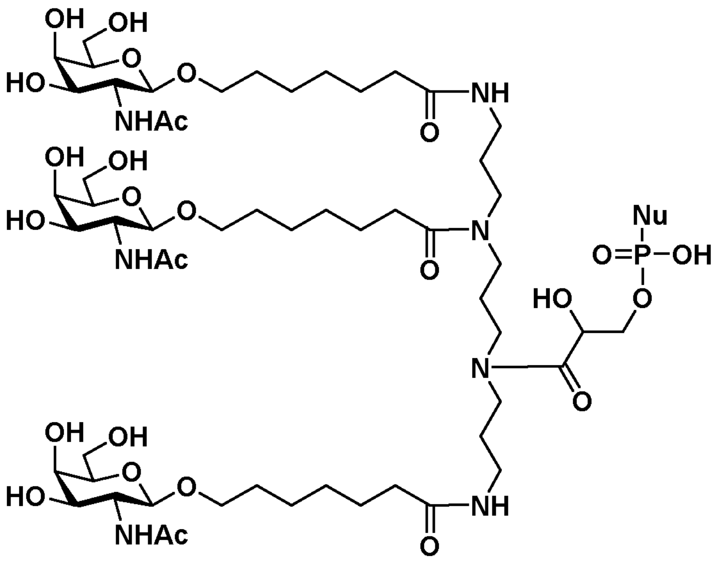

Formula (407)

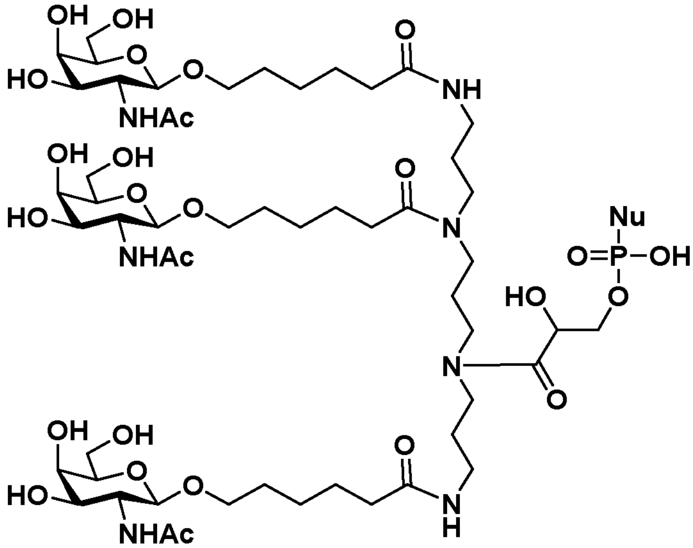

Formula (408)

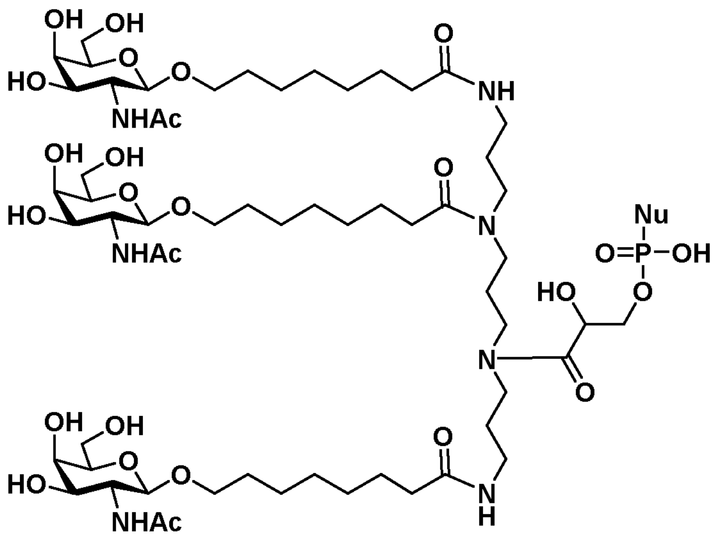

Formula (409)

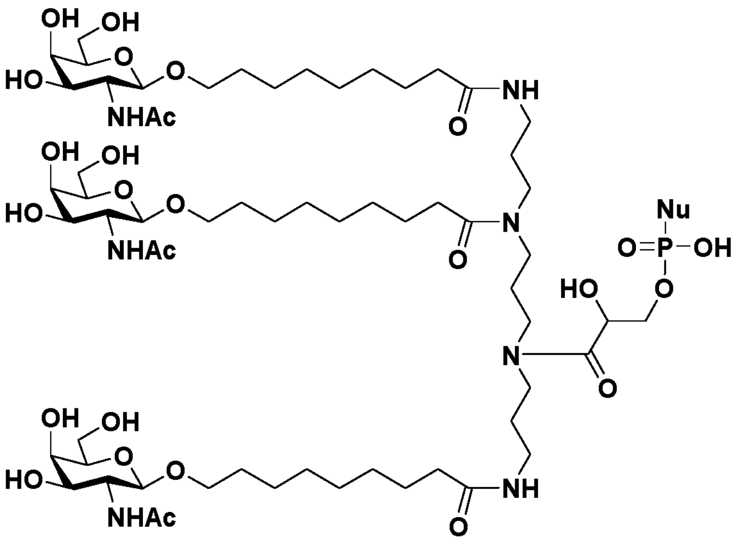

Formula (410)

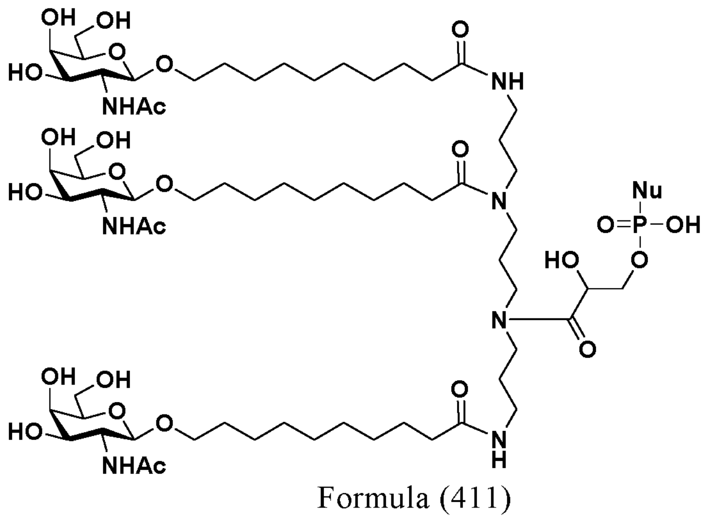

Formula (411)

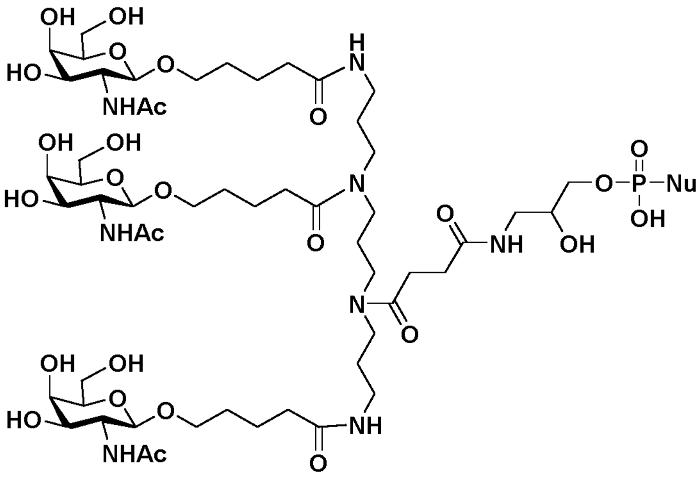

Formula (412)

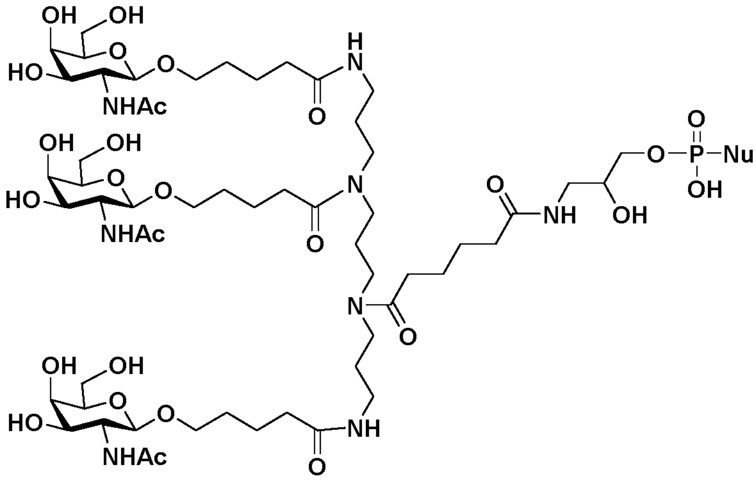

Formula (413)

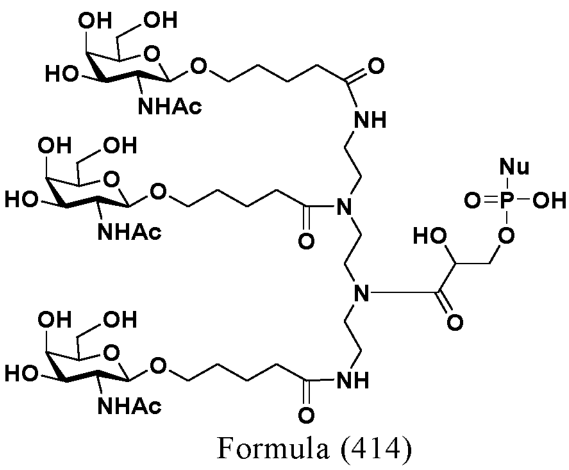

Formula (414)

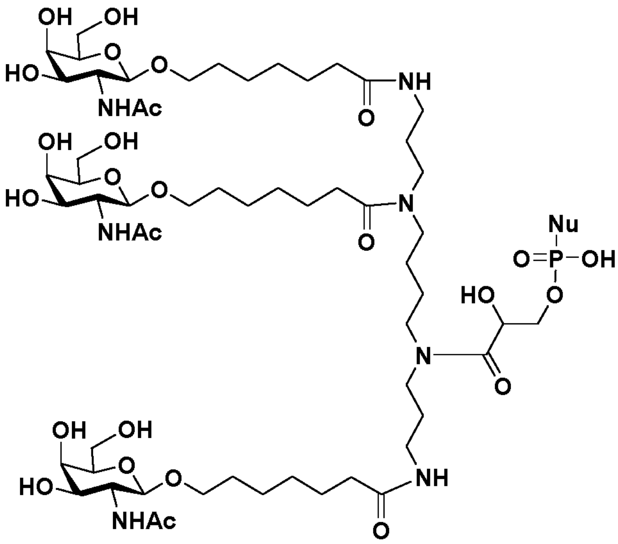

Formula (415)

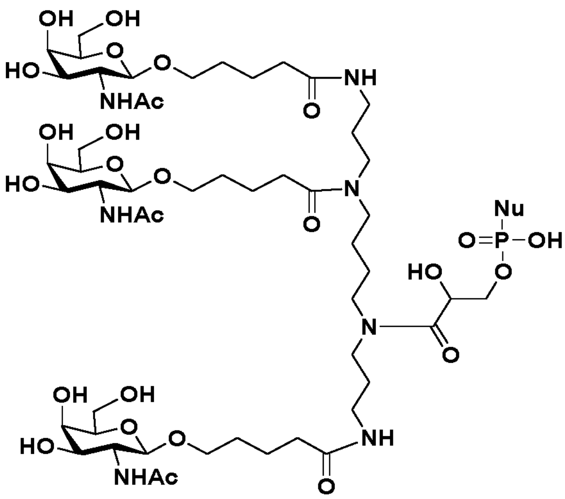

Formula (416)

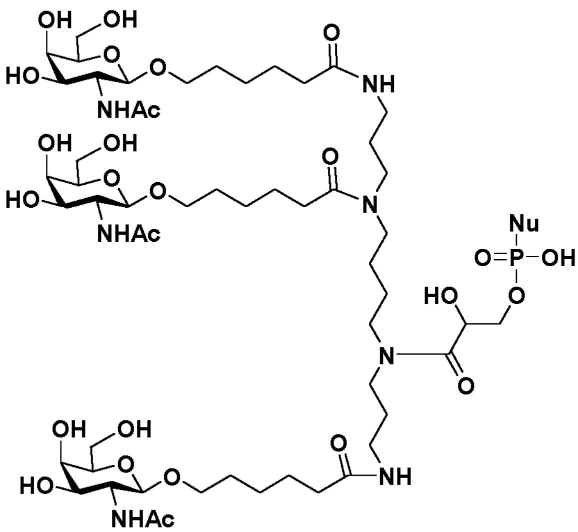

Formula (417)

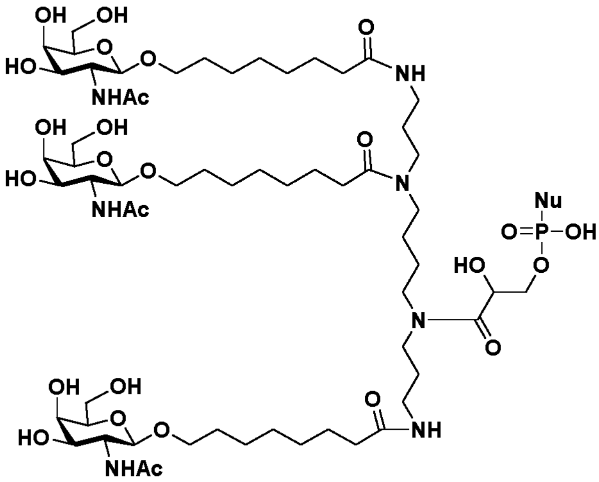

Formula (418)

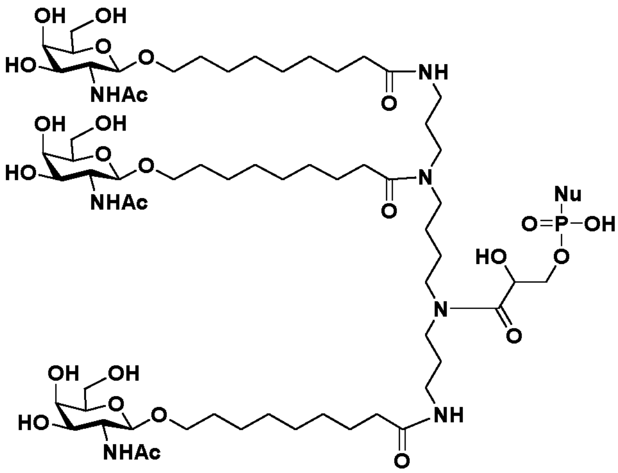

Formula (419)

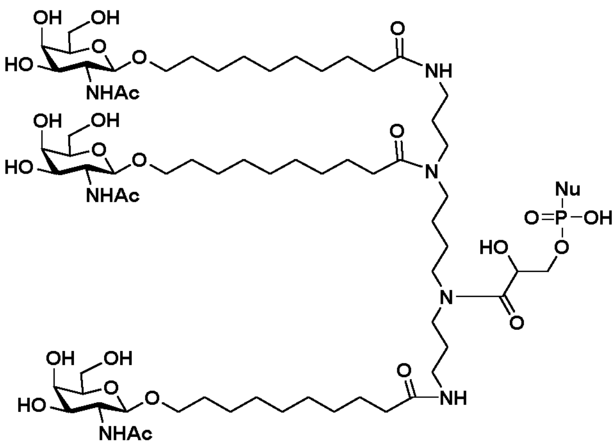

Formula (420)

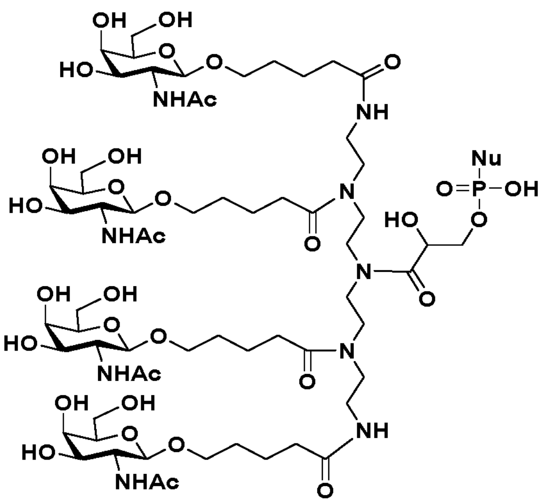

Formula (421)

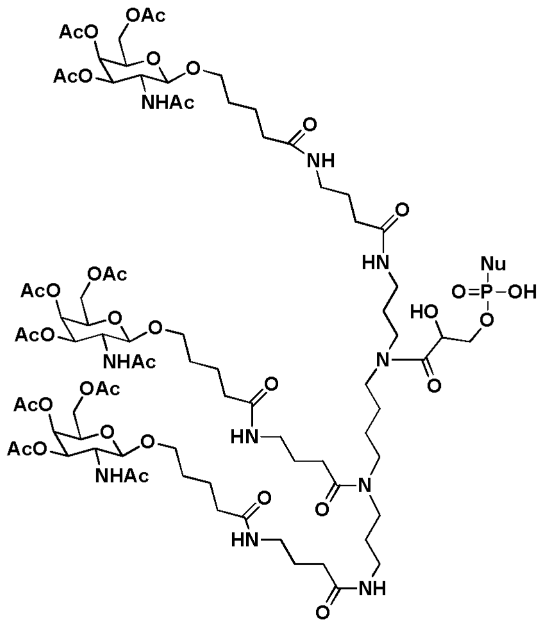

Formula (422)

In some embodiments, the P atom in Formula A59 may be linked to any possible position in the siRNA sequence. For example, the P atom in Formula A59 may be linked to any nucleotide in the sense or antisense strand of the siRNA. In some embodiments, the P atom in Formula A59 is linked to any nucleotide in the sense strand of the siRNA. In some embodiments, the P atom in Formula A59 may be linked to a terminal region of the sense or antisense strand of the siRNA. In some embodiments, the P atom in Formula A59 is linked to a terminal region of the sense strand of the siRNA. Said terminal region refers to the first 4 nucleotides counted from one terminal of the sense or antisense strand. In some embodiments, the P atom in Formula A59 is linked to either terminal of the sense or antisense strand of the siRNA. In some embodiments, the P atom in Formula A59 is linked to 3' terminal of the sense strand of the siRNA. In the case where the P atom in Formula A59 is linked to the above position of the sense strand of the siRNA, after having entered into cells, the siRNA conjugate as shown by Formula (308) can release a separate antisense strand of the siRNA during unwinding, thereby blocking the translation of the APOC3 mRNA into a protein and inhibiting the expression of apolipoprotein C3 gene.

In some embodiments, the P atom in Formula A59 may be linked to any possible position of a nucleotide in the siRNA, for example, position 5', position 2', position 3', or the base of the nucleotide. In some embodiments, the P atom in Formula A59 may be linked to position 2', 3', or 5' of a nucleotide in the siRNA by forming a phosphodiester bond. In some embodiments, the P atom in Formula A59 is linked to an oxygen atom formed by dehydrogenation of 3'-hydroxy of the nucleotide at 3' terminal of the sense strand in the siRNA (in this case, the P atom in Formula A59 may be also regarded as the P atom in the phosphate group of the siRNA), or the P atom in Formula A59 is linked to a nucleotide by substituting a hydrogen atom in 2'-hydroxy of a nucleotide of the sense strand in the siRNA, or the P atom in Formula A59 is linked to a nucleotide by substituting a hydrogen atom in 5'-hydroxy of the nucleotide at 5' terminal of the sense strand in the siRNA.

The inventors of the present disclosure have surprisingly found that the siRNA conjugate of the present disclosure exhibits significantly improved stability in plasma and low off-target effect, without significantly reduced silencing activity against APOC3 mRNA, and further shows higher inhibitory effect on blood lipid. Thus, in some embodiments, the siRNA in the siRNA conjugates of the present disclosure is shown in Tables 1, 2, 3, 4, and 5:

**Table 1 The sequences of first siRNAs in the conjugates of the present disclosure**

| siRNA NO. | SEQ ID NO: | Sequence direction 5'-3' |
|---|---|---|
| siAPa1 | 9 | UUAAAAGGGACAGUAUUCU |
| | 10 | AGAAUACUGUCCCUUUUAAGC |
| siAPa2 | 11 | GCUUAAAAGGGACAGUAUUCU |
| | 12 | AGAAUACUGUCCCUUUUAAGCAA |
| siAPa1-M1 | 61 | UmUmAmAmAfAmGfGfGfAmCmAmGmUmAmUmUmCmUm |
| | 62 | AmGfAmAmUmAfCmUfGfUmCmCmCmUfUmUfUmAmAmGmCm |
| siAPa2-M1 | 63 | GmCmUmUmAmAmAfAmGfGfGfAmCmAmGmUmAmUmUmCmUm |
| | 64 | AmGfAmAmUmAfCmUfGfUmCmCmCmUfUmUfUmAmAmGmCmAmAm |
| siAPa1-M2 | 65 | UmUmAmAmAfAmGfGfGfAmCmAmGmUmAmUmUmCmUm |
| | 66 | AmGfAmAmUmAfCmUmGmUmCmCmCmUfUmUfUmAmAmGmCm |
| siAPa2-M2 | 67 | GmCmUmUmAmAmAfAmGfGfGfAmCmAmGmUmAmUmUmCmUm |
| | 68 | AmGfAmAmUmAfCmUmGmUmCmCmCmUfUmUfUmAmAmGmCmAmAm |
| siAPa1-M3 | 69 | UmUmAmAmAmAmGfGfGfAmCmAmGmUmAmUmUmCmUm |
| | 70 | AmGfAmAmUmAfCmUmGmUmCmCmCmUfUmUfUmAmAmGmCm |
| siAPa2-M3 | 71 | GmCmUmUmAmAmAmAmGfGfGfAmCmAmGmUmAmUmUmCmUm |
| | 72 | AmGfAmAmUmAfCmUmGmUmCmCmCmUfUmUfUmAmAmGmCmAmAm |
| siAPa1-M1S | 121 | UmsUmsAmAmAfAmGfGfGfAmCmAmGmUmAmUmUmCmUm |
| | 122 | AmsGfsAmAmUmAfCmUfGfUmCmCmCmUfUmUfUmAmAmsGmsCm |

(continued)

| siRNA NO. | SEQ ID NO: | Sequence direction 5'-3' |
|---|---|---|
| siAPa2-M1S | 123 | GmsCmsUmUmAmAmAfAmGfGfGfAmCmAmGmUmAmUmUmCmUm |
| | 124 | AmsGfsAmAmUmAfCmUfGfUmCmCmCmUfUmUfUmAmAmGmCmsAmsAm |
| siAPa1-M2S | 125 | UmsUmsAmAmAfAmGfGfGfAmCmAmGmUmAmUmUmCmUm |
| | 126 | AmsGfsAmAmUmAfCmUmGmUmCmCmCmUfUmUfUmAmAmsGmsCm |
| siAPa2-M2S | 127 | GmsCmsUmUmAmAmAfAmGfGfGfAmCmAmGmUmAmUmUmCmUm |
| | 128 | AmsGfsAmAmUmAfCmUmGmUmCmCmCmUfUmUfUmAmAmGmCmsAmsAm |
| siAPa-M3S | 129 | UmsUmsAmAmAmAmGfGfGfAmCmAmGmUmAmUmUmCmUm |
| | 130 | AmsGfsAmAmUmAfCmUmGmUmCmCmCmUfUmUfUmAmAmsGmsCm |
| siAPa2-M3S | 131 | GmsCmsUmUmAmAmAmAmGfGfGfAmCmAmGmUmAmUmUmCmUm |
| | 132 | AmsGfsAmAmUmAfCmUmGmUmCmCmCmUfUmUfUmAmAmGmCmsAmsAm |
| siAPa1-M1P1 | 181 | UmUmAmAmAfAmGfGfGfAmCmAmGmUmAmUmUmCmUm |
| | 182 | P1-AmGfAmAmUmAfCmUfGfUmCmCmCmUfUmUfUmAmAmGmCm |
| siAPa2-M1P1 | 183 | GmCmUmUmAmAmAfAmGfGfGfAmCmAmGmUmAmUmUmCmUm |
| | 184 | P1-AmGfAmAmUmAfCmUfGfUmCmCmCmUfUmUfUmAmAmGmCmAmAm |
| siAPa1-M2P1 | 185 | UmUmAmAmAfAmGfGfGfAmCmAmGmUmAmUmUmCmUm |
| | 186 | P1-AmGfAmAmUmAfCmUmGmUmCmCmCmUfUmUfUmAmAmGmCm |
| siAPa2-M2P1 | 187 | GmCmUmUmAmAmAfAmGfGfGfAmCmAmGmUmAmUmUmCmUm |
| | 188 | P1-AmGfAmAmUmAfCmUmGmUmCmCmCmUfUmUfUmAmAmGmCmAmAm |

(continued)

| siRNA NO. | SEQ ID NO: | Sequence direction 5'-3' |
|---|---|---|
| siAPa1-M3P1 | 189 | UmUmAmAmAmAmGfGfGfAmCmAmGmUmAmUmUmCmUm |
| | 190 | P1-AmGfAmAmUmAfCmUmGmUmCmCmCmUfUmUfUmAmAmGmCm |
| siAPa2-M3P1 | 191 | GmCmUmUmAmAmAmAmGfGfGfAmCmAmGmUmAmUmUmCmUm |
| | 192 | P1-AmGfAmAmUmAfCmUmGmUmCmCmCmUfUmUfUmAmAmGmCmAmAm |
| siAPa1-M1SP1 | 193 | UmsUmsAmAmAfAmGfGfGfAmCmAmGmUmAmUmUmCmUm |
| | 194 | P1-AmsGfsAmAmUmAfCmUfGfUmCmCmCmUfUmUfUmAmAmsGmsCm |
| siAPa2-M1SP1 | 195 | GmsCmsUmUmAmAmAfAmGfGfGfAmCmAmGmUmAmUmUmCmUm |
| | 196 | P1-AmsGfsAmAmUmAfCmUfGfUmCmCmCmUfUmUfUmAmAmGmCmsAmsAm |
| siAPa1-M2SP1 | 197 | UmsUmsAmAmAfAmGfGfGfAmCmAmGmUmAmUmUmCmUm |
| | 198 | P1-AmsGfsAmAmUmAfCmUmGmUmCmCmCmUfUmUfUmAmAmsGmsCm |
| siAPa2-M2SP1 | 199 | GmsCmsUmUmAmAmAfAmGfGfGfAmCmAmGmUmAmUmUmCmUm |
| | 200 | P1-AmsGfsAmAmUmAfCmUmGmUmCmCmCmUfUmUfUmAmAmGmCmsAmsAm |
| siAPa1-M3SP1 | 201 | UmsUmsAmAmAmAmGfGfGfAmCmAmGmUmAmUmUmCmUm |
| | 202 | P1-AmsGfsAmAmUmAfCmUmGmUmCmCmCmUfUmUfUmAmAmsGmsCm |
| siAPa2-M3SP1 | 203 | GmsCmsUmUmAmAmAmAmGfGfGfAmCmAmGmUmAmUmUmCmUm |
| | 204 | P1-AmsGfsAmAmUmAfCmUmGmUmCmCmCmUfUmUfUmAmAmGmCmsAmsAm |

(continued)

| siRNA NO. | SEQ ID NO: | Sequence direction 5'-3' |
|---|---|---|
| siAPa1U-M1P1 | 327 | UmUmAmAmAfAmGfGfGfAmCmAmGmUmAmUmUmCmAm |
| | 328 | P1-UmGfAmAmUmAfCmUfGfUmCmCmCmUfUmUfUmAmAmGmCm |
| siAPa2U-M1P1 | 329 | GmCmUmUmAmAmAfAmGfGfGfAmCmAmGmUmAmUmUmCmAm |
| | 330 | P1-UmGfAmAmUmAfCmUfGfUmCmCmCmUfUmUfUmAmAmGmCmAmAm |
| siAPa1 U -M2P1 | 331 | UmUmAmAmAfAmGfGfGfAmCmAmGmUmAmUmUmCmAm |
| | 332 | P1-UmGfAmAmUmAfCmUmGmUmCmCmCmUfUmUfUmAmAmGmCm |
| siAPa2U -M2P1 | 333 | GmCmUmUmAmAmAfAmGfGfGfAmCmAmGmUmAmUmUmCmAm |
| | 334 | P1-UmGfAmAmUmAfCmUmGmUmCmCmCmUfUmUfUmAmAmGmCmAmAm |
| siAPa1 U -M3P1 | 335 | UmUmAmAmAmAmGfGfGfAmCmAmGmUmAmUmUmCmAm |
| | 336 | P1-UmGfAmAmUmAfCmUmGmUmCmCmCmUfUmUfUmAmAmGmCm |
| siAPa2U -M3P1 | 337 | GmCmUmUmAmAmAmAmGfGfGfAmCmAmGmUmAmUmUmCmAm |
| | 338 | P1-UmGfAmAmUmAfCmUmGmUmCmCmCmUfUmUfUmAmAmGmCmAmAm |
| siAPa1 U -M1SP1 | 339 | UmsUmsAmAmAfAmGfGfGfAmCmAmGmUmAmUmUmCmAm |
| | 340 | P1-UmsGfsAmAmUmAfCmUfGfUmCmCmCmUfUmUfUmAmAmsGmsCm |
| siAPa2U -M1SP1 | 341 | GmsCmsUmUmAmAmAfAmGfGfGfAmCmAmGmUmAmUmUmCmAm |
| | 342 | P1-UmsGfsAmAmUmAfCmUfGfUmCmCmCmUfUmUfUmAmAmGmCmsAmsAm |

(continued)

| siRNA NO. | SEQ ID NO: | Sequence direction 5'-3' |
|---|---|---|
| siAPa1 U -M2SP1 | 343 | UmsUmsAmAmAfAmGfGfGfAmCmAmGmUmAmUmUmCmAm |
| | 344 | P1-UmsGfsAmAmUmAfCmUmGmUmCmCmCmUfUmUfUmAmAmsGmsCm |
| siAPa2U -M2SP1 | 345 | GmsCmsUmUmAmAmAfAmGfGfGfAmCmAmGmUmAmUmUmCmAm |
| | 346 | P1-UmsGfsAmAmUmAfCmUmGmUmCmCmCmUfUmUfUmAmAmGmCmsAmsAm |
| siAPa1 U -M3SP1 | 347 | UmsUmsAmAmAmAmGfGfGfAmCmAmGmUmAmUmUmCmAm |
| | 348 | P1-UmsGfsAmAmUmAfCmUmGmUmCmCmCmUfUmUfUmAmAmsGmsCm |
| siAPa2U -M3SP1 | 349 | GmsCmsUmUmAmAmAmAmGfGfGfAmCmAmGmUmAmUmUmCmAm |
| | 350 | P1-UmsGfsAmAmUmAfCmUmGmUmCmCmCmUfUmUfUmAmAmGmCmsAmsAm |

Table 2 The sequences of second siRNAs in the conjugates of the present disclosure

| siRNA NO. | SEQ ID NO: | Sequence direction 5'-3' |
|---|---|---|
| siAPb1 | 21 | ACAGUAUUCUCAGUGCUCU |
| | 22 | AGAGCACUGAGAAUACUGUCC |
| siAPb2 | 23 | GGACAGUAUUCUCAGUGCUCU |
| | 24 | AGAGCACUGAGAAUACUGUCCCU |
| siAPb1-M1 | 73 | AmCmAmGmUfAmUfUfCfUmCmAmGmUmGmCmUmCmUm |
| | 74 | AmGfAmGmCmAfCmUfGfAmGmAmAmUfAmCfUmGmUmCmCm |
| siAPb2-M1 | 75 | GmGmAmCmAmGmUfAmUfUfCfUmCmAmGmUmGmCmUmCmUm |
| | 76 | AmGfAmGmCmAfCmUfGfAmGmAmAmUfAmCfUmGmUmCmCmCmUm |
| siAPb1-M2 | 77 | AmCmAmGmUfAmUfUfCfUmCmAmGmUmGmCmUmCmUm |
| | 78 | AmGfAmGmCmAfCmUmGmAmGmAmAmUfAmCfUmGmUmCmCm |

(continued)

| siRNA NO. | SEQ ID NO: | Sequence direction 5'-3' |
|---|---|---|
| siAPb2-M2 | 79 | GmGmAmCmAmGmUfAmUfUfCfUmCmAmGmUmGmCmUmCmUm |
| | 80 | AmGfAmGmCmAfCmUmGmAmGmAmAmUfAmCfUmGmUmCmCmCmUm |
| siAPb1-M3 | 81 | AmCmAmGmUmAmUfUfCfUmCmAmGmUmGmCmUmCmUm |
| | 82 | AmGfAmGmCmAfCmUmGmAmGmAmAmUfAmCfUmGmUmCmCm |
| siAPb2-M3 | 83 | GmGmAmCmAmGmUmAmUfUfCfUmCmAmGmUmGmCmUmCmUm |
| | 84 | AmGfAmGmCmAfCmUmGmAmGmAmAmUfAmCfUmGmUmCmCmCmUm |
| siAPb 1-M1S | 133 | AmsCmsAmGmUfAmUfUfCfUmCmAmGmUmGmCmUmCmUm |
| | 134 | AmsGfsAmGmCmAfCmUfGfAmGmAmAmUfAmCfUmGmUmsCmsCm |
| siAPb2-M1S | 135 | GmsGmsAmCmAmGmUfAmUfUfCfUmCmAmGmUmGmCmUmCmUm |
| | 136 | AmsGfsAmGmCmAfCmUfGfAmGmAmAmUfAmCfUmGmUmCmCmsCmsUm |
| siAPb1-M2S | 137 | AmsCmsAmGmUfAmUfUfCfUmCmAmGmUmGmCmUmCmUm |
| | 138 | AmsGfsAmGmCmAfCmUmGmAmGmAmAmUfAmCfUmGmUmsCmsCm |
| siAPb2-M2S | 139 | GmsGmsAmCmAmGmUfAmUfUfCfUmCmAmGmUmGmCmUmCmUm |
| | 140 | AmsGfsAmGmCmAfCmUmGmAmGmAmAmUfAmCfUmGmUmCmCmsCmsUm |
| siAPb1-M3S | 141 | AmsCmsAmGmUmAmUfUfCfUmCmAmGmUmGmCmUmCmUm |
| | 142 | AmsGfsAmGmCmAfCmUmGmAmGmAmAmUfAmCfUmGmUmsCmsCm |
| siAPb2-M3S | 143 | GmsGmsAmCmAmGmUmAmUfUfCfUmCmAmGmUmGmCmUmCmUm |
| | 144 | AmsGfsAmGmCmAfCmUmGmAmGmAmAmUfAmCfUmGmUmCmCmsCmsUm |
| siAPb1-M1P1 | 205 | AmCmAmGmUfAmUfUfCfUmCmAmGmUmGmCmUmCmUm |
| | 206 | P1-AmGfAmGmCmAfCmUfGfAmGmAmAmUfAmCfUmGmUmCmCm |

(continued)

| siRNA NO. | SEQ ID NO: | Sequence direction 5'-3' |
|---|---|---|
| siAPb2-M1P1 | 207 | GmGmAmCmAmGmUfAmUfUfCfUmCmAmGmUmGmCmUmCmUm |
| | 208 | P1-AmGfAmGmCmAfCmUfGfAmGmAmAmUfAmCfUmGmUmCmCmCmUm |
| siAPb1-M2P1 | 209 | AmCmAmGmUfAmUfUfCfUmCmAmGmUmGmCmUmCmUm |
| | 210 | P1-AmGfAmGmCmAfCmUmGmAmGmAmAmUfAmCfUmGmUmCmCm |
| siAPb2-M2P1 | 211 | GmGmAmCmAmGmUfAmUfUfCfUmCmAmGmUmGmCmUmCmUm |
| | 212 | P1-AmGfAmGmCmAfCmUmGmAmGmAmAmUfAmCfUmGmUmCmCmCmUm |
| siAPb1-M3P1 | 213 | AmCmAmGmUmAmUfUfCfUmCmAmGmUmGmCmUmCmUm |
| | 214 | P1-AmGfAmGmCmAfCmUmGmAmGmAmAmUfAmCfUmGmUmCmCm |
| siAPb2-M3P1 | 215 | GmGmAmCmAmGmUmAmUfUfCfUmCmAmGmUmGmCmUmCmUm |
| | 216 | P1-AmGfAmGmCmAfCmUmGmAmGmAmAmUfAmCfUmGmUmCmCmCmUm |
| siAPb1-M1SP1 | 217 | AmsCmsAmGmUfAmUfUfCfUmCmAmGmUmGmCmUmCmUm |
| | 218 | P1-AmsGfsAmGmCmAfCmUfGfAmGmAmAmUfAmCfUmGmUmsCmsCm |
| siAPb2-M1SP1 | 219 | GmsGmsAmCmAmGmUfAmUfUfCfUmCmAmGmUmGmCmUmCmUm |
| | 220 | P1-AmsGfsAmGmCmAfCmUfGfAmGmAmAmUfAmCfUmGmUmCmCmsCmsUm |
| siAPb1-M2SP1 | 221 | AmsCmsAmGmUfAmUfUfCfUmCmAmGmUmGmCmUmCmUm |
| | 222 | P1-AmsGfsAmGmCmAfCmUmGmAmGmAmAmUfAmCfUmGmUmCmCmsCmsUm |

(continued)

| siRNA NO. | SEQ ID NO: | Sequence direction 5'-3' |
|---|---|---|
| siAPb2-M2SP1 | 223 | GmsGmsAmCmAmGmUfAmUfUfCfUmCmAmGmUmGmCmUmCmUm |
| | 224 | P1-AmsGfsAmGmCmAfCmUmGmAmGmAmAmUfAmCfUmGmUmCmCmsCmsUm |
| siAPb1-M3SP1 | 225 | AmsCmsAmGmUmAmUfUfCfUmCmAmGmUmGmCmUmCmUm |
| | 226 | P1-AmsGfsAmGmCmAfCmUmGmAmGmAmAmUfAmCfUmGmUmsCmsCm |
| siAPb2-M3SP1 | 227 | GmsGmsAmCmAmGmUmAmUfUfCfUmCmAmGmUmGmCmUmCmUm |
| | 228 | P1-AmsGfsAmGmCmAfCmUmGmAmGmAmAmUfAmCfUmGmUmCmCmsCmsUm |
| siAPb1U-M1P1 | 351 | AmCmAmGmUfAmUfUfCfUmCmAmGmUmGmCmUmCmAm |
| | 352 | P1-UmGfAmGmCmAfCmUfGfAmGmAmAmUfAmCfUmGmUmCmCm |
| siAPb2U-M1P1 | 353 | GmGmAmCmAmGmUfAmUfUfCfUmCmAmGmUmGmCmUmCmAm |
| | 354 | P1-UmGfAmGmCmAfCmUfGfAmGmAmAmUfAmCfUmGmUmCmCmCmUm |
| siAPb 1U-M2P1 | 355 | AmCmAmGmUfAmUfUfCfUmCmAmGmUmGmCmUmCmAm |
| | 356 | P1-UmGfAmGmCmAfCmUmGmAmGmAmAmUfAmCfUmGmUmCmCm |
| siAPb2U-M2P1 | 357 | GmGmAmCmAmGmUfAmUfUfCfUmCmAmGmUmGmCmUmCmAm |
| | 358 | P1-UmGfAmGmCmAfCmUmGmAmGmAmAmUfAmCfUmGmUmCmCmCmUm |
| siAPb1U-M3P1 | 359 | AmCmAmGmUmAmUfUfCfUmCmAmGmUmGmCmUmCmAm |
| | 360 | P1-UmGfAmGmCmAfCmUmGmAmGmAmAmUfAmCfUmGmUmCmCm |

(continued)

| siRNA NO. | SEQ ID NO: | Sequence direction 5'-3' |
|---|---|---|
| siAPb2U-M3P1 | 361 | GmGmAmCmAmGmUmAmUfUfCfUmCmAmGmUmGmCmUmCmAm |
| | 362 | P1-UmGfAmGmCmAfCmUmGmAmGmAmAmUfAmCfUmGmUmCmCmCmUm |
| siAPb1U-M1SP1 | 363 | AmsCmsAmGmUfAmUfUfCfUmCmAmGmUmGmCmUmCmAm |
| | 364 | P1-UmsGfsAmGmCmAfCmUfGfAmGmAmAmUfAmCfUmGmUmsCmsCm |
| siAPb2U-M1SP1 | 365 | GmsGmsAmCmAmGmUfAmUfUfCfUmCmAmGmUmGmCmUmCmAm |
| | 366 | P1-UmsGfsAmGmCmAfCmUfGfAmGmAmAmUfAmCfUmGmUmCmCmsCmsUm |
| siAPb1U-M2SP1 | 367 | AmsCmsAmGmUfAmUfUfCfUmCmAmGmUmGmCmUmCmAm |
| | 368 | P1-UmsGfsAmGmCmAfCmUmGmAmGmAmAmUfAmCfUmGmUmCmCmsCmsUm |
| siAPb2U-M2SP1 | 369 | GmsGmsAmCmAmGmUfAmUfUfCfUmCmAmGmUmGmCmUmCmAm |
| | 370 | P1-UmsGfsAmGmCmAfCmUmGmAmGmAmAmUfAmCfUmGmUmCmCmsCmsUm |
| siAPb1U-M3SP1 | 371 | AmsCmsAmGmUmAmUfUfCfUmCmAmGmUmGmCmUmCmAm |
| | 372 | P1-UmsGfsAmGmCmAfCmUmGmAmGmAmAmUfAmCfUmGmUmsCmsCm |
| siAPb2U-M3SP1 | 373 | GmsGmsAmCmAmGmUmAmUfUfCfUmCmAmGmUmGmCmUmCmAm |
| | 374 | P1-UmsGfsAmGmCmAfCmUmGmAmGmAmAmUfAmCfUmGmUmCmCmsCmsUm |

**Table 3 The sequences of third siRNAs in the conjugates of the present disclosure**

| siRNA NO. | SEQ ID NO: | Sequence direction 5'-3' |
|---|---|---|
| siAPc1 | 33 | UAUUCUCAGUGCUCUCCUA |
| | 34 | UAGGAGAGCACUGAGAAUACU |

(continued)

| siRNA NO. | SEQ ID NO: | Sequence direction 5'-3' |
|---|---|---|
| siAPc2 | 35 | AGUAUUCUCAGUGCUCUCCUA |
| | 36 | UAGGAGAGCACUGAGAAUACUGU |
| siAPc1-M1 | 85 | UmAmUmUmCfUmCfAfGfUmGmCmUmCmUmCmCmUmAm |
| | 86 | UmAfGmGmAmGfAmGfCfAmCmUmGmAfGmAfAmUmAmCmUm |
| siAPc2-M1 | 87 | AmGmUmAmUmUmCfUmCfAfGfUmGmCmUmCmUmCmCmUmAm |
| | 88 | UmAfGmGmAmGfAmGfCfAmCmUmGmAfGmAfAmUmAmCmUmGmUm |
| siAPc1-M2 | 89 | UmAmUmUmCfUmCfAfGfUmGmCmUmCmUmCmCmUmAm |
| | 90 | UmAfGmGmAmGfAmGmCmAmCmUmGmAfGmAfAmUmAmCmUm |
| siAPc2-M2 | 91 | AmGmUmAmUmUmCfUmCfAfGfUmGmCmUmCmUmCmCmUmAm |
| | 92 | UmAfGmGmAmGfAmGmCmAmCmUmGmAfGmAfAmUmAmCmUmGmUm |
| siAPc1-M3 | 93 | UmAmUmUmCmUmCfAfGfUmGmCmUmCmUmCmCmUmAm |
| | 94 | UmAfGmGmAmGfAmGmCmAmCmUmGmAfGmAfAmUmAmCmUm |
| siAPc2-M3 | 95 | AmGmUmAmUmUmCmUmCfAfGfUmGmCmUmCmUmCmCmUmAm |
| | 96 | UmAfGmGmAmGfAmGmCmAmCmUmGmAfGmAfAmUmAmCmUmGmUm |
| siAPc1-M1S | 145 | UmsAmsUmUmCfUmCfAfGfUmGmCmUmCmUmCmCmUmAm |
| | 146 | UmsAfsGmGmAmGfAmGfCfAmCmUmGmAfGmAfAmUmAmsCmsUm |
| siAPc2-M1S | 147 | AmsGmsUmAmUmUmCfUmCfAfGfUmGmCmUmCmUmCmCmUmAm |
| | 148 | UmsAfsGmGmAmGfAmGfCfAmCmUmGmAfGmAfAmUmAmCmUmsGmsUm |
| siAPc1-M2S | 149 | UmsAmsUmUmCfUmCfAfGfUmGmCmUmCmUmCmCmUmAm |
| | 150 | UmsAfsGmGmAmGfAmGmCmAmCmUmGmAfGmAfAmUmAmsCmsUm |
| siAPc2-M2S | 151 | AmsGmsUmAmUmUmCfUmCfAfGfUmGmCmUmCmUmCmCmUmAm |
| | 152 | UmsAfsGmGmAmGfAmGmCmAmCmUmGmAfGmAfAmUmAmCmUmsGmsUm |

(continued)

| siRNA NO. | SEQ ID NO: | Sequence direction 5'-3' |
|---|---|---|
| siAPc1-M3S | 153 | UmsAmsUmUmCmUmCfAfGfUmGmCmUmCmUmCmCmUmAm |
| | 154 | UmsAfsGmGmAmGfAmGmCmAmCmUmGmAfGmAfAmUmAmsCmsUm |
| siAPc2-M3S | 155 | AmsGmsUmAmUmUmCmUmCfAfGfUmGmCmUmCmUmCmCmUmAm |
| | 156 | UmsAfsGmGmAmGfAmGmCmAmCmUmGmAfGmAfAmUmAmCmUmsGmsUm |
| siAPc1-M1P1 | 229 | UmAmUmUmCfUmCfAfGfUmGmCmUmCmUmCmCmUmAm |
| | 230 | P1-UmAfGmGmAmGfAmGfCfAmCmUmGmAfGmAfAmUmAmCmUm |
| siAPc2-M1P1 | 231 | AmGmUmAmUmUmCfUmCfAfGfUmGmCmUmCmUmCmCmUmAm |
| | 232 | P1-UmAfGmGmAmGfAmGfCfAmCmUmGmAfGmAfAmUmAmCmUmGmUm |
| siAPc1-M2P1 | 233 | UmAmUmUmCfUmCfAfGfUmGmCmUmCmUmCmCmUmAm |
| | 234 | P1-UmAfGmGmAmGfAmGmCmAmCmUmGmAfGmAfAmUmAmCmUm |
| siAPc2-M2P1 | 235 | AmGmUmAmUmUmCfUmCfAfGfUmGmCmUmCmUmCmCmUmAm |
| | 236 | P1-UmAfGmGmAmGfAmGmCmAmCmUmGmAfGmAfAmUmAmCmUmGmUm |
| siAPc1-M3P1 | 237 | UmAmUmUmCmUmCfAfGfUmGmCmUmCmUmCmCmUmAm |
| | 238 | P1-UmAfGmGmAmGfAmGmCmAmCmUmGmAfGmAfAmUmAmCmUm |
| siAPc2-M3P1 | 239 | AmGmUmAmUmUmCmUmCfAfGfUmGmCmUmCmUmCmCmUmAm |
| | 240 | P1-UmAfGmGmAmGfAmGmCmAmCmUmGmAfGmAfAmUmAmCmUmGmUm |
| siAPc1-M1SP1 | 241 | UmsAmsUmUmCfUmCfAfGfUmGmCmUmCmUmCmCmUmAm |
| | 242 | P1-UmsAfsGmGmAmGfAmGfCfAmCmUmGmAfGmAfAmUmAmsCmsUm |

(continued)

| siRNA NO. | SEQ ID NO: | Sequence direction 5'-3' |
|---|---|---|
| siAPc2-M1SP1 | 243 | AmsGmsUmAmUmUmCfUmCfAfGfUmGmCmUmCmUmCmCmUmAm |
| | 244 | P1-UmsAfsGmGmAmGfAmGfCfAmCmUmGmAfGmAfAmUmAmCmUmsGmsUm |
| siAPc1-M2SP1 | 245 | UmsAmsUmUmCfUmCfAfGfUmGmCmUmCmUmCmCmUmAm |
| | 246 | P1-UmsAfsGmGmAmGfAmGmCmAmCmUmGmAfGmAfAmUmAmsCmsUm |
| siAPc2-M2SP1 | 247 | AmsGmsUmAmUmUmCfUmCfAfGfUmGmCmUmCmUmCmCmUmAm |
| | 248 | P1-UmsAfsGmGmAmGfAmGmCmAmCmUmGmAfGmAfAmUmAmCmUmsGmsUm |
| siAPc1-M3SP1 | 249 | UmsAmsUm UmCmUmCfAfGfUmGmCmUmCmUmCmCmUmAm |
| | 250 | P1-UmsAfsGmGmAmGfAmGmCmAmCmUmGmAfGmAfAmUmAmsCmsUm |
| siAPc2-M3SP1 | 251 | AmsGmsUmAmUmUmCmUmCfAfGfUmGmCmUmCmUmCmCmUmAm |
| | 252 | P1-UmsAfsGmGmAmGfAmGmCmAmCmUmGmAfGmAfAmUmAmCmUmsGmsUm |

**Table 4 The sequences of fourth siRNAs in the conjugates of the present disclosure**

| siRNA NO. | SEQ ID NO: | Sequence direction 5'-3' |
|---|---|---|
| siAPd1 | 45 | AGUAUUCUCAGUGCUCUCC |
| | 46 | GGAGAGCACUGAGAAUACUGU |
| siAPd2 | 47 | ACAGUAUUCUCAGUGCUCUCC |
| | 48 | GGAGAGCACUGAGAAUACUGUCC |
| siAPd1-M1 | 97 | AmGmUmAmUfUmCfUfCfAmGmUmGmCmUmCmUmCmCm |
| | 98 | GmGfAmGmAmGfCmAfCfUmGmAmGmAfAmUfAmCmUmGmUm |
| siAPd2-M1 | 99 | AmCmAmGmUmAmUfUmCfUfCfAmGmUmGmCmUmCmUmCmCm |
| | 100 | GmGfAmGmAmGfCmAfCfUmGmAmGmAfAmUfAmCmUmGmUmCmCm |

(continued)

| siRNA NO. | SEQ ID NO: | Sequence direction 5'-3' |
|---|---|---|
| siAPd1-M2 | 101 | AmGmUmAmUfUmCfUfCfAmGmUmGmCmUmCmUmCmCm |
| | 102 | GmGfAmGmAmGfCmAmCmUmGmAmGmAfAmUfAmCmUmGmUm |
| siAPd2-M2 | 103 | AmCmAmGmUmAmUfUmCfUfCfAmGmUmGmCmUmCmUmCmCm |
| | 104 | GmGfAmGmAmGfCmAmCmUmGmAmGmAfAmUfAmCmUmGmUmCmCm |
| siAPd1-M3 | 105 | AmGmUmAmUmUmCfUfCfAmGmUmGmCmUmCmUmCmCm |
| | 106 | GmGfAmGmAmGfCmAmCmUmGmAmGmAfAmUfAmCmUmGmUm |
| siAPd2-M3 | 107 | AmCmAmGmUmAmUmUmCfUfCfAmGmUmGmCmUmCmUmCmCm |
| | 108 | GmGfAmGmAmGfCmAmCmUmGmAmGmAfAmUfAmCmUmGmUmCmCm |
| siAPd1-M1S | 157 | AmsGmsUmAmUfUmCfUfCfAmGm UmGmCmUmCmUmCmCm |
| | 158 | GmsGfsAmGmAmGfCmAfCfUmGmAmGmAfAmUfAmCmUmsGmsUm |
| siAPd2-M1S | 159 | AmsCmsAmGmUmAmUfUmCfUfCfAmGmUmGmCmUmCmUmCmCm |
| | 160 | GmsGfsAmGmAmGfCmAfCfUmGmAmGmAfAmUfAmCmUmGmUmsCmsCm |
| siAPd1-M2S | 161 | AmsGmsUmAmUmAmUfUmCfUfCfAmGmUmGmCmUmCmUmCmCm |
| | 162 | GmsGfsAmGmAmGfCmAmCmUmGmAmGmAfAmUfAmCmUmsGmsUm |
| siAPd2-M2S | 163 | AmsCmsAmGmUmAmUfUmCfUfCfAmGmUmGmCmUmCmUmCmCm |
| | 164 | GmsGfsAmGmAmGfCmAmCmUmGmAmGmAfAmUfAmCmUmGmUmsCmsCm |
| siAPd1-M3S | 165 | AmsGmsUmAmUmUmCfUfCfAmGmUmGmCmUmCmUmCmCm |
| | 166 | GmsGfsAmGmAmGfCmAmCmUmGmAmGmAfAmUfAmCmUmsGmsUm |
| siAPd2-M3S | 167 | AmsCmsAmGmUmAmUmUmCfUfCfAmGmUmGmCmUmCmUmCmCm |
| | 168 | GmsGfsAmGmAmGfCmAmCmUmGmAmGmAfAmUfAmCmUmGmUmsCmsCm |

(continued)

| siRNA NO. | SEQ ID NO: | Sequence direction 5'-3' |
|---|---|---|
| siAPd1-M1P1 | 253 | AmGmUmAmUfUmCfUfCfAmGmUmGmCmUmCmUmCmCm |
| | 254 | P1-GmGfAmGmAmGfCmAfCfUmGmAmGmAfAmUfAmCmUmGmUm |
| siAPd2-M1P1 | 255 | AmCmAmGmUmAmUfUmCfUfCfAmGmUmGmCmUmCmUmCmCm |
| | 256 | P1-GmGfAmGmAmGfCmAfCfUmGmAmGmAfAmUfAmCmUmGmUmCmCm |
| siAPd1-M2P1 | 257 | AmGmUmAmUfUmCfUfCfAmGmUmGmCmUmCmUmCmCm |
| | 258 | P1-GmGfAmGmAmGfCmAmCmUmGmAmGmAfAmUfAmCmUmGmUm |
| siAPd2-M2P1 | 259 | AmCmAmGmUmAmUfUmCfUfCfAmGmUmGmCmUmCmUmCmCm |
| | 260 | P1-GmGfAmGmAmGfCmAmCmUmGmAmGmAfAmUfAmCmUmGmUmCmCm |
| siAPd1-M3P1 | 261 | AmGmUmAmUmUmCfUfCfAmGmUmGmCmUmCmUmCmCm |
| | 262 | P1-GmGfAmGmAmGfCmAmCmUmGmAmGmAfAmUfAmCmUmGmUm |
| siAPd2-M3P1 | 263 | AmCmAmGmUmAmUmUmCfUfCfAmGmUmGmCmUmCmUmCmCm |
| | 264 | P1-GmGfAmGmAmGfCmAmCmUmGmAmGmAfAmUfAmCmUmGmUmCmCm |
| siAPd1-M1SP1 | 265 | AmsGmsUmAmUfUmCfUfCfAmGmUmGmCmUmCmUmCmCm |
| | 266 | P1-GmsGfsAmGmAmGfCmAfCfUmGmAmGmAfAmUfAmCmUmsGmsUm |
| siAPd2-M1SP1 | 267 | AmsCmsAmGmUmAmUfUmCfUfCfAmGmUmGmCmUmCmUmCmCm |
| | 268 | P1-GmsGfsAmGmAmGfCmAfCfUmGmAmGmAfAmUfAmCmUmGmUmsCmsCm |

(continued)

| siRNA NO. | SEQ ID NO: | Sequence direction 5'-3' |
|---|---|---|
| siAPd1-M2SP1 | 269 | AmsGmsUmAmUfUmCfUfCfAmGm UmGmCmUmCmUmCmCm |
| | 270 | P1-GmsGfsAmGmAmGfCmAmCmUmGmAmGmAfAmUfAmCmUmsGmsUm |
| siAPd2-M2SP1 | 271 | AmsCmsAmGmUmAmUfUmCfUfCfAmGmUmGmCmUmCmUmCmCm |
| | 272 | P1-GmsGfsAmGmAmGfCmAmCmUmGmAmGmAfAmUfAmCmUmGmUmsCmsCm |
| siAPd1-M3SP1 | 273 | AmsGmsUmAmUmUmCfUfCfAmGm UmGmCmUmCmUmCmCm |
| | 274 | P1-GmsGfsAmGmAmGfCmAmCmUmGmAmGmAfAmUfAmCmUmsGmsUm |
| siAPd2-M3SP1 | 275 | AmsCmsAmGmUmAmUmUmCfUfCfAmGmUmGmCmUmCmUmCmCm |
| | 276 | P1-GmsGfsAmGmAmGfCmAmCmUmGmAmGmAfAmUfAmCmUmGmUmsCmsCm |
| siAPd1 U-M1P1 | 375 | AmGmUmAmUfUmCfUfCfAmGmUmGmCmUmCmUmCmAm |
| | 376 | P1-UmGfAmGmAmGfCmAfCfUmGmAmGmAfAmUfAmCmUmGmUm |
| siAPd2U-M1P1 | 377 | AmCmAmGmUmAmUfUmCfUfCfAmGmUmGmCmUmCmUmCmAm |
| | 378 | P1-UmGfAmGmAmGfCmAfCfUmGmAmGmAfAmUfAmCmUmGmUmCmCm |
| siAPd1 U-M2P1 | 379 | AmGmUmAmUfUmCfUfCfAmGmUmGmCmUmCmUmCmAm |
| | 380 | P1-UmGfAmGmAmGfCmAmCmUmGmAmGmAfAmUfAmCmUmGmUm |
| siAPd2U-M2P1 | 381 | AmCmAmGmUmAmUfUmCfUfCfAmGmUmGmCmUmCmUmCmAm |
| | 382 | P1-UmGfAmGmAmGfCmAmCmUmGmAmGmAfAmUfAmCmUmGmUmCmCm |

(continued)

| siRNA NO. | SEQ ID NO: | Sequence direction 5'-3' |
|---|---|---|
| siAPd1 U -M3P1 | 383 | AmGmUmAmUmUmCfUfCfAmGmUmGmCmUmCmUmCmAm |
| | 384 | P1-UmGfAmGmAmGfCmAmCmUmGmAmGmAfAmUfAmCmUmGmUm |
| siAPd2U -M3P1 | 385 | AmCmAmGmUmAmUmUmCfUfCfAmGmUmGmCmUmCmUmCmAm |
| | 386 | P1-UmGfAmGmAmGfCmAmCmUmGmAmGmAfAmUfAmCmUmGmUmCmCm |
| siAPd1 U -M1SP1 | 387 | AmsGmsUmAmUfUmCfUfCfAmGm UmGmCmUmCmUmCmAm |
| | 388 | P1-UmsGfsAmGmAmGfCmAfCfUmGmAmGmAfAmUfAmCmUmsGmsUm |
| siAPd2U -M1SP1 | 389 | AmsCmsAmGmUmAmUfUmCfUfCfAmGmUmGmCmUmCmUmCmAm |
| | 390 | P1-UmsGfsAmGmAmGfCmAfCfUmGmAmGmAfAmUfAmCmUmGmUmsCmsCm |
| siAPd1 U -M2SP1 | 391 | AmsGmsUmAmUfUmCfUfCfAmGmUmGmCmUmCmUmCmAm |
| | 392 | P1-UmsGfsAmGmAmGfCmAmCmUmGmAmGmAfAmUfAmCmUmsGmsUm |
| siAPd2U -M2SP1 | 393 | AmsCmsAmGmUmAmUfUmCfUfCfAmGmUmGmCmUmCmUmCmAm |
| | 394 | P1-UmsGfsAmGmAmGfCmAmCmUmGmAmGmAfAmUfAmCmUmGmUmsCmsCm |
| siAPd1 U -M3SP1 | 395 | AmsGmsUmAmUmUmCfUfCfAmGmUmGmCmUmCmUmCmAm |
| | 396 | P1-UmsGfsAmGmAmGfCmAmCmUmGmAmGmAfAmUfAmCmUmsGmsUm |
| siAPd2U -M3SP1 | 397 | AmsCmsAmGmUmAmUmUmCfUfCfAmGmUmGmCmUmCmUmCmAm |
| | 398 | P1-UmsGfsAmGmAmGfCmAmCmUmGmAmGmAfAmUfAmCmUmGmUmsCmsCm |

**Table 5 The sequences of fifth siRNAs in the conjugates of the present disclosure**

| siRNA NO. | SEQ ID NO: | Sequence direction 5'-3' |
|---|---|---|
| siAPe1 | 57 | GGACAGUAUUCUCAGUGCU |
| | 58 | AGCACUGAGAAUACUGUCCCU |
| siAPe2 | 59 | AGGGACAGUAUUCUCAGUGCU |
| | 60 | AGCACUGAGAAUACUGUCCCUUU |
| siAPe1-M1 | 109 | GmGmAmCmAfGmUfAfUfUmCmUmCmAmGmUmGmCmUm |
| | 110 | AmGfCmAmCmUfGmAfGfAmAmUmAmCfUmGfUmCmCmCmUm |
| siAPe2-M1 | 111 | AmGmGmGmAmCmAfGmUfAfUfUmCmUmCmAmGmUmGmCmUm |
| | 112 | AmGfCmAmCmUfGmAfGfAmAmUmAmCfUmGfUmCmCmCmUmUmUm |
| siAPe1-M2 | 113 | GmGmAmCmAfGmUfAfUfUmCmUmCmAmGmUmGmCmUm |
| | 114 | AmGfCmAmCmUfGmAmGmAmAmUmAmCfUmGfUmCmCmCmUm |
| siAPe2-M2 | 115 | AmGmGmGmAmCmAfGmUfAfUfUmCmUmCmAmGmUmGmCmUm |
| | 116 | AmGfCmAmCmUfGmAmGmAmAmUmAmCfUmGfUmCmCmCmUmUmUm |
| siAPe1-M3 | 117 | GmGmAmCmAmGmUfAfUfUmCmUmCmAmGmUmGmCmUm |
| | 118 | AmGfCmAmCmUfGmAmGmAmAmUmAmCfUmGfUmCmCmCmUm |
| siAPe2-M3 | 119 | AmGmGmGmAmCmAmGmUfAfUfUmCmUmCmAmGmUmGmCmUm |
| | 120 | AmGfCmAmCmUfGmAmGmAmAmUmAmCfUmGfUmCmCmCmUmUmUm |
| siAPe1-M1S | 169 | GmsGmsAmCmAfGmUfAfUfUmCmUmCmAmGmUmGmCmUm |
| | 170 | AmsGfsCmAmCmUfGmAfGfAmAmUmAmCfUmGfUmCmCmsCmsUm |
| siAPe2-M1S | 171 | AmsGmsGmGmAmCmAfGmUfAfUfUmCmUmCmAmGmUmGmCmUm |
| | 172 | AmsGfsCmAmCmUfGmAfGfAmAmUmAmCfUmGfUmCmCmCmUmsUmsUm |
| siAPe1-M2S | 173 | GmsGmsAmCmAfGmUfAfUfUmCmUmCmAmGmUmGmCmUm |
| | 174 | AmsGfsCmAmCmUfGmAmGmAmAmUmAmCfUmGfUmCmCmsCmsUm |

(continued)

| siRNA NO. | SEQ ID NO: | Sequence direction 5'-3' |
|---|---|---|
| siAPe2-M2S | 175 | AmsGmsGmGmAmCmAfGmUfAfUfUmCmUmCmAmGmUmGmCmUm |
| | 176 | AmsGfsCmAmCmUfGmAmGmAmAmUmAmCfUmGfUmCmCmCmUmsUmsUm |
| siAPe1-M3S | 177 | GmsGmsAmCmAmGmUfAfUfUmCmUmCmAmGmUmGmCmUm |
| | 178 | AmsGfsCmAmCmUfGmAmGmAmAmUmAmCfUmGfUmCmCmsCmsUm |
| siAPe2-M3S | 179 | AmsGmsGmGmAmCmAmGmUfAfUfUmCmUmCmAmGmUmGmCmUm |
| | 180 | AmsGfsCmAmCmUfGmAmGmAmAmUmAmCfUmGfUmCmCmCmUmsUmsUm |
| siAPe1-M1P1 | 277 | GmGmAmCmAfGmUfAfUfUmCmUmCmAmGmUmGmCmUm |
| | 278 | P1-AmGfCmAmCmUfGmAfGfAmAmUmAmCfUmGfUmCmCmCmUm |
| siAPe2-M1P1 | 279 | AmGmGmGmAmCmAfGmUfAfUfUmCmUmCmAmGmUmGmCmUm |
| | 280 | P1-AmGfCmAmCmUfGmAfGfAmAmUmAmCfUmGfUmCmCmCmUmUmUm |
| siAPe1-M2P1 | 281 | GmGmAmCmAfGmUfAfUfUmCmUmCmAmGmUmGmCmUm |
| | 282 | P1-AmGfCmAmCmUfGmAmGmAmAmUmAmCfUmGfUmCmCmCmUm |
| siAPe2-M2P1 | 283 | AmGmGmGmAmCmAfGmUfAfUfUmCmUmCmAmGmUmGmCmUm |
| | 284 | P1-AmGfCmAmCmUfGmAmGmAmAmUmAmCfUmGfUmCmCmCmUmUmUm |
| siAPe1-M3P1 | 285 | GmGmAmCmAmGmUfAfUfUmCmUmCmAmGmUmGmCmUm |
| | 286 | P1-AmGfCmAmCmUfGmAmGmAmAmUmAmCfUmGfUmCmCmCmUm |
| siAPe2-M3P1 | 287 | AmGmGmGmAmCmAmGmUfAfUfUmCmUmCmAmGmUmGmC |

(continued)

| siRNA NO. | SEQ ID NO: | Sequence direction 5'-3' |
|---|---|---|
| | | mUm |
| | 288 | P1-AmGfCmAmCmUfGmAmGmAmAmUmAmCfUmGfUmCmCmCmUmUmUm |
| siAPe1-M1SP1 | 289 | GmsGmsAmCmAfGmUfAfUfUmCmUmCmAmGmUmGmCmUm |
| | 290 | P1-AmsGfsCmAmCmUfGmAfGfAmAmUmAmCfUmGfUmCmCmsCmsUm |
| siAPe2-M1SP1 | 291 | AmsGmsGmGmAmCmAfGmUfAfUfUmCmUmCmAmGmUmGmCmUm |
| | 292 | P1-AmsGfsCmAmCmUfGmAfGfAmAmUmAmCfUmGfUmCmCmCmUmsUmsUm |
| siAPe1-M2SP1 | 293 | GmsGmsAmCmAfGmUfAfUfUmCmUmCmAmGmUmGmCmUm |
| | 294 | P1-AmsGfsCmAmCmUfGmAmGmAmAmUmAmCfUmGfUmCmCmsCmsUm |
| siAPe2-M2SP1 | 295 | AmsGmsGmGmAmCmAfGmUfAfUfUmCmUmCmAmGmUmGmCmUm |
| | 296 | P1-AmsGfsCmAmCmUfGmAmGmAmAmUmAmCfUmGfUmCmCmCmUmsUmsUm |
| siAPe1-M3SP1 | 297 | GmsGmsAmCmAmGmUfAfUfUmCmUmCmAmGmUmGmCmUm |
| | 298 | P1-AmsGfsCmAmCmUfGmAmGmAmAmUmAmCfUmGfUmCmCmsCmsUm |
| siAPe2-M3SP1 | 299 | AmsGmsGmGmAmCmAmGmUfAfUfUmCmUmCmAmGmUmGmCmUm |
| | 300 | P1-AmsGfsCmAmCmUfGmAmGmAmAmUmAmCfUmGfUmCmCmCmUmsUmsUm |
| siAPe1 U -M1P1 | 399 | GmGmAmCmAfGmUfAfUfUmCmUmCmAmGmUmGmCmAm |
| | 400 | P1-UmGfCmAmCmUfGmAfGfAmAmUmAmCfUmGfUmCmCmCmUm |

(continued)

| siRNA NO. | SEQ ID NO: | Sequence direction 5'-3' |
|---|---|---|
| siAPe2U -M1P1 | 401 | AmGmGmGmAmCmAfGmUfAfUfUmCmUmCmAmGmUmGmCmAm |
| | 402 | P1-UmGfCmAmCmUfGmAfGfAmAmUmAmCfUmGfUmCmCmCmUmUmUm |
| siAPe1 U -M2P1 | 403 | GmGmAmCmAfGmUfAfUfUmCmUmCmAmGmUmGmCmAm |
| | 404 | P1-UmGfCmAmCmUfGmAmGmAmAmUmAmCfUmGfUmCmCmCmUm |
| siAPe2U -M2P1 | 405 | AmGmGmGmAmCmAfGmUfAfUfUmCmUmCmAmGmUmGmCmAm |
| | 406 | P1-UmGfCmAmCmUfGmAmGmAmAmUmAmCfUmGfUmCmCmCmUmUmUm |
| siAPe1 U -M3P1 | 407 | GmGmAmCmAmGmUfAfUfUmCmUmCmAmGmUmGmCmAm |
| | 408 | P1-UmGfCmAmCmUfGmAmGmAmAmUmAmCfUmGfUmCmCmCmUm |
| siAPe2U -M3P1 | 409 | AmGmGmGmAmCmAmGmUfAfUfUmCmUmCmAmGmUmGmCmAm |
| | 410 | P1-UmGfCmAmCmUfGmAmGmAmAmUmAmCfUmGfUmCmCmCmUmUmUm |
| siAPe1 U -M1SP1 | 411 | GmsGmsAmCmAfGmUfAfUfUmCmUmCmAmGmUmGmCmAm |
| | 412 | P1-UmsGfsCmAmCmUfGmAfGfAmAmUmAmCfUmGfUmCmCmsCmsUm |
| siAPe2U -M1SP1 | 413 | AmsGmsGmGmAmCmAfGmUfAfUfUmCmUmCmAmGmUmGmCmAm |
| | 414 | P1-UmsGfsCmAmCmUfGmAfGfAmAmUmAmCfUmGfUmCmCmCmUmsUmsUm |
| siAPe1U -M2SP1 | 415 | GmsGmsAmCmAfGmUfAfUfUmCmUmCmAmGmUmGmCmAm |
| | 416 | P1-UmsGfsCmAmCmUfGmAmGmAmAmUmAmCfUmGfUmCmCmsCmsUm |

(continued)

| siRNA NO. | SEQ ID NO: | Sequence direction 5'-3' |
|---|---|---|
| siAPe2U -M2SP1 | 417 | AmsGmsGmGmAmCmAfGmUfAfUfUmCmUmCmAmGmUmGmCmAm |
| | 418 | P1-UmsGfsCmAmCmUfGmAmGmAmAmUmAmCfUmGfUmCmCmCmUmsUmsUm |
| siAPe1 U -M3SP1 | 419 | GmsGmsAmCmAmGmUfAfUfUmCmUmCmAmGmUmGmCmAm |
| | 420 | P1-UmsGfsCmAmCmUfGmAmGmAmAmUmAmCfUmGfUmCmCmsCmsUm |
| siAPe2U -M3SP1 | 421 | AmsGmsGmGmAmCmAmGmUfAfUfUmCmUmCmAmGmUmGmCmAm |
| | 422 | P1-UmsGfsCmAmCmUfGmAmGmAmAmUmAmCfUmGfUmCmCmCmUmsUmsUm |

In the siRNA or siRNA conjugate of the present disclosure, each pair of adjacent nucleotides is linked via a phosphodiester bond or phosphorothioate diester bond. The non-bridging oxygen or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as a metal cation, an ammonium cation $NH_4^+$ or an organic ammonium cation. In order to increase solubility, in one embodiment, the cation is one or more selected from an alkali metal cation, an ammonium cation formed by a tertiary amine and a quaternary ammonium cation. The alkali metal ion may be $K^+$ and/or $Na^+$, and the cation formed by a tertiary amine may be an ammonium cation formed by triethylamine and/or an ammonium cation formed by N,N-diisopropylethylamine. Thus, the siRNA and the siRNA conjugate of the present disclosure can be at least partially present in the form of salt. In one embodiment, the non-bridging oxygen atom or sulfur atom in the phosphodiester bond or phosphorothioate diester bond at least partly binds to sodium ion, and thus the siRNA and the siRNA conjugate of the present disclosure are present or partially present in the form of sodium salt.

Those skilled in the art clearly know that a modified nucleotide group can be introduced into the siRNA of the present disclosure by a nucleoside monomer with a corresponding modification. The methods for preparing a nucleoside monomer having the corresponding modification and the methods for introducing a modified nucleotide into a siRNA are also well-known to those skilled in the art. All modified nucleoside monomers may be either commercially available or prepared by known methods.

## Preparation of the siRNA conjugate as shown by Formula (308)

The siRNA conjugate as shown by Formula (308) can be prepared by any appropriate synthetic routes.

In some embodiments, the siRNA conjugate as shown by Formula (308) can be prepared by the following method, comprising: sequentially linking nucleoside monomers in 3' to 5' direction according to the type and sequence of the nucleotides in the sense strand and antisense strands of the siRNA respectively, under the condition for phosphoramidite solid phase synthesis, wherein the step of linking each nucleoside monomer includes a four-step reaction of deprotection, coupling, capping, and oxidation or sulfurization; isolating the sense strand and the antisense strand of the siRNA; and annealing; wherein the siRNA is the above siRNA of the present disclosure.

Moreover, the method further comprises: contacting the compound as shown by Formula (321) with a nucleoside monomer or a nucleotide sequence attached to a solid phase support under coupling reaction condition and in the presence of a coupling agent, thereby linking the compound as shown by Formula (321) to the nucleotide sequence via a coupling reaction. Hereinafter, the compound as shown by Formula (321) is also referred to as a conjugation molecule.

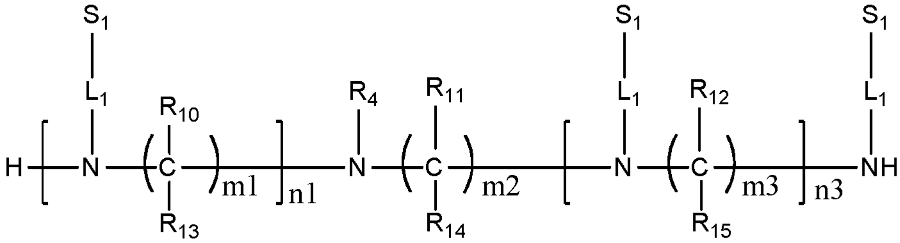

Formula (321)

wherein,

$R_4$ is a group capable of binding to the siRNA represented by Nu in the compound as shown by Formula (308). In some embodiments, $R_4$ is a group capable of binding to the siRNA represented by Nu via a covalent bond. In some embodiments, $R_4$ is a group comprising any functional group that may be conjugated to the siRNA represented by Nu via a phosphodiester bond by a reaction;

Each $S_1$ is independently a group formed by substituting all active hydroxyls in $M_1$ with the group YCOO-, wherein each Y is independently one selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl; in some embodiments, Y is methyl.

The definitions and options of n1, n3, m1, m2, m3, $R_{10}$, $R_{11}$, $R_{12}$, $R_{13}$, $R_{14}$, $R_{15}$, $L_1$, and $M_1$ are respectively as described above.

$R_4$ is selected to achieve the linkage to the N atom on a nitrogenous backbone and to provide a suitable reaction site for synthesizing the siRNA conjugate as shown by Formula (308). In some embodiments, $R_4$ comprises a $R_2$ linking group or a protected $R_2$ linking group, and a functional group than can react with a siRNA to form a structure as shown by Formula (A59).

In some embodiments, $R_4$ comprises a first functional group that can react with a group on the siRNA represented by Nu or a nucleoside monomer to form a phosphite ester, and a second functional group that can react with a hydroxy group or an amino group to form a covalent bond with, or comprises a solid phase support linked by the covalent bond. In some embodiments, the first functional group is a phosphoramidite, a hydroxy or a protected hydroxy. In some embodiments, the second functional group is a phosphoramidite, a carboxyl or a carboxylate salt. In some embodiments, the second functional group is a solid phase support linked to the rest of the molecule via a covalent bond which is formed by a hydroxy group or an amino group. In some embodiments, the solid phase support is linked via a phosphoester bond, a carboxyl ester bond or an amide bond. In some embodiments, the solid phase support is a resin.

In some embodiments, the first functional group comprises hydroxy, $-OR_k$ or a group as shown by Formula (C3); the second functional group comprises a group as shown by Formula (C1), (C2), (C3), (C1'), or (C3'):

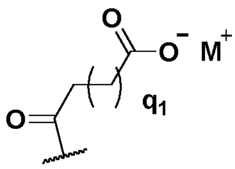
,
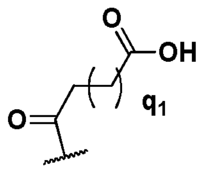
,
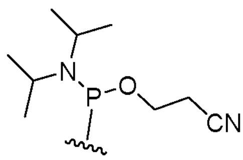
,

(C1) (C2) (C3)

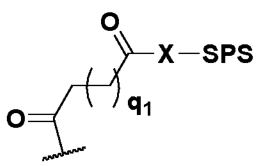
,
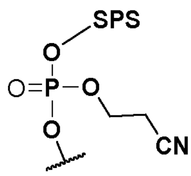
,

(C1') (C3')

wherein $q_1$ is an integer of 1-4, X is O or NH, $M^+$ is a cation, $R_k$ is a hydroxy protection group, SPS represents a solid

phase support, and represents the site where a group is attached to the rest of the molecule.

In some embodiments, the first functional group comprises a phosphoramidite group, such as the group as shown by Formula (C3). The phosphoramidite group can form a phosphite ester with a hydroxy at any position on a nucleotide (such as a 2'- hydroxy or 3'-hydroxy) by a coupling reaction, and the phosphite ester can form a phosphodiester bond or phosphorothioate ester bond as shown by Formula (A59) via oxidation or sulfurization, so as to conjugate the conjugation molecule to a siRNA. Here, even if the second functional group does not exist, the compound as shown by Formula (321) could still be conjugated to the nucleotide, while not affecting the obtaining of the siRNA conjugate as shown by Formula (308). Under such circumstances, after obtaining a sense or antisense strand of the siRNA by a method such as phosphoramidite solid phase synthesis, the compound as shown by Formula (321) is reacted with a hydroxy on the nucleotide at the terminal of the nucleotide sequence, and a phosphodiester bond linkage or a phosphorothioate bond linkage is formed in the subsequent oxidation or sulfurization process, thereby conjugating the compound as shown by Formula (321) to the siRNA.

In some embodiments, the first functional group comprises a protected hydroxy. In some embodiments, the second functional group comprises a group that can react with a solid phase support to provide a conjugation molecule comprising a solid phase support. In some embodiments, the second functional group comprises a carboxyl, a carboxylate salt or a phosphoramidite, such as the functional group as shown by Formula (C1), (C2) or (C3). When the second functional group comprises a carboxyl or a carboxylate salt, the compound of Formula (321) can react with a hydroxy or an amino group on a solid phase support (such as a resin) via esterification or amidation reaction, to form a conjugation molecule comprising a solid phase support linked via a carboxylate ester bond. When the second functional group comprises a phosphoramidite functional group, the compound of Formula (321) can couple with a hydroxy group on a universal solid phase support (such as a resin), and form a conjugation molecule comprising a solid phase support linked via a phosphodiester bond by oxidation. Next, starting from the above product linked to a solid phase support, the nucleoside monomers are linked sequentially through a phosphoramidite solid phase synthesis method, so as to obtain a sense strand or an antisense strand of the siRNA linked to a conjugation group. In the process of phosphoramidite solid phase synthesis, the first functional group is deprotected, and then coupled with a phosphoramidite group on a nucleoside monomer under coupling reaction condition.

In some embodiments, the first functional group comprises a hydroxy or a protected hydroxy group; the second functional group comprises a solid phase support linked via a carboxylate ester bond, an amide bond, or a phosphoester bond, as shown by Formula (C1') or (C3'). In this case, starting from the compound of Formula (321) in place of a solid phase support, the nucleoside monomers are linked sequentially through a phosphoramidite solid phase synthesis method, so as to obtain a sense strand or an antisense strand of the siRNA linked to a conjugation group.

In some embodiments, the carboxylate may be expressed as $-COO^-M^+$, wherein $M^+$ is a cation such as one selected from a metal cation, an ammonium cation $NH_4^+$ and an organic ammonium cation. In one embodiment, the metal cation may be an alkali metal cation, such as $K^+$ or $Na^+$. In order to increase solubility and facilitate the reaction, in some embodiments, the organic ammonium cation is an ammonium cation formed by a tertiary amine or a quaternary ammonium cation, such as an ammonium cation formed by triethylamine or an ammonium cation formed by N,N-diisopropylethylamine. In some embodiments, the carboxylate is a triethylamine carboxylate or an N,N-diisopropylethylamine carboxylate.

In some embodiments, $R_4$ comprises the structure as shown by Formula (B9), (B10), (B9'), (B10'), (B11), (B12), (B11'), or B(12'):

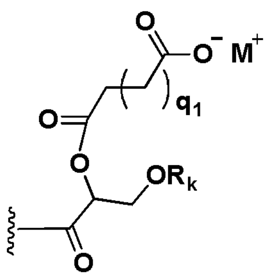
,
(B9)

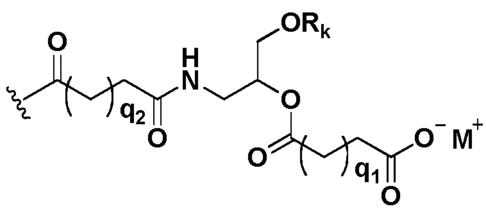
,
(B10)

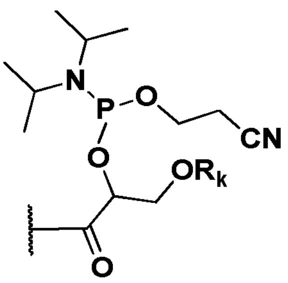
,
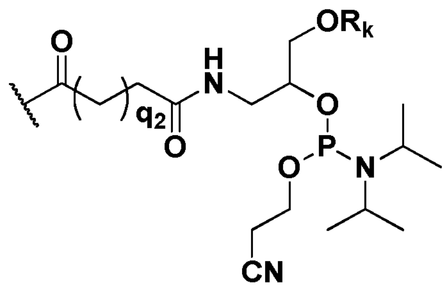
,

(B9') (B10')

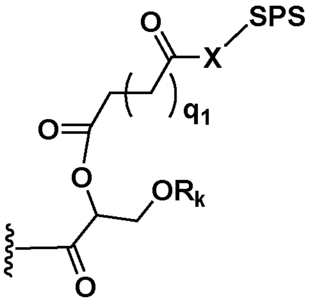
,
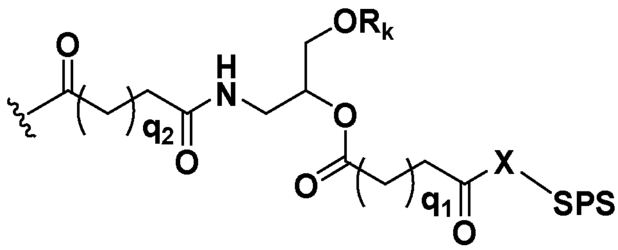
,

(B11) (B12)

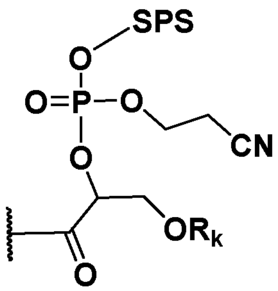
,
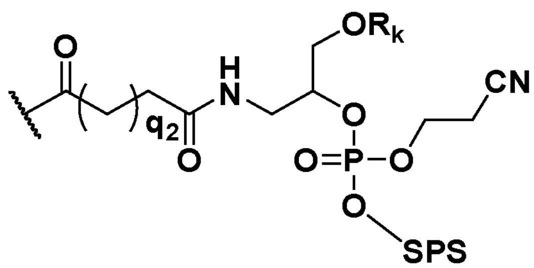
,

(B11') (B12')

wherein $q_1$ is an integer of 1-4, $q_2$ is an integer of 1-10, X is O or NH, $M^+$ is a cation, $R_k$ is a hydroxy protection group, SPS represents a solid phase support, and 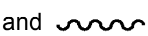 represents the site where the group is linked to the rest of the molecule. In some embodiments, $q_1$ is 1 or 2. In some embodiments, $q_2$ is an integer of 1-5. In some embodiments, $R_4$ comprises a structure as shown by Formula (B9) or (B10). In some embodiments, $R_4$ comprises a structure as shown by Formula (B11) or (B12).

In some embodiments, $R_k$ is one or more of Tr (trityl), MMTr (4-methoxytrityl), DMTr (4,4'-dimethoxytrityl), and TMTr (4,4',4'-trimethoxytrityl). In some embodiments, $R_k$ may be DMTr, i.e., 4,4'-dimethoxytrityl.

The definition of $L_1$ is as described above.

In some embodiments, $L_1$ is used to link the $M_1$ targeting group to the N atom on the nitrogenous backbone, thereby providing liver targeting function for the siRNA conjugate as shown by Formula (308). In some embodiments, $L_1$ comprises any one of Formulae (A1)-(A26), and any combination thereof.

According to the above description, those skilled in the art that would easily understand that as compared with the well-known phosphoramidite solid phase synthesis method in the art, the siRNA conjugate as shown by Formula (308) in which the conjugation molecule is linked to any possible position of the nucleotide sequence can be obtained by using the above first functional group and an optional second functional group. For example, the conjugation molecule is linked to a terminal region of the nucleotide sequence, or to a terminal of the nucleotide sequence. Correspondingly, unless otherwise specified, in the following description regarding preparation of the conjugate and/or the conjugation molecule, when referring to the reactions such as "deprotection", "coupling", "capping", "oxidation", "sulfurization", it will

be understood that the reaction conditions and agents involved in the well-known phosphoramidite solid phase synthesis method in the art would also apply to these reactions. Exemplary reaction conditions and agents will be described in detail hereinafter.

In some embodiments, each $S_1$ is independently a $M_1$. In some embodiments, each $S_1$ is independently a group formed by protecting at least one active hydroxyl group in $M_1$ with a hydroxyl protection group. In some embodiments, each $S_1$ is independently a group formed by protecting all existing active hydroxyl groups in $M_1$ with hydroxyl protection groups. In some embodiments, any hydroxyl protection group known to a skilled one may be used to protect the active hydroxyl group in $M_1$. In some embodiments, the protected hydroxy is expressed as the Formula YCOO-, wherein each Y is independently selected from the group consisting of $C_1$-$C_{10}$ alkyl and $C_6$-$C_{10}$ aryl, which is optionally substituted with one or more substituents selected from the group consisting of halo and $C_1$-$C_6$ alkyl. In some embodiments, each Y is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and $C_1$-$C_6$ alkylphenyl.

In some embodiments, each $S_1$ is independently selected from the group consisting of Formulae (A46)- (A54):

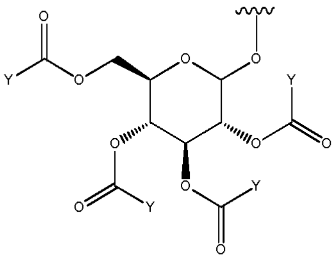
,
(A46)

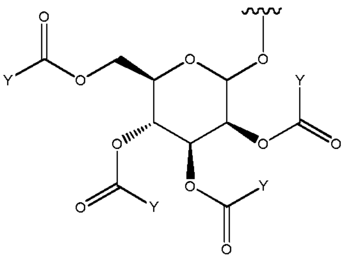
,
(A47)

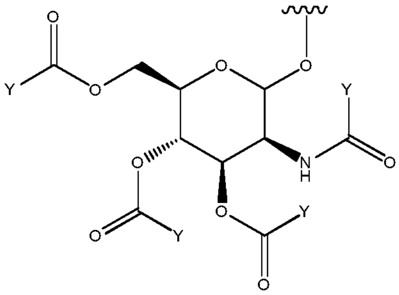
,
(A48)

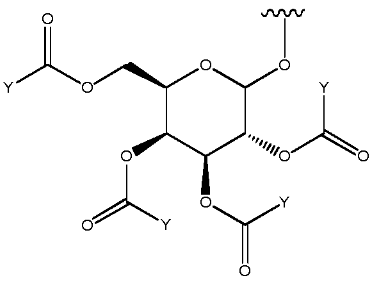
,
(A49)

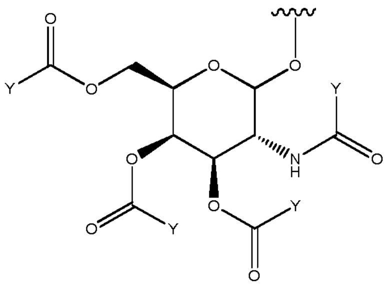
,
(A50)

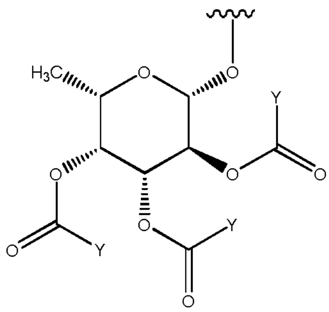
,
(A51)

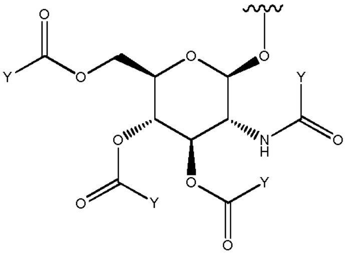
,
(A52)

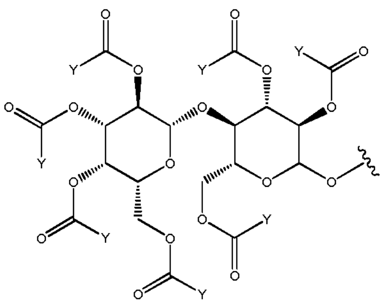
,
(A53)

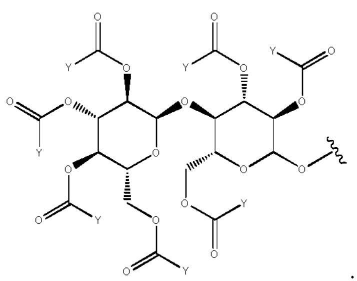
.
(A54)

In some embodiments, $S_1$ is A49 or A50.

In some embodiments, each Y is independently one selected from methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is methyl.

As mentioned above, the method for preparing the siRNA conjugate as shown by Formula (308) further comprises the following steps: synthesizing the other strand of the siRNA (for example, when a sense strand of the siRNA linked to a conjugation group is synthesized in the above step, the method further comprises synthesizing an antisense strand of the siRNA according to the solid phase synthesis method, *vice versa*), isolating the sense strand and the antisense strand, and annealing. In particular, in the step of isolating, the solid phase support linked to the nucleotide sequence and/or the conjugation molecule is cleaved, and the necessary protection group is removed (in this case, each $S_1$ group in the compound of Formula (321) is converted to the corresponding $M_1$ targeting group), to afford a sense strand (or an antisense strand) of the siRNA linked to a conjugation group and the corresponding antisense strand (or sense strand). The sense strand and the antisense strand are annealed to form a double-strand RNA structure, thereby affording the siRNA conjugate as shown by Formula (308).

In some embodiments, the method for preparing the siRNA conjugate as shown by Formula (308) comprises the following steps: contacting the compound as shown by Formula (321) with the first nucleoside monomer at 3' terminal of the sense strand or the antisense strand under coupling reaction condition in the presence of a coupling agent, thereby linking the compound as shown by Formula (321) to the first nucleotide in the sequence; sequentially linking nucleoside monomers in 3' to 5' direction to synthesize a sense or antisense strand of the siRNA according to the type and sequence of the nucleotides in the desired sense or antisense strand under the condition for phosphoramidite solid phase synthesis, wherein the compound of Formula (321) is a compound in which $R_4$ comprises a first functional group and a second functional group, wherein the first functional group comprises a protected hydroxyl and the second functional group has a structure as shown by Formula (C1') or (C3'), and the compound of (321) is deprotected before being linked to the first nucleoside monomer; and the linking of each nucleoside monomer comprises a four-step reaction of deprotection, coupling, capping, and oxidation or sulfurization; thus obtaining a sense or antisense strand of the nucleic acid linked to a conjugation group; sequentially linking nucleoside monomers in 3' to 5' direction to synthesize an antisense or sense strand of the nucleic acid according to the type and sequence of the nucleotides in the sense or antisense strand under the condition for phosphoramidite solid phase synthesis; wherein the linking of each nucleoside monomer includes a four-step reaction of deprotection, coupling, capping, and oxidation or sulfurization; removing the protection group and cleaving the solid phase support; isolating and purifying the sense strand and the antisense strand of the nucleic acid; and annealing.

In some embodiments, the method for preparing the siRNA conjugate as shown by Formula (308) comprises the following steps: according to the type and sequence of the nucleotides in the sense or antisense strand of the double-strand siRNA, sequentially linking nucleoside monomers in 3' to 5' direction to synthesize the antisense and sense strand; wherein the linking of each nucleoside monomer includes a four-step reaction of deprotection, coupling, capping, and oxidation or sulfurization, to obtain the sense strand linked to the solid phase support and the antisense strand linked to the solid phase support; contacting the compound as shown by Formula (321) with the sense strand linked to the solid phase support or the antisense strand linked to the solid phase support under coupling reaction condition in the presence of a coupling agent, thereby linking the compound as shown by Formula (321) to the sense strand or antisense strand; wherein the compound as shown by Formula (321) is a compound in which $R_4$ comprises a first functional group which is a phosphoramidite group; removing the protection group and cleaving the solid phase support; respectively isolating and purifying the sense strand or the antisense strand of the siRNA; and annealing, wherein the sense or antisense strand of the siRNA is linked to a conjugation group.

In some embodiments, the P atom in the Formula A59 is linked to the 3' terminal of the sense strand of the siRNA, and the method for preparing the siRNA conjugate as shown by Formula (308) comprises:

(1) removing the hydroxyl protection group $P_k$ in the compound of Formula (321), wherein the compound of Formula (321) is a compound in which $R_4$ comprises a first functional group comprising a protected hydroxyl $OR_k$, and a second functional group having a structure as shown by Formulas (C1') or (C3'); contacting the deprotected product with a nucleoside monomer to afford a nucleoside monomer linked to a solid phase support via a conjugation molecule under coupling reaction condition in the presence of a coupling agent;
(2) starting from the nucleoside monomer linked to a solid phase support via the conjugation molecule, synthesizing a sense strand of the siRNA in 3' to 5' direction by a phosphoramidite solid phase synthesis method;
(3) synthesizing an antisense strand of the siRNA by a phosphoramidite solid phase synthesis method; and
(4) isolating the sense strand and the antisense strand of the siRNA and annealing the same to afford the siRNA conjugate as shown by Formula (308).

Therein, in step (1), the method for removing the protection group $R_k$ in the compound of Formula (321) comprises contacting the compound of Formula (321) with a deprotection agent under deprotection condition. The deprotection condition comprises a temperature of 0-50°C, and in some embodiments, 15-35°C, and a reaction time of 30-300 seconds, and in some embodiments, 50-150 seconds. The deprotection agent may be one or more selected from trifluoroacetic acid, trichloroacetic acid, dichloroacetic acid, and monochloroacetic acid, and in some embodiments,

dichloroacetic acid. The molar ratio of the deprotection agent to the compound as shown by Formula (321) is 10:1 to 1000:1, and in some embodiments, 50:1 to 500:1.

The coupling reaction condition and the coupling agent may be any condition and agent suitable for the above coupling reaction. In some embodiments, the same condition and agent as those of the coupling reaction in the solid phase synthesis method can be used.

In some embodiments, the coupling reaction condition comprises a reaction temperature of 0-50°C, and in some embodiments, 15-35°C. The molar ratio of the compound of Formula (321) to the nucleoside monomer is 1:1 to 1:50, and in some embodiments, 1:2 to 1:5. The molar ratio of the compound of Formula (321) to the coupling agent may be 1:1 to 1:50, and in some embodiments, 1:3 to 1:10. The reaction time is 200-3,000 seconds, and in some embodiments, 500-1,500 seconds. The coupling agent is one or more selected from 1H-tetrazole, 5-ethylthio-1H-tetrazole and 5-benzylthio-1H-tetrazole, and in some embodiments, is 5-ethylthio-1H-tetrazole. The coupling reaction may be performed in an organic solvent. The organic solvent is one or more selected from anhydrous acetonitrile, anhydrous DMF and anhydrous dichloromethane, and in some embodiments, is anhydrous acetonitrile. With respect to the compound as shown by Formula (321), the amount of the organic solvent is 3-50 L/mol, and in some embodiments, 5-20 L/mol.

In step (2), starting from the nucleoside monomer linked to a solid phase support via a conjugation molecule prepared in the above steps, a sense strand S of the second siRNA conjugate is synthesized in 3' to 5' direction by the phosphoramidite solid phase synthesis method. In this case, the conjugation molecule is linked to 3' terminal of the resultant sense strand.

Other conditions for the solid phase synthesis in steps (2) and (3), including the deprotection condition for the nucleoside monomer, the type and amount of the deprotection agent, the coupling reaction condition, the type and amount of the coupling agent, the capping reaction condition, the type and amount of the capping agent, the oxidation reaction condition, the type and amount of the oxidation agent, the sulfurization reaction condition, and the type and amount of the sulfurization agent, adopt various conventional agents, amounts, and conditions in the art.

In some embodiments, for example, the solid phase synthesis in steps (2) and (3) can use the following conditions:

The deprotection condition for the nucleoside monomer comprises a reaction temperature of 0-50°C, and in some embodiments, 15-35°C, and a reaction time of 30-300 seconds, and in some embodiments, 50-150 seconds. The deprotection agent may be one or more selected from trifluoroacetic acid, trichloroacetic acid, dichloroacetic acid, and monochloroacetic acid, and in some embodiments, is dichloroacetic acid. The molar ratio of the deprotection agent to the protection group 4,4'-dimethoxytrityl on the solid phase support is 2:1 to 100:1, and in some embodiments, 3:1 to 50:1.

The coupling reaction condition comprises a reaction temperature of 0-50°C, and in some embodiments, 15-35°C. The molar ratio of the nucleic acid sequence linked to the solid phase support to the nucleoside monomer is 1:1 to 1:50, and in some embodiments, 1:5 to 1:15. The molar ratio of the nucleic acid sequence linked to the solid phase support to the coupling agent is 1:1 to 1:100, and in some embodiments, 1:50 to 1:80. The selection of the reaction time and the coupling agent is the same as above.

The capping reaction condition comprises a reaction temperature of 0-50°C, and in some embodiments, 15-35 °C, and a reaction time of 5-500 seconds, and in some embodiments, 10-100 seconds. The selection of the capping agent is the same as above. The molar ratio of the total amount of the capping agent to the nucleic acid sequence linked to the solid phase support is 1:100 to 100:1, and in some embodiments, is 1:10 to 10:1. In the case where the capping agent uses equimolar acetic anhydride and N-methylimidazole, the molar ratio of acetic anhydride, N-methylimidazole, and the nucleic acid sequence linked to the solid phase support may be 1:1:10 - 10:10:1, and in some embodiments, is 1:1:2 - 2:2:1.

The oxidation reaction condition comprises a reaction temperature of 0-50 °C, and in some embodiments, 15-35°C, and a reaction time of 1-100 seconds, and in some embodiments, 5-50 seconds. In some embodiments, the oxidation agent is iodine (in some embodiments provided as iodine water). The molar ratio of the oxidation agent to the nucleic acid sequence linked to the solid phase support in the coupling step may be 1:1 to 100:1, and in some embodiments, is 5:1 to 50:1. In some embodiments, the oxidation reaction is performed in a mixed solvent in which the ratio of tetrahydrofuran: water: pyridine is 3:1:1-1:1:3. The sulfurization reaction condition comprises a reaction temperature of 0-50 °C, and in some embodiments, 15-35°C, and a reaction time of 50-2,000 seconds, and in some embodiments, 100-1,000 seconds. in some embodiments, the sulfurization agent is xanthane hydride. The molar ratio of the sulfurization agent to the nucleic acid sequence linked to the solid phase support in the coupling step is 10:1 to 1,000:1, and in some embodiments, is 10:1 to 500:1. In some embodiments, the sulfurization reaction is performed in a mixed solvent in which the ratio of acetonitrile: pyridine is 1:3-3:1.

The method further comprises isolating the sense strand and the antisense strand of the siRNA after linking all nucleoside monomers and before the annealing. Methods for isolation are well-known to those skilled in the art and generally comprise cleaving the synthesized nucleotide sequence from the solid phase support, removing the protection groups on the bases, phosphate groups and ligands, purifying, and desalting.

The conventional cleavage and deprotection methods in the synthesis of siRNAs can be used to cleave the

synthesized nucleotide sequence from the solid phase support, and remove the protection groups on the bases, phosphate groups and ligands. For example, the resultant nucleotide sequence linked to the solid phase support is contacted with concentrated aqueous ammonia; during deprotection, the protection group YCOO- in groups A46-A54 is converted to a hydroxyl group, and thus the $S_1$ groups are converted to corresponding $M_1$ groups, providing the conjugate as shown by Formula (308); wherein the concentrated aqueous ammonia may be aqueous ammonia of a concentration of 25-30 wt%. With respect to the target siRNA sequence, the amount of the concentrated aqueous ammonia may be 0.2 ml/μmol-0.8 ml/μmol.

When there is at least one 2'-TBDMS protection on the synthesized nucleotide sequence, the method further comprises contacting the nucleotide sequence removed from the solid phase support with triethylamine trihydrofluoride to remove the 2'-TBDMS protection. Here, the resultant target siRNA sequence comprises the corresponding nucleoside having free 2'-hydroxy. With respect to the target siRNA sequence, the amount of pure triethylamine trihydrofluoride is 0.4 ml/μmol-1.0 ml/μmol. As such, the siRNA conjugate as shown by Formula (308) can be obtained.

Methods for purification and desalination are well-known to those skilled in the art. For example, nucleic acid purification may be performed using a preparative ion chromatography purification column with a gradient elution of NaBr or NaCl; after collection and combination of the product, the desalination may be performed using a reverse phase chromatography purification column.

In the resultant siRNA conjugate as shown by Formula (308), the non-bridging oxygen or sulfur atom in the phosphodiester bond or phosphorothioate diester bond between the nucleotides substantially binds to sodium ion, and the siRNA conjugate is substantially present in the form of a sodium salt. The well-known ion-exchange methods may be used, in which the sodium ion may be replaced with hydrogen ion and/or other cations, thereby providing other forms of siRNA conjugates as shown by Formula (308). The cations are as described above.

During synthesis, the purity and molecular weight of the nucleic acid sequence may be determined at any time, in order to better control the synthesis quality. Such determination methods are well-known to those skilled in the art. For example, the purity of the nucleic acid may be determined by ion exchange chromatography, and the molecular weight may be determined by liquid chromatography-mass spectrometry (LC-MS).

Methods for annealing are also well-known to those skilled in the art. For example, the synthesized sense strand (S strand) and the antisense strand (AS strand) may be simply mixed in water for injection at an equimolar ratio, heated to 70-95°C, and then cooled at room temperature to form a double-stranded structure via hydrogen bond. Hence, the siRNA conjugate as shown by Formula (308) can be obtained.

After having obtained the conjugate, in some embodiments, the synthesized siRNA conjugate as shown by Formula (308) can also be characterized by the means such as molecular weight detection using the methods such as liquid chromatography-mass spectrometry, to confirm that the synthesized siRNA conjugate is the designed siRNA conjugate as shown by Formula (308) of interest, and the sequence of the synthesized siRNA is the sequence of the desired siRNA sequence, for example, one of the sequences listed in Tables 1-5.

The compound as shown by Formula (321) may be obtained by the following preparation method, comprising: contacting a compound as shown by Formula (313) with a cyclic anhydride in an organic solvent under esterification reaction condition in the presence of a base and an esterification catalyst; isolating the compound as shown by Formula (321) by ion exchange:

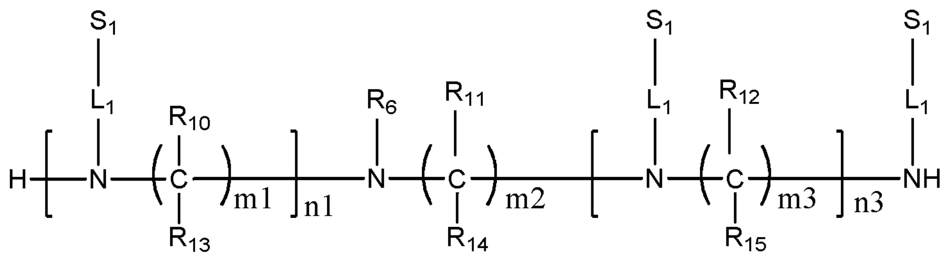

,

Formula (313)

wherein the definitions and options of n1, n3, m1, m2, m3, $R_{10}$, $R_{11}$, $R_{12}$, $R_{13}$, $R_{14}$, $R_{15}$, $L_1$, and

$S_1$ are respectively as described above;

$R_6$ is a group for providing $R_4$ of Formula (321); in some embodiments, $R_6$ has a structure as shown by Formula (A61):

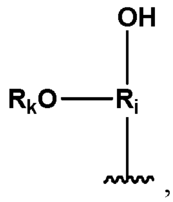

(A61)

wherein $R_i$ is any group capable of linking to the N atom on the nitrogenous backbone, linking to $R_kO$ and linking to a free hydroxy group; $R_k$ is a hydroxy protection group. In this case, a compound as shown by Formula (321) is obtained, wherein $R_4$ comprises a first functional group which comprises a hydroxy protection group and a second functional group which comprises a structure as shown by Formula (C1) or (C2).

The esterification reaction condition includes a reaction temperature of 0-100°C and a reaction time of 8-48 hours. In some embodiments, the esterification reaction condition comprises a reaction temperature of 10-40 °C and a reaction time of 20-30 hours.

In some embodiments, the organic solvent comprises one or more of an epoxy solvent, an ether solvent, an haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tertbutyl ether, and the haloalkane solvent is one or more of dichloromethane, trichloromethane and 1,2-dichloroethane. In some embodiments, the organic solvent is dichloromethane. With respect to the compound as shown by Formula (313), the amount of the organic solvent is 3-50 L/mol, and in some embodiments, 5-20 L/mol.

In some embodiments, the cyclic anhydride is one of succinic anhydride, glutaric anhydride, adipic anhydride or pimelic anhydride, and in some embodiments, the cyclic anhydride is succinic anhydride. The molar ratio of the cyclic anhydride to the compound as shown by Formula (313) is 1:1 to 10:1, and in some embodiments, 2:1 to 5:1.

The esterification catalyst may be any catalyst capable of catalyzing esterification reaction, such as 4-dimethylaminopyridine. The molar ratio of the catalyst to the compound as shown by Formula (313) is 1:1 to 10:1, and in some embodiments, is 2:1 to 5:1.

In some embodiments, the base may be any inorganic base, organic base or combination thereof. Considering solubility and product stability, the base may be, for example, an organic base of tertiary amine. In some embodiments, the organic base of tertiary amine is triethylamine or N,N-diisopropylethylamine. The molar ratio of the organic base of tertiary amine to the compound as shown by Formula (313) is 1:1 to 20:1, and in some embodiments, 3:1 to 10:1.

The ion exchange serves the function of converting the compound as shown by Formula (321) into a desired form of carboxylic acid or carboxylic salt and the methods of ion exchange are well-known to those skilled in the art. The above conjugation molecule in which the cation is $M^+$ may be obtained by using suitable ion exchange solution and ion exchange condition, which are omitted herein. In some embodiments, the ion exchange reaction is performed using a triethylamine phosphate solution, and the concentration of the triethylamine phosphate solution is 0.2-0.8 M. In some embodiments, the concentration of the triethylamine phosphate solution is 0.4-0.6 M, and with respect to the compound as shown by Formula (313), the amount of the triethylamine phosphate solution is 3-6 L/mol, and in further embodiments, 4-5 L/mol.

The compound of Formula (321) may be isolated from the reaction mixture using any suitable isolation methods. In some embodiments, the compound of Formula (321) may be isolated by removal of solvent via evaporation followed by chromatography. For example, the following two chromatographic conditions can be used for isolation: (1) normal phase purification of silica gel: 200-300 mesh silica gel filler, gradient elution of 1 wt%o triethylamine in dichloromethane: methanol = 100:18 - 100:20; or (2) reverse phase purification: C18 and C8 reverse phase filler, gradient elution of methanol: acetonitrile = 0.1: 1 - 1:0.1. In some embodiments, the solvent may be directly removed to obtain a crude product of the compound of Formula (321), which may be directly used in subsequent reactions.

In some embodiments, the method for preparing the compound of Formula (321) further comprises: further contacting the product obtained by the above ion exchanging reaction with a solid phase support with amino or hydroxy groups in an organic solvent under condensation reaction condition in the presence of a condensation agent and an organic base of tertiary amine. In this case, a compound as shown by Formula (321) is obtained, wherein $R_4$ comprises a first functional group which comprises a hydroxy protection group and a second functional group which comprises a structure as shown by Formula (C1').

The solid phase support is one of the supports used in solid phase synthesis of siRNA, some of which are well-known to those skilled in the art. For example, the solid phase support may be selected from the solid phase supports containing active hydroxy or amino functional group(s), and in some embodiments, is an amino or hydroxy resin. In

some embodiments, the amino or hydroxy resin has the following parameters: particle size of 100-400 mesh, and surface amino or hydroxy loading of 0.2 - 0.5 mmol/g. The ratio of the compound as shown by Formula (321) to the solid phase support is 10 - 400 μmol compound/g solid phase support. In some embodiments, the ratio of the compound of Formula (321) to the solid phase support is 50 μmol/g to 200 μmol/g.

The organic solvent may be any suitable solvent or mixed solvent known to those skilled in the art. In some embodiments, the organic solvent is one or more of acetonitrile, an epoxy solvent, an ether solvent, an haloalkane solvent, dimethyl sulfoxide, N,N-dimethylformamide, and N,N-diisopropylethylamine. In some embodiments, the epoxy solvent is dioxane and/or tetrahydrofuran; the ether solvent is diethyl ether and/or methyl tertbutyl ether; the haloalkane solvent is one or more of dichloromethane, trichloromethane and 1,2-dichloroethane. In some embodiments, the organic solvent is acetonitrile. With respect to the compound of Formula (321), the amount of the organic solvent is 20-200 L/mol, and in some embodiments, 50-100 L/mol.

In some embodiments, the condensation agent may be benzotriazol-1-yl-oxytripyrrolidino phosphonium hexafluorophosphate (PyBop), 3-diethoxyphosphoryl-1,2,3-benzotrizin-4(3H)-one and/or O-benzotriazol-tetramethyluronium hexafluorophosphate. In some embodiments, the condensation agent is O-benzotriazol-tetramethyluronium hexafluorophosphate. The molar ratio of the condensation agent to the compound as shown by Formula (321) is 1:1 to 20:1, and in some embodiments, 1:1 to 5:1.

In some embodiments, the organic base of tertiary amine is triethylamine and/or N,N-diisopropylethylamine, and in some embodiments, N,N-diisopropylethylamine. The molar ratio of the organic base of tertiary amine to the compound as shown by Formula (321) is 1:1 to 20:1, and in some embodiments, 1:1 to 5:1.

In some embodiments, the method for preparing the compound of Formula (321) further comprises: contacting the resultant condensation product with a capping agent and an acylation catalyst in an organic solvent under capping reaction condition, and isolating the compound of Formula (321). The capping reaction is used to remove any active functional group that does not completely react, so as to avoid producing unnecessary by-products in subsequent reactions. The capping reaction condition comprises a reaction temperature of 0-50°C, and in some embodiments, 15-35°C, and a reaction time of 1-10 hours, and in some embodiments, 3-6 hours. The capping agent may be the capping agent used in solid phase synthesis of siRNA, which are well-known to those skilled in the art.

In some embodiments, the capping agent is composed of a capping agent 1 (cap1) and a capping agent 2 (cap2). The cap1 is N-methylimidazole, and in some embodiments, provided as a mixed solution of N-methylimidazole in pyridine/acetonitrile, wherein the volume ratio of pyridine to acetonitrile is 1:10 to 1:1, and in some embodiments, 1:3 to 1:1. In some embodiments, the ratio of the total volume of pyridine and acetonitrile to the volume of N-methylimidazole is 1:1 to 10:1, and in some embodiments, 3:1 to 7:1. The cap2 is acetic anhydride, and in some embodiments, provided as a solution of acetic anhydride in acetonitrile, wherein the volume ratio of acetic anhydride to acetonitrile is 1:1 to 1:10, and in further embodiments, 1:2 to 1:6.

In some embodiments, the ratio of the volume of the mixed solution of N-methylimidazole in pyridine/acetonitrile to the weight of the compound of Formula (321) is 5 ml/g-50 ml/g, and in some embodiments, 15 ml/g-30 ml/g. The ratio of the volume of the solution of acetic anhydride in acetonitrile to the weight of the compound of Formula (321) is 0.5 ml/g-10 ml/g, and in some embodiments, 1 ml/g-5 ml/g.

In some embodiments, the capping agent comprises equimolar acetic anhydride and N-methylimidazole. The organic solvent is one or more of acetonitrile, an epoxy solvent, an ether solvent, an haloalkane solvent, dimethyl sulfoxide, N,N-dimethylformamide, and N,N-diisopropylethylamine. In some embodiments, the organic solvent is acetonitrile. With respect to the compound of Formula (321), the amount of the organic solvent is 10-50 L/mol, and in some embodiments, 5-30 L/mol.

In some embodiments, the acylation catalyst may be selected from any catalyst that may be used for esterification condensation or amidation condensation, such as alkaline heterocyclic compounds. In some embodiments, the acylation catalyst is 4-dimethylaminopyridine. The weight ratio of the catalyst to the compound as shown by Formula (321) may be 0.001:1 to 1:1, and in some embodiments, 0.01:1 to 0.1:1.

In some embodiments, the compound of Formula (321) may be isolated from the reaction mixture by any suitable separation methods. In some embodiments, the compound of Formula (321) may be obtained by thoroughly washing with an organic solvent and filtering to remove unreacted reactants, excess capping agent and other impurities, wherein the organic solvent is selected from acetonitrile, dichloromethane and methanol. In some embodiments, the organic solvent is acetonitrile.

In some embodiments, the preparation method of the conjugation molecule as shown by Formula (321) comprises contacting a compound as shown by Formula (313) with a phosphorodiamidite in an organic solvent under coupling reaction condition in the presence of a coupling agent, and isolating the compound as shown by Formula (321). In this case, a compound as shown by Formula (321) is obtained, wherein $R_4$ comprises a first functional group comprising a hydroxy protection group and a second functional group comprising a structure as shown by Formula (C3).

In some embodiments, the coupling reaction condition comprises: a reaction temperature of 0-50°C, such as 15-35°C; the molar ratio of the compound of Formula (313) to the phosphorodiamidite of 1:1 to 1:50, such as 1:5 to 1:15;

the molar ratio of the compound of Formula (313) to the coupling agent of 1:1 to 1:100, such as 1:50 to 1:80; and a reaction time of 200-3,000 seconds, such as 500-1,500 seconds. The phosphorodiamidite may be, for example, bis(diisopropylamino)(2-cyanoethoxy)phosphine, which may be commercially available or synthesized according to the methods well-known in the art. The coupling agent is one or more selected from 1H-tetrazole, 5-ethylthio-1H-tetrazole and 5-benzylthio-1H-tetrazole, such as 5-ethylthio-1H-tetrazole. The coupling reaction may be performed in an organic solvent. The organic solvent is one or more selected from anhydrous acetonitrile, anhydrous DMF and anhydrous dichloromethane, such as anhydrous acetonitrile. With respect to the compound of Formula (313), the amount of the organic solvent is 3-50 L/mol, such as 5-20 L/mol. By coupling reaction, the hydroxy group in the compound of Formula (313) reacts with the phosphorodiamidite to form a phosphoramidite group. In some embodiments, the solvent may be directly removed to afford a crude product of the compound of Formula (321), which may be directly used in subsequent reactions.

In some embodiments, the preparation method of the compound of Formula (321) further comprises the following steps: further contacting the isolated product with a solid phase support with hydroxy groups in an organic solvent under coupling reaction condition in the presence of a coupling agent, followed by capping, oxidation, and isolation, to afford the compound of Formula (321), wherein $R_4$ comprises a first functional group comprising a hydroxy protection group and a second functional group comprising a structure as shown by Formula (C3').

In some embodiments, the solid phase support is a solid support well-known in the art for solid phase synthesis of nucleic acid, such as, a deprotected commercially available universal solid phase support (NittoPhase®HL UnyLinker™ 300 Oligonucleotide Synthesis Support, Kinovate Life Sciences, as shown by Formula B80):

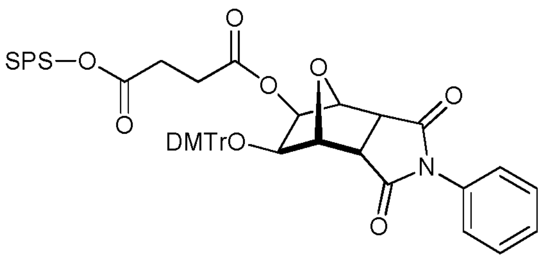

(B80)

A deprotection reaction is well-known to those skilled in the art. In some embodiments, the deprotection condition comprises a temperature of 0-50°C, such as 15-35°C, and a reaction time of 30-300 seconds, such as 50-150 seconds. The deprotection agent may be one or more selected from of trifluoroacetic acid, trichloroacetic acid, dichloroacetic acid, and monochloroacetic acid. In some embodiments, the deprotection agent is dichloroacetic acid. The molar ratio of the deprotection agent to the protection group -DMTr (4,4'-dimethoxytrityl) on the solid phase support is 2:1 to 100:1, such as 3:1 to 50:1. Through such deprotection, reactive free hydroxy groups are obtained on the surface of the solid phase support, for facilitating the subsequent coupling reaction.

The coupling reaction condition and the coupling agent may be selected as above. Through the coupling reaction, the free hydroxy groups formed in the deprotection react with the phosphoramidite groups, so as to form a phosphite ester linkage.

In some embodiments, the capping reaction condition comprises a temperature of 0-50 °C, such as 15-35 °C, and a reaction time of 5-500 seconds, such as 10-100 seconds. The capping reaction is carried out in the presence of a capping agent. The selection and amount of the capping agent are as described above.

The oxidation reaction condition comprises a temperature of 0-50 °C, such as 15-35 °C, and a reaction time of 1-100 seconds, such as 5-50 seconds. The oxidation agent may be, for example, iodine (in some embodiments, provided as iodine water). The molar ratio of the oxidation agent to the nucleic acid sequence linked to the solid phase support is 1:1 to 100:1, such as, may be 5:1 to 50:1. In some embodiments, the oxidation reaction is performed in a mixed solvent in which the ratio of tetrahydrofuran: water: pyridine = 3:1:1 - 1:1:3.

In some embodiments, $R_6$ is one of the groups of Formula B7 or B8:

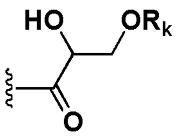

,

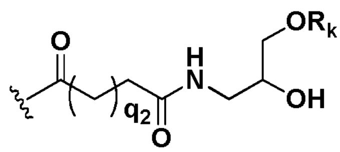

,

(B7) (B8)

wherein the definition of $q_2$ is as described above.

In this case, the compound as shown by Formula (313) may be obtained by the following preparation method, comprising: contacting the compound as shown by Formula (314) with a compound as shown by Formula (A-1) or (A-2) in an organic solvent under amidation reaction condition in the presence of a condensation agent for amidation reaction and an organic base of tertiary amine, followed by isolation:

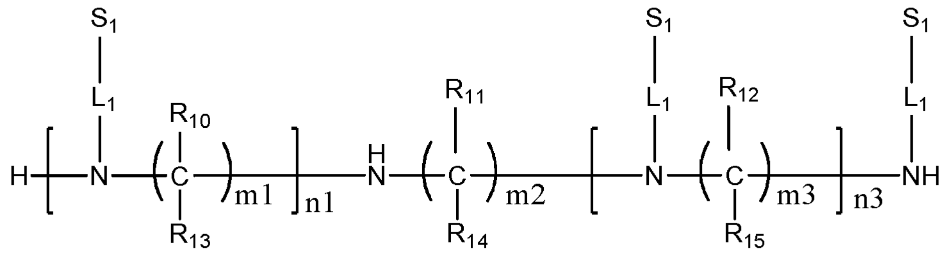

,

Formula (314)

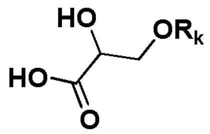

, 

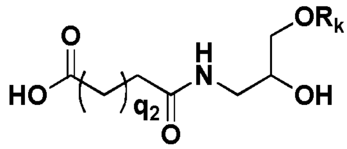

,

(A-1) (A-2)

wherein the definitions and options of n1, n3, m1, m2, m3, $R_{10}$, $R_{11}$, $R_{12}$, $R_{13}$, $R_{14}$, $R_{15}$, $L_1$, $S_1$, $q_2$, and $R_k$ are respectively as described above.

The amidation reaction condition comprises a reaction temperature of 0-100 °C and a reaction time of 1-48 hours. In some embodiments, the amidation reaction condition comprises a reaction temperature of 10-40 °C and a reaction time of 2-16 hours.

In some embodiments, the organic solvent is one or more of an alcohol solvent, an epoxy solvent, an ether solvent, an haloalkane solvent, dimethyl sulfoxide, N,N-dimethylformamide, and N,N-diisopropylethylamine. In some embodiments, the alcohol solvent is one or more of methanol, ethanol and propanol, and in some embodiments, ethanol. In some embodiments, the epoxy solvent is dioxane and/or tetrahydrofuran. In some embodiments, the ether solvent is diethyl ether and/or methyl tert-butyl ether. In some embodiments, the haloalkane solvent is one or more of dichloromethane, trichloromethane and 1,2-dichloroethane. In some embodiments, the organic solvent is dichloromethane. With respect to the compound of Formula (314), the amount of the organic solvent is 3-50 L/mol, and in some embodiments, 3-20 L/mol.

In some embodiments, the condensation agent for amidation reaction is benzotriazol-1-yl-oxytripyrrolidinophosphonium hexafluorophosphate, 3-diethoxyphosphoryl-1,2,3-benzotrizin-4(3H)-one, 4-(4,6-dimethoxytriazin-2-yl)-4-methylmorpholine hydrochloride, 2-ethoxy-1-ethoxycarbonyl-1,2-dihydroquinoline (EEDQ), or O-benzotriazol-tetramethyluronium hexafluorophosphate, and in further embodiments, 3-diethoxyphosphoryl-1,2,3-benzotrizin-4(3H)-one. The molar ratio of the condensation agent for amidation reaction to the compound as shown by Formula (314) is 1:1 to 10:1, and in some embodiments, 2.5:1 to 5:1.

In some embodiments, the organic base of tertiary amine is triethylamine or N,N-diisopropylethylamine, and in some embodiments, N,N-diisopropylethylamine. The molar ratio of the organic base of tertiary amine to the compound as shown by Formula (314) is 3:1 to 20:1, and in some embodiments, 5:1 to 10:1.

The compounds of Formula (A-1) and (A-2) may be prepared by any suitable means. For example, when $R_k$ is a DMTr group, the compound of Formula (A-1) may be prepared by reacting calcium glycerate with DMTrCl. Similarly, the compound of Formula (A-2) may be prepared by firstly contacting 3-amino-1,2-propanediol with a cyclic anhydride and then reacting with DMTrCl, wherein the cyclic anhydride may have 4-13 carbon atoms, and in some embodiments, 4-8 carbon atoms. Those skilled in the art would easily understand that the selections of different cyclic anhydrides correspond to different values for $q_2$ in the compound of Formula (A-2). For example, when the cyclic anhydride is succinic anhydride, $q_2$=1; when the cyclic anhydride is glutaric anhydride, $q_2$=2, and so on.

In some variations, the compound of Formula (313) can also be prepared by sequentially reacting the compound as shown by Formula (314) with the cyclic anhydride, 3-amino-1,2-propanediol and DMTrCl. Those skilled in the art would easily understand that these variations would not affect the structure and function of the compound of Formula

(313), and these variations are readily realized by those skilled in the art on the basis of the above methods.

Similarly, the compound of Formula (313) may be isolated from the reaction mixture by any suitable isolation methods. In some embodiments, the compound of Formula (313) may be isolated by removal of solvent via evaporation followed by chromatography. For example, the following chromatographic conditions may be used for isolation: (1) normal phase purification of silica gel: 200-300 mesh silica gel filler, with gradient elution of petroleum ether: ethyl acetate: dichloromethane: N,N-dimethylformamide = 1:1:1:0.5 - 1:1:1:0.6; and (2) reverse phase purification: C18 and C8 reverse phase fillers, with gradient elution of methanol: acetonitrile = 0.1:1 - 1:0.1. In some embodiments, the solvent may be directly removed to afford a crude product of the compound of Formula (313), which may be directly used in subsequent reactions.

In some embodiments, the compound as shown by Formula (314) may be obtained by the following preparation method, comprising: contacting the compound as shown by Formula (320) with the compound as shown by Formula (316) in an organic solvent under condensation reaction condition in the presence of a condensation agent for amidation reaction and an organic base of tertiary amine, followed by isolation:

$S_1$-$L_1$-OH

Formula (316)

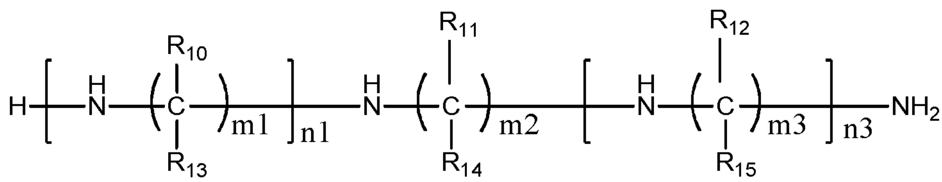
,

Formula (320)

wherein the definitions and options of n1, n3, m1, m2, m3, $R_{10}$, $R_{11}$, $R_{12}$, $R_{13}$, $R_{14}$, and $R_{15}$ are respectively as described above.

The compound of Formula (316) can be, such as, those disclosed in J. Am. Chem. Soc. 2014, 136, 16958-16961. Alternatively, the compounds of Formula (316) may be prepared by those skilled in the art via various methods. For example, some compounds of Formula (316) may be prepared according to the method disclosed in Example 1 of the US patent US8106022B2, which is incorporated herein by reference in its entirety.

In some embodiments, the condensation reaction condition comprises a reaction temperature of 0-100 °C and a reaction time of 0.1-24 hours, and in some embodiments, a reaction temperature of 10-40 °C and a reaction time of 0.5-16 hours.

Considering the structure of the desired product compound of Formula (314), the molar ratio of the compound as shown by Formula (316) to the compound as shown by Formula (320) should be determined based on the sum of n1 and n3 in Formula (320). In some embodiments, for example, when n1+n3= 3, to ensure complete reaction without any excess, the molar ratio of the compound as shown by Formula (316) to the compound as shown by Formula (320) may be 3:1 to 3.5:1, and in some embodiments, 3.01:1 to 3.15:1.

In some embodiments, the organic solvent is one or more of acetonitrile, an epoxy solvent, an ether solvent, an haloalkane solvent, dimethyl sulfoxide, N,N-dimethylformamide, and N,N-diisopropylethylamine. In some embodiments, the epoxy solvent is dioxane and/or tetrahydrofuran. In some embodiments, the ether solvent is diethyl ether and/or methyl tert-butyl ether. In some embodiments, the haloalkane solvent is one or more of dichloromethane, trichloromethane and 1,2-dichloroethane. In some embodiments, the organic solvent is dichloromethane. With respect to the compound of Formula (320), the amount of the organic solvent may be 3-50 L/mol, and in some embodiments, 5-20 L/mol.

In some embodiments, the condensing agent for amidation reaction is benzotriazol-1-yl-oxytripyrrolidinophosphonium hexafluorophosphate, 3-diethoxyphosphoryloxy-1,2,3-benzotrizin-4(3H)-one (DEPBT), O-benzotriazol-tetramethyluronium hexafluorophosphate, 4-(4,6-dimethoxytriazin-2-yl)-4-methylmorpholine hydrochloride, or 1-hydroxybenzotriazole, and in further embodiments, is a mixture of benzotriazol-1-yl-oxytripyrrolidinophosphonium hexafluorophosphate and 1-hydroxybenzotriazole, wherein benzotriazol-1-yl-oxytripyrrolidinophosphonium hexafluorophosphate and 1-hydroxybenzotriazole are used in equimolar amounts. The molar ratio of the total condensing agent for amidation reaction to the compound as shown by Formula (316) may be 1:1 to 3:1, and in some embodiments, 1.05:1 to 1.5:1.

The organic base of tertiary amine may be N-methylmorpholine, triethylamine or N,N-diisopropylethylamine,

and in some embodiments, N-methylmorpholine. The molar ratio of the organic base of tertiary amine to the compound as shown by Formula (316) may be 2:1 to 10:1, and in some embodiments, 2:1 to 5:1.

Similarly, the compound as shown by Formula (314) may be isolated from the reaction mixture by any suitable isolation method. In some embodiments, the compound as shown by Formula (314) may be isolated by removal of solvent via evaporation followed by chromatography, for example, using the following two chromatographic conditions for isolation: (1) normal phase purification of silica gel: 200-300 mesh silica gel filler, with gradient elution of dichloromethane: methanol = 100:5 - 100:7; and (2) reverse phase purification: $C_{18}$ and $C_8$ reverse phase fillers, with gradient elution of methanol: acetonitrile = 0.1:1 - 1:0.1. In some embodiments, the solvent may be directly removed to afford a crude product of the compound of Formula (314), which may be directly used in subsequent reactions.

The compound of Formula (320) may be commercially available, or prepared by those skilled in the art via known methods. For example, in the case where m1=m2=m3=3, n1=1, n3=2, and $R_{10}$, $R_{11}$, $R_{12}$, $R_{13}$, $R_{14}$, and $R_{15}$ are all H, the compound of Formula (320) may be commercially available from Alfa Aesar Inc.

The siRNA conjugate of the present disclosure may also be used in combination with other pharmaceutically acceptable excipients, which may be one or more of various conventional formulations or compounds in the art. For details, please refer to the above description of the pharmaceutical compositions of the present disclosure.

## Use of the siRNA, the pharmaceutical composition and the conjugate comprising the siRNA of the present disclosure

In some embodiments, the present disclosure provides the use of the siRNA and/or the pharmaceutical composition and/or the siRNA conjugate of the present disclosure in the manufacture of a medicament for treating and/or preventing dyslipidemia.

In some embodiments, the present disclosure provides a method for preventing and/or treating dyslipidemia, comprising administering an effective amount of the siRNA and/or the pharmaceutical composition and/or the siRNA conjugate of the present disclosure to a subject in need thereof.

The purpose of preventing and/or treating dyslipidemia may be achieved through the mechanism of RNA interference by administering the siRNA active ingredient of the present disclosure to a subject in need thereof. Therefore, the siRNA and/or the pharmaceutical composition and/or the siRNA conjugate of the present disclosure may be used for preventing and/or treating dyslipidemia, or for preparing a medicament for preventing and/or treating dyslipidemia.

The dyslipidemia refers to abnormal blood lipid caused by over-expression of APOC3 gene in hepatocytes, and generally manifests itself as increased level(s) of any one or all of lipids (such as triglyceride and cholesterol) and/or lipoprotein in blood. A high level of blood lipid is highly associated with hypertension, cardiovascular disease, diabetes, and other pathological conditions. Hypertriglyceridemia is associated with atherosclerosis, and would lead to pancreatitis. The dyslipidemia of the present disclosure includes, but is not limited to, hypercholesteremia, hypertriglyceridemia or atherosclerosis.

As used herein, the term "administration/administer" refers to the delivery of the siRNA and/or the pharmaceutical composition and/or the siRNA conjugate of the present disclosure into a subject's body by a method or a route that at least partly locates the siRNA and/or the pharmaceutical composition and/or the siRNA conjugate of the present disclosure at a desired site to produce a desired effect. Suitable administration routes for the methods of the present disclosure include topical administration and systemic administration. In general, topical administration results in the delivery of more siRNA conjugate to a particular site as compared with the whole body of the subject; whereas systemic administration results in the delivery of the siRNA and/or the pharmaceutical composition and/or the siRNA conjugate of the present disclosure to substantially the whole body of the subject. Considering that the present disclosure is intended to provide a means for preventing and/or treating dyslipidemia, in some embodiments, an administration mode capable of delivering a medicament to the liver.

The administration to a subject may be achieved by any suitable routes known in the art, including but not limited to, oral and parenteral routes, such as intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency may be once or more times daily, weekly, biweekly, triweekly, monthly, bimonthly, quarterly, semiannually, or yearly.

The dose of the siRNA or the pharmaceutical composition or the siRNA conjugate of the present disclosure may be a conventional dose in the art, which may be determined according to various parameters, especially age, weight and gender of a subject. Toxicity and efficacy may be determined in cell cultures or experimental animals by standard pharmaceutical procedures, for example, by determining $LD_{50}$ (the lethal dose that causes 50% population death) and $ED_{50}$ (the dose that can cause 50% of the maximum response intensity in a graded response, and that causes 50% of the experimental subjects to have a positive response in a qualitative response). The dose range for human may be derived based on data obtained from cell culture analysis and animal studies.

When the siRNA, the pharmaceutical composition and/or the siRNA conjugate of the present disclosure is administered, for example, to male or female, 6 to 12 weeks old, C57BL/6J mice of 18 to 25 g body weight or ob/ob mice of 30 to 45 g body weight, (i) for the siRNA conjugate, the amount of the siRNA thereof may be 0.001 to 100 mg/kg body weight, in further embodiments 0.01 to 50 mg/kg body weight, in more further embodiments 0.05 to 20 mg/kg body weight, in some embodiments 0.05 to 15 mg/kg body weight, and in other embodiments 0.1 to 10 mg/kg body weight; (ii) for a pharmaceutical composition formed from the siRNA and the pharmaceutically acceptable carrier, the amount of the siRNA thereof may be 0.001 to 50 mg/kg body weight, in further embodiments 0.01 to 10 mg/kg body weight, in more further embodiments 0.05 to 5 mg/kg body weight, and in still more further embodiments 0.1 to 3 mg/kg body weight, based on the amount of the siRNA.

In some embodiments, the present disclosure provides a method of inhibiting the expression of APOC3 gene in hepatocytes, comprising contacting an effective amount of the siRNA and/or the pharmaceutical composition and/or the siRNA conjugate of the present disclosure with the hepatocytes, and introducing the siRNA and/or the pharmaceutical composition and/or the siRNA conjugate of the present disclosure into the hepatocytes, so as to realize the purpose of inhibiting the expression of APOC3 gene in hepatocytes through the mechanism of RNA interference. The hepatocytes may be selected from hepatoma cell lines (such as Hep3B, HepG2 and Huh7), and isolated primary hepatocytes. In some embodiments, the hepatocytes are Huh7 hepatoma cells.

In the case where the expression of APOC3 gene in a cell is inhibited by the method of the present disclosure, the amount of the siRNA in the modified siRNA and/or the pharmaceutical composition and/or the siRNA conjugate of the present disclosure is generally such an amount that is sufficient to reduce the expression of the target gene and results in an extracellular concentration of 1 pM to 1 μM, or 0.01 nM to 100 nM, or 0.05 nM to 50 nM, or 0.05 nM to about 5 nM on the surface of the target cells. The amount required to achieve this topical concentration will vary with various factors, including the delivery method, the delivery site, the number of cell layers between the delivery site and the target cells or tissues, the delivery route (topical or systemic), etc. The concentration at the delivery site may be significantly higher than that on the surface of the target cells or tissues.

## Kit

The present disclosure provides a kit comprising an effective amount of at least one of the modified siRNA, the pharmaceutical composition, and the siRNA conjugate of the present disclosure.

In some embodiments, the kit of the present disclosure may provide the modified siRNA in a container. In some embodiments, the kit of the present disclosure may comprise a container containing a pharmaceutically acceptable excipient. In some embodiments, the kit may further comprise other ingredients, such as stabilizers or preservatives. In some embodiments, the kit of the present disclosure may comprise at least one additional therapeutic agent in other container different from the container for providing the modified siRNA of the present disclosure. In some embodiments, the kit may comprise an instruction for mixing the modified siRNA with pharmaceutically acceptable carriers and/or excipients or other ingredients (if present).

In the kit of the present disclosure, the modified siRNA and the pharmaceutically acceptable carrier and/or excipient, as well as the modified siRNA, the pharmaceutical composition, and/or the siRNA conjugate and/or the conjugate, and/or the pharmaceutically acceptable exceipient may be provided in any form, such as in a liquid form, a dry form or a lyophilized form. In some embodiments, the modified siRNA and the pharmaceutically acceptable carrier and/or excipient, and the pharmaceutical composition and/or conjugate and optional pharmaceutically acceptable excipient(s) are substantially pure and/or sterilized. In some embodiments, the kit of the present disclosure may provide sterilized water.

Hereinafter, the present disclosure will be further illustrated by way of examples, but will not be limited thereto in any respect.

## Examples

Unless otherwise specified, the reagents and culture media used in following examples are all commercially available, and the procedures used such as nucleic acid electrophoresis and real-time PCR are all performed according to the methods described in Molecular Cloning (Cold Spring Harbor Laboratory Press (1989)).

Huh7 cells were purchased from the Stem Cell Bank of Chinese Academy of Science and cultured in DMEM complete media (Hyclone company) containing 10% fetal bovine serum (FBS, Hyclone company) and 1% non-essential amino acid (NEAA, Corning company) at 37°C in an incubator containing 5% $CO_2$/95% air.

When the siRNA or the siRNA conjugate against APOC3 gene synthesized in the present disclosure or the siRNA or the siRNA conjugate as negative control was used to transfect cells, Lipofectamine™2000 (Invitrogen) was used as a transfection reagent. The specific procedures could refer to the instruction provided by the manufacturer.

Unless otherwise specified, ratios of reagents provided below are all calculated by volume ratio (v/v).

The animal models used are as follows:

Human APOC3 transgenic mice: B6; CBA-Tg(APOC3)3707Bres/J, purchased from Jackson Laboratory, US;

All the experimental data are expressed as ,X, and the data are analyzed with Graphpad prism 5.0 statistical analysis software.

**Preparation Example 1: The preparations of Conjugates 1-11**

In this preparation example, Conjugates 1-11 were synthesized. The conjugates were those formed by conjugating L-9 conjugation molecule to the siRNAs as shown in Table 7.

(1-1) Synthesis of Compound L-10:

Compound L-10 was synthesized according to the following method:

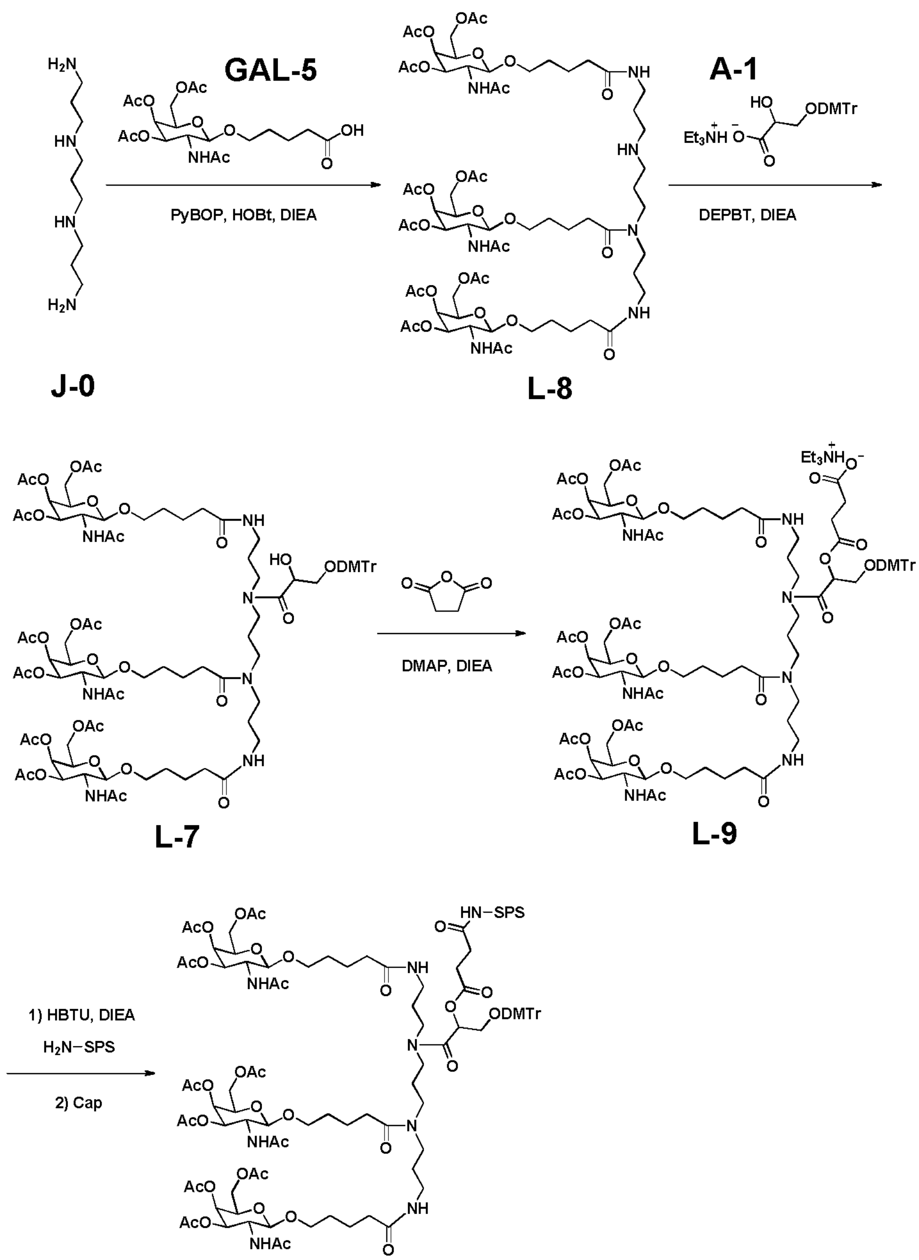


J-0 L-8

L-7 L-9

L-10

(1-1-1) Synthesis of GAL-5 (a terminal segment of the conjugation molecule)

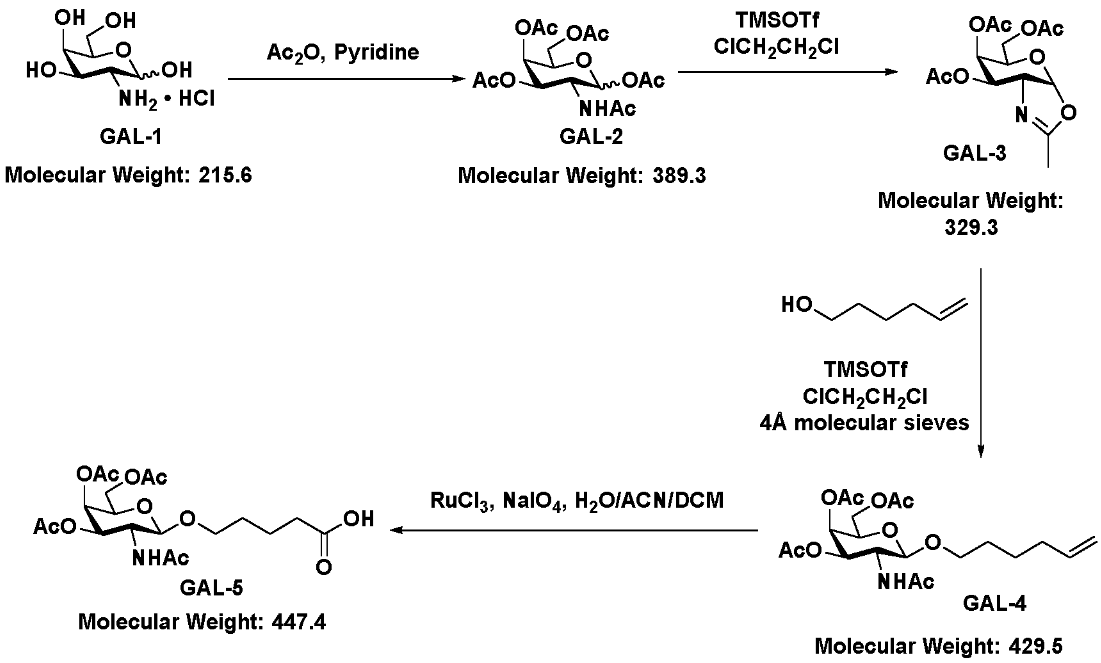


(1-1-1a) Synthesis of GAL-2

100.0 g of GAL-1 (N-acetyl-D-galactosamine hydrochloride, CAS No.: 1772-03-8, purchased from Ning Bo hongxiang bio-chem Co., Ltd., 463.8 mmol) was dissolved in 1000 ml of anhydrous pyridine, to which 540 ml of acetic anhydride (purchased from Enox Inc., 5565.6 mmol) was added in an ice water bath to react under stirring at room temperature for 1.5 hours. The resultant reaction solution was poured into 10 L of ice water and subjected to suction filtration under reduced pressure. The residue was washed with 2 L of ice water, and then added with a mixed solvent of acetonitrile/toluene (v/v ratio of acetonitrile: toluene = 1:1) until completely dissolved. The solvent was removed by evaporation to give 130.0 g of product GAL-2 as a white solid.

(1-1-1b) Synthesis of GAL-3

GAL-2 (35.1 g, 90.0 mmol) obtained in step (1-1-1a) was dissolved in 213 ml of anhydrous 1,2-dichloroethane, to which 24.0 g of TMSOTf (CAS No.: 27607-77-8, purchased from Macklin Inc., 108.0 mmol) was added in an ice water bath under nitrogen atmosphere to react at room temperature overnight.

The reaction solution was added with 400 ml dichloromethane for dilution, filtered with diatomite, and then added with 1L saturated aqueous sodium bicarbonate solution and stirred evenly. An organic phase was isolated. The aqueous phase remained was extracted twice, each with 300 ml of dichloroethane. The organic phases were combined and washed with 300 ml of saturated aqueous sodium bicarbonate solution and 300 ml of saturated brine, respectively. The organic phase was isolated and dried with anhydrous sodium sulfate. The solvent was removed by evaporation to dryness under reduced pressure to give 26.9 g of product GAL-3 as a light yellow viscous syrup.

(1-1-1c) Synthesis of GAL-4

GAL-3 (26.9 g, 81.7 mmol) obtained in step (1-1-1b) was dissolved in 136 ml of anhydrous 1,2-dichloroethane, added with 30 g of dry 4Å molecular sieve powder followed by 9.0 g of 5-hexen-1-ol (CAS No.: 821-41-0, purchased from Adamas-beta Inc., 89.9 mmol), and stirred at room temperature for 30 minutes. 9.08 g of TMSOTf (40.9 mmol) was added in an ice bath under nitrogen atmosphere to react under stirring at room temperature overnight. The 4Å molecular sieve powder was removed by filtration. The filtrate was added with 300 ml dichloroethane for dilution, filtered with diatomite, and then added with 500 ml of saturated aqueous sodium bicarbonate solution and stirred for 10 minutes for washing. An organic phase was isolated. The aqueous phase was extracted once with 300 ml of dichloroethane.

The organic phases were combined and washed with 300 ml of saturated aqueous sodium bicarbonate solution and 300 ml of saturated brine, respectively. The organic phase was isolated and dried with anhydrous sodium sulfate. The solvent was removed by evaporation to dryness under reduced pressure to give 41.3g of product GAL-4 as a yellow syrup, which was directly used in the next oxidation reaction without purification.

(1-1-1d) Synthesis of GAL-5

GAL-4 (14.9 g, 34.7 mmol) obtained according to the method described in step (1-1-1c) was dissolved in a mixed solvent of 77 ml of dichloromethane and 77 ml of acetonitrile, added with 103 ml of deionized water and 29.7 g of sodium periodate (CAS No.: 7790-28-5, purchased from Aladdin Inc., 138.8 mmol) respectively, and stirred in an ice bath for 10 minutes. Ruthenium trichloride (CAS No.: 14898-67-0, available from Energy Chemical, 238 mg, 1.145 mmol) was added to react at room temperature overnight. The resultant reaction solution was diluted by adding 300 ml of water under stirring, and adjusted to a pH of about 7.5 by adding saturated sodium bicarbonate. The organic phase was isolated and discarded. The aqueous phase was extracted three times, each with 200 ml of dichloromethane, and the organic phase was discarded. The aqueous phase was adjusted to a pH of about 3 with citric acid solids and extracted three times, each with 200 ml of dichloromethane, and the resultant organic phases were combined and dried with anhydrous sodium sulfate. The solvent was removed by evaporation to dryness under reduced pressure to give 6.85 g of product GAL-5 as a white foamy solid. $^1$H NMR (400 MHz, DMSO) δ 12.01 (br, 1H), 7.83 (d, J = 9.2 Hz, 1H), 5.21 (d, J = 3.2 Hz, 1H), 4.96 (dd, J = 11.2, 3.2 Hz, 1H), 4.49 (d, J = 8.4 Hz, 1H), 4.07 - 3.95 (m, 3H), 3.92-3.85 (m, 1H), 3.74 - 3.67 (m, 1H), 3.48 - 3.39 (m, 1H), 2.20 (t, J = 6.8 Hz, 2H), 2.11 (s, 3H), 2.00 (s, 3H), 1.90 (s, 3H), 1.77 (s, 3H), 1.55 - 1.45 (m, 4H).

(1-1-2) Synthesis of L-8

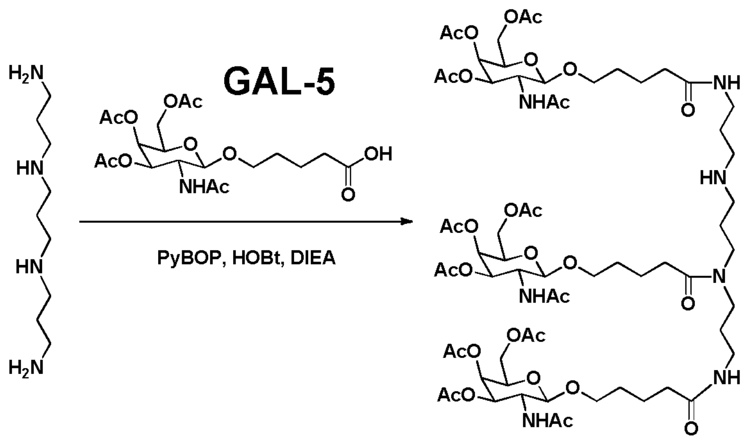


J-0 L-8

J-0 (9.886 g, 52.5 mmol, purchased from Alfa Aesar Inc.) and GAL-5 (72.819 g, 162.75 mmol, obtained by combining several batches of products) obtained in step (1-1-1) were dissolved in 525 ml of dichloromethane, and added with diisopropylethylamine (DIEA, 44.782 g, 346.50 mmol), benzotriazol-1-yl-oxytripyrrolidino phosphonium hexafluorophosphate (PyBOP, 90.158 g, 173.25 mmol) and hydroxybenzotriazole (HOBt, 23.410 g, 173.25mmol) to react at room temperature for 4 hours. The resultant reaction solution was washed by adding 20 ml of saturated sodium bicarbonate solution and 200 ml of saturated brine. The aqueous phase was extracted twice, each with 100 ml of dichloromethane. The organic phases were combined, dried with anhydrous sodium sulfate, and filtered. Then the solvent was removed by evaporation to dryness under reduced pressure to give a crude product. The crude product was purified by using a normal phase silica gel column (200-300 mesh). The column was added with 10 wt% triethylamine for neutralizing the acidity of silica gel, equilibrated with 1wt%o triethylamine, and eluted with a gradient elution of dichloromethane: methanol = 100:25-100:40. The eluate was collected, and the solvent was removed by evaporation to dryness under reduced pressure to give 38.8 g of pure product L-8. $^1$H NMR (400 MHz, DMSO) δ 7.84 (d, J = 9.0 Hz, 3H), 7.27 - 7.23 (m, 1H), 7.13 - 7.18 (m, 1H), 5.22 (d, J = 3.1 Hz, 3H), 4.97 (dd, J = 11.3, 3.1 Hz, 3H), 4.48 (d, J = 8.4 Hz, 3H), 4.09 - 3.98 (m, 9H), 3.88 (dd, J = 19.3, 9.3 Hz, 3H), 3.75 - 3.66 (m, 3H), 3.44 - 3.38 (m, 3H), 3.17 - 3.30 (m, 4H), 3.10 - 2.97 (m, 4H), 2.35 - 2.20 (m, 6H), 2.15 - 2.08 (m, 9H), 2.07 - 1.98 (m, 13H), 1.94 - 1.87 (m, 9H), 1.81 - 1.74 (m, 9H), 1.65 - 1.42 (m, 18H). MS m/z: $C_{85}H_{119}N_7O_{30}$, $[M+H]^+$, calculated: 1477.59, measured: 1477.23.

(1-1-3)

(1-1-3a) Synthesis of A-1

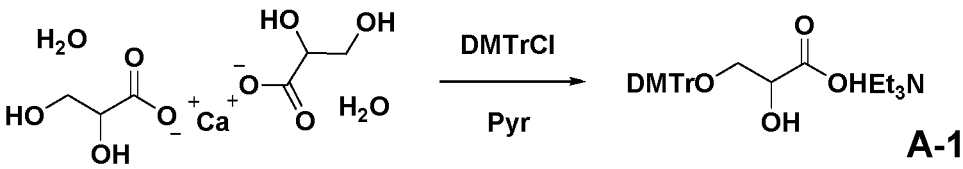


A-1

Molecular Weight: 286.25 Molecular Weight: 509.64

DMTrCl (4,4'-dimethoxytrityl chloride, 101.65 g, 300 mmol) was dissolved in 1000 ml of anhydrous pyridine, and added with calcium DL-glycerate hydrate (28.63 g, 100 mmol) to react at 45°C for 20 hours. The resultant reaction solution was filtered. The residue was rinsed with 200 ml of DCM, and the filtrate was concentrated to dryness under reduced pressure. The residue was redissolved in 500 ml of dichloromethane and washed twice, each with 200 ml of 0.5 M triethylamine phosphate (pH = 7-8). The aqueous phase was extracted twice, each with 200 ml of dichloromethane. The organic phases were combined, dried with anhydrous sodium sulfate, and filtered. The solvent was removed by evaporation to dryness under reduced pressure, and the residue was purified by using a normal phase silica gel column (200-300 mesh). The column was eluted with a gradient elution of petroleum ether: ethyl acetate: dichloromethane: methanol = 1:1:1:0.35 - 1:1:1:0.55. The eluate was collected, and the solvent was removed by evaporation to dryness under reduced pressure. The residue was redissolved in 600 ml of dichloromethane, and washed once with 200 ml of 0.5 M triethylamine phosphate. The aqueous phase was extracted once with 200 ml of dichloromethane. The organic phases were combined, dried with anhydrous sodium sulfate, and filtered. The solvent was removed by evaporation to dryness under reduced pressure, and the residue was dried under reduced pressure in a vacuum oil pump overnight to give 50.7 g of product A-1 as a white solid. $^1H$ NMR (400 MHz, DMSO-d6) $\delta$ 7.46 (ddd, J = 6.5, 2.3, 1.1 Hz, 1H), 7.40 - 7.28 (m, 7H), 6.89 - 6.81 (m, 4H), 4.84 (d, J = 5.0 Hz, 1H), 4.36 - 4.24 (m, 1H), 4.29 (s, 6H), 3.92 (dd, J = 12.4, 7.0 Hz, 1H), 3.67 (dd, J = 12.3, 7.0 Hz, 1H), 2.52 (q, J = 6.3 Hz, 6H), 1.03 (t, J = 6.3 Hz, 9H). MS m/z: $C_{24}H_{23}O_6$, $[M-H]^-$, calculated: 407.15, measured: 406.92.

(1-1-3b) Synthesis of L-7

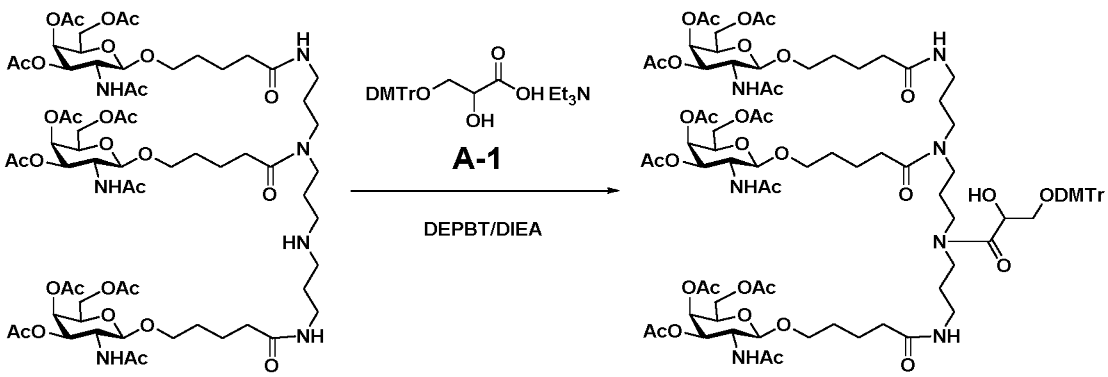


L-8 L-7

L-8 (40 g, 27.09 mmol, obtained by combining several batches of products) obtained in step (1-1-2) and A-1 (41.418 g, 81.27 mmol) obtained in step (1-1-3a) were mixed and dissolved in 271 ml of dichloromethane, added with 3-diethoxyphosphoryl-1,2,3-benzotrizin-4(3H)-one (DEPBT) (24.318 g, 81.37 mmol), and further added with diisopropylethylamine (21.007 g, 162.54 mmol) to react under stirring at 25 °C for 1.5 hours. The organic phase was washed with 800 ml of saturated sodium bicarbonate. The aqueous phase was extracted three times, each with 50 ml of dichloromethane. The organic phase was washed with 150 ml of saturated brine, and the aqueous phase was extracted once with 50 ml of dichloromethane, and the organic phases were combined, dried with anhydrous sodium sulfate and filtered. The solvent

was removed by evaporation to dryness under reduced pressure, and the residue was foam-dried in a vacuum oil pump overnight to give a crude product. The crude product was subjected to a column purification. The column was filled with 2 kg normal phase silica gel (200-300 mesh), added with 200 ml triethylamine for neutralizing the acidity of silica gel, equilibrated with petroleum ether containing 1 wt% triethylamine, and eluted with a gradient elution of petroleum ether: ethyl acetate: dichloromethane: N,N-dimethylformamide = 1:1:1:0.5 - 1:1:1:0.6. The eluate was collected, and the solvent was removed by evaporation to dryness under reduced pressure to give 40.4 g of pure product L-7. $^1$H NMR (400 MHz, DMSO) δ7.90 - 7.78 (m, 4H), 7.75 - 7.64 (m, 1H), 7.38 - 7.18 (m, 9H), 6.91 - 6.83 (m, 4H), 5.25 - 5.10 (m, 4H), 4.97 (dd, *J* = 11.2, 3.2 Hz, 3H), 4.48 - 4.30 (m, 4H), 4.02 (s, 9H), 3.93 - 3.84 (m, 3H), 3.76 - 3.66 (m, 9H), 3.45 - 3.35 (m, 3H), 3.24 - 2.98 (m, 10H), 2.30 - 2.20 (m, 2H), 2.11 - 1.88 (m, 31H), 1.80 - 1.40 (m, 28H). MS m/z: $C_{90}H_{128}N_7O_{35}$, $[M-DMTr]^+$, calculated: 1564.65, measured: 1564.88.

(1-1-4) Synthesis of L-9

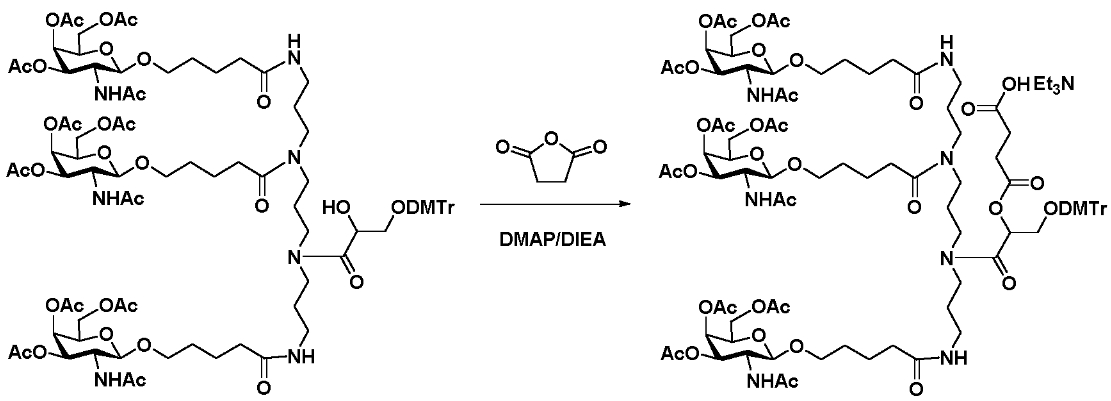


L-7 L-9

L-7 (40g, 21.4247mmol) obtained in step (1-1-3b), succinic anhydride (4.288g, 42.8494mmol) and 4-dimethylaminopyridine (DMAP, 5.235g, 42.8494mmol) were mixed and dissolved in 215 ml of dichloromethane, further added with diisopropylethylamine (DIEA, 13.845g, 107.1235mmol), and stirred at 25°C for 24 hours. The resultant reaction solution was washed with 800 ml of 0.5 M triethylamine phosphate. The aqueous phase was extracted three times, each with 5 ml of dichloromethane. The organic phases were combined and evaporated to dryness under reduced pressure to give a crude product. The crude product was subjected to a column purification. The columan was filled with 1 kg normal phase silica gel (200-300 mesh), added with 1 wt% triethylamine for neutralizing the acidity of silica gel, equilibrated with dichloromethane, and eluted with a gradient elution of 1wt‰ triethylamine in dichloromethane: methanol = 100:18-100:20. The eluate was collected, and the solvent was removed by evaporation to dryness under reduced pressure to give 31.0 g of pure product L-9 conjugation molecule. $^1$H NMR (400 MHz, DMSO) δ 8.58 (d, *J* = 4.2 Hz, 1H), 7.94 - 7.82 (m, 3H), 7.41 - 7.29 (m, 5H), 7.22 (d, *J* = 8.1 Hz, 5H), 6.89 (d, *J* = 8.3 Hz, 4H), 5.49 - 5.37 (m, 1H), 5.21 (d, *J* = 3.0 Hz, 3H), 4.97 (d, *J* = 11.1 Hz, 3H), 4.49 (d, *J* = 8.2 Hz, 3H), 4.02 (s, 9H), 3.88 (dd, *J* = 19.4, 9.4 Hz, 3H), 3.77 - 3.65 (m, 9H), 3.50 - 3.39 (m, 6H), 3.11 - 2.90 (m, 5H), 2.61 - 2.54 (m, 4H), 2.47 - 2.41 (m, 2H), 2.26 - 2.17 (m, 2H), 2.15 - 1.95 (m, 22H), 1.92 - 1.84 (m, 9H), 1.80- 1.70 (m, 10H), 1.65 - 1.35 (m, 17H), 1.31 - 1.19 (m, 4H), 0.96 (t, *J* = 7.1 Hz, 9H). MS m/z: $C_{94}H_{13}2N_7O_{38}$, $[M-DMTr]^+$, calculated: 1664.72, measured: 1665.03.

(1-1-5) Synthesis of Compound L-10

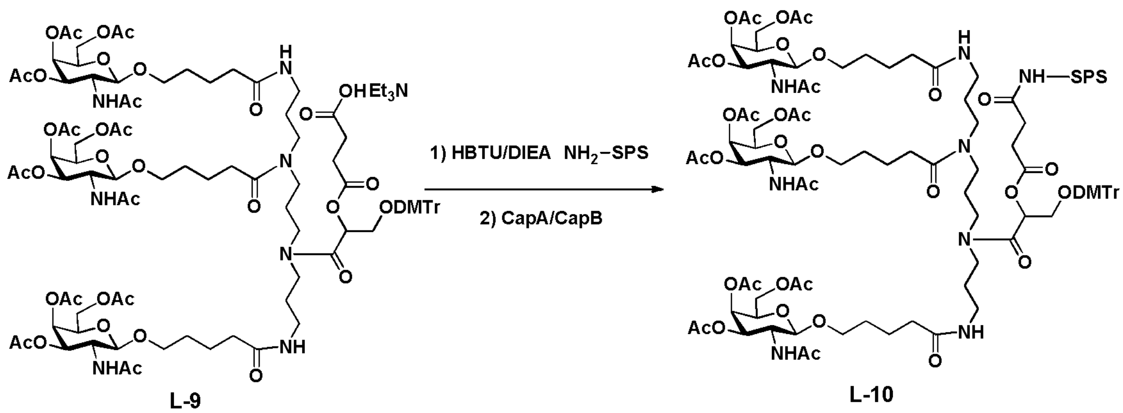


L-9

L-10

In this step, Compound L-10 was prepared by linking the L-9 conjugation molecule to a solid phase support.

The L-9 conjugation molecule (22.751 g, 11 mmol) obtained in step (1-1-4), O-benzotriazol-tetramethyluronium hexafluorophosphate (HBTU, 6.257 g, 16.5 mmol) and diisopropylethylamine (DIEA, 2.843 g, 22 mmol) were mixed and dissolved in 900 ml of acetonitrile, and stirred at room temperature for 5 minutes. The resultant reaction solution was added with Aminomethyl resin ($H_2$NResin, 88 g, 100-200 mesh, amino loading: 400 μmol/g, purchased from Tianjin Nankai HECHENG S&T Co., Ltd.). A reaction was performed on a shaker at 25°C and at a rotation speed of 150 rpm/min for 18 hours, followed by filtration. The residue was rinsed twice (each with 300 ml of DCM) and three times (each with 300 ml of acetonitrile), and dried in a vacuum oil pump for 18 hours. Then starting materials (CapA, CapB, 4-dimethylaminopyridine (DMAP) and acetonitrile) were added according to the charge ratio as shown in Table 6 for a capping reaction. The reaction was performed on a shaker at 25°C and at a rotation speed of 150 rpm/min for 5 hours. The reaction liquid was filtered. The residue was rinsed three times, each with 300 ml of acetonitrile. The solvent was removed by evaporation to dryness under reduced pressure, and the residue was dried under reduced pressure in a vacuum oil pump overnight to give 102 g of Compound L-10 (i.e., the L-9 conjugation molecule linked to a solid phase support), with a loading of 90.8 μmol/g.

Table 6 The charge ratio of capping reaction

| Starting Materials | Amount | Specs | Lot No. | Manufacturer |
|---|---|---|---|---|
| CapA | 1980 ml | -- | -- | -- |
| CapB | 220 ml | -- | -- | -- |
| DMAP | 1.100 g | analytical pure | 11422139 | Aladdin |
| Acetonitrile | 220 ml | spectroscopic pure | 015161001 | CINC (Shanghai) Co., Ltd |

In the above table, Cap A and Cap B are solutions of capping agents. Cap A is a mixed solution of 20% by volume of N-methylimidazole in pyridine/acetonitrile, wherein the volume ratio of pyridine to acetonitrile is 3:5. Cap B is a solution of 20% by volume of acetic anhydride in acetonitrile.

(1-2) Synthesis of sense strands of Conjugates 1-11

Nucleoside monomers were linked one by one in 3' to 5' direction according to the arrangement sequences of nucleotides in the sense strands of Conjugates 1-11 the phosphoramidite solid phase synthesis method, starting the cycles from the Compound L-10 prepared in the above step. The linking of each nucleoside monomer included a four-step reaction of deprotection, coupling, capping, and oxidation or sulfurization. Therein, when two nucleotides are linked via a phosphoester bond, a four-step reaction of deprotection, coupling, capping, and oxidation was included during linking of the later nucleoside monomer; and when two nucleotides is linked via a phosphorothioate linkage, a four-step reaction of deprotection, coupling, capping, and sulfurization was included during linking of the later nucleoside monomer. The synthesis conditions are as follows.

The nucleoside monomers are provided in a 0.1 M acetonitrile solution. The condition for deprotection reaction in each step is identical, i.e., a temperature of 25 °C, a reaction time of 70 seconds, a solution of dichloroacetic acid in dichloromethane (3% v/v) as a deprotection reagent, and a molar ratio of dichloroacetic acid to the protection group 4,4'-dimethoxytrityl on the solid phase support of 5:1.

The condition for coupling reaction in each step is identical, including a temperature of 25°C, a molar ratio of the nucleic acid sequence linked to the solid phase support to nucleoside monomers of 1:10, a molar ratio of the nucleic acid sequence linked to the solid phase support to a coupling reagent of 1:65, a reaction time of 600 seconds, and 0.5 M acetonitrile solution of 5-ethylthio-1H-tetrazole (ETT) as a coupling reagent.

The condition for capping reaction in each step is identical, including a temperature of 25°C, a reaction time of 15 seconds, a mixed solution of Cap A and Cap B in a molar ratio of 1:1 as a solution of capping agent, and a molar ratio of the capping agent to the nucleic acid sequence linked to the solid phase support of 1:1:1 (acetic anhydride: N-methylimidazole: the nucleic acid sequence linked to the solid phase support).

The condition for oxidation reaction in each step is identical, including a temperature of 25 °C, a reaction time of 15 seconds, and 0.05 M iodine water as an oxidation reagent; and a molar ratio of iodine to the nucleic acid sequence linked to the solid phase support in the coupling step of 30:1. The reaction is carried out in a mixed solvent of tetrahydrofuran: water: pyridine (3:1:1).

The condition for sulfurization reaction in each step is identical, including a temperature of 25 °C, a reaction time of 300 seconds, and xanthane hydride as a sulfurization reagent; and a molar ratio of the sulfurization reagent to the nucleic acid sequence linked to the solid phase support in the coupling step of 120:1. The reaction is carried out in a mixed solvent of acetonitrile: pyridine (1:1).

The conditions for cleavage and deprotection are as follows: adding the synthesized nucleotide sequence linked to the support into 25 wt% aqueous ammonia to react at 55 °C for 16 hours, wherein the amount of the aqueous ammonia is 0.5 ml/μmol. The liquid was removed, and the residue is concentrated to dryness in vacuum.

Purification and desalination: purification of the nucleic acid is achieved by using a preparative ion chromatography purification column (Source 15Q) with a gradient elution of NaCl. Specifically, eluent A is 20 mM sodium phosphate (pH 8.1), solvent is water/acetonitrile in 9:1 (v/v); eluent B is 1.5 M sodium chloride, 20 mM sodium phosphate (pH 8.1), solvent is water/acetonitrile in 9:1 (v/v); elution gradient: the ratio of eluent A: eluent B = 100:0-50:50. The eluate is collected, combined and desalted by using a reverse phase chromatography purification column. The specific condition includes: using a Sephadex column for desalination (filler: Sephadex G25) and eluting with deionized water.

Detection: the purity is determined by ion exchange chromatography (IEX-HPLC); and the molecular weight of the product was analyzed by Liquid Chromatography-Mass Spectrometry (LC-MS). The fact that the measured values were in conformity with the calculated values indicates that the sense strands S of Conjugates 1-11 of which 3' terminal was conjugated to the L-9 conjugation molecule were synthesized.

(1-3) Synthesis of antisense strands

(1-3A) Preparation of antisense strands of Conjugates 1-5

Antisense strands AS of Conjugates 1-5 were synthesized respectively by linking nucleoside monomers one by one in 3' to 5' direction according to the arrangement sequences of nucleotides in the antisense strands of Conjugates 1-5 in the phosphoramidite solid phase synthesis method, starting the cycles from a universal solid phase support (UnyLinker™ loaded NittoPhase®HL Solid Supports, Kinovate Life Sciences Inc.). The reaction conditions of deprotection, coupling, capping, oxidation or sulfurization, cleavage and deprotection, and purification and desalting in the solid phase synthesis method were the same as those used for the synthesis of the sense strands.

Detection: the purity was detected by ion exchange chromatography (IEX-HPLC); and the molecular weight was analyzed by liquid chromatography-mass spectrometry (LC-MS). The fact that the measured values were in conformity with the calculated values indicates that the antisense strands AS having the target sequences were synthesized.

Therein, the vinyl phosphate and 2'-methoxy modified uracil monomer (VP-Um) was synthesized according to the following method:

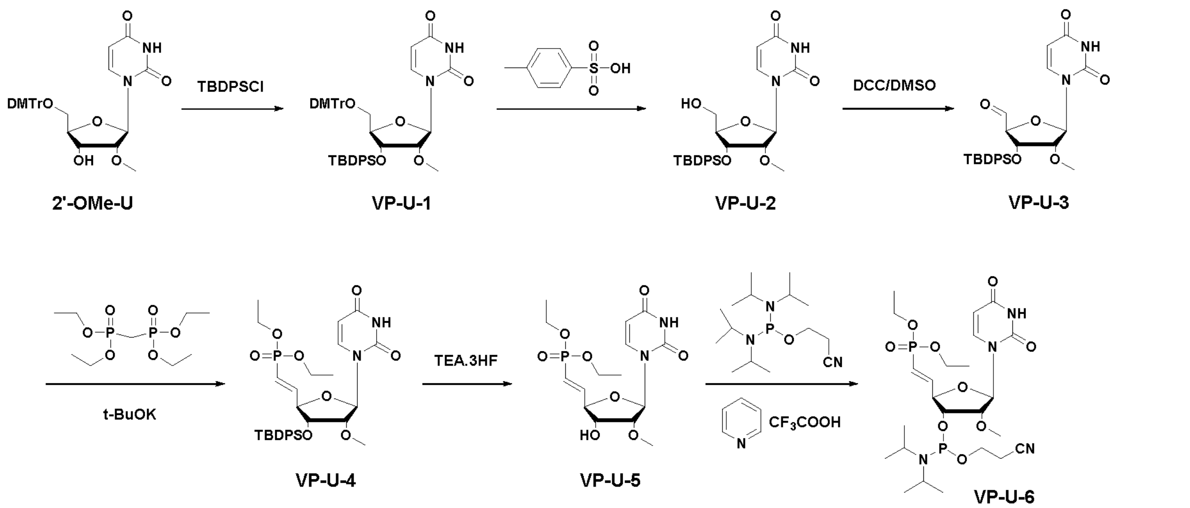


2'-OMe-U VP-U-1 VP-U-2 VP-U-3

VP-U-4 VP-U-5 VP-U-6

(1-3-1) Synthesis of VP-U-2

A VP-U-2 molecule was synthesized according to the following method:

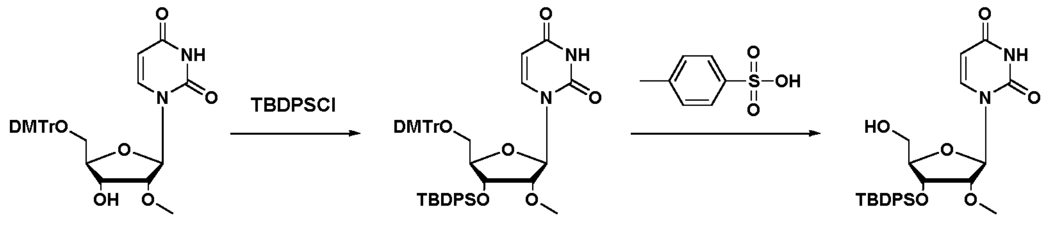


2'-OMe-U VP-U-1 VP-U-2

A 2'-methoxy modified uracil nucleoside (2'-OMe-U, 51.30 g, 91.6 mmol), tert-butyl diphenylchlorosilane (TBDPSCl, 50.35 g, 183.2 mmol), and imidazole (12.47 g, 183.2 mmol) were mixed and dissolved in 450 ml of N,N-dimethylformamide (DMF) to react under stirring at room temperature for 20 hours. DMF was removed by evaporation, and the residue was dissolved with 600 ml of dichloromethane and washed with 300 ml of saturated sodium bicarbonate. The aqueous phase was extracted three times, each with 300 ml of dichloromethane (DCM). The organic phases were combined, washed with 5% oxalic acid until the pH of the aqueous phase was <5. The solvent was evaporated to dryness to give a crude product VP-U-1, which was directly used in the subsequent synthesis of VP-U-2.

The crude product VP-U-1 was dissolved in 100 ml of dichloromethane, and then stirred for 10 minutes in an ice bath. 450 ml of 2% p-toluenesulfonic acid solution (the solvent is a mixed solvent of methanol and dichloromethane in a volume ratio of 3:7) pre-cooled in a refrigerator at 4 °C was added to react for 10 minutes. The reaction was quenched by addition of 200 ml of saturated sodium bicarbonate. The organic phase was washed to pH=8 by addition of saturated sodium bicarbonate solution. The aqueous phases were combined and extracted twice, each with 200 ml of dichloromethane. The organic phases were combined and washed once with 200 ml of saturated brine. The solvent was removed by evaporation to dryness, and the residue was purified by using a normal phase silica gel column (200-300 mesh). The column was packed with petroleum ether and eluted with a gradient elution of petroleum ether: ethyl acetate: dichloromethane: methanol = 1:1:1:0.05-1:1:1:0.25. The eluate was collected, the solvent was removed by evaporation to dryness under reduced pressure, and the residue was foam-dried in a vacuum oil pump to give a total of 40.00 g of pure product VP-U-2. $^1H$ NMR (400 MHz, DMSO-d6) $\delta$ 7.96 (d, J = 7.8 Hz, 1H), 7.64 (dtd, J = 5.1, 4.0, 2.2 Hz, 4H), 7.41-7.30 (m, 6H), 6.79 (d, J = 4.7 Hz, 1H), 5.73 (d, J = 7.6 Hz, 1H), 4.94 (t, J = 7.0 Hz, 1H), 4.12 (td, J = 4.6, 3.9 Hz, 1H), 4.05 (dd, J = 4.8, 4.0 Hz, 1H), 3.96 (t, J = 4.7 Hz, 1H), 3.68 (ddd, J = 11.8, 7.0, 4.6 Hz, 1H), 3.57 - 3.46 (m, 1H), 3.39 (s, 3H), 1.05 (s, 8H). MS m/z: $C_{26}H_{33}N_2O_6Si$, $[M+H]^+$, calculated: 497.21, measured: 497.45.

(1-3-2) Synthesis of VP-U-4

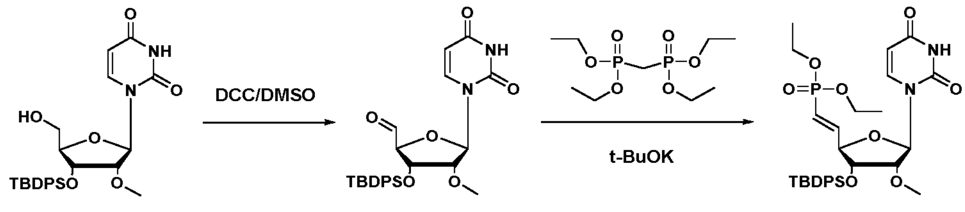


VP-U-2 VP-U-3 VP-U-4

VP-U-2 (19.84 g, 40.0 mmol), dicyclohexylcarbodiimide (DCC, 16.48 g, 80.0 mmol), pyridine (4.20 g, 53.2 mmol), and trifluoroacetic acid (6.61 g, 53.2 mmol) were mixed and dissolved in 200 ml of dimethyl sulfoxide (DMSO) to react under stirring at room temperature for 20 hours. Separately, tetraethyl methylenediphosphate (21.44 g, 74.4 mmol) was dissolved in 120 ml of THF, cooled in an ice bath, added with t-BuOK (11.36 g, 101.2 mmol) at a temperature of the ice bath to react for 10 min, warmed to room temperature to react for 0.5 hour and added into the above reaction solution over about 1 hour. The reaction was carried out at a temperature of the ice bath for 1 hour and then warmed to room temperature to react for 18 hours. The reaction was quenched by addition of water. The aqueous phase was extracted three times, each with 200 ml of dichloromethane. The organic phases were combined and washed once with 200 ml of saturated brine. The solvent was evaporated to dryness, and the residue was purified by using a normal phase silica gel column (200-300 mesh). The column was packed with petroleum ether and eluted with a gradient elution of petroleum ether: ethyl acetate = 1:1-1:4. The eluate was collected. The solvent was removed by evaporation to dryness under reduced pressure, and the residue was foam-dried in a vacuum oil pump to give a total of 14.00 g of pure product VP-U-4. $^1$H NMR (400 MHz, DMSO-d6) δ 7.96 (d, J = 7.8 Hz, 1H), 7.64 (dtd, J = 5.1, 4.0, 2.2 Hz, 4H), 7.41 - 7.30 (m, 6H), 6.82 - 6.71 (m, 2H), 5.90 (ddd, J = 25.9, 15.0, 1.0 Hz, 1H), 5.73 (d, J = 7.6 Hz, 1H), 4.36 - 4.21 (m, 3H), 4.18 (t, J = 4.9 Hz, 1H), 4.05 (ddq, J = 9.7, 8.5, 6.9 Hz, 2H), 3.87 (t, J = 4.8 Hz, 1H), 3.39 (s, 3H), 1.32 (td, J = 6.9, 0.7 Hz, 6H), 1.05 (s, 8H). MS m/z: $C_{31}H_{42}N_2O_8PSi$, [M+H]+, calculated: 629.24, measured: 629.51.

(1-3-3) Synthesis of VP-U-5

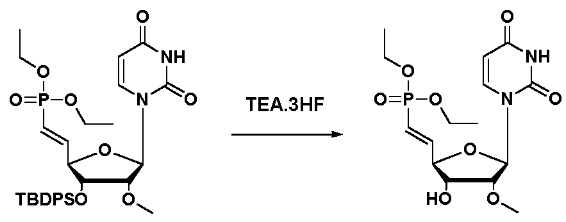


VP-U-4 VP-U-5

VP-U-4 (14.00 g, 22.29 mmol) was dissolved in 100 ml of tetrahydrofuran, added with triethylamine trihydrofluoride (17.96 g, 111.45 mmol), and stirred at room temperature for 20 hours to react completely. The solvent was directly evaporated to dryness, the residue was dissolved in dichoromethane, and then the solvent was evaporated to dryness again. The above dissolution and evaporation steps were performed twice, each with 50 ml of dichloromethane, to give a crude product. The crude product was purified by using a normal phase silica gel column (200-300 mesh). The column was packed with petroleum ether and eluted with a gradient elution of petroleum ether: ethyl acetate: dichloromethane: methanol = 1:1:1:0.05-1:1:1:0.25. The eluate was collected, the solvent was removed by evaporation to dryness under reduced pressure, and the residue was foam-dried in a vacuum oil pump to give a total of 6.70 g of pure product VP-U-5. $^1$H NMR (400 MHz, DMSO-d6) δ 7.96 (d, J = 7.8 Hz, 1H), 6.77 (dd, J = 15.0, 6.2 Hz, 1H), 5.99 - 5.82 (m, 2H), 5.73 (d, J = 7.6 Hz, 1H), 5.27 (d, J = 5.1 Hz, 1H), 5.10 (dd, J = 5.3, 4.7 Hz, 1H), 4.29 (ddq, J = 9.8, 8.6, 7.0 Hz, 2H), 4.17 (ddd, J = 6.2, 5.2, 1.0 Hz, 1H), 4.12 - 3.98 (m, 3H), 3.39 (s, 2H), 1.32 (td, J = 6.9, 0.6 Hz, 6H). MS m/z: $C_{15}H_{24}N_2O_8P$, $[M+H]^+$, calculated: 391.13, measured: 391.38.

(1-3-4) Synthesis of VP-U-6

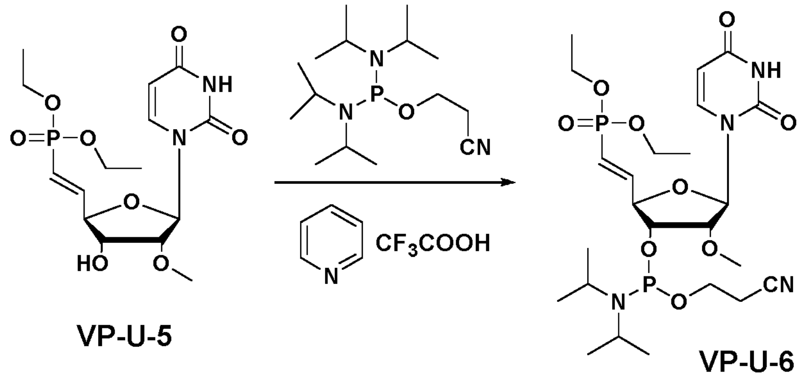


VP-U-5 VP-U-6

VP-U-5 (391 mg, 1.0 mmol), pyridine trifluoroacetate (0.232 g, 1.2 mmol), N-methylimidazole (0.099 g, 1.2 mmol), and bis(diisopropylamino)(2-cyanoethoxy)phosphine (0.452 g, 1.5 mmol) were added into 10 ml of anhydrous dichloromethane under argon atmosphere to react under stirring at room temperature for 5 hours. The solvent was evaporated to dryness, and then the residue was purified by column chromatography (200-300 mesh normal phase silica gel, with a gradient elution of dichloromethane: acetonitrile (containing 0.5 wt% triethylamine) = 3:1-1:3). The eluate was collected and concentrated to remove the solvent to give a total of 508 mg of target product VP-U-6. $^{31}P$ NMR (161 MHz, DMSO-d6) $\delta$ 150.34, 150.29, 17.07, 15.50. MS m/z: $C_{24}H_{41}N_4O_9P_2$, $[M+H]^+$, calculated: 591.23, measured: 591.55. The above data indicated that VP-U-6 was the target product VP-Um, which was involved in the synthesis of RNA strands as a nucleoside monomer.

(1-3B) Preparation of antisense strands of Conjugates 6 and 8-11

In addition to linking the corresponding nucleoside monomers one by one according to the nucleotide sequences as shown in Table 7 respetively, the method for synthesizing the antisense strands of Conjugates 6 and 8-11 differs from that of the antisense strand of Conjugate 4 only in the modification of the the first nucleotide at 5' terminal. During preparation of the antisense strands according to the phosphoramidite solid phase synthesis method, after the linking of 2'-methoxy modified guanine nucleoside monomer (Gm) as the last nucleoside monomer, the monomer of Formula (CPR-I) (purchased from Suzhou GenePharma Inc. as Cat#13-2601-XX) was linked to 5' terminal of the antisense strand by a four-step reaction of deprotection, coupling, capping, and oxidation, so as to form a 5'-phosphate modification.

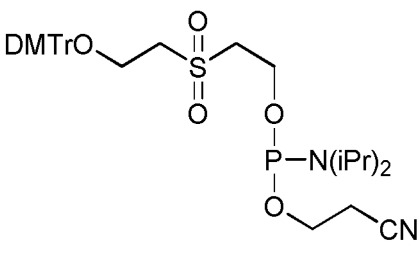
(CPR-I)

During the synthesis, the universal solid phase support, the conditions of deprotection, coupling, capping, oxidation or sulfurization reaction, cleavage and deprotection, purification and desalting used were the same as those used in the synthesis of the sense strand.

The purity was detected by ion exchange chromatography (IEX-HPLC); and the molecular weight was analyzed by liquid chromatography-mass spectrometry (LC-MS). The fact that the measured values were in conformity with the calculated values indicates that the antisense strands AS having the target sequences were synthesized.

(1-3C) Preparation of an antisense strand of Conjugate 7

An antisense strand of Conjugate 7 with a 5'-phosphorothioate modification was prepared by using the same synthesis method as that in step (1-3B), except that the oxidation reaction condition was replaced with a sulfurization reaction condition in the linking of the CPR-I monomer.

(1-4) Synthesis of Conjugates 1-11

For Conjugate 1, the S strand and AS strand were respectively dissolved in water for injection to give a solution of 40 mg/mL. They were mixed in an equimolar ratio, heated at 50 °C for 15 min, cooled at room temperature to produce an annealed product, and then lyophilized to give a lyophilized powder. After the conjugate was diluted to a concentration of 0.2 mg/mL with ultra-pure water (homemade by Milli-Q ultra-pure water instrument, with resistivity of 18.2MΩ*cm (25°C)), the molecular weight was determined by a liquid chromatography-mass spectrometry (LC-MS) instrument (purchased from Waters Corp., model: LCT Premier). The fact that the measured values were in conformity with the calculated values indicates that the synthesized Conjugate 1 was the designed target double-stranded nucleic acid sequence with the L-9 conjugation molecule.

Conjugates 2-11 were prepared by the same method except that the sense strand of Conjugate 1 was replaced with the sense strands of Conjugates 2-11 as prepared above, and the antisense strand of Conjugate 1 was replaced with the antisense strands of Conjugates 2-11 as prepared above. The molecular weights of the resultant Conjugates 2-11 were respectively determined to confirm that that the measured values were in conformity with the calculated values, indicating that the synthesized conjugates were the designed target double-stranded nucleic acid sequences with the L-9 conjugation molecule.

Conjugates 1-11 have the structure as shown by Formula (403).

Table 7: siRNA conjugates

| Conjugate NO: | siRNA NO: | siRNA Sequences (sequence direction 5'-3') | | SEQ ID NO |
|---|---|---|---|---|
| Conjugate 1 | siAPa1U M3SVP | S | UmsUmsAmAmAmAmGfGfGfAmCmAm GmUmAmUmUmCmAm | 301 |
| | | AS | VP-UmsGfsAmAmUmAfCmUmGmUmCmCm CmUfUmUfUmAmAmsGmsCm | 302 |
| Conjugate 2 | siAPelU M3SVP | S | GmsGmsAmCmAmGmUfAfUfUmCmUm CmAmGmUmGmCmAm | 303 |
| | | AS | VP-UmsGfsCmAmCmUfGmAmGmAmAmUm AmCfUmGfUmCmCmsCmsUm | 304 |
| Conjugate 3 | siAPb 1U M3SVP | S | AmsCmsAmGmUmAmUfUfCfUmCmAm GmUmGmCmUmCmAm | 305 |
| | | AS | VP-UmsGfsAmGmCmAfCmUmGmAmGmAm AmUfAmCfUmGmUmsCmsCm | 306 |
| Conjugate 4 | siAPd1U M3SVP | S | AmsGmsUmAmUmUmCfUfCfAmGmUm GmCmUmCmUmCmAm | 307 |
| | | AS | VP-UmsGfsAmGmAmGfCmAmCmUmGmAm GmAfAmUfAmCmUmsGmsUm | 308 |
| Conjugate 5 | siAPc1M 3SVP | S | UmsAmsUmUmCmUmCfAfGfUmGmCm UmCmUmCmCmUmAm | 309 |
| | | AS | VP-UmsAfsGmGmAmGfAmGmCmAmCmUm GmAfGmAfAmUmAmsCmsUm | 310 |

(continued)

| Conjugate NO: | siRNA NO: | siRNA Sequences (sequence direction 5'-3') | | SEQ ID NO |
|---|---|---|---|---|
| Conjugate 6 | siAPd1U M3SP | S | AmsGmsUmAmUmUmCfUfCfAmGmUm GmCmUmCmUmCmAm | 311 |
| | | AS | P-UmsGfsAmGmAmGfCmAmCmUmGmAm GmAfAmUfAmCmUmsGmsUm | 312 |
| Conjugate 7 | siAPd1U M3SPs | S | AmsGmsUmAmUmUmCfUfCfAmGmUm GmCmUmCmUmCmAm | 313 |
| | | AS | Ps-UmsGfsAmGmAmGfCmAmCmUmGmAm GmAfAmUfAmCmUmsGmsUm | 314 |
| Conjugate 8 | siAPa1M 3SP | S | UmsUmsAmAmAmAmGfGfGfAmCmAm GmUmAmUmUmCmUm | 423 |
| | | AS | P-AmsGfsAmAmUmAfCmUmGmUmCmCm CmUfUmUfUmAmAmsGmsCm | 424 |
| Conjugate 9 | siAPelM 3SP | S | GmsGmsAmCmAmGmUfAfUfUmCmUm CmAmGmUmGmCmUm | 425 |
| | | AS | P-AmsGfsCmAmCmUfGmAmGmAmAmUm AmCfUmGfUmCmCmsCmsUm | 426 |
| Conjugate 10 | siAPb1 M3SP | S | AmsCmsAmGmUmAmUfUfCfUmCmAm GmUmGmCmUmCmUm | 427 |
| | | AS | P-AmsGfsAmGmCmAfCmUmGmAmGmAm AmUfAmCfUmGmUmsCmsCm | 428 |
| Conjugate 11 | siAPc1M 3SP | S | UmsAmsUmUmCmUmCfAfGfUmGmCm UmCmUmCmCmUmAm | 429 |
| | | AS | P-UmsAfsGmGmAmGfAmGmCmAmCmUm GmAfGmAfAmUmAmsCmsUm | 430 |
| Comparative Conjugate 1 | 65704 | S | GmsCmsUmUmAmAmAfAmGfGfGfAmCm AmGmUmAmUmUmCmAm | 315 |
| | | AS | UmsGfsAmAmUmAmCmUmGmUmCmCm CmUfUmUmUmAmAmGmCmsAmsAm | 316 |
| Comparative Conjugate 2 | 69535 | S | GmsCmsUmUmAmAmAmAmGfGmGfAmC mAmGmUmAmUmUmCmAm | 317 |
| | | AS | UmsGfsAmAmUmAmCmUmGmUmCmCfC mUfUmUmUmAmAmGmCmsAmsAm | 318 |

**Preparation Example 2 Preparation of Comparative Conjugates 1 and 2**

In the present preparation example, Comparative Conjugates 1 and 2 were synthesized. The conjugated siRNAs in these conjugates had the sequences as shown in Table 7. The conjugation molecule to be conjugated refers to the $(GalNAc)_3$ conjugation molecule as synthesized in step (2-1) below. The conjugates respectively had the same structures as those of Compounds AD-65704 and AD-69535 in WO2016081444A1.

(2-1) Synthesis of $(GalNAc)_3$ conjugation molecule

Compound 30, i.e., the conjugation molecule (referred to as $(GalNAc)_3$ conjugation molecule) containing the above-mentioned linker -$(L^A)_3$-trihydroxymethylaminomethane-$L^B$- and N-acetylgalactosamine molecule as the targeting group (wherein each $L^A$ can be linked to one N-acetylgalactosamine molecule so that one linker can be linked to three N-acetylgalactosamine molecules), was synthesized according to the method described in Example 17 of WO2014025805A1. The chemical reaction formulae involved in the synthesis and the structure of $(GalNAc)_3$ conjugation molecule were shown below:

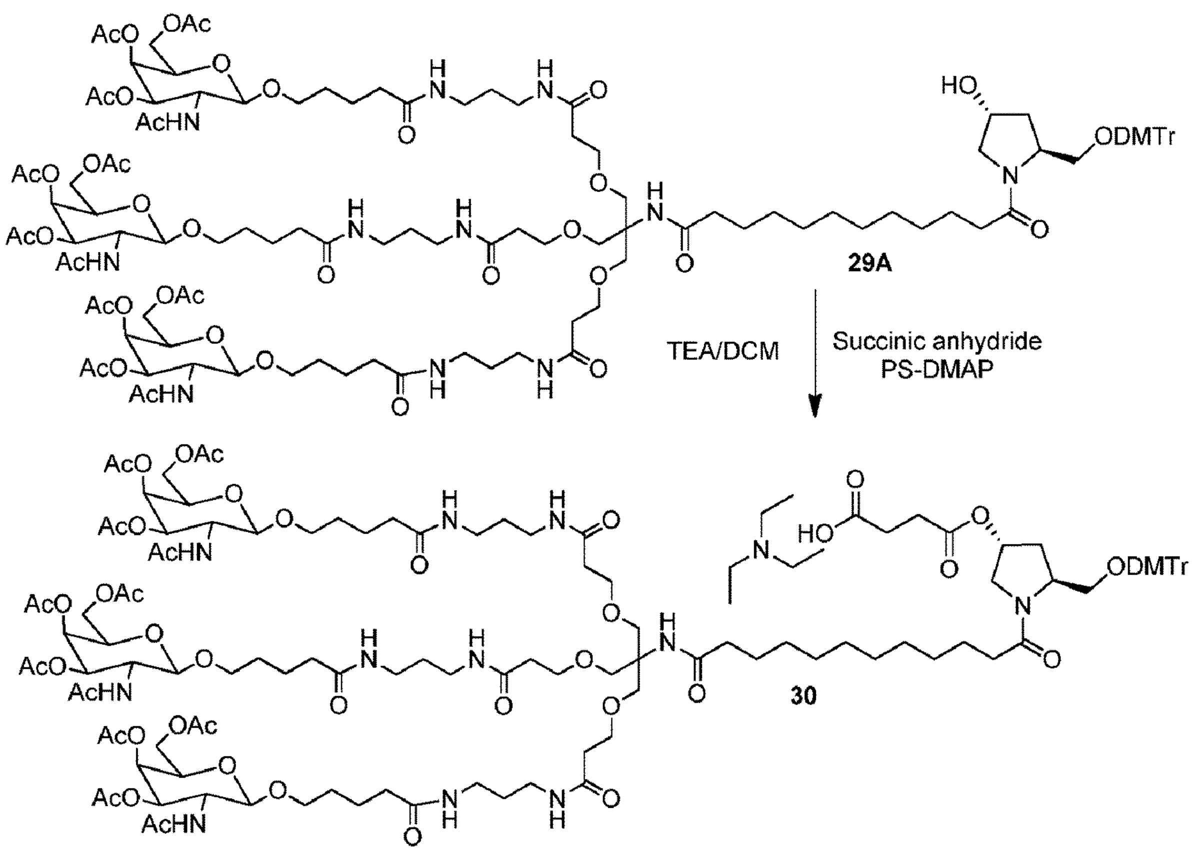

29A
30

(2-2) Preparation of the $(GalNAc)_3$ conjugation molecule linked to a solid phase support

The $(GalNAc)_3$ conjugation molecule linked to a solid phase support was prepared by using the same method as that in step (1-1-5) of Preparation Example 1, except that the L-9 conjungation molecule was replaced with the $(GalNAc)_3$ conjugation molecule, thereby obtaining the $(GalNAc)_3$ conjugation molecule linked to a solid phase support.

(2-3) Synthesis of Comparative Conjugates 1 and 2

Comparative Conjugates 1 and 2 were prepared by the same methods as those in steps (1-2), (1-3A) and (1-4) of Preparation Example 1, except that 1) the compound obtained in step (2-2) was used to start the synthesis of the sense strand; 2) during the preparation of the antisense strands according to the phosphoramidite solid phase synthesis method, 2'-methoxy modified uracil nucleoside monomer (Um) was linked as the last nucleoside monomer; and 3) the conjugated siRNAs respectively had the sequences as shown by Nos. $(GalNAc)_3$-65704 and $(GalNAc)_3$-69535 in Table 7.
The molecular weight was determined by a liquid chromatography-mass spectrometry (LC-MS) instrument

(purchased from Waters Corp., model: LCT Premier). The fact that the measured values were in conformity with the calculated values confirms that the synthesized conjugates were the designed target compounds having the structures as shown by Formula (305).

**Experimental Example 1: Detection of the inhibitory activity of the siRNA conjugates in *in vitro* cell system**

**Experimental Example 1-1 The Inhibitory effect of the siRNA conjugates on the expression level of APOC3 mRNA in Huh 7**

In this experimental example, the inhibition efficiencies of Conjugates 1-5 against the expression level of APOC3 mRNA in *in vitro* Huh 7 cell lines were investigated.

Conjugates 1-5 were transfected to human hepatoma cell lines Huh7 by using Lipofectamine™ 2000. The final concentrations of the siRNA conjugates (based on the amount of the siRNA) were 0.5 nM, 0.125 nM and 0.03125 nM, respectively. The siRNA conjugates were provided in the form of solutions. In particular, prior to the experiment, the siRNA conjugates were dissolved in DEPC water to give solutions with the desired concentrations. The cells not being transfected with the siRNA conjugates were used as a blank control, with two replicate wells per concentration.

The expression levels of APOC3 mRNAs in Huh 7 cells transfected with Conjugates 1-5 at various concentrations were measured by Quantitative Real-Time PCR, respectively. Specific steps were as follows: 24 hours after cultivation of the transfected cells, the total RNA was extracted and obtained by using Trizol (Thermo Fisher) according to the standard procedure for total RNA extraction; 1 μg of the total RNA was taken and reverse transcribed into cDNA by using a reverse transcription kit (Promega, Cat No. A3500) according to the procedures in the instruction thereof. The expression level of APOC3 mRNA was measured by using 2×Ultra SYBR Mixture (with ROX) kit (Beijing Cowin Biosciences Co., Ltd, Cat No. CW0956) with the cDNA as a template according to the procedures in the instruction. Therein, the PCR primers of for amplifying APOC3 and GAPDH as an internal control gene are shown in Table 8.

Table 8 Sequences of the primers for detection

| Genes | Upstream Primers | Downstream Primers |
|---|---|---|
| Human APOC3 | 5'-GTGACCGATGGCTTCAGTTC-3' (SEQ ID NO: 319) | 5'-ATGGATAGGCAGGTGGACTT-3' (SEQ ID NO: 320) |
| Human GAPDH | 5'-GGTCGGAGTCAACGGATTT- 3' (EQ ID NO: 321) | 5'-CCAGCATCGCCCCACTTGA-3' (SEQ ID NO: 322) |

The expression level of APOC3 mRNA was calculated by the following equation:

The expression level of APOC3 mRNA = (the expression level of APOC3 mRNA in the test group/the expression level of GAPDH mRNA in the test group)/(the expression level of APOC3 mRNA in the control group/the expression level of GAPDH mRNA in the control group) × 100%.

The inhibition rates of the conjugates against the expression level of APOC3 mRNA were calculated by the following equation:
The inhibition rate = (1 - the expression level of APOC3 mRNA) × 100%. Therein, the test groups are respectively the Huh7 cells treated with Conjugates 1-5 at various concentrations, and the control group is the Huh 7 cells untreated with the conjugates.

Figure 1 is a histogram showing the expression levels of APOC3 mRNA in untransfected Huh7 cells and in Huh7 cells transfected with different conjugates at different final concentrations (the expression levels used those of human GAPDH as reference, and were normalized against the blank control).

As can be seen from Figure 1, the siRNA conjugates of the present disclosure exhibited higher inhibitory activity in Huh7 cell lines; the conjugates at 0.5 nM showed inhibition rates of more than 60% against the expression levels of APOC3 mRNA. All of the siRNA conjugates of the present disclosure at all concentration exhibited higher inhibitory activity than that of Comparative Conjugate 1.

**Experimental Example 1-2: Determination of $IC_{50}$ of siRNA conjugates against APOC3 mRNA in Huh7 cell lines**

This experimental example determined the $IC_{50}$ values of Conjugates 6 and 8-11 against APOC3 mRNA in Huh7 cell lines.

Conjugates 6 and 8-11 were transfected by the same method as that in Experimental Example 1-1, and the expression level of APOC3 mRNA in each group was measured, except that the final concentrations of the conjugates (calculated based on the concentrations of siRNAs) were from 3 nM to 0.004 nM (three-fold serial dilutions of 7 concentrations). The dose-response curves were plotted by the expression levels of APOC3 mRNA measured at different concentrations of the conjugates, and the curves were fitted using the function log(inhibitor) vs. response-Variable slope of Graphpad 5.0 software. The $IC_{50}$ of each conjugate was calculated based on the dose-response curve.

$$Y = \mathrm{Bot} + \frac{\mathrm{Top} - \mathrm{Bot}}{1 + 10^{(\mathrm{LogIC_{50}} - X) \times \mathrm{HillSlope}}}$$

wherein:

Y is the expression level of remaining mRNA,
X is the logarithm of the concentration of transfected conjugates,
Bot is the Y value at the bottom of the steady stage,
Top is the Y value at the top of the steady stage,
$LogIC_{50}$ is the X value at which Y is the median value between the bottom and the top, and
HillSlope is the slope of the curve.

The $IC_{50}$ values of Conjugates 6 and 8-11 in *in vitro* Huh 7 cells were measured to be 0.01002 nM, 0.008159 nM, 0.05375 nM, 0.01933 nM, and 0.01949 nM, respectively, indicating that the siRNA conjugates of the present disclosure exhibited higher inhibitory activity against the expression levels of the target mRNA *in vitro.*

**Experimental Example 2 This experiment investigated the *in vivo* effects of reducing blood lipid content of the siRNA conjugates of the present disclosure.**

**Experimental Example 2-1 This experiment investigated the *in vivo* effects of Conjugates 2, 4 and 5 for reducing the contents of total cholesterol (CHO) and triglyceride (TG) in serum of human APOC3 transgenic mice.**

Human APOC3 transgenic mice (B6; CBA-Tg(APOC3)3707Bres/J) with serum TG content > 2mmol/L were randomly divided into groups (7 mice per group) as follows: (1) normal saline control group; (2) Conjugate 2: 3 mg/kg group; (3) Conjugate 2: 1 mg/kg group; (4) Conjugate 4: 3mg/kg group; (5) Conjugate 4: 1 mg/kg group; (6) Conjugate 5: 3mg/kg group; and (7) Conjugate 5: 1mg/kg group. The administration dosages for all animals were calculated according to the body weight and subcutaneously injected as single administration, wherein the siRNA conjugates were provided in 0.9 wt% NaCl aqueous solutions with concentrations of 0.6 mg/ml and 0.2 mg/ml. In particular, prior to the experiments, the siRNA conjugates were dissolved in the 0.9 wt% NaCl aqueous solution to give solutions with the desired concentrations. The administration volumes for normal saline and the siRNA conjugates were 5 mL/kg.

The blood was taken from mouse orbital venous plexus before administration (marked as day 0) and on days 7, 14, 21, 28, 35, 42, 49, 63, 77, 91, 112, 133, 147, 161, 175, and 189 after administration respectively. The contents of total cholesterol (CHO) and triglyceride (TG) in serum were measured at each time point.

About 100 μl blood was taken from the orbit each time, and the serum was not less than 20 μl after centrifugation. The contents of total cholesterol (CHO) and triglyceride (TG) in serum were further measured by using a PM1P000/3 full-automatic serum biochemical analyzer (SABA).

The normalized blood lipid level = (the blood lipid content in the test group after administration/the blood lipid content in the test group before administration) × 100%.

The inhibition rate against blood lipid level = (1 - the blood lipid content in the test group after administration/the blood lipid content in the test group before administration) × 100%. Blood lipid refers to total cholesterol (CHO) or triglyceride (TG).

Figures 2A to 2D are diagrams showing the changes of total cholesterol (CHO) level and triglyceride (TG) level over time in serum of human APOC3 trangenic mice administered with normal saline and Conjugates 2, 4 and 5 at different doses. In these figures, the test group before administration was marked as day D0; and the respective CHO levels and TG levels in serum were normalized relative to the value on day D0.

As can be seen from Figure 2A, the three conjugates in the 3 mg/kg dose groups after single administration

show inhibition rates of 70% to 90% against TG over a period of up to 77 days, and inhibition rates of about 50% or lower against TG over a period of up to 147 days.

As can be seen from Figure 2B, the three conjugates in the 1 mg/kg dose groups show inhibition rates of about 80% against TG on day 7 after single administration, and continuously show the effects of reducing the TG content by no less than 50% over a period of up to 49 days.

As can be seen from Figure 2C, after single administration, the three conjugates in the 3 mg/kg dose groups substantially maintain inhibition rates of about 50% against CHO over a period of up to 77 days.

As can be seen from Figure 2D, the three conjugates in the 1 mg/kg dose groups still show the effects of reducing CHO content by at least about 50% on day 35 after single administration.

The results of Figures 2A to 2D show that Conjugates 2, 4 and 5 show the effects of significantly reducing the contents of TG and CHO in mouse serum at different time points after administration, indicating that the siRNA conjugates of the present disclosure could stably and effectively reduce blood lipid level over a longer time period.

**Experimental Example 2-2: This experiment investigated the *in vivo* effects of Conjugate 1 for reducing the contents of total cholesterol (CHO) and triglyceride (TG) in serum of human APOC3 transgenic mice.**

The measurement was conducted by the same method as that in Experimental Example 2-1, except that each test group included 6 mice; the conjugates to be administered were Conjugate 1 and Comparative Conjugate 2; and the test continued until day 112 after administration.

Figures 3A to 3B are diagrams showing the changes of total cholesterol (CHO) level and triglyceride (TG) level over time in serum of human APOC3 trangenic mice administered with normal saline and Conjugate 1 and Comparative Conjugate 2 at different doses. In these figures, the test group before administration was marked as day D0; and the respective CHO levels and TG levels in serum were normalized relative to the value on day D0.

As can be seen from Figures 3A to 3B, in both of the 3 mg/kg dose group and the 1 mg/kg dose group, Conjugate 1 could significantly reduce the TG and CHO levels in the transgenic mice over a period of up to 112 days, and such reduction effects are significantly superior to that of Comparative conjugative 2. In both of the 3 mg/kg dose group and the 1 mg/kg dose group, Conjugate 1 shows inhibition rates of 50% or higher against TG and CHO over 56 days after single administration, and the inhibitory effects on TG are more significant: the two doses of Conjugate 1 continuously maintain the TG level of about 50% over a period of up to 112 days.

**Experimental Example 2-3: This experiment investigated the *in vivo* effects of Conjugate 3 for reducing the contents of total cholesterol (CHO) and triglyceride (TG) in serum of human APOC3 transgenic mice.**

The measurement was conducted by the same method as that in Experimental Example 2-1, except that each test group included 6 mice; the conjugate to be administered was Conjugate 3; Conjugate 3 was dissolved in 1×PBS buffer solution in place of normal saline (NS) to give a solution with the desired concentration; 1×PBS buffer solution was used as the blank control in the experiment; and the test continued until day 112 after administration.

Figures 4A to 4B are diagrams showing the changes of the total cholesterol (CHO) level and triglyceride (TG) level over time in serum of human APOC3 trangenic mice administered with PBS and Conjugate 3 at different doses. In these figures, the test group before administration was marked as day D0; and the respective CHO levels and TG levels in serum were normalized relative to the value on day D0.

As can be seen from Figure 4A, in the 3 mg/kg dose group, Conjugate 3 shows an inhibition rate of up to 93.6% against TG on day 14 after single administration, and still shows an inhibition rate of 66.4% against TG on day 98 after administration. In the 1 mg/kg dose group, Conjugate 3 shows an inhibition rate of up to 93.3% against TG on day 14 after single administration, and still shows an inhibition rate of 37.7% against TG on day 98 after administration.

As can be seen from Figure 4B, in the 3 mg/kg dose group, Conjugate 3 shows an inhibition rate of up to 63.0% against CHO on day 7 after single administration, and still shows an inhibition rate of 49.2% against CHO on day 98 after administration. In the 1 mg/kg dose group, Conjugate 3 shows an inhibition rate of up to 52.2% against CHO on day 7 after single administration.

The results of Figures 4A to 4B show that Conjugate 3 could significantly reduce the TG and CHO levels in the transgenic mice over a period of up to 98 days, and such reduction effects have obvious dose-dependent effect.

**Experimental Example 2-4: This experiment investigated the *in vivo* effects of Conjugate 4 for reducing the contents of total cholesterol (CHO) and triglyceride (TG) in serum of human APOC3 transgenic mice.**

The measurement was conducted by the same method as that in Experimental Example 2-1, except that each test group included 8 mice; the conjugate to be administered was Conjugate 4; Conjugate 4 was administered at three doses of 0.3 mg/kg, 1 mg/kg and 3 mg/kg, respectively, while the administration volume remained unchanged; and the

test continued until day 133 after administration.

Figures 5A to 5B are diagrams showing the changes of the total cholesterol (CHO) level and triglyceride (TG) level over time in serum of human APOC3 trangenic mice administered with normal saline and Conjugate 4 at different doses. In these figures, the test group before administration was marked as day D0; and the respective of CHO levels and TG levels in serum were normalized relative to the value on day D0. As can be seen from the results of Figures 5A to 5B, Conjugate 4 at the three doses shows the effects of significantly reducing lipid in human APOC3 trangenic mice, and the reduction effects are dose-dependent. Conjugate 4 in the 3 mg/kg dose group still shows an inhibition rate of up to 50% against TG on day 133.

**Experimental Example 2-5: This experiment investigated the *in vivo* effects of Conjugates 4, 6 and 7 for reducing the contents of total cholesterol (CHO) and triglyceride (TG) in serum of human APOC3 transgenic mice.**

The measurement was conducted by the same method as that in Experimental Example 2-1, except that each test group included 6 mice; the conjugates to be administered were Conjugates 4, 6 and 7 and Comparative Conjugate 2; and the test continued until day 112 after administration.

Figures 6A to 6D are diagrams showing the changes of the total cholesterol (CHO) level and triglyceride (TG) level in serum of human APOC3 trangenic mice administered with normal saline and Conjugates 4, 6 and 7 and Comparative Conjugate 2 at different doses. In these figures, the test group before administration was marked as day D0; and the respective of CHO levels and TG levels in serum were normalized relative to the value on day D0.

As can be seen from the results of Figures 6A to 6D, Conjugates 4, 6 and 7 at the two doses show the effects of significantly reducing blood lipid in human APOC3 trangenic mice. Conjugates 4, 6 and 7 in the 3 mg/kg dose group continuously maintain inhibition rates of 50% or higher against TG and inhibition rates of 30% or higher against CHO on day 84 after administration. It is noteworthy that Conjugates 4, 6 and 7 at the doses of 3 mg/kg and 1 mg/kg continuously show higher inhibitory effects on TG than those of Comparative Conjugate 2; and the same tendency is also observed for the inhibitory effect on CHO.

**Experimental Example 2-6: This experiment investigated the *in vivo* effects of Conjugates 8 and 9 at different doses for reducing the contents of total cholesterol (CHO) and triglyceride (TG) in serum of human APOC3 transgenic mice.**

The measurement was conducted by the same method as that in Experimental Example 2-1, except that each test group included 8 mice; the conjugates to be administered were Conjugates 8 and 9; the conjugates were administered at five doses of 0.1 mg/kg, 0.3 mg/kg, 1 mg/kg, 3 mg/kg, and 9 mg/kg, respectively; and the administration volume remained unchanged while the concentrations of the conjugate solutions were correspondingly adjusted; and the test continued until day 147 after administration.

Figures 7A to 7D are diagrams showing the the changes of the total cholesterol (CHO) level and triglyceride (TG) level over time in serum of human APOC3 trangenic mice administered with normal saline and Conjugates 8 and 9 at different doses. In these figures, the test group before administration was marked as day D0; and the respective of CHO levels and TG levels in serum were normalized relative to the value on day D0.

As can be seen from the results of Figures 7A to 7D, Conjugates 8 and 9 at the five doses show the effects of significantly reducing lipid in human APOC3 trangenic mice, and the reduction effects are dose-dependent.

Further, $ED_{50}$ values of Conjugates 8 and 9 for inhibiting TG level at different time points in human APOC3 transgenic mice were measured based on the inhibition rates of the conjugates at different doses against TG at different time points.

The dose-response curves were plotted according to the serum TG levels measured at different time points when using Conjugates 8 and 9 at different doses, and the curves were fitted using the function log(inhibitor) vs. response-Variable slope of Graphpad 5.0 software. The $ED_{50}$ value of each conjugate was calculated based on the dose-response curve.

$$Y = \mathrm{Bot} + \frac{\mathrm{Top} - \mathrm{Bot}}{1 + 10^{(\mathrm{LogED_{50}} - \mathrm{X}) \times \mathrm{HillSlope}}}$$

wherein:

Y is the measured serum TG level (normalized relative to the TG level on day D0),
X is the logarithm of the dose of transfected conjugates at the same time point,
Bot is the Y value at the bottom of the steady stage,

Top is the Y value at the top of the steady stage,
$LogED_{50}$ is the X value at which Y is the median value between the bottom and the top, and
HillSlope is the slope of the curve.

The calculated $ED_{50}$ values of Conjugates 8 and 9 for inhibiting TG at different time points after single administration were shown in Table 9.

Table 9 $ED_{50}$ values (mg/kg) of Conjugates 8 and 9 for inhibiting TG at different time points

| Conjugate | Day 7 | Day 14 | Day 21 | Day 28 | Day 35 | Day 42 | Day 56 | Day 70 |
|---|---|---|---|---|---|---|---|---|
| Conjugate 8 | 0.27 | 0.16 | 0.53 | 0.94 | 0.90 | 1.22 | 1.95 | 3.93 |
| Conjugate 9 | 0.32 | 0.11 | 0.60 | 0.58 | 0.64 | 0.73 | 1.11 | 2.82 |

According to the results shown in Table 9, it can be seen that for human APOC3 transgenic mouse, even half a month after administration, a single subcutaneous injection of 0.16 mg/kg Conjugate 8 or 0.11 mg/kg Conjugate 9 could still realize the efficacy of reducing half of the TG content; and even one month after administration, a single subcutaneous injection of less than 1 mg/kg of the conjugates of the present disclosure could still realize the effect of reducing half of the TG content.

**Experimental Example 3: This experimental example investigated inhibition rate of Conjugate 4 against the expression level of APOC3 mRNA in the *in vivo* liver tissue of human APOC3 transgenic mice.**

Prior to the experiments, the serum triglyceride (TG) contents in human APOC3 transgenic mice (B6; CBA-Tg(APOC3)3707Bres/J) were measured. Transgenic mice with serum TG content > 2 mmol/L were selected and randomly divided into groups. Each of the test groups included 5 mice; and the blank control group (administered with NS) included 4 mice. Conjugate 4 and normal saline (NS) were respectively administered to the mice in groups. The administration dosages for all animals were calculated according to the body weight and subcutaneously injected as single administration. The siRNA conjugate was administered at two doses of 1 mg/kg and 0.1 mg/kg (based on the amount of the siRNA), wherein the siRNA conjugates were provided in 0.9 wt% NaCl aqueous solutions with concentrations of 0.2 mg/ml and 0.02 mg/ml concentration and the administration volume is 5 mL/kg. The mice were sacrificed on day 14 after administration and the liver tissues were collected and stored in RNA later (Sigma Aldrich). Subsequently, the liver tissues were homogenized with a tissue homogenizer, and then the total RNAs in the liver tissues were extracted and obtained by using Trizol (Thermo Fisher) according to the standard procedure for total RNA extraction.

The expression levels of APOC3 mRNA in liver tissues were measured by the same Quantitative Real-Time PCR method as that used in Experimental Example 1-1, except that in this fluorescent qPCR method, β-actin gene was used as an internal control gene, and the expression levels of APOC3 and β-actin were determined by using the primers for APOC3 and β-actin, respectively.

The sequences of the primers for detection were shown in Table 10.

Table 10: Sequences of the primers for detection

| Genes | Upstream Primers | Downstream Primers |
|---|---|---|
| Human APOC3 | 5'-GTGACCGATGGCTTCAGTTC-3' (SEQ ID NO: 323) | 5'- ATGGATAGGCAGGTGGACTT-3' (SEQ ID NO: 324) |
| Mouse β-actin | 5'-AGCTTCTTTGCAGCTCCTTCGTTG-3' (SEQ ID NO: 325) | 5'-TTCTGACCCATTCCCACCATCACA-3' (SEQ ID NO: 326) |

The expression levels of APOC3 mRNA in liver and the inhibition rate of the conjugate against APOC3 mRNA were calculated by the same methods as those used in Experimental Example 1-1. In this experiment, the mice in the control group were administered with normal Saline (NS); and the mice in the test groups were administered with the siRNA conjugate at different concentrations, respectively.

Figure 8 is a scatterplot showing the expression levels of APOC3 mRNA in the *in vivo* liver tissues of human APOC3 trangenic mice administered with normal saline (NS) and Conjugate 4 at different doses (the expression levels used those of mouse β-actin gene as reference, and were normalized against the blank control NS).

The results shows that single administration of 1 mg/kg and 0.1 mg/kg of Conjugate 4 could have significantly inhibitory effect on human APOC3 gene in transgenic mice. In particular, 1 mg/kg Conjugate 4 shows an inhibition rate of up to 82.0% against APOC3 mRNA, and 0.1 mg/kg Conjugate 4 shows an inhibition rate of 40.4% against APOC3 mRNA.

Some embodiments of the present disclosure are described in detail above, but the present disclosure is not limited to the specific details of the above embodiments. Various simple variations to the technical solutions of the present disclosure can be made within the scope of the technical concept of the present disclosure, and these simple variations are also within the scope of the present disclosure.

It is to be noted that each of the specific technical features described in the above embodiments can be combined in any suitable manner provided that no contradiction is caused. In order to avoid unnecessary repetition, various possible combination manners are no longer described in the present disclosure.

In addition, various different embodiments of the present disclosure may also be combined as long as it does not deviate from the idea of the present disclosure, which should also be regarded as the disclosure of the present disclosure.

## INCORPORATION BY REFERENCE

All publications, patents and patent applications mentioned in this description are incorporated herein by reference to the extent as if each publication, patent and patent application were specifically and separately incorporated herein by reference.

**Claims**

1. A siRNA conjugate having a structure as shown by Formula (308):

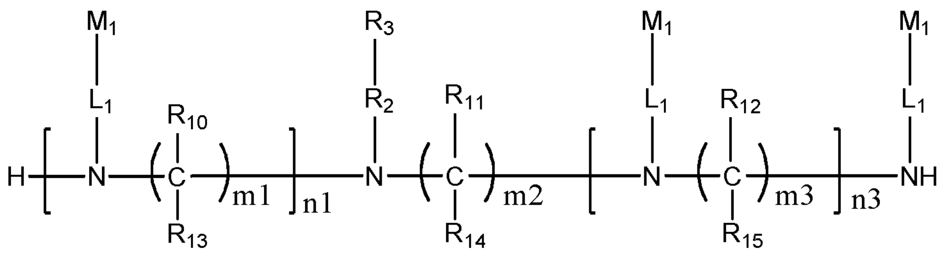

Formula (308),

wherein

n1 is an integer of 1-3, and n3 is an integer of 0-4;
m1, m2, and m3 independently of one another are an integer of 2-10;
$R_{10}$, $R_{11}$, $R_{12}$, $R_{13}$, $R_{14}$, and $R_{15}$ independently of one another are H, or selected from the group consisting of $C_1$-$C_{10}$ alkyl, $C_1$-$C_{10}$ haloalkyl, and $C_1$-$C_{10}$ alkoxy;
$R_3$ is a group having a structure as shown by Formula (A59):

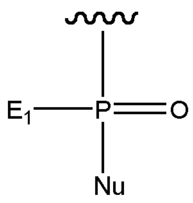

Formula (A59)

wherein,

$E_1$ is OH, SH or $BH_2$;
Nu is a siRNA;
the siRNA comprises a sense strand and an antisense strand; each nucleotide in the siRNA is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; the nucleotide sequence I and the nucleotide sequence II are the sequences selected from one of the following groups i)-v):

i) the nucleotide sequence I and the nucleotide sequence as shown by SEQ ID NO: 1 have an equal length and no more than 3 nucleotide differences, and the nucleotide sequence II and the nucleotide sequence as shown by SEQ ID NO: 2 have an equal length and no more than 3 nucleotide differences; the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$, wherein $Z_{a4}$ is the first nucleotide at 5' terminal of the antisense strand; or

ii) the nucleotide sequence I and the nucleotide sequence as shown by SEQ ID NO: 13 have an equal length and no more than 3 nucleotide differences, and the nucleotide sequence II and the nucleotide sequence as shown by SEQ ID NO: 14 have an equal length and no more than 3 nucleotide differences; the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$, wherein $Z_{b4}$ is the first nucleotide at 5' terminal of the antisense strand; or

iii) the nucleotide sequence I and the nucleotide sequence as shown by SEQ ID NO: 25 have an equal length and no more than 3 nucleotide differences, and the nucleotide sequence II and the nucleotide sequence as shown by SEQ ID NO: 26 have an equal length and no more than 3 nucleotide differences; the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$, wherein $Z_{c4}$ is the first nucleotide at 5' terminal of the antisense strand; or

iv) the nucleotide sequence I and the nucleotide sequence as shown by SEQ ID NO: 37 have an equal length and no more than 3 nucleotide differences, and the nucleotide sequence II and the nucleotide sequence as shown by SEQ ID NO: 38 have an equal length and no more than 3 nucleotide differences; the nucleotide sequence I comprises a nucleotide $Z_{d3}$ at the position corresponding to $Z_{d1}$; the nucleotide sequence II comprises a nucleotide $Z_{d4}$ at the position corresponding to $Z_{d2}$, wherein $Z_{d4}$ is the first nucleotide at 5' terminal of the antisense strand;

or

v) the nucleotide sequence I and the nucleotide sequence as shown by SEQ ID NO: 49 have an equal length and no more than 3 nucleotide differences, and the nucleotide sequence II and the nucleotide sequence as shown by SEQ ID NO: 50 have an equal length and no more than 3 nucleotide differences; the nucleotide sequence I comprises a nucleotide $Z_{e3}$ at the position corresponding to $Z_{e1}$; the nucleotide sequence II comprises a nucleotide $Z_{e4}$ at the position corresponding to $Z_{e2}$, wherein $Z_{e4}$ is the first nucleotide at 5' terminal of the antisense strand;

$R_2$ is a linear alkylene of 1 to 20 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(0)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl,-$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl);

each $L_1$ is a linear alkylene of 1 to 70 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl,-$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl);

 represents the site where a group is linked to the rest of the molecule; and $M_1$ represents a targeting group.

**2.** The siRNA conjugate according to claim 1, wherein each $L_1$ is independently selected from the linkage combinations of one or more of the groups of Formulae (A1)-(A26):

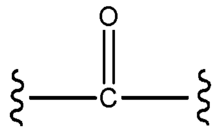, 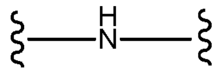, 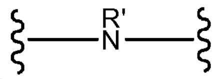, 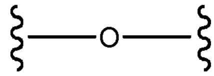,

(A1) (A2) (A3) (A4)

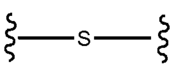, 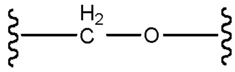, 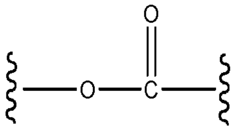, 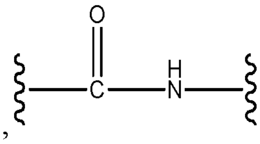,

(A5) (A6) (A7) (A8)

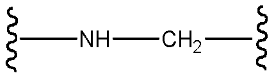, 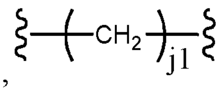, 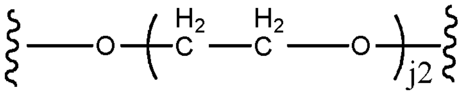,

(A9) (A10) (A11)

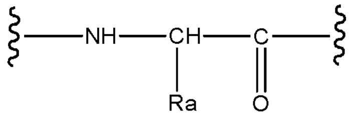, 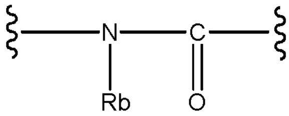, 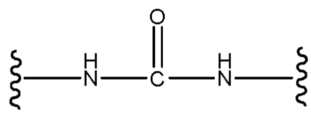,

(A12) (A13) (A14)

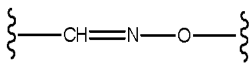, 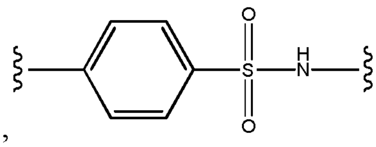, 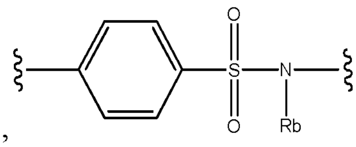,

(A15) (A16) (A17)

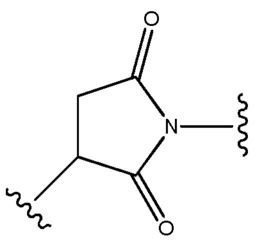, 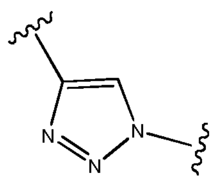, 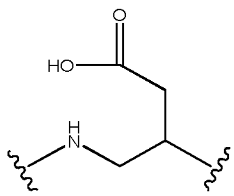, 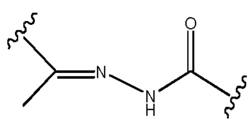,

(A18) (A19) (A20) (A21)

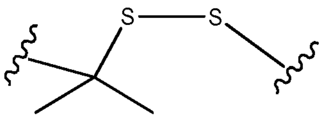 , 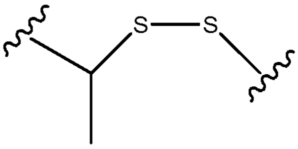 , 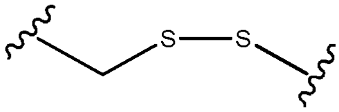 ,

(A22) (A23) (A24)

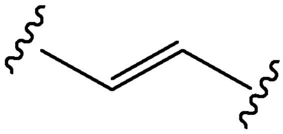 , and 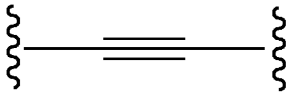 ;

(A25) (A26)

wherein j1 is an integer of 1-20;

j2 is an integer of 1-20;
R' is a $C_1$-$C_{10}$ alkyl;
Ra is selected from the group consisting of the groups of Formulae (A27)- (A45) or any combination thereof:

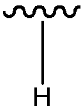 , 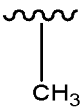 , 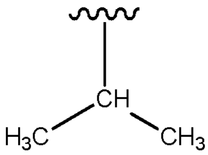 , 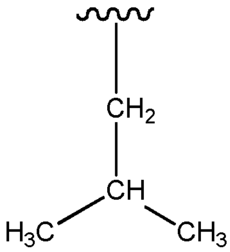 , 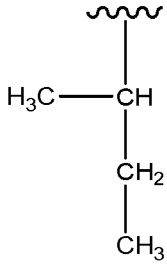 , 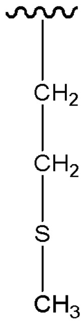 ,

(A27) (A28) (A29) (A30) (A31) (A32)

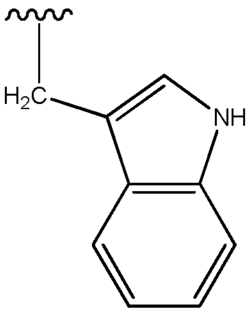 , 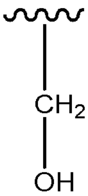 , 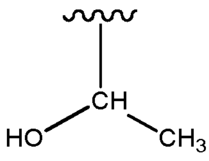 , 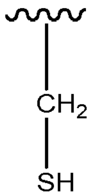 , 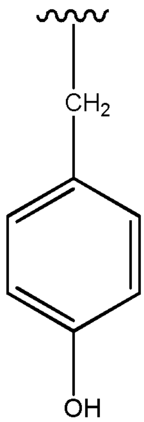 ,

(A33) (A34) (A35) (A36) (A37)

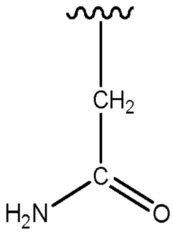,
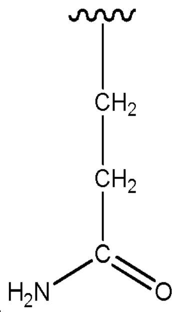,
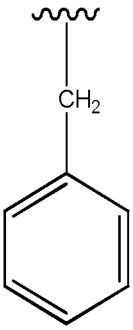,
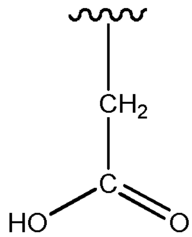,
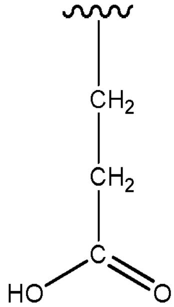,

(A38) (A39) (A40) (A41) (A42)

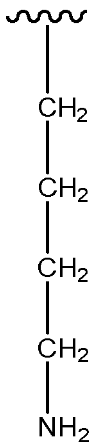,
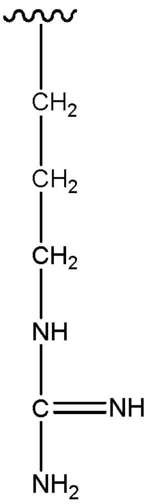, and
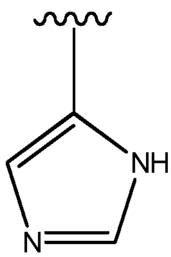;

(A43) (A44) (A45)

Rb is a $C_1$-$C_{10}$ alkyl; or
$L_1$ is selected from the linkage combinations of one or more of Formulae (A1), (A4), (A5), (A6), (A8), (A10), (A11), and (A13); or
$L_1$ is selected from the linkage combinations of at least two of Formulae (A1), (A4), (A8), (A10) and (A11); or
$L_1$ is selected from the linkage combinations of at least two of Formulae (A1), (A8), and (A10); or
$L_1$ has a length of 3 to 25 atoms; or
$L_1$ has a length of 4 to 15 atoms.

**3.** The siRNA conjugate according to claim 2, wherein j 1 is an integer of 2-10; j2 is an integer of 2-10; R' is a $C_1$-$C_4$ alkyl; Ra is one of A27, A28, A29, A30, and A31; and Rb is a $C_1$-$C_5$ alkyl; or
j 1 is is an integer of 3-5; j2 is an integer of 3-5; R' is one of methyl, ethyl, and isopropyl; Ra is a group as shown by Formula A27 or A28; and Rb is one of methyl, ethyl, isopropyl, and butyl.

**4.** The siRNA conjugate according to any one of claims 1-3, wherein n1 is an integer of 1-2; n3 is an integer of 0-1; and n1+n3 = 2-3.

**5.** The siRNA conjugate according to any one of claims 1-4, wherein m1, m2 and m3 independently of one another are an integer of 2-5, and/or m1 = m2 = m3.

**6.** The siRNA conjugate according to any one of claims 1-5, wherein each of the targeting groups is independently a ligand that has affinity to the asialoglycoprotein receptor on the surface of mammalian hepatocyte; or

each of the targeting groups is independently an asialoglycoprotein or a saccharide; or
each of the targeting groups is independently selected from the group consisting of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-P-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, L-4-thioribose; or
at least one or each of the targeting groups is galactose or N-acetylgalactosamine.

**7.** The siRNA conjugate according to any one of claims 1-6, wherein $R_{10}$, $R_{11}$, $R_{12}$, $R_{13}$, $R_{14}$, and $R_{15}$ independently of one another are H, methyl or ethyl; or
$R_{10}$, $R_{11}$, $R_{12}$, $R_{13}$, $R_{14}$, and $R_{15}$ are H.

**8.** The siRNA conjugate according to any one of claims 1-7, wherein $R_2$ comprises both a site linking to the N atom on the nitrogenous backbone and a site linking to the P atom in $R_3$; or

in $R_2$, the site linking to the N atom on the nitrogenous backbone forms an amide bond with the N atom, and the site linking to the P atom in $R_3$ forms a phosphoester bond with the P atom;
or $R_2$ is a functional group having a structure as shown by any one of Fromula (B5),(B6),(B5') or (B6).

**9.** The siRNA conjugate according to any one of claims 1-8, wherein the conjugate has a structure as shown by Formula (403), (404), (405), (406), (407), (408), (409), (410), (411), (412), (413), (414), (415), (416), (417), (418), (419), (420), (421), or (422).

**10.** The siRNA conjugate according to any one of claims 1-9, wherein the P atom in Formula A59 is linked to a terminal region of the sense or antisense strand of the siRNA, and the terminal region refers to the first 4 nucleotides counted from one terminal of the sense or antisense strand; or

the P atom in Formula A59 is linked to one terminal of the sense or antisense strand of the siRNA; or
the P atom in Formula A59 is linked to 3' terminal of the sense strand of the siRNA; or
the P atom in Formula A59 is linked to position 2', 3', or 5' of a nucleotide in the siRNA by forming a phosphodiester bond.

**11.** The siRNA conjugate according to claim 1, wherein

i) the nucleotide sequence I has no more than 1 nucleotide difference from the nucleotide sequence as shown by SEQ ID NO: 1, and/or the nucleotide sequence II has no more than 1 nucleotide difference from the nucleotide sequence as shown by SEQ ID NO: 2; or
ii) the nucleotide sequence I has no more than 1 nucleotide difference from the nucleotide sequence as shown by SEQ ID NO: 13, and/or the nucleotide sequence II has no more than 1 nucleotide difference from the nucleotide sequence as shown by SEQ ID NO: 14; or
iii) the nucleotide sequence I has no more than 1 nucleotide difference from the nucleotide sequence as shown by SEQ ID NO: 25, and/or the nucleotide sequence II has no more than 1 nucleotide difference from the nucleotide sequence as shown by SEQ ID NO: 26; or
iv) the nucleotide sequence I has no more than 1 nucleotide difference from the nucleotide sequence as shown by SEQ ID NO: 37, and/or the nucleotide sequence II has no more than 1 nucleotide difference from the nucleotide sequence as shown by SEQ ID NO: 38; or
v) the nucleotide sequence I has no more than 1 nucleotide difference from the nucleotide sequence as shown by SEQ ID NO: 49, and/or the nucleotide sequence II has no more than 1 nucleotide difference from the nucleotide sequence as shown by SEQ ID NO: 50.

**12.** The siRNA conjugate according to claim 1 or 11, wherein

i) the nucleotide difference between the nucleotide sequence II and the nucleotide sequence as shown by SEQ

ID NO: 2 includes a difference at the position $Z_{a4}$, and $Z_{a4}$ is selected from A, C or G; or
ii) the nucleotide difference between the nucleotide sequence II and the nucleotide sequence as shown by SEQ ID NO: 14 includes a difference at the position $Z_{b4}$, and $Z_{b4}$ is selected from A, C or G; or
iii) the nucleotide difference between the nucleotide sequence II and the nucleotide sequence as shown by SEQ ID NO: 26 includes a difference at the position $Z_{c4}$, and $Z_{c4}$ is selected from A, C or G; or
iv) the nucleotide difference between the nucleotide sequence II and the nucleotide sequence as shown by SEQ ID NO: 38 includes a difference at the position $Z_{d4}$, and $Z_{d4}$ is selected from A, C or G; or
v) the nucleotide difference between the nucleotide sequence II and the nucleotide sequence as shown by SEQ ID NO: 50 includes a difference at the position $Z_{e4}$, and $Z_{e4}$ is selected from A, C or G.

**13.** The siRNA conjugate according to claim 12, wherein $Z_{a3}$ is a neucleotide complementary to $Z_{a4}$; or

$Z_{b3}$ is a neucleotide complementary to $Z_{b4}$; or
$Z_{c3}$ is a neucleotide complementary to $Z_{c4}$; or
$Z_{d3}$ is a neucleotide complementary to $Z_{d4}$; or
$Z_{e3}$ is a neucleotide complementary to $Z_{e4}$.

**14.** The siRNA conjugate according to any one of claims 11-13, wherein the nucleotide sequence I and the nucleotide sequence II are basically reverse complementary, substantially reverse complementary, or completely reverse complementary to each other; the "basically reverse complementary" means that there is no more than 3 base mispairings between two nucleotide sequences; the "substantially reverse complementary" means that there is no more than 1 base mispairing between two nucleotide sequences; and the "completely reverse complementary" means that there is no mispairing between two nucleotide sequences.

**15.** The siRNA conjugate according to any one of claims 11-14, wherein the sense strand further comprises a nucleotide sequence III, the antisense strand further comprises a nucleotide sequence IV; and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; the nucleotide sequence III and the nucleotide sequence IV have an equal length, and are substantially reverse complementary or completely reverse complementary to each other; the "substantially reverse complementary" means that there is no more than 1 base mispairing between two nucleotide sequences; and the "completely reverse complementary" means that there is no mispairing between two nucleotide sequences.

**16.** The siRNA conjugate according to claim 15, wherein i) the nucleotide sequence I is the nucleotide sequence as shown by SEQ ID NO: 3; the nucleotide sequence II is the nucleotide sequence as shown by SEQ ID NO: 4; and the nucleotide sequence III and the nucleotide sequence IV both have a length of 1 nucleotide, and the base of the nucleotide sequence III is C; or

the nucleotide sequence III and the nucleotide sequence IV both have a length of 2 nucleotides, and in the direction from 5' terminal to 3' terminal, the bases of the nucleotide sequence III is GC; or
the nucleotide sequence III and the nucleotide sequence IV both have a length of 3 nucleotides, and in the direction from 5' terminal to 3' terminal, the bases of the nucleotide sequence III is UGC; or
the nucleotide sequence III and the nucleotide sequence IV both have a length of 4 nucleotides, and in the direction from 5' terminal to 3' terminal, the bases of the nucleotide sequence III is UUGC; or

ii) the nucleotide sequence I is the nucleotide sequence as shown by SEQ ID NO: 15; the nucleotide sequence II is the nucleotide sequence as shown by SEQ ID NO: 16; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is GG; or
the nucleotide sequence III and the nucleotide sequence IV both have a length of 3 nucleotides, and in the direction from 5' terminal to 3' terminal, the bases of the nucleotide sequence III is AGG; or
the nucleotide sequence III and the nucleotide sequence IV both have a length of 4 nucleotides, and in the direction from 5' terminal to 3' terminal, the bases of the nucleotide sequence III is AGGG; or

iii) the nucleotide sequence I is the nucleotide sequence as shown by SEQ ID NO: 25; the nucleotide

sequence II is the nucleotide sequence as shown by SEQ ID NO: 26; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is AG; or
the nucleotide sequence III and the nucleotide sequence IV both have a length of 3 nucleotides, and in the direction from 5' terminal to 3' terminal, the bases of the nucleotide sequence III is CAG; or
the nucleotide sequence III and the nucleotide sequence IV both have a length of 4 nucleotides, and in the direction from 5' terminal to 3' terminal, the bases of the nucleotide sequence III is ACAG; or

iv) the nucleotide sequence I is the nucleotide sequence as shown by SEQ ID NO: 39; the nucleotide sequence II is the nucleotide sequence as shown by SEQ ID NO: 40; and the nucleotide sequence III and the nucleotide sequence IV both have a length of 1 nucleotide, and the base of the nucleotide sequence III is C; or

the nucleotide sequence III and the nucleotide sequence IV both have a length of 2 nucleotides, and in the direction from 5' terminal to 3' terminal, the bases of the nucleotide sequence III is AC; or
the nucleotide sequence III and the nucleotide sequence IV both have a length of 3 nucleotides, and in the direction from 5' terminal to 3' terminal, the bases of the nucleotide sequence III is GAC; or
the nucleotide sequence III and the nucleotide sequence IV both have a length of 4 nucleotides, and in the direction from 5' terminal to 3' terminal, the bases of the nucleotide sequence III is GGAC; or

v) the nucleotide sequence I is the nucleotide sequence as shown by SEQ ID NO: 51; the nucleotide sequence II is the nucleotide sequence as shown by SEQ ID NO: 52; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is AG; or
the nucleotide sequence III and the nucleotide sequence IV both have a length of 3 nucleotides, and in the direction from 5' terminal to 3' terminal, the bases of the nucleotide sequence III is AAG; or
the nucleotide sequence III and the nucleotide sequence IV both have a length of 4 nucleotides, and in the direction from 5' terminal to 3' terminal, the bases of the nucleotide sequence III is AAAG.

**17.** The siRNA conjugate according to claim 16, wherein the nucleotide sequence III and the nucleotide sequence IV are completely reverse complementary to each other.

**18.** The siRNA conjugate according to any one of claims 11-17, wherein the antisense strand further comprises a nucleotide sequence V, which has a length of 1 to 3 nucleotides and is linked to 3' terminal of the antisense strand, thereby forming a 3' overhang terminal of the antisense strand; or

the nucleotide sequence V has a length of 2 nucleotides; or
the nucleotide sequence V is 2 consecutive thymine deoxyribonucleotides or 2 consecutive uracil ribonucleotides; or
the nucleotide sequence V is complementary to the nucleotides at the corresponding positions of the target mRNA.

**19.** The siRNA conjugate according to any one of claims 11-18, wherein the sense strand of the siRNA comprises the nucleotide sequence as shown by SEQ ID NO: 5, and the antisense strand of the siRNA comprises the nucleotide sequence as shown by SEQ ID NO: 6; or

the sense strand of the siRNA comprises the nucleotide sequence as shown by SEQ ID NO: 7, and the antisense strand of the siRNA comprises the nucleotide sequence as shown by SEQ ID NO: 8; or
the sense strand of the siRNA comprises the nucleotide sequence as shown by SEQ ID NO: 17, and the antisense strand of the siRNA comprises the nucleotide sequence as shown by SEQ ID NO: 18; or
the sense strand of the siRNA comprises the nucleotide sequence as shown by SEQ ID NO: 19, and the antisense strand of the siRNA comprises the nucleotide sequence as shown by SEQ ID NO: 20; or

the sense strand of the siRNA comprises the nucleotide sequence as shown by SEQ ID NO: 29, and the antisense strand of the siRNA comprises the nucleotide sequence as shown by SEQ ID NO: 30; or
the sense strand of the siRNA comprises the nucleotide sequence as shown by SEQ ID NO: 31, and the antisense strand of the siRNA comprises the nucleotide sequence as shown by SEQ ID NO: 32; or
the sense strand of the siRNA comprises the nucleotide sequence as shown by SEQ ID NO: 41, and the antisense strand of the siRNA comprises the nucleotide sequence as shown by SEQ ID NO: 42; or
the sense strand of the siRNA comprises the nucleotide sequence as shown by SEQ ID NO: 43, and the antisense strand of the siRNA comprises the nucleotide sequence as shown by SEQ ID NO: 44; or
the sense strand of the siRNA comprises the nucleotide sequence as shown by SEQ ID NO: 53, and the antisense strand of the siRNA comprises the nucleotide sequence as shown by SEQ ID NO: 54; or
the sense strand of the siRNA comprises the nucleotide sequence as shown by SEQ ID NO: 55, and the antisense strand of the siRNA comprises the nucleotide sequence as shown by SEQ ID NO: 56.

**20.** The siRNA conjugate according to any one of claims 11-19, wherein the siRNA is siAPa1, siAPa2, siAPb1, siAPb2, siAPc1, siAPc2, siAPd1, siAPd2, siAPe1, or siAPe2.

**21.** The siRNA conjugate according to any one of claims 11-20, wherein at least one nucleotide in the sense strand or the antisense strand is a modified nucleotide, and/or at least one phosphate group is a phosphate group with modified group(s).

**22.** The siRNA conjugate according to claim 21, wherein each nucleotide in the sense strand and antisense strand is independently a fluoro modified nucleotide or a non-fluoro modified nucleotide; or

the fluoro modified nucleotides are located in the nucleotide sequence I and the nucleotide sequence II; and in the direction from 5' terminal to 3' terminal, the nucleotides at positions 7, 8 and 9 of the nucleotide sequence I are fluoro modified nucleotides; and in the direction from 5'terminal to 3' terminal, the nucleotides at positions 2, 6, 14, and 16 of the nucleotide sequence II are fluoro modified nucleotides; or
in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14 and 16 or at positions 2, 6, 8, 9, 14, and 16 of the nucleotide sequence II in the antisense strand are fluoro modified nucleotides, and the nucleotides at the other positions in the antisense strand are non-fluoro modified nucleotides.

**23.** The siRNA conjugate according to claim 21 or 22, wherein each non-fluoro modified nucleotide is independently a nucleotide formed by substituting the 2' -hydroxy of the ribose group of the nucleotide with a non-fluoro group, or a nucleotide analogue; or

the nucleotide formed by substituting the 2' -hydroxy of the ribose group with a non-fluoro group is selected from the group consisting of 2' -alkoxy modified nucleotide, 2' -substituted alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide; and the nucleotide analogue is selected from the group consisting of an isonucleotide, LNA, ENA, cET, UNA, and GNA; or
each non-fluoro modified nucleotide is a methoxy modified nucleotide, wherein the methoxy modified nucleotide refers to a nucleotide formed by substituting 2'-hydroxy of the ribose group with a methoxy group.

**24.** The siRNA conjugate according to any one of claims 21-23, wherein in the direction from 5' terminal to 3' terminal, the nucleotides at positions 5, 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the other positions in the antisense strand of the siRNA are methoxy modified nucleotides; or

in the direction from 5' terminal to 3' terminal, the nucleotides at positions 5, 7, 8 and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand

are methoxy modified nucleotides; or

in the direction from 5' terminal to 3' terminal, the nucleotides at positions 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides.

**25.** The siRNA conjugate according to any one of claims 11-24, wherein the siRNA is any one of siAPa1-M1, si APa2-M1, siAPa1-M2, siAPa2-M2, siAPa1-M3, siAPa2-M3, siAPb1-M1, siAPb2-M1, siAPb1-M2, siAPb2-M2, siAPb1-M3, siAPb2-M3, siAPc1-M1, siAPc2-M1, siAPc1-M2, siAPc2-M2, siAPc1-M3, siAPc2-M3, siAPd1-M1, siAPd2-M1, siAPd1-M2, siAPd2-M2, siAPd1-M3, siAPd2-M3, siAPe1-M1, siAPe2-M1, siAPe1-M2, siAPe2-M2, siAPe1-M3, and siAPe2-M3.

**26.** The siRNA conjugate according to any one of claims 11-25, wherein the phosphate group with modified group(s) is a phosphorothioate group formed by substituting at least one oxygen atom in a phosphodiester bond in a phosphate group with a sulfur atom; or the phosphate group with modified group(s) is a phosphorothioate group having a structure as shown by Formula (1):

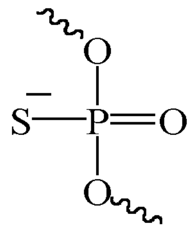

Formula (1).

**27.** The siRNA conjugate according to claim 26, wherein in the siRNA, the phosphorothioate linkage is located in at least one of the group consisting of the following positions:

the position between the first and second nucleotides at 5' terminal of the sense strand;
the position between the second and third nucleotides at 5' terminal of the sense strand;
the position between the first and second nucleotides at 3' terminal of the sense strand;
the position between the second and third nucleotides at 3' terminal of the sense strand;
the position between the first and second nucleotides at 5' terminal of the antisense strand;
the position between the second and third nucleotides at 5' terminal of the antisense strand;
the position between the first and second nucleotides at 3' terminal of the antisense strand; and
the position between the second and third nucleotides at 3' terminal of the antisense strand.

**28.** The siRNA conjugate according to any one of claims 11-27, wherein the siRNA is any one of siAPa1-M1S, siAPa2-M1S, siAPa1-M2S, siAPa2-M2S, siAPa1-M3S, siAPa2-M3S, siAPb1-M1S, siAPb2-M1S, siAPb1-M2S, siAPb2-M2S, siAPb1-M3S, siAPb2-M3S, siAPc1-M1S, siAPc2-M1S, siAPc1-M2S, siAPc2-M2S, siAPc1-M3S, siAPc2-M3S, siAPd1-M1S, siAPd2-M1S, siAPd1-M2S, siAPd2-M2S, siAPd1-M3S, siAPd2-M3S, siAPe1-M1S, siAPe2-M1S, siAPe1-M2S, siAPe2-M2S, siAPe1-M3S, and siAPe2-M3S.

**29.** The siRNA conjugate according to any one of claims 11-28, wherein the nucleotide at 5'-terminal of the antisense strand of the siRNA is a 5'-phosphate nucleotide or a 5'-phosphate analogue modified nucleotide; or

the 5' -phosphate nucleotide is a nucleotide having a structure as shown by Formula (2); and the 5'-phosphate analogue modified nucleotide is a nucleotide having a structure as shown by any one of Formulae (3) to (6):

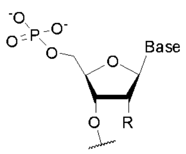 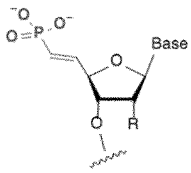 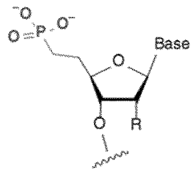 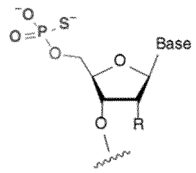 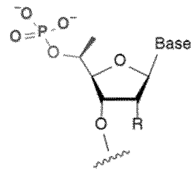

Formula (2) Formula (3) Formula (4) Formula (5) Formula (6)

wherein R is selected from H, OH, methoxy or F; "Base" represents a base selected from A, U, C, G, or T.

**30.** The siRNA conjugate according to any one of claims 11-29, wherein the siRNA is any one of siAPa1-M1P1, siAPa2-M1P1, siAPa1-M2P1, siAPa2-M2P1, siAPa1-M3P1, siAPa2-M3P1, siAPa1-M1SP1, siAPa2-M1SP1, siAPa1-M2SP1, siAPa2-M2SP1, siAPa1-M3SP1, siAPa2-M3SP1, siAPa1U-M1P1, siAPa2U-M1P1, siAPa1U-M2P1, siAPa2U-M2P1, siAPa1U-M3P1, siAPa2U-M3P1, siAPa1U-M1SP1, siAPa2U-M1SP1, siAPa1U-M2SP1, siAPa2U-M2SP1, siAPa1U-M3SP1, siAPa2U-M3SP1, siAPb1-M1P1, siAPb2-M1P1, siAPb1-M2P1, siAPb2-M2P1, siAPb1-M3P1, siAPb2-M3P1, siAPb1-M1SP1, siAPb2-MlSP1, siAPb1-M2SP1, siAPb2-M2SP1, siAPb1-M3SP1, siAPb2-M3SP1, siAPb1U-M1P1, siAPb2U-M1P1, siAPb1U-M2P1, siAPb2U-M2P1, siAPb1U-M3P1, siAPb2U-M3P1, siAPb1U-M1SP1, siAPb2U-M1SP1, siAPb1U-M2SP1, siAPb2U-M2SP1, siAPb1U-M3SP1, siAPb2U-M3SP1, siAPc1-M1P1, siAPc2-M1P1, siAPc1-M2P1, siAPc2-M2P1, siAPc1-M3P1, siAPc2-M3P1, siAPc1-M1SP1, siAPc2-M1SP1, siAPc1-M2SP1, siAPc2-M2SP1, siAPc1-M3SP1, siAPc2-M3SP1, siAPd1-M1P1, siAPd2-M1P1, siAPd1-M2P1, siAPd2-M2P1, siAPd1-M3P1, siAPd2-M3P1, siAPd1-M1SP1, siAPd2-M1SP1, siAPd1-M2SP1, siAPd2-M2SP1, siAPd1-M3SP1, siAPd2-M3SP1, siAPd1U-M1P1, siAPd2U-M1P1, siAPd1U-M2P1, siAPd2U-M2P1, siAPd1U-M3P1, siAPd2U-M3P1, siAPd1U-M1SP1, siAPd2U-M1SP1, siAPd1U-M2SP1, siAPd2U-M2SP1, siAPd1U-M3SP1, siAPd2U-M3SP1, siAPe1-M1P1, siAPe2-M1P1, siAPe1-M2P1, siAPe2-M2P1, siAPe1-M3P1, siAPe2-M3P1, siAPe1-M1SP1, siAPe2-M1SP1, siAPe1-M2SP1, siAPe2-M2SP1, siAPe1-M3SP1, siAPe2-M3SP1, siAPe1U-M1P1, siAPe2U-M1P1, siAPe1U-M2P1, siAPe2U-M2P1, siAPe1U-M3P1, siAPe2U-M3P1, siAPe1U-M1SP1, siAPe2U-M1SP1, siAPe1U-M2SP1, siAPe2U-M2SP1, siAPe1U-M3SP1, and siAPe2U-M3SP1.

**31.** A siRNA comprising a sense strand and an antisense strand; each nucleotide in the ense strand and antisense strand is independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; the fluoro modified nucleotides are located in the nucleotide sequence I and the nucleotide sequence II; and in the direction from 5' terminal to 3' terminal, the nucleotides at positions 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14, and 16 of the nucleotide sequence II in the antisense strand are fluoro modified nucleotides, and the nucleotides at the other positions in the antisense strand are non-fluoro modified nucleotides; and

i) the nucleotide sequence I and the nucleotide sequence as shown by SEQ ID NO: 1 have an equal length and no more than 3 nucleotide differences, and the nucleotide sequence II and the nucleotide sequence as shown by SEQ ID NO: 2 have an equal length and no more than 3 nucleotide differences; the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$, wherein $Z_{a4}$ is the first nucleotide at 5' terminal of the antisense strand; or

ii) the nucleotide sequence I and the nucleotide sequence as shown by SEQ ID NO: 13 have an equal length and no more than 3 nucleotide differences, and the nucleotide sequence II and the nucleotide sequence as shown by SEQ ID NO: 14 have an equal length and no more than 3 nucleotide differences; the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$, wherein $Z_{b4}$ is the first nucleotide at 5' terminal of the antisense strand; or

iii) the nucleotide sequence I and the nucleotide sequence as shown by SEQ ID NO: 25 have an equal length and no more than 3 nucleotide differences, and the nucleotide sequence II and the nucleotide sequence as shown by SEQ ID NO: 26 have an equal length and no more than 3 nucleotide differences; the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$, wherein $Z_{c4}$ is the first nucleotide at 5' terminal of the antisense strand; or

iv) the nucleotide sequence I and the nucleotide sequence as shown by SEQ ID NO: 37 have an equal length and no more than 3 nucleotide differences, and the nucleotide sequence II and the nucleotide sequence as shown by SEQ ID NO: 38 have an equal length and no more than 3 nucleotide differences; the nucleotide sequence I comprises a nucleotide $Z_{d3}$ at the position corresponding to $Z_{d1}$; the nucleotide sequence II comprises a nucleotide $Z_{d4}$ at the position corresponding to $Z_{d2}$, wherein $Z_{d4}$ is the first nucleotide at 5' terminal of the antisense strand; or

v) the nucleotide sequence I and the nucleotide sequence as shown by SEQ ID NO: 49 have an equal length and no more than 3 nucleotide differences, and the nucleotide sequence II and the nucleotide sequence as shown by SEQ ID NO: 50 have an equal length and no more than 3 nucleotide differences; the nucleotide sequence I comprises a nucleotide $Z_{e3}$ at the position corresponding to $Z_{e1}$; the nucleotide sequence II comprises a nucleotide $Z_{e4}$ at the position corresponding to $Z_{e2}$, wherein $Z_{e4}$ is the first nucleotide at 5' terminal of the antisense strand.

**32.** The siRNA according to claim 31, wherein each non-fluoro modified nucleotide is independently a nucleotide formed by substituting the 2'-hydroxy of the ribose group thereof with a non-fluoro group, or a nucleotide analogue.

**33.** The siRNA according to claim 32, wherein the nucleotide formed by substituting the 2' - hydroxy of the ribose group with a non-fluoro group is selected from the group consisting of 2'-alkoxy modified nucleotide, 2' -substituted alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide; and the nucleotide analogue is selected from the group consisting of an isonucleotide, LNA, ENA, cET, UNA, and GNA.

**34.** The siRNA according to any one of claims 31-33, wherein each non-fluoro modified nucleotide is a methoxy modified nucleotide, wherein the methoxy modified nucleotide refers to a nucleotide formed by substituting 2'-hydroxy of the ribose group with a methoxy group.

**35.** The siRNA according to any one of claims 31-34, wherein

i) the nucleotide sequence I has no more than 1 nucleotide difference from the nucleotide sequence as shown by SEQ ID NO: 1, and/or the nucleotide sequence II has no more than 1 nucleotide difference from the nucleotide sequence as shown by SEQ ID NO: 2; or

ii) the nucleotide sequence I has no more than 1 nucleotide difference from the nucleotide sequence as shown by SEQ ID NO: 13, and/or the nucleotide sequence II has no more than 1 nucleotide difference from the nucleotide sequence as shown by SEQ ID NO: 14; or

iii) the nucleotide sequence I has no more than 1 nucleotide difference from the nucleotide sequence as shown by SEQ ID NO: 25, and/or the nucleotide sequence II has no more than 1 nucleotide difference from the nucleotide sequence as shown by SEQ ID NO: 26; or

iv) the nucleotide sequence I has no more than 1 nucleotide difference from the nucleotide sequence as shown by SEQ ID NO: 37, and/or the nucleotide sequence II has no more than 1 nucleotide difference from the nucleotide sequence as shown by SEQ ID NO: 38; or

v) the nucleotide sequence I has no more than 1 nucleotide difference from the nucleotide sequence as shown by SEQ ID NO: 49, and/or the nucleotide sequence II has no more than 1 nucleotide difference from the nucleotide sequence as shown by SEQ ID NO: 50.

**36.** The siRNA according to any one of claims 31-35, wherein

i) the nucleotide difference between the nucleotide sequence II and the nucleotide sequence as shown by SEQ ID NO: 2 includes a difference at the position $Z_{a4}$, and $Z_{a4}$ is selected from A, C or G; or

ii) the nucleotide difference between the nucleotide sequence II and the nucleotide sequence as shown by SEQ ID NO: 14 includes a difference at the position $Z_{b4}$, and $Z_{b4}$ is selected from A, C or G; or

iii) the nucleotide difference between the nucleotide sequence II and the nucleotide sequence as shown by SEQ ID NO: 26 includes a difference at the position $Z_{c4}$, and $Z_{c4}$ is selected from A, C or G; or

iv) the nucleotide difference between the nucleotide sequence II and the nucleotide sequence as shown by SEQ ID NO: 38 includes a difference at the position $Z_{d4}$, and $Z_{d4}$ is selected from A, C or G; or

v) the nucleotide difference between the nucleotide sequence II and the nucleotide sequence as shown by SEQ ID NO: 50 includes a difference at the position $Z_{e4}$, and $Z_{e4}$ is selected from A, C or G.

**37.** The siRNA according to claim 36, wherein $Z_{a3}$ is a neucleotide complementary to $Z_{a4}$; or

$Z_{b3}$ is a neucleotide complementary to $Z_{b4}$; or
$Z_{c3}$ is a neucleotide complementary to $Z_{c4}$; or
$Z_{d3}$ is a neucleotide complementary to $Z_{d4}$; or
$Z_{e3}$ is a neucleotide complementary to $Z_{e4}$.

**38.** The siRNA according to any one of claims 31-37, wherein the nucleotide sequence I and the nucleotide sequence II are basically reverse complementary, substantially reverse complementary, or completely reverse complementary to each other; the "basically reverse complementary" means that there is no more than 3 base mispairings between two nucleotide sequences; the "substantially reverse complementary" means that there is no more than 1 base mispairing between two nucleotide sequences; and the "completely reverse complementary" means that there is no mispairing between two nucleotide sequences.

**39.** The siRNA according to any one of claims 31-38, wherein the sense strand further comprises a nucleotide sequence III, the antisense strand further comprises a nucleotide sequence IV; and the nucleotide sequence III and the nucleotide sequence IV independently of one another have a length of 1 to 4 nucleotides; the nucleotide sequence III is linked to 5' terminal of the nucleotide sequence I, the nucleotide sequence IV is linked to 3' terminal of the nucleotide sequence II; the nucleotide sequence III and the nucleotide sequence IV have an equal length, and are substantially reverse complementary or completely reverse complementary to each other; the "substantially reverse complementary" means that there is no more than 1 base mispairing between two nucleotide sequences; and the "completely reverse complementary" means that there is no mispairing between two nucleotide sequences.

**40.** The siRNA according to any one of claims 31-39, wherein

i) the nucleotide sequence I is the nucleotide sequence as shown by SEQ ID NO: 3; the nucleotide sequence II is the nucleotide sequence as shown by SEQ ID NO: 4; and the nucleotide sequence III and the nucleotide sequence IV both have a length of 1 nucleotide, and the base of the nucleotide sequence III is C; or

the nucleotide sequence III and the nucleotide sequence IV both have a length of 2 nucleotides, and in the direction from 5' terminal to 3' terminal, the bases of the nucleotide sequence III is GC; or
the nucleotide sequence III and the nucleotide sequence IV both have a length of 3 nucleotides, and in the direction from 5' terminal to 3' terminal, the bases of the nucleotide sequence III is UGC; or
the nucleotide sequence III and the nucleotide sequence IV both have a length of 4 nucleotides, and in the direction from 5' terminal to 3' terminal, the bases of the nucleotide sequence III is UUGC; or

ii) the nucleotide sequence I is the nucleotide sequence as shown by SEQ ID NO: 15; the nucleotide sequence II is the nucleotide sequence as shown by SEQ ID NO: 16; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is GG; or
the nucleotide sequence III and the nucleotide sequence IV both have a length of 3 nucleotides, and in the direction from 5' terminal to 3' terminal, the bases of the nucleotide sequence III is AGG; or
the nucleotide sequence III and the nucleotide sequence IV both have a length of 4 nucleotides, and in the direction from 5' terminal to 3' terminal, the bases of the nucleotide sequence III is AGGG; or

iii) the nucleotide sequence I is the nucleotide sequence as shown by SEQ ID NO: 25; the nucleotide sequence II is the nucleotide sequence as shown by SEQ ID NO: 26; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is AG; or
the nucleotide sequence III and the nucleotide sequence IV both have a length of 3 nucleotides, and in the direction from 5' terminal to 3' terminal, the bases of the nucleotide sequence III is CAG; or
the nucleotide sequence III and the nucleotide sequence IV both have a length of 4 nucleotides, and in the direction from 5' terminal to 3' terminal, the bases of the nucleotide sequence III is ACAG; or

iv) the nucleotide sequence I is the nucleotide sequence as shown by SEQ ID NO: 39; the nucleotide sequence II is the nucleotide sequence as shown by SEQ ID NO: 40; and the nucleotide sequence III and the nucleotide

sequence IV both have a length of 1 nucleotide, and the base of the nucleotide sequence III is C; or

the nucleotide sequence III and the nucleotide sequence IV both have a length of 2 nucleotides, and in the direction from 5' terminal to 3' terminal, the bases of the nucleotide sequence III is AC; or
the nucleotide sequence III and the nucleotide sequence IV both have a length of 3 nucleotides, and in the direction from 5' terminal to 3' terminal, the bases of the nucleotide sequence III is GAC; or
the nucleotide sequence III and the nucleotide sequence IV both have a length of 4 nucleotides, and in the direction from 5' terminal to 3' terminal, the bases of the nucleotide sequence III is GGAC; or

v) the nucleotide sequence I is the nucleotide sequence as shown by SEQ ID NO: 51; the nucleotide sequence II is the nucleotide sequence as shown by SEQ ID NO: 52; and the nucleotide sequence III and the nucleotide sequence IV both have a length of 1 nucleotide, and the base of the nucleotide sequence III is G; or

the nucleotide sequence III and the nucleotide sequence IV both have a length of 2 nucleotides, and in the direction from 5' terminal to 3' terminal, the bases of the nucleotide sequence III is AG; or
the nucleotide sequence III and the nucleotide sequence IV both have a length of 3 nucleotides, and in the direction from 5' terminal to 3' terminal, the bases of the nucleotide sequence III is AAG; or
the nucleotide sequence III and the nucleotide sequence IV both have a length of 4 nucleotides, and in the direction from 5' terminal to 3' terminal, the bases of the nucleotide sequence III is AAAG.

**41.** The siRNA according to claim 40, wherein the nucleotide sequence III and the nucleotide sequence IV are completely reverse complementary to each other.

**42.** The siRNA according to any one of claims 31-41, wherein the antisense strand further comprises nucleotide sequence V, which has a length of 1 to 3 nucleotides and is linked to 3' terminal of the antisense strand, thereby forming a 3' overhang terminal of the antisense strand; or

the nucleotide sequence V has a length of 2 nucleotides; or
the nucleotide sequence V is 2 consecutive thymine deoxyribonucleotides or 2 consecutive uracil ribonucleotides; or
the nucleotide sequence V is complementary to the nucleotides at the corresponding positions of the target mRNA.

**43.** The siRNA according to any one of claims 31-42, wherein the sense strand of the siRNA comprises the nucleotide sequence as shown by SEQ ID NO: 5, and the antisense strand of the siRNA comprises the nucleotide sequence as shown by SEQ ID NO: 6; or

the sense strand of the siRNA comprises the nucleotide sequence as shown by SEQ ID NO: 7, and the antisense strand of the siRNA comprises the nucleotide sequence as shown by SEQ ID NO: 8; or
the sense strand of the siRNA comprises the nucleotide sequence as shown by SEQ ID NO: 17, and the antisense strand of the siRNA comprises the nucleotide sequence as shown by SEQ ID NO: 18; or
the sense strand of the siRNA comprises the nucleotide sequence as shown by SEQ ID NO: 19, and the antisense strand of the siRNA comprises the nucleotide sequence as shown by SEQ ID NO: 20; or
the sense strand of the siRNA comprises the nucleotide sequence as shown by SEQ ID NO: 29, and the antisense strand of the siRNA comprises the nucleotide sequence as shown by SEQ ID NO: 30; or
the sense strand of the siRNA comprises the nucleotide sequence as shown by SEQ ID NO: 31, and the antisense strand of the siRNA comprises the nucleotide sequence as shown by SEQ ID NO: 32; or
the sense strand of the siRNA comprises the nucleotide sequence as shown by SEQ ID NO: 41, and the antisense strand of the siRNA comprises the nucleotide sequence as shown by SEQ ID NO: 42; or
the sense strand of the siRNA comprises the nucleotide sequence as shown by SEQ ID NO: 43, and the antisense strand of the siRNA comprises the nucleotide sequence as shown by SEQ ID NO: 44; or
the sense strand of the siRNA comprises the nucleotide sequence as shown by SEQ ID NO: 53, and the antisense strand of the siRNA comprises the nucleotide sequence as shown by SEQ ID NO: 54; or
the sense strand of the siRNA comprises the nucleotide sequence as shown by SEQ ID NO: 55, and the antisense strand of the siRNA comprises the nucleotide sequence as shown by SEQ ID NO: 56.

**44.** The siRNA according to any one of claims 31-43, wherein the siRNA has the nucleotide sequence as shown by siAPa1, siAPa2, siAPb1, siAPb2, siAPc1, siAPc2, siAPd1, siAPd2, siAPe1, or siAPe2.

**45.** The siRNA according to any one of claims 31-44, wherein the siRNA is any one of siAPa1-M3, siAPa2-M3, siAPb1-M3, siAPb2-M3, siAPc1-M3, siAPc2-M3, siAPd1-M3, siAPd2-M3, siAPe1-M3, and siAPe2-M3.

**46.** The siRNA according to any one of claims 31-45, wherein at least one phosphate group in the sense strand or the antisense strand is a phosphate group with modified group(s).

**47.** The siRNA according to claim 46, wherein the phosphate group with modified group(s) is a phosphorothioate group formed by substituting at least one oxygen atom in a phosphodiester bond in a phosphate group with a sulfur atom; or the phosphate group with modified group(s) is a phosphorothioate group having a structure as shown by Formula (1):

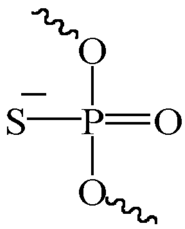

Formula (1).

**48.** The siRNA according to claim 47, wherein in the siRNA, the phosphorothioate linkage is located in at least one of the group consisting of the following positions:

the position between the first and second nucleotides at 5’ terminal of the sense strand;
the position between the second and third nucleotides at 5’ terminal of the sense strand;
the position between the first and second nucleotides at 3’ terminal of the sense strand;
the position between the second and third nucleotides at 3’ terminal of the sense strand;
the position between the first and second nucleotides at 5’ terminal of the antisense strand;
the position between the second and third nucleotides at 5’ terminal of the antisense strand;
the position between the first and second nucleotides at 3’ terminal of the antisense strand; and
the position between the second and third nucleotides at 3’ terminal of the antisense strand.

**49.** The siRNA according to any one of claims 31-48, wherein the siRNA is any one of siAPa1-M3S, siAPa2-M3S, siAPb1-M3S, siAPb2-M3S, siAPc1-M3S, siAPc2-M3S, siAPd1-M3S, siAPd2-M3S, siAPe1-M3S and siAPe2-M3S.

**50.** The siRNA according to any one of claims 31-49, wherein the nucleotide at 5’-terminal of

the antisense strand of the siRNA is a 5’-phosphate nucleotide or a 5’-phosphate analogue modified nucleotide; or the 5’ -phosphate nucleotide is a nucleotide having a structure as shown by Formula (2); and the 5’-phosphate analogue modified nucleotide is a nucleotide having a structure as shown by any one of Formulae (3) to (6):

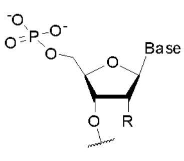
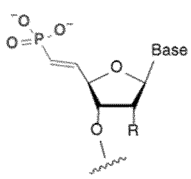
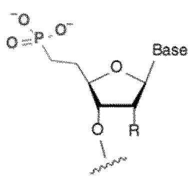
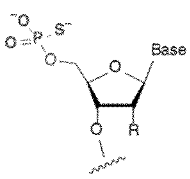
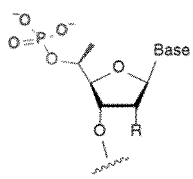

Formula (2) Formula (3) Formula (4) Formula (5) Formula (6)

wherein R is selected from H, OH, methoxy, or F;
"Base" represents a base selected from A, U, C, G, or T.

**51.** The siRNA according to any one of claims 31-50, wherein the siRNA is any one of siAPa1-M3P1, siAPa2-M3P1, siAPa1-M3SP1, siAPa2-M3SP1, siAPa1U-M3P1, siAPa2U-M3P1, siAPa1U-M3SP1, si APa2U-M3SP1, siAPb1-M3P1, siAPb2-M3P1, siAPb1-M3SP1, siAPb2-M3SP1, siAPb1U-M3P1, siAPb2U-M3P1, siAPb1U-M3SP1, siAPb2U-M3SP1, siAPc1-M3P1, siAPc2-M3P1, siAPc1-M3SP1, siAPc2-M3SP1, siAPd1-M3P1, siAPd2-M3P1, siAPd1-M3SP1, siAPd2-M3SP1, siAPd1U-M3P1, siAPd2U-M3P1, siAPd1U-M3SP1, siAPd2U-M3SP1, siAPe1-M3P1, siAPe2-M3P1, siAPe1-M3SP1, siAPe2-M3SP1, siAPe1U-M3P1, siAPe2U-M3P1, siAPe1U-M3SP1, and siAPe2U-M3SP1.

**52.** The siRNA according to any one of claims 31-51, wherein the siRNA is any one of siAPa1UM3SVP, siAPe1UM3SVP, siAPb1UM3SVP, siAPd1UM3SVP, siAPc1M3SVP, siAPd1UM3SP, siAPd1UM3SPs, siAPa1M3SP, siAPe1M3SP, siAPb1M3SP and siAPc1M3SP.

**53.** A pharmaceutical composition, comprising the siRNA according to any one of claims 31-52, and a pharmaceutically acceptable carrier.

**54.** A siRNA conjugate, comprising the siRNA according to any one of claims 31-52, and a conjugation group conjugatively linked to the siRNA.

**55.** Use of the siRNA conjugate according to any one of claims 1-30 and 54, the siRNA according to any one of claims 31-52 and/or the pharmaceutical composition according to claim 53 in the manufacture of a medicament for treating and/or preventing dyslipidemia.

**56.** A method for treating and/or preventing dyslipidemia, comprising administering an effective amount of the siRNA conjugate according to any one of claims 1-30 and 54, the siRNA according to any one of claims 31-52 and/or the pharmaceutical composition according to claim 53 to a subject suffering from dyslipidemia.

**57.** A method for inhibiting the expression of APOC3 gene in hepatocytes, comprising contacting an effective amount of the siRNA conjugate according to any one of claims 1-30 and 54, the siRNA according to any one of claims 31-52 and/or the pharmaceutical composition according to claim 53 with the hepatocytes.

**58.** A kit, comprising the siRNA conjugate according to any one of claims 1-30 and 54, the siRNA according to any one of claims 31-52 and/or the pharmaceutical composition according to claim 53.

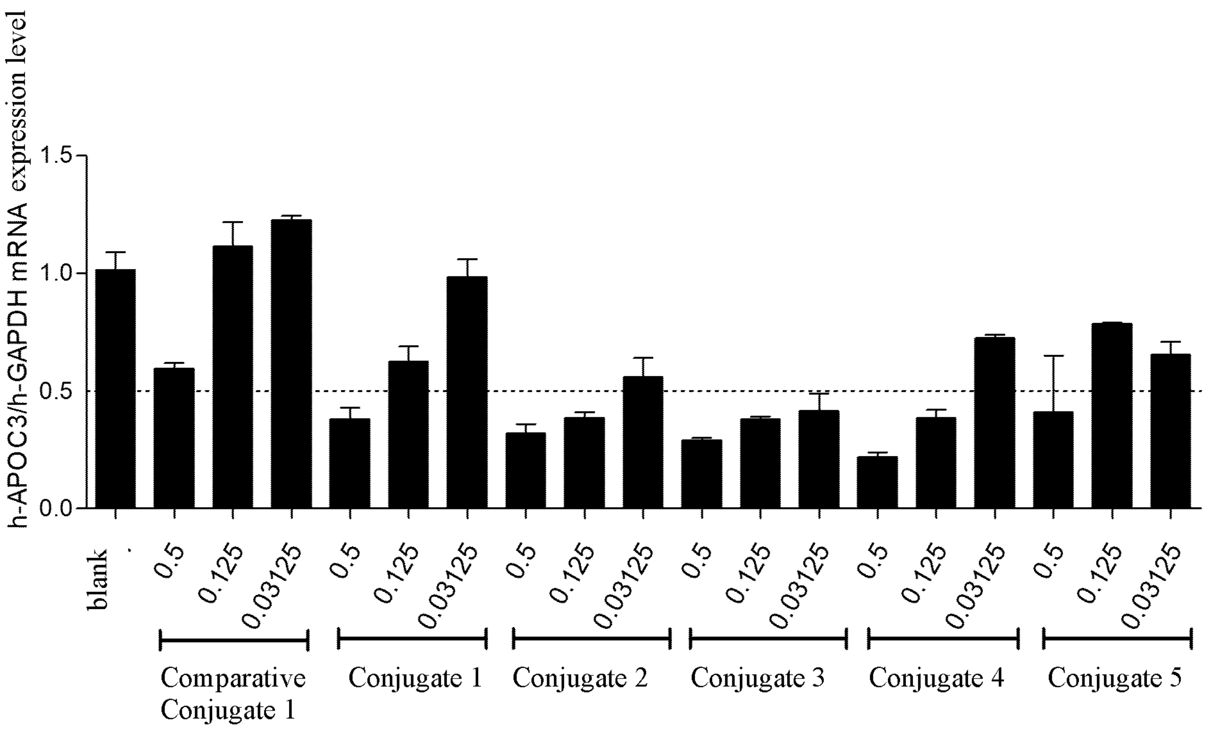
h-APOC3/h-GAPDH mRNA expression level
1.5
1.0
0.5
0.0
blank
0.5
0.125
0.03125
0.5
0.125
0.03125
0.5
0.125
0.03125
0.5
0.125
0.03125
0.5
0.125
0.03125
0.5
0.125
0.03125
Comparative Conjugate 1
Conjugate 1
Conjugate 2
Conjugate 3
Conjugate 4
Conjugate 5

Figure 1

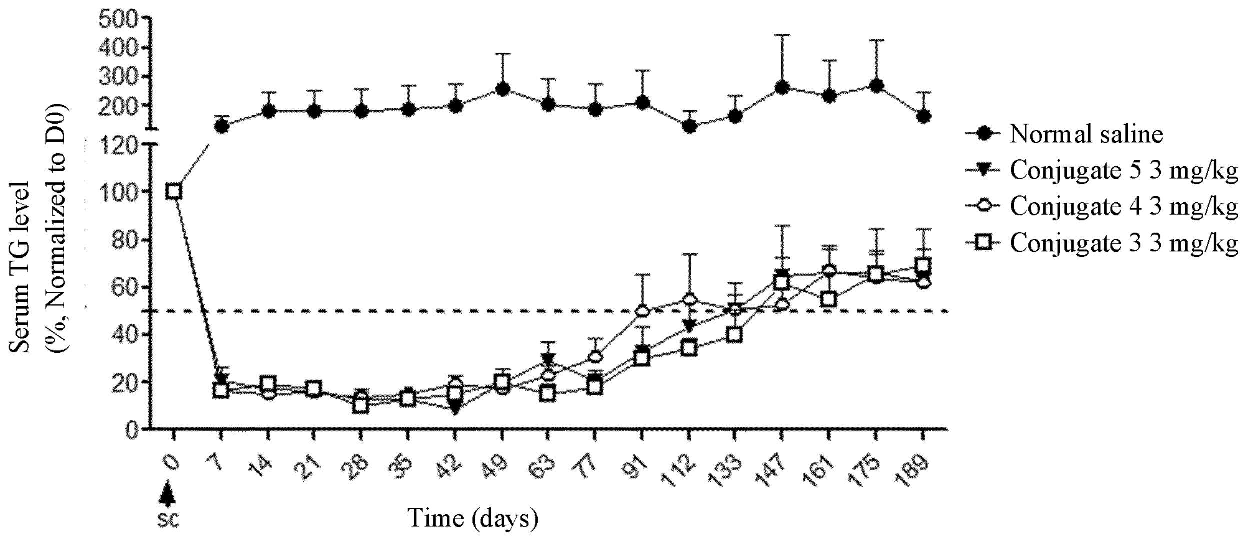


**Figure 2A**

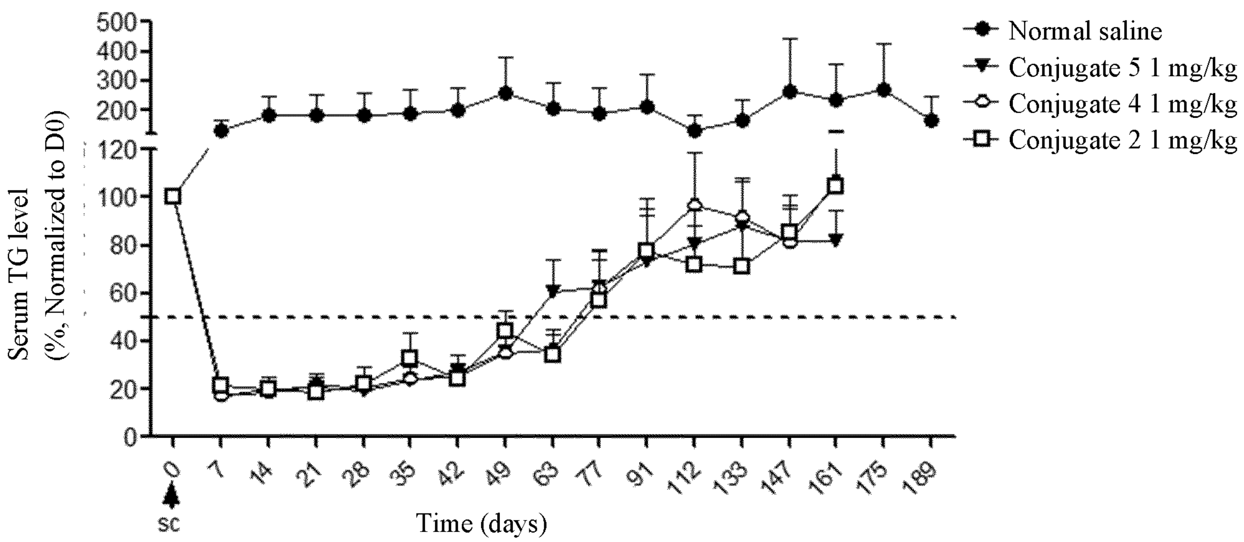


**Figure 2B**

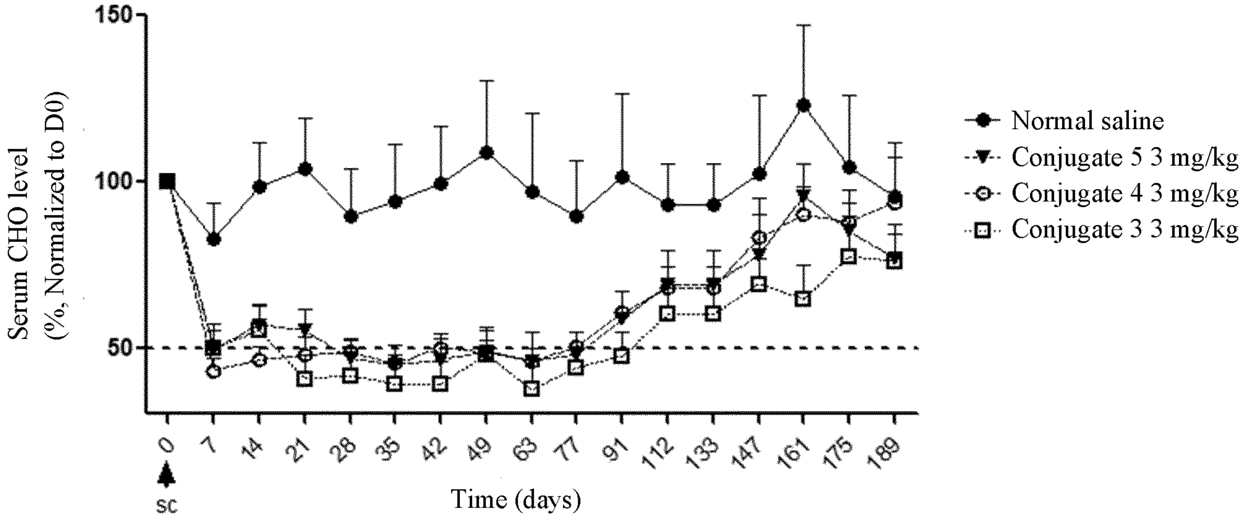


**Figure 2C**

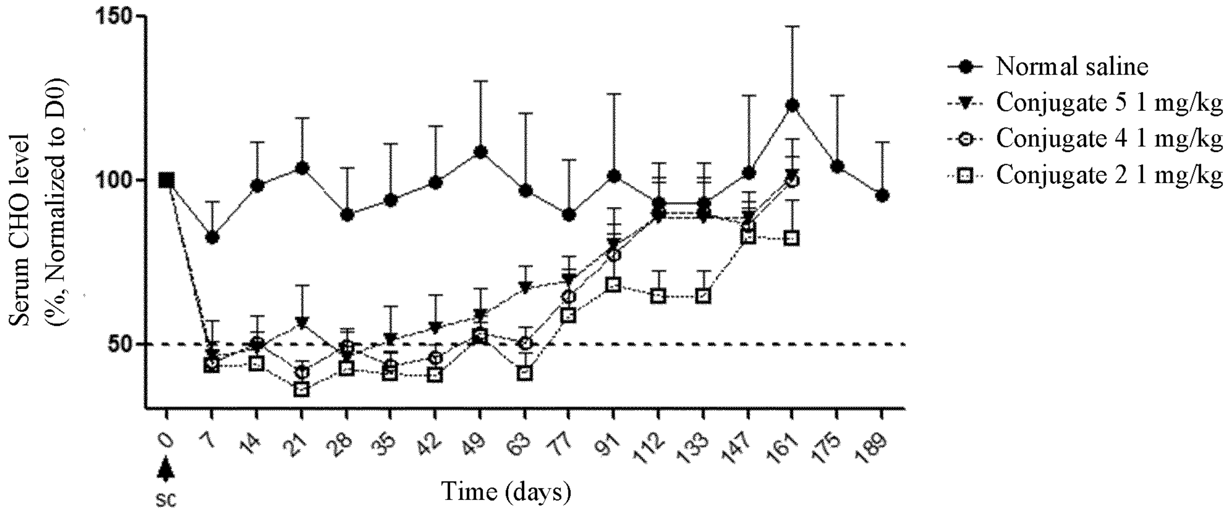


**Figure 2D**

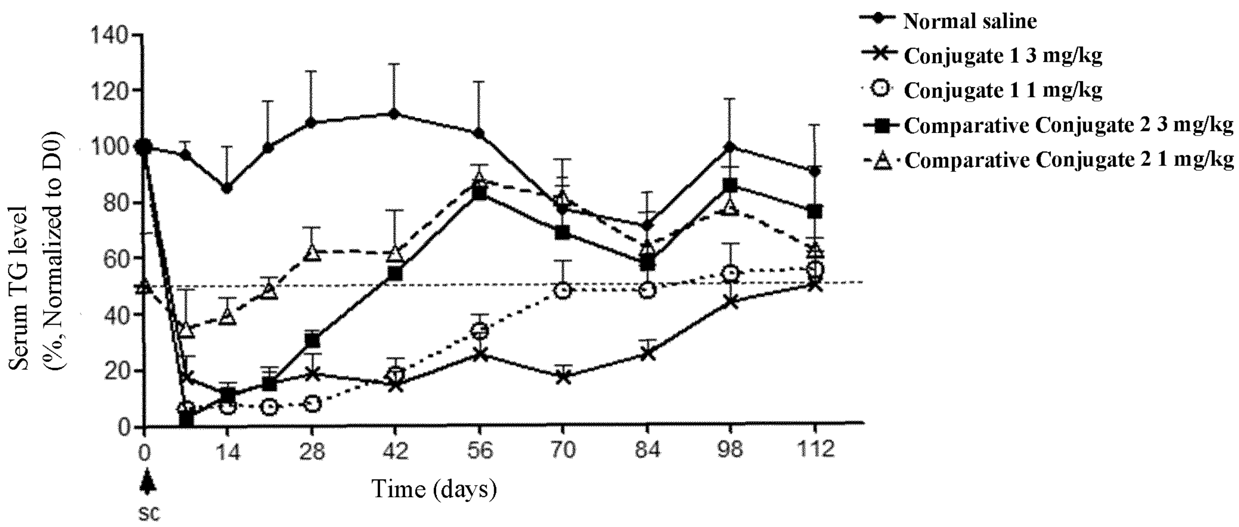


Figure 3A

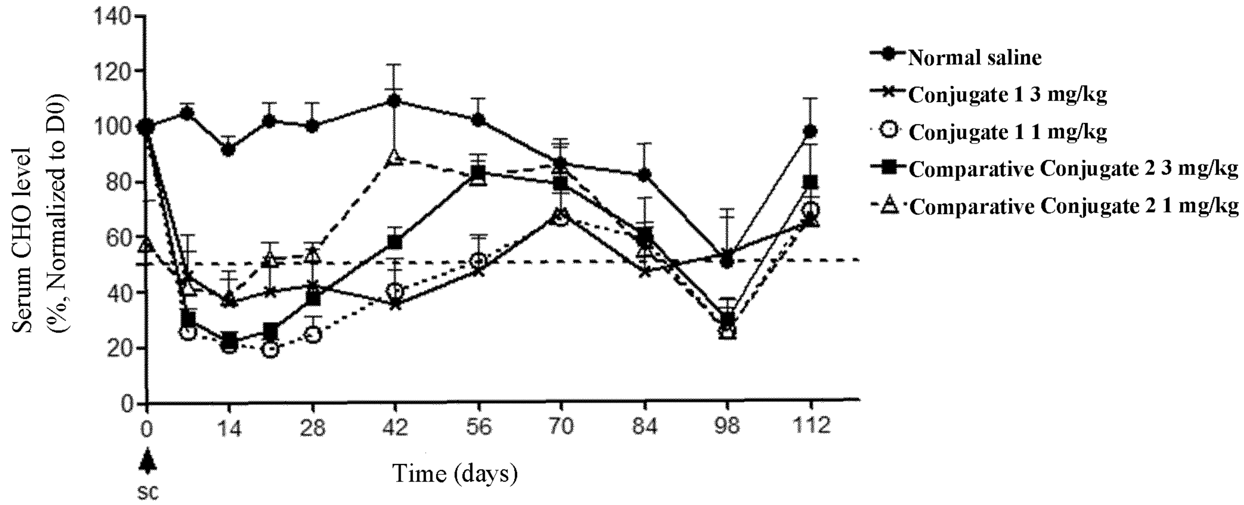


Figure 3B

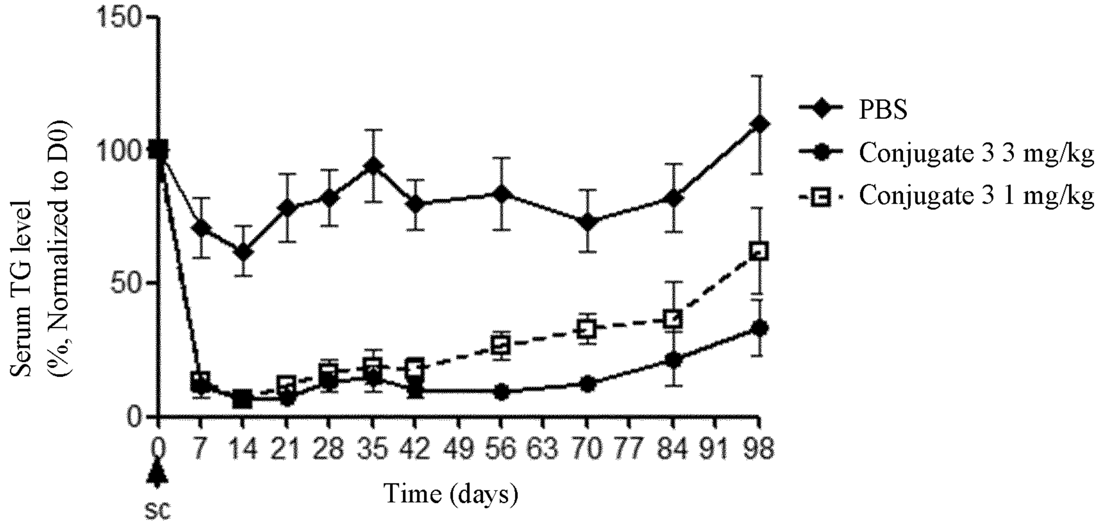


**Figure 4A**

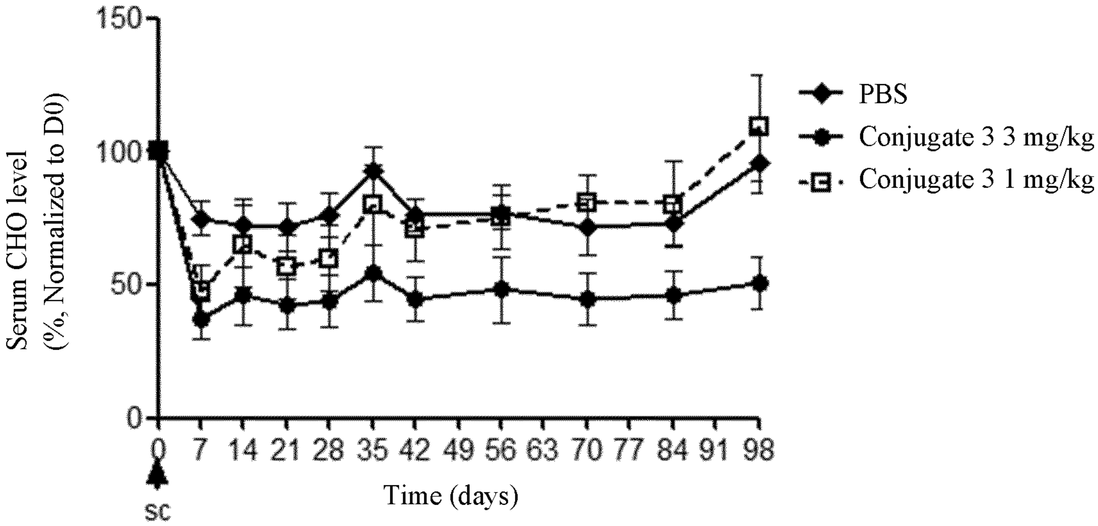


**Figure 4B**

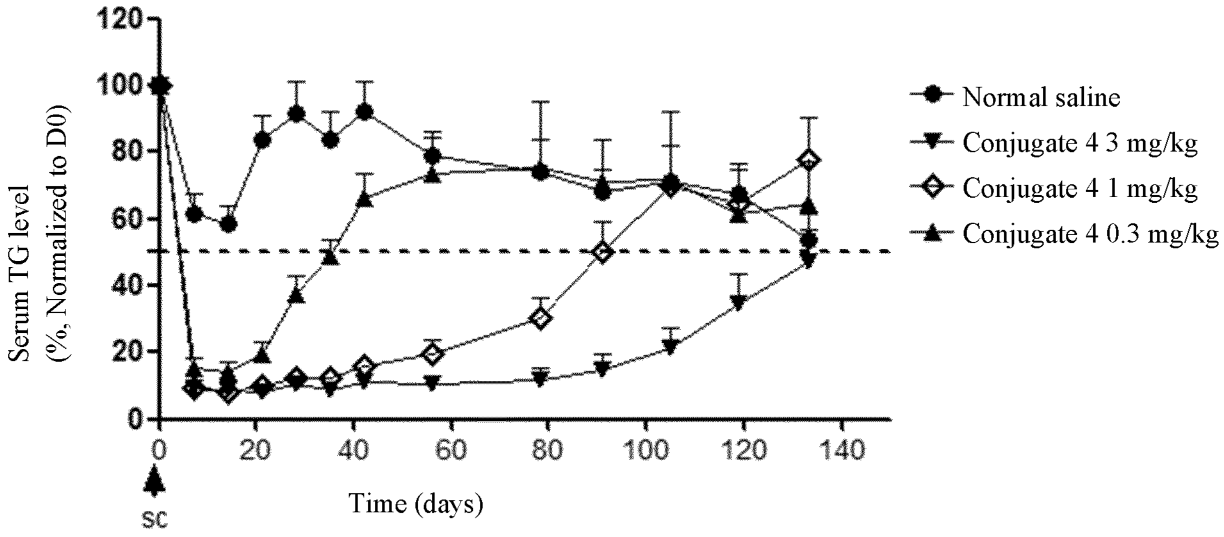


**Figure 5A**

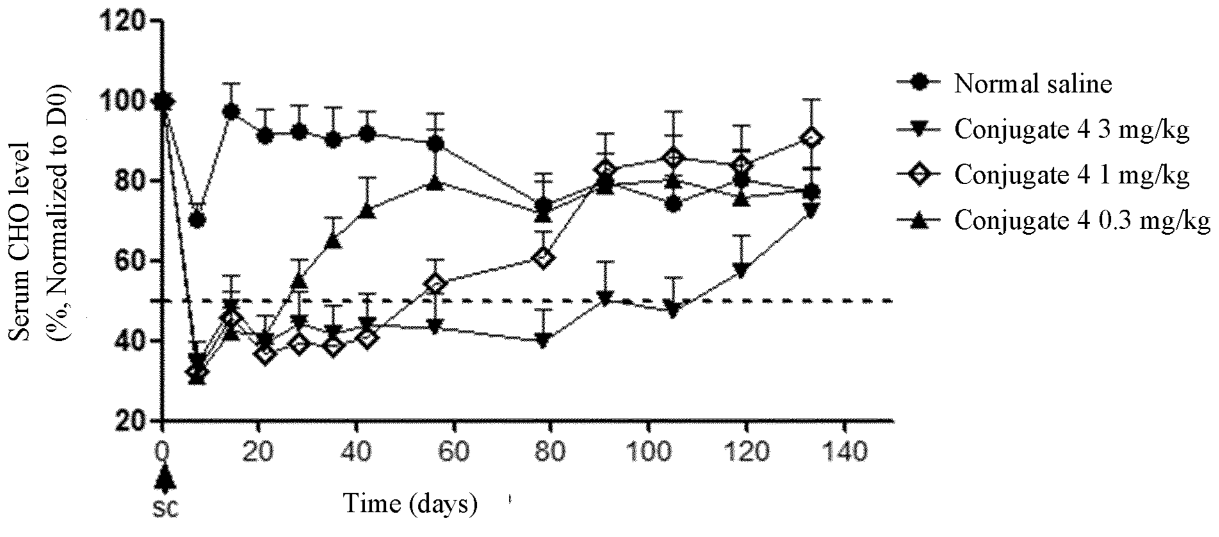


**Figure 5B**

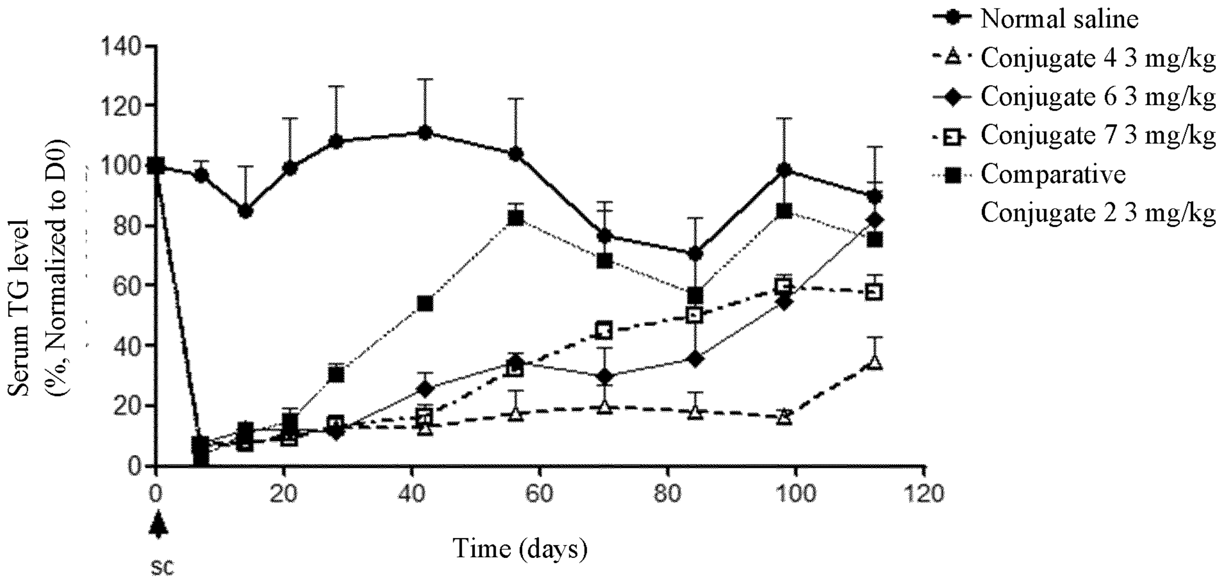


**Figure 6A**

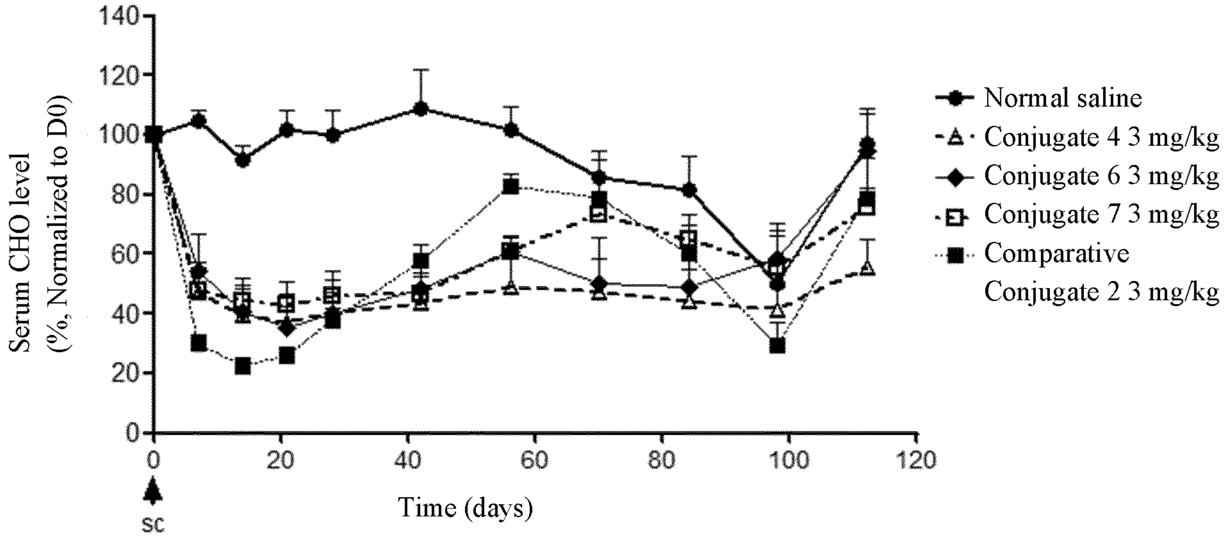


**Figure 6B**

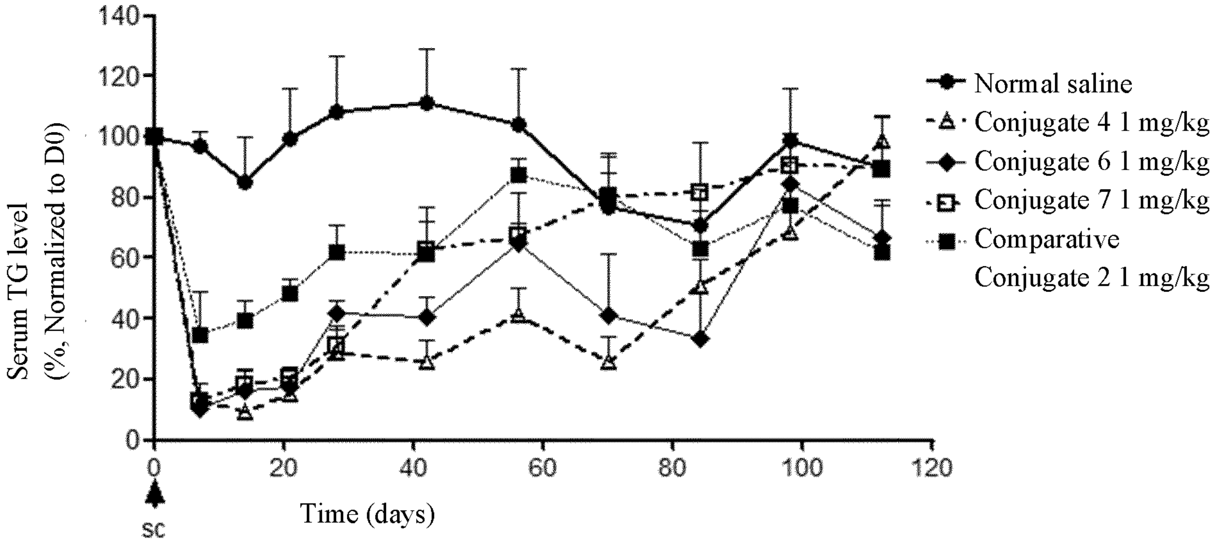


**Figure 6C**

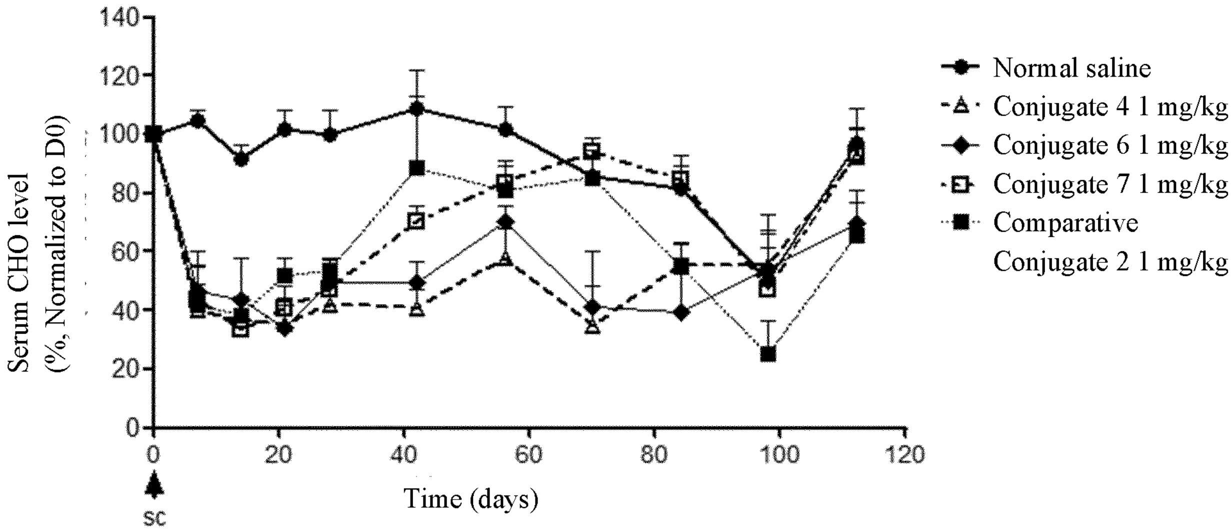


**Figure 6D**

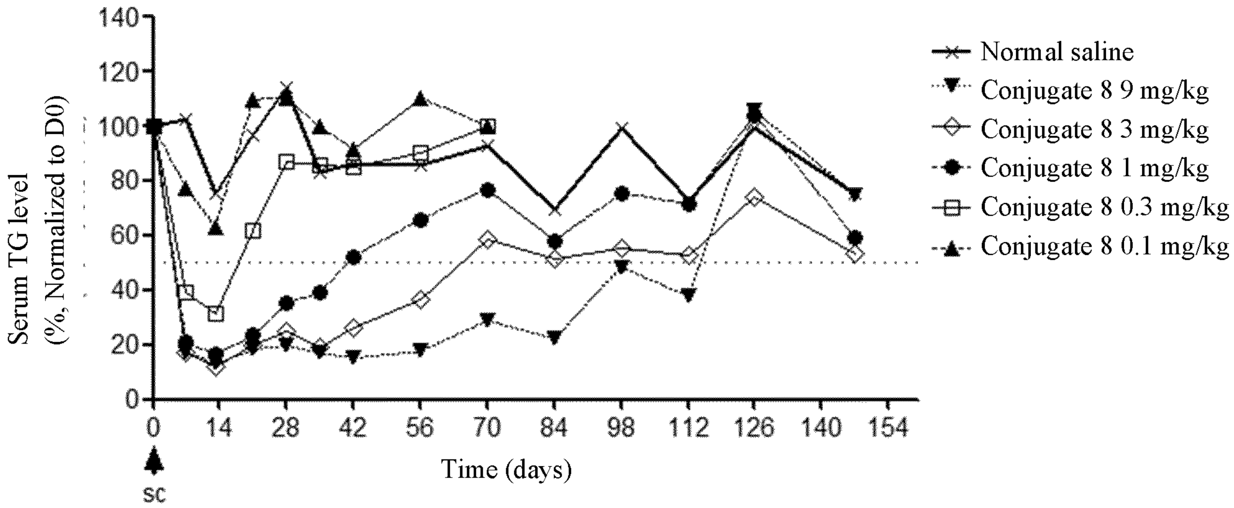


**Figure 7A**

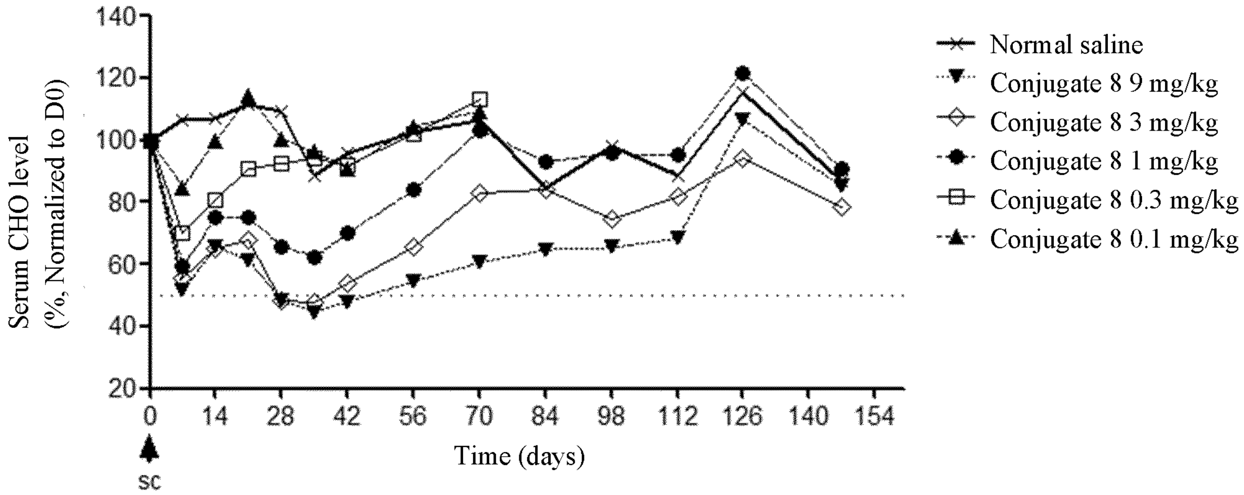


**Figure 7B**

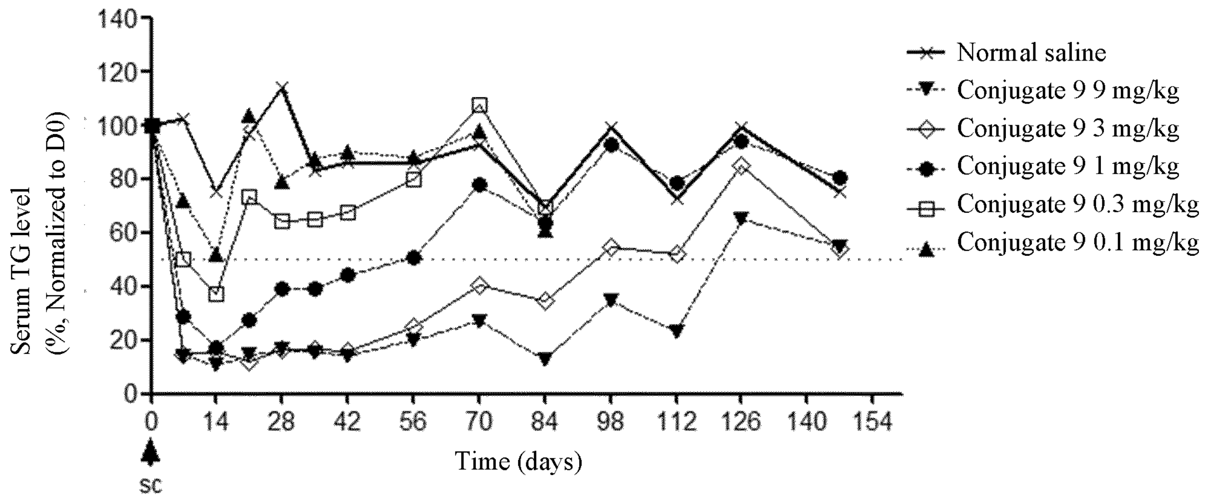


**Figure 7C**

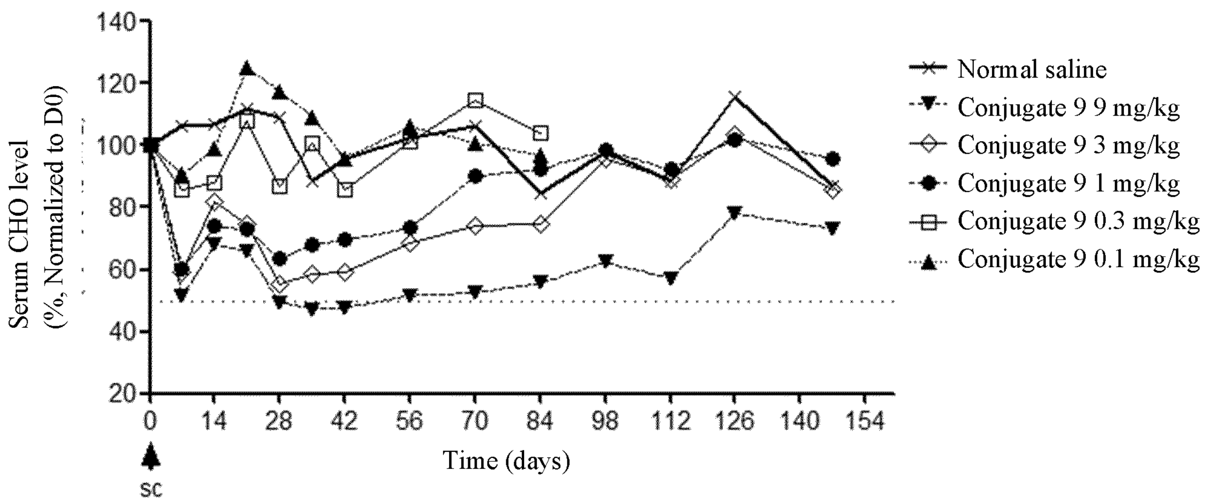


**Figure 7D**

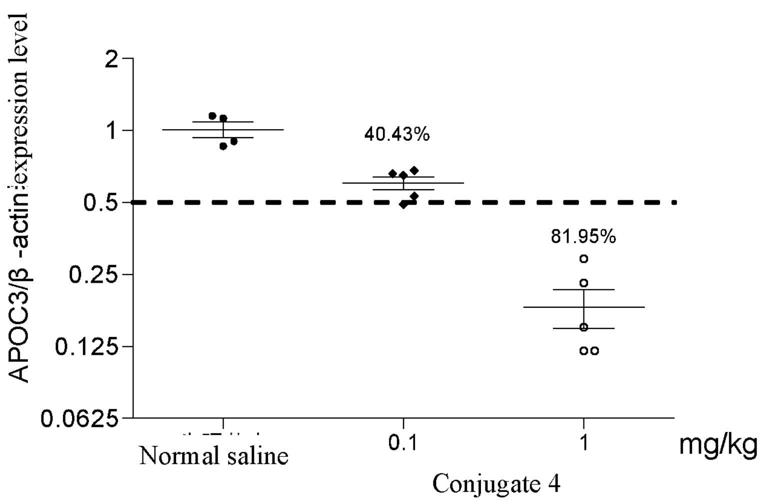


**Figure 8**

**INTERNATIONAL SEARCH REPORT**

International application No.
**PCT/CN2019/129016**

**A. CLASSIFICATION OF SUBJECT MATTER**

A61K 48/00(2006.01)i; C12N 15/113(2010.01)i; A61P 3/06(2006.01)i

According to International Patent Classification (IPC) or to both national classification and IPC

**B. FIELDS SEARCHED**

Minimum documentation searched (classification system followed by classification symbols)

A61K; C12N; A61P

Documentation searched other than minimum documentation to the extent that such documents are included in the fields searched

Electronic data base consulted during the international search (name of data base and, where practicable, search terms used)

VEN, CNABS, PubMed, ISI Web of Knowledge, NCBI Genbank, EMBL-EBI, CNKI: 申请人/发明人, siRNA, ApoC3, 缀合物, 载脂蛋白C3, 偶联物, 修饰, conjugate, modification, SEQ ID NOs: 1, 2, 13, 14, 25, 26, 37, 38, 49, and 50

**C. DOCUMENTS CONSIDERED TO BE RELEVANT**

| Category* | Citation of document, with indication, where appropriate, of the relevant passages | Relevant to claim No. |
|---|---|---|
| PY | TW 201925471 A (SUZHOU RIBO LIFE SCIENCE CO., LTD.) 01 July 2019 (2019-07-01)<br>claims 1-100 | 1-30, 55-58 |
| PY | TW 201929905 A (SUZHOU RIBO LIFE SCIENCE CO., LTD.) 01 August 2019 (2019-08-01)<br>claims 1-114 | 1-30, 55-58 |
| X | CN 105392488 A (ISIS PHARMACEUTICALS, INC.) 09 March 2016 (2016-03-09)<br>the abstract, and sequences 171-173 | 31-54 |
| Y | CN 105392488 A (ISIS PHARMACEUTICALS, INC.) 09 March 2016 (2016-03-09)<br>the abstract, and sequences 171-173 | 1-30, 55-58 |
| A | CN 107109405 A (SOLSTICE BIOLOG LTD.) 29 August 2017 (2017-08-29)<br>entire document | 1-58 |
| A | WO 2010147992 A1 (ALNYLAM PHARMACEUTICALS, INC.) 23 December 2010 (2010-12-23)<br>entire document | 1-58 |
| A | WO 2017055627 A1 (INSERM INSTITUT NAT. DE LA SANTé ET DE LA RECH. MéDICALE et al.) 06 April 2017 (2017-04-06)<br>entire document | 1-58 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 March 2020** | **26 March 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/129016**

**C. DOCUMENTS CONSIDERED TO BE RELEVANT**

| Category* | Citation of document, with indication, where appropriate, of the relevant passages | Relevant to claim No. |
|---|---|---|
| A | NORDESTGAARD, B. G. et al. "Advances in lipid-lowering therapy through gene-silencing technologies" *ATURE REVIEWS CARDIOLOGY,* Vol. 15, 08 February 2018 (2018-02-08), pp. 261-272 | 1-58 |

Form PCT/ISA/210 (second sheet) (January 2015)

**INTERNATIONAL SEARCH REPORT**

International application No.
**PCT/CN2019/129016**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2019/129016**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **56-57**
because they relate to subject matter not required to be searched by this Authority, namely:

[1] Claims 56 and 57 relate to a method for treating and/or preventing dyslipidemia and a method for inhibiting APOC3 gene expression in liver cells, which fall under the treatment methods for diseases defined in PCT Rule 39.1(iv); the present report is made on the basis of the pharmaceutical use of siRNA.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/129016**

| Patent document cited in search report | | | Publication date (day/month/year) | Patent family member(s) | | | Publication date (day/month/year) |
|---|---|---|---|---|---|---|---|
| TW | 201925471 | A | 01 July 2019 | WO | 2019105419 | A1 | 06 June 2019 |
| TW | 201929905 | A | 01 August 2019 | WO | 2019128611 | A1 | 04 July 2019 |
| CN | 105392488 | A | 09 March 2016 | CA | 2921518 | A1 | 06 November 2014 |
| | | | | US | 2016090596 | A1 | 31 March 2016 |
| | | | | US | 2019055554 | A1 | 21 February 2019 |
| | | | | JP | 6592486 | B2 | 16 October 2019 |
| | | | | RU | 2015151199 | A | 05 June 2017 |
| | | | | EP | 2992097 | A2 | 09 March 2016 |
| | | | | NZ | 631537 | A | 26 May 2017 |
| | | | | CN | 105378082 | A | 02 March 2016 |
| | | | | EP | 2991656 | B1 | 18 December 2019 |
| | | | | AU | 2017200365 | A1 | 23 February 2017 |
| | | | | RU | 2018136140 | A | 17 December 2018 |
| | | | | US | 2018273953 | A1 | 27 September 2018 |
| | | | | RU | 2015151200 | A3 | 14 January 2019 |
| | | | | MX | 2015015264 | A | 12 August 2016 |
| | | | | PE | 14302016 | A1 | 06 January 2017 |
| | | | | US | 2016076030 | A1 | 17 March 2016 |
| | | | | HK | 1221475 | A1 | 02 June 2017 |
| | | | | AU | 2014259750 | A1 | 22 October 2015 |
| | | | | RU | 2015151199 | A3 | 27 March 2018 |
| | | | | AU | 2018267625 | A1 | 13 December 2018 |
| | | | | CR | 20150612 | A | 03 March 2016 |
| | | | | US | 9181550 | B2 | 10 November 2015 |
| | | | | JP | 2018027091 | A | 22 February 2018 |
| | | | | RU | 2019110030 | A | 06 May 2019 |
| | | | | US | 2018273952 | A1 | 27 September 2018 |
| | | | | WO | 2014179627 | A9 | 26 February 2015 |
| | | | | KR | 20180051678 | A | 16 May 2018 |
| | | | | PL | 2992098 | T3 | 30 September 2019 |
| | | | | AU | 2019202598 | A1 | 02 May 2019 |
| | | | | WO | 2014179629 | A8 | 02 June 2016 |
| | | | | AU | 2017203436 | A1 | 08 June 2017 |
| | | | | EP | 2992009 | A1 | 09 March 2016 |
| | | | | IL | 264241 | D0 | 28 February 2019 |
| | | | | AU | 2017200365 | C1 | 18 April 2019 |
| | | | | PH | 12015502493 | A1 | 22 February 2016 |
| | | | | SG | 11201508870V | A | 27 November 2015 |
| | | | | EP | 2991661 | A4 | 15 February 2017 |
| | | | | AU | 2017200950 | B2 | 17 January 2019 |
| | | | | AU | 2014259755 | B2 | 30 August 2018 |
| | | | | JP | 6456362 | B2 | 23 January 2019 |
| | | | | ES | 2730015 | T3 | 07 November 2019 |
| | | | | KR | 20160002976 | A | 08 January 2016 |
| | | | | PE | 20152002 | A1 | 21 January 2016 |
| | | | | IL | 263843 | D0 | 31 January 2019 |
| | | | | HK | 1221404 | A1 | 02 June 2017 |
| | | | | NZ | 631552 | A | 24 February 2017 |
| | | | | EA | 201592093 | A1 | 30 June 2016 |
| | | | | US | 9181549 | B2 | 10 November 2015 |

Form PCT/ISA/210 (patent family annex) (January 2015)

**INTERNATIONAL SEARCH REPORT**
**Information on patent family members**

International application No.
**PCT/CN2019/129016**

| Patent document cited in search report | Publication date (day/month/year) | Patent family member(s) | Publication date (day/month/year) |
| --- | --- | --- | --- |
| | | US 9932581 B2 | 03 April 2018 |
| | | RU 2670614 C9 | 23 November 2018 |
| CN 107109405 A | 29 August 2017 | US 2017114341 A1 | 27 April 2017 |
| | | CA 2950960 A1 | 10 December 2015 |
| | | WO 2015188197 A2 | 10 December 2015 |
| | | WO 2015188197 A3 | 25 February 2016 |
| | | EP 3152308 A2 | 12 April 2017 |
| | | AU 2015269053 A1 | 22 December 2016 |
| | | EP 3152308 A4 | 27 December 2017 |
| | | JP 2017522046 A | 10 August 2017 |
| WO 2010147992 A1 | 23 December 2010 | US 2012244207 A1 | 27 September 2012 |
| | | US 10053689 B2 | 21 August 2018 |
| | | US 2019153443 A1 | 23 May 2019 |
| | | US 9051567 B2 | 09 June 2015 |
| | | US 2016024498 A1 | 28 January 2016 |
| WO 2017055627 A1 | 06 April 2017 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2009082607 A2
- CN 105378082 A
- WO 2015006740 A2
- WO 2014025805 A1
- US 8106022 B2
- WO 2016081444 A1


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **WATTS, J.K. ; G. F. DELEAVEY ; M. J. DAMHA.** Chemically Modified siRNA: tools and applications. *Drug Discov Today,* 2008, vol. 13 (19-20), 842-55
- **ANASTASIA KHVOROVA ; JONATHAN K. WATTS.** The chemical evolution of oligonucleotide therapies of clinical utility. *Nature Biotechnology,* 2017, vol. 35 (3), 238-48
- **MUTHIAH MANOHARAN.** siRNA conjugates carrying sequentially assembled trivalent N-acetylgalactosamine linked through nucleosides elicit robust gene silencing in vivo in hepatocytes. *ACS Chemical biology,* 2015, vol. 10 (5), 1181-7
- **RAJEEV et al.** *ChemBioChem,* 2015, vol. 16, 903-908
- *J. Am. Chem. Soc.,* 2014, vol. 136, 16958-16961
- Molecular Cloning. Cold Spring Harbor Laboratory Press, 1989
- *CHEMICAL ABSTRACTS,* 1772-03-8
- *CHEMICAL ABSTRACTS,* 27607-77-8
- *CHEMICAL ABSTRACTS,* 821-41-0
- *CHEMICAL ABSTRACTS,* 7790-28-5
- *CHEMICAL ABSTRACTS,* 14898-67-0

Sequence listing

<110> SUZHOU RIBO LIFE SCIENCE CO., LTD.

<120> NUCLEIC ACID, COMPOSITION AND CONJUGATE CONTAINING NUCLEIC ACID, PREPARATION METHOD THEREFOR AND USE THEREOF

<130> CP1181115P-CN

<150> CN 201811622633.0
<151> 2018-12-28

<160> 430

<210> 1
<211> 19
<212> RNA
<213> Artificial Sequence

<220>
<221> misc-feature
<222> (19)..(19)
<223> n is Za1, Za1 is A

<400> 1
uuaaaaggga caguauucn 19

<210> 2
<211> 19
<212> RNA
<213> Artificial Sequence

<220>
<221> misc-feature
<222> (1)..(1)
<223> n is Za2, Za2 is U

<400> 2
ngaauacugu cccuuuuaa 19

<210> 3
<211> 19
<212> RNA
<213> Artificial Sequence

<220>
<221> misc-feature
<222> (19)..(19)
<223> n is Za3, Za3 is selected from A, U, G or C

<400> 3
uuaaaaggga caguauucn 19

<210> 4
<211> 19
<212> RNA
<213> Artificial Sequence

<220>
<221> misc-feature
<222> (1)..(1)
<223> n is Za4, Za4 is a nucleotide complementary to Za3, Za3 is selected from A, U, G or C

<400> 4
ngaauacugu cccuuuuaa 19

<210> 5
<211> 19
<212> RNA
<213> Artificial Sequence

<220>
<221> misc-feature
<222> (19)..(19)
<223> n is Za3, Za3 is selected from A, U, G or C

<400> 5
uuaaaaggga caguauucn 19

<210> 6
<211> 21
<212> RNA
<213> Artificial Sequence

<220>
<221> misc-feature
<222> (1)..(1)
<223> n is Za4, Za4 is a nucleotide complementary to Za3, Za3 is selected from A, U, G or C

<400> 6
ngaauacugu cccuuuuaag c 21

<210> 7
<211> 21
<212> RNA
<213> Artificial Sequence

<220>
<221> misc-feature
<222> (21)..(21)
<223> n is Za3, Za3 is selected from A, U, G or C

<400> 7
gcuuaaaagg gacaguauuc n 21

<210> 8
<211> 23
<212> RNA
<213> Artificial Sequence

<220>
<221> misc-feature
<222> (1)..(1)
<223> n is Za4, Za4 is a nucleotide complementary to Za3, Za3 is selected from A, U, G or C

<400> 8
ngaauacugu cccuuuuaag caa 23

<210> 9
<211> 19
<212> RNA
<213> Artificial Sequence

<400> 9
uuaaaaggga caguauucu 19

<210> 10
<211> 21
<212> RNA
<213> Artificial Sequence

<400> 10
agaauacugu cccuuuuaag c 21

<210> 11
<211> 21
<212> RNA
<213> Artificial Sequence

<400> 11
gcuuaaaagg gacaguauuc u 21

<210> 12
<211> 23
<212> RNA
<213> Artificial Sequence

<400> 12
agaauacugu cccuuuuaag caa 23

<210> 13
<211> 19
<212> RNA
<213> Artificial Sequence

<220>
<221> misc-feature
<222> (19)..(19)
<223> n is Zb1, Zb1 is A

<400> 13
acaguauucu cagugcucn 19

<210> 14
<211> 19
<212> RNA
<213> Artificial Sequence

<220>
<221> misc-feature
<222> (1)..(1)
<223> n is Zb2, Zb2 is U

<400> 14
ngagcacuga gaauacugu 19

<210> 15
<211> 19
<212> RNA
<213> Artificial Sequence

<220>
<221> misc-feature
<222> (19)..(19)
<223> n is Zb3, Zb3 is selected from A, U, G or C

<400> 15
acaguauucu cagugcucn 19

<210> 16
<211> 19
<212> RNA
<213> Artificial Sequence

<220>
<221> misc-feature
<222> (1)..(1)
<223> n is Zb4, Zb4 is a nucleotide complementary to Zb3, Zb3 is selected from A, U, G or C

<400> 16
ngagcacuga gaauacugu 19

<210> 17
<211> 19
<212> RNA
<213> Artificial Sequence

<220>
<221> misc-feature
<222> (19)..(19)
<223> n is Zb3, Zb3 is selected from A, U, G or C

<400> 17
acaguauucu cagugcucn 19

<210> 18
<211> 21
<212> RNA
<213> Artificial Sequence

<220>
<221> misc-feature
<222> (1)..(1)
<223> n is Zb4, Zb4 is a nucleotide complementary to Zb3, Zb3 is selected from A, U, G or C

<400> 18
ngagcacuga gaauacuguc c 21

<210> 19
<211> 21
<212> RNA
<213> Artificial Sequence

<220>
<221> misc-feature
<222> (21)..(21)
<223> n is Zb3, Zb3 is selected from A, U, G or C

<400> 19
ggacaguauu cucagugcuc n 21

<210> 20
<211> 23
<212> RNA
<213> Artificial Sequence

<220>

<221> misc-feature
<222> (1)..(1)
<223> n is Zb4, Zb4 is a nucleotide complementary to Zb3, Zb3 is selected from A, U, G or C

<400> 20
ngagcacuga gaauacuguc ccu 23

<210> 21
<211> 19
<212> RNA
<213> Artificial Sequence

<400> 21
acaguauucu cagugcucu 19

<210> 22
<211> 21
<212> RNA
<213> Artificial Sequence

<400> 22
agagcacuga gaauacuguc c 21

<210> 23
<211> 21
<212> RNA
<213> Artificial Sequence

<400> 23
ggacaguauu cucagugcuc u 21

<210> 24
<211> 23
<212> RNA
<213> Artificial Sequence

<400> 24
agagcacuga gaauacuguc ccu 23

<210> 25
<211> 19
<212> RNA
<213> Artificial Sequence

<220>
<221> misc-feature
<222> (19)..(19)
<223> n is Zc1, Zc1 is A

<400> 25
uauucucagu gcucuccun 19

<210> 26
<211> 19
<212> RNA
<213> Artificial Sequence

<220>
<221> misc-feature
<222> (1)..(1)
<223> n is Zc2, Zc2 is U

<400> 26
naggagagca cugagaaua 19

<210> 27
<211> 19
<212> RNA
<213> Artificial Sequence

<220>
<221> misc-feature
<222> (19)..(19)
<223> n is Zc3, Zc3 is selected from A, U, G or C

<400> 27
uauucucagu gcucuccun 19

<210> 28
<211> 19
<212> RNA
<213> Artificial Sequence

<220>
<221> misc-feature
<222> (1)..(1)
<223> n is Zc4, Zc4 is a nucleotide complementary to Zc3, Zc3 is selected from A, U, G or C

<400> 28
naggagagca cugagaaua 19

<210> 29
<211> 19
<212> RNA
<213> Artificial Sequence

<220>
<221> misc-feature
<222> (19)..(19)
<223> n is Zc3, Zc3 is selected from A, U, G or C

<400> 29
uauucucagu gcucuccun 19

<210> 30
<211> 21
<212> RNA
<213> Artificial Sequence

<220>
<221> misc-feature
<222> (1)..(1)
<223> n is Zc4, Zc4 is a nucleotide complementary to Zc3, Zc3 is selected from A, U, G or C

<400> 30
naggagagca cugagaauac u 21

<210> 31
<211> 21
<212> RNA
<213> Artificial Sequence

<220>

<221> misc-feature
<222> (21)..(21)
<223> n is Zc3, Zc3 is selected from A, U, G or C

<400> 31
aguauucuca gugcucuccu n 21

<210> 32
<211> 23
<212> RNA
<213> Artificial Sequence

<220>
<221> misc-feature
<222> (1)..(1)
<223> n is Zc4, Zc4 is a nucleotide complementary to Zc3, Zc3 is selected from A, U, G or C

<400> 32
naggagagca cugagaauac ugu 23

<210> 33
<211> 19
<212> RNA
<213> Artificial Sequence

<400> 33
uauucucagu gcucuccua 19

<210> 34
<211> 21
<212> RNA
<213> Artificial Sequence

<400> 34
uaggagagca cugagaauac u 21

<210> 35
<211> 21
<212> RNA
<213> Artificial Sequence

<400> 35
aguauucuca gugcucuccu a 21

<210> 36
<211> 23
<212> RNA
<213> Artificial Sequence

<400> 36
uaggagagca cugagaauac ugu 23

<210> 37
<211> 19
<212> RNA
<213> Artificial Sequence

<220>
<221> misc-feature
<222> (19)..(19)
<223> n is Zd1, Zd1 is A

<400> 37
aguauucuca gugcucucn 19

<210> 38
<211> 19
<212> RNA
<213> Artificial Sequence

<220>
<221> misc-feature
<222> (1)..(1)
<223> n is Zd2, Zd2 is U

<400> 38
ngagagcacu gagaauacu 19

<210> 39
<211> 19
<212> RNA
<213> Artificial Sequence

<220>
<221> misc-feature
<222> (19)..(19)
<223> n is Zd3, Zd3 is selected from A, U, G or C

<400> 39
aguauucuca gugcucucn 19

<210> 40
<211> 19
<212> RNA
<213> Artificial Sequence

<220>
<221> misc-feature
<222> (1)..(1)
<223> n is Zd4, Zd4 is a nucleotide complementary to Zd3, Zd3 is selected from A, U, G or C

<400> 40
ngagagcacu gagaauacu 19

<210> 41
<211> 19
<212> RNA
<213> Artificial Sequence

<220>
<221> misc-feature
<222> (19)..(19)
<223> n is Zd3, Zd3 is selected from A, U, G or C

<400> 41
aguauucuca gugcucucn 19

<210> 42
<211> 21
<212> RNA
<213> Artificial Sequence

<220>
<221> misc-feature

<222> (1)..(1)
<223> n is Zd4, Zd4 is a nucleotide complementary to Zd3, Zd3 is selected from A, U, G or C

<400> 42
ngagagcacu gagaauacug u 21

<210> 43
<211> 21
<212> RNA
<213> Artificial Sequence

<220>
<221> misc-feature
<222> (21)..(21)
<223> n is Zd3, Zd3 is selected from A, U, G or C

<400> 43
acaguauucu cagugcucuc n 21

<210> 44
<211> 23
<212> RNA
<213> Artificial Sequence

<220>
<221> misc-feature
<222> (1)..(1)
<223> n is Zd4, Zd4 is a nucleotide complementary to Zd3, Zd3 is selected from A, U, G or C

<400> 44
ngagagcacu gagaauacug ucc 23

<210> 45
<211> 19
<212> RNA
<213> Artificial Sequence

<400> 45
aguauucuca gugcucucc 19

<210> 46
<211> 21
<212> RNA
<213> Artificial Sequence

<400> 46
ggagagcacu gagaa uacug u 21

<210> 47
<211> 21
<212> RNA
<213> Artificial Sequence

<400> 47
acaguauucu cagugcucuc c 21

<210> 48
<211> 23
<212> RNA
<213> Artificial Sequence

<400> 48
ggagagcacu gagaauacug ucc 23

<210> 49
<211> 19
<212> RNA
<213> Artificial Sequence

<220>
<221> misc-feature
<222> (19)..(19)
<223> n is Ze1, Ze1 is A

<400> 49
ggacaguauu cucagugcn 19

<210> 50
<211> 19
<212> RNA
<213> Artificial Sequence

<220>
<221> misc-feature
<222> (1)..(1)
<223> n is Ze2, Ze2 is U

<400> 50
ngcacugaga auacugucc 19

<210> 51
<211> 19
<212> RNA
<213> Artificial Sequence

<220>
<221> misc-feature
<222> (19)..(19)
<223> n is Ze3, Ze3 is selected from A, U, G or C

<400> 51
ggacaguauu cucagugcn 19

<210> 52
<211> 19
<212> RNA
<213> Artificial Sequence

<220>
<221> misc-feature
<222> (1)..(1)
<223> n is Ze4, Ze4 is a nucleotide complementary to Ze3, Ze3 is selected from A, U, G or C

<400> 52
ngcacugaga auacugucc 19

<210> 53
<211> 19
<212> RNA
<213> Artificial Sequence

<220>
<221> misc-feature

<222> (19)..(19)
<223> n is Ze3, Ze3 is selected from A, U, G or C

<400> 53
ggacaguauu cucagugcn 19

<210> 54
<211> 21
<212> RNA
<213> Artificial Sequence

<220>
<221> misc-feature
<222> (1)..(1)
<223> n is Ze4, Ze4 is a nucleotide complementary to Ze3, Ze3 is selected from A, U, G or C

<400> 54
ngcacugaga auacuguccc u 21

<210> 55
<211> 21
<212> RNA
<213> Artificial Sequence

<220>
<221> misc-feature
<222> (21)..(21)
<223> n is Ze3, Ze3 is selected from A, U, G or C

<400> 55
agggacagua uucucagugc n 21

<210> 56
<211> 23
<212> RNA
<213> Artificial Sequence

<220>
<221> misc-feature
<222> (1)..(1)
<223> n is Ze4, Ze4 is a nucleotide complementary to Ze3, Ze3 is selected from A, U, G or C

<400> 56
ngcacugaga auacuguccc uuu 23

<210> 57
<211> 19
<212> RNA
<213> Artificial Sequence

<400> 57
ggacaguauu cucagugcu 19

<210> 58
<211> 21
<212> RNA
<213> Artificial Sequence

<400> 58
agcacugaga auacuguccc u 21

<210> 59
<211> 21
<212> RNA
<213> Artificial Sequence

<400> 59
agggacagua uucucagugc u 21

<210> 60
<211> 23
<212> RNA
<213> Artificial Sequence

<400> 60
agcacugaga auacuguccc uuu 23

<210> 61
<211> 19
<212> RNA
<213> Artificial Sequence

<400> 61
uuaaaaggga caguauucu 19

<210> 62
<211> 21
<212> RNA
<213> Artificial Sequence

<400> 62
agaauacugu cccuuuuaag c 21

<210> 63
<211> 21
<212> RNA
<213> Artificial Sequence

<400> 63
gcuuaaaagg gacaguauuc u 21

<210> 64
<211> 23
<212> RNA
<213> Artificial Sequence

<400> 64
agaauacugu cccuuuuaag caa 23

<210> 65
<211> 19
<212> RNA
<213> Artificial Sequence

<400> 65
uuaaaaggga caguauucu 19

<210> 66
<211> 21
<212> RNA
<213> Artificial Sequence

<400> 66
agaauacugu cccuuuuaag c 21

<210> 67
<211> 21
<212> RNA
<213> Artificial Sequence

<400> 67
gcuuaaaagg gacaguauuc u 21

<210> 68
<211> 23
<212> RNA
<213> Artificial Sequence

<400> 68
agaauacugu cccuuuuaag caa 23

<210> 69
<211> 19
<212> RNA
<213> Artificial Sequence

<400> 69
uuaaaaggga caguauucu 19

<210> 70
<211> 21
<212> RNA
<213> Artificial Sequence

<400> 70
agaauacugu cccuuuuaag c 21

<210> 71
<211> 21
<212> RNA
<213> Artificial Sequence

<400> 71
gcuuaaaagg gacaguauuc u 21

<210> 72
<211> 23
<212> RNA
<213> Artificial Sequence

<400> 72
agaauacugu cccuuuuaag caa 23

<210> 73
<211> 19
<212> RNA
<213> Artificial Sequence

<400> 73
acaguauucu cagugcucu 19

<210> 74
<211> 21
<212> RNA
<213> Artificial Sequence

<400> 74

agagcacuga gaauacuguc c 21

<210> 75
<211> 21
<212> RNA
<213> Artificial Sequence

<400> 75
ggacaguauu cucagugcuc u 21

<210> 76
<211> 23
<212> RNA
<213> Artificial Sequence

<400> 76
agagcacuga gaauacuguc ccu 23

<210> 77
<211> 19
<212> RNA
<213> Artificial Sequence

<400> 77
acaguauucu cagugcucu 19

<210> 78
<211> 21
<212> RNA
<213> Artificial Sequence

<400> 78
agagcacuga gaauacuguc c 21

<210> 79
<211> 21
<212> RNA
<213> Artificial Sequence

<400> 79
ggacaguauu cucagugcuc u 21

<210> 80
<211> 23
<212> RNA
<213> Artificial Sequence

<400> 80
agagcacuga gaauacuguc ccu 23

<210> 81
<211> 19
<212> RNA
<213> Artificial Sequence

<400> 81
acaguauucu cagugcucu 19

<210> 82
<211> 21
<212> RNA
<213> Artificial Sequence

<400> 82
agagcacuga gaauacuguc c 21

<210> 83
<211> 21
<212> RNA
<213> Artificial Sequence

<400> 83
ggacaguauu cucagugcuc u 21

<210> 84
<211> 23
<212> RNA
<213> Artificial Sequence

<400> 84
agagcacuga gaauacuguc ccu 23

<210> 85
<211> 19
<212> RNA
<213> Artificial Sequence

<400> 85
uauucucagu gcucuccua 19

<210> 86
<211> 21
<212> RNA
<213> Artificial Sequence

<400> 86
uaggagagca cugagaauac u 21

<210> 87
<211> 21
<212> RNA
<213> Artificial Sequence

<400> 87
aguauucuca gugcucuccu a 21

<210> 88
<211> 23
<212> RNA
<213> Artificial Sequence

<400> 88
uaggagagca cugagaauac ugu 23

<210> 89
<211> 19
<212> RNA
<213> Artificial Sequence

<400> 89
uauucucagu gcucuccua 19

<210> 90
<211> 21
<212> RNA
<213> Artificial Sequence

<400> 90
uaggagagca cugagaauac u 21

<210> 91
<211> 21
<212> RNA
<213> Artificial Sequence

<400> 91
aguauucuca gugcucuccu a 21

<210> 92
<211> 23
<212> RNA
<213> Artificial Sequence

<400> 92
uaggagagca cugagaauac ugu 23

<210> 93
<211> 19
<212> RNA
<213> Artificial Sequence

<400> 93
uauucucagu gcucuccua 19

<210> 94
<211> 21
<212> RNA
<213> Artificial Sequence

<400> 94
uaggagagca cugagaauac u 21

<210> 95
<211> 21
<212> RNA
<213> Artificial Sequence

<400> 95
aguauucuca gugcucuccu a 21

<210> 96
<211> 23
<212> RNA
<213> Artificial Sequence

<400> 96
uaggagagca cugagaauac ugu 23

<210> 97
<211> 19
<212> RNA
<213> Artificial Sequence

<400> 97
aguauucuca gugcucucc 19

<210> 98
<211> 21
<212> RNA

<213>  Artificial Sequence

<400>  98
ggagagcacu gagaauacug u                                                21

<210>  99
<211>  21
<212>  RNA
<213>  Artificial Sequence

<400>  99
acaguauucu cagugcucuc c                                                21

<210>  100
<211>  23
<212>  RNA
<213>  Artificial Sequence

<400>  100
ggagagcacu gagaauacug ucc                                              23

<210>  101
<211>  19
<212>  RNA
<213>  Artificial Sequence

<400>  101
aguauucuca gugcucucc                                                   19

<210>  102
<211>  21
<212>  RNA
<213>  Artificial Sequence

<400>  102
ggagagcacu gagaauacug u                                                21

<210>  103
<211>  21
<212>  RNA
<213>  Artificial Sequence

<400>  103
acaguauucu cagugcucuc c                                                21

<210>  104
<211>  23
<212>  RNA
<213>  Artificial Sequence

<400>  104
ggagagcacu gagaauacug ucc                                              23

<210>  105
<211>  19
<212>  RNA
<213>  Artificial Sequence

<400>  105
aguauucuca gugcucucc                                                   19

<210>  106
<211>  21

<212> RNA
<213> Artificial Sequence

<400> 106
ggagagcacu gagaauacug u 21

<210> 107
<211> 21
<212> RNA
<213> Artificial Sequence

<400> 107
acaguauucu cagugcucuc c 21

<210> 108
<211> 23
<212> RNA
<213> Artificial Sequence

<400> 108
ggagagcacu gagaauacug ucc 23

<210> 109
<211> 19
<212> RNA
<213> Artificial Sequence

<400> 109
ggacaguauu cucagugcu 19

<210> 110
<211> 21
<212> RNA
<213> Artificial Sequence

<400> 110
agcacugaga auacuguccc u 21

<210> 111
<211> 21
<212> RNA
<213> Artificial Sequence

<400> 111
agggacagua uucucagugc u 21

<210> 112
<211> 23
<212> RNA
<213> Artificial Sequence

<400> 112
agcacugaga auacuguccc uuu 23

<210> 113
<211> 19
<212> RNA
<213> Artificial Sequence

<400> 113
ggacaguauu cucagugcu 19

<210> 114

<211> 21
<212> RNA
<213> Artificial Sequence

<400> 114
agcacugaga auacuguccc u 21

<210> 115
<211> 21
<212> RNA
<213> Artificial Sequence

<400> 115
agggacagua uucucagugc u 21

<210> 116
<211> 23
<212> RNA
<213> Artificial Sequence

<400> 116
agcacugaga auacuguccc uuu 23

<210> 117
<211> 19
<212> RNA
<213> Artificial Sequence

<400> 117
ggacaguauu cucagugcu 19

<210> 118
<211> 21
<212> RNA
<213> Artificial Sequence

<400> 118
agcacugaga auacuguccc u 21

<210> 119
<211> 21
<212> RNA
<213> Artificial Sequence

<400> 119
agggacagua uucucagugc u 21

<210> 120
<211> 23
<212> RNA
<213> Artificial Sequence

<400> 120
agcacugaga auacuguccc uuu 23

<210> 121
<211> 19
<212> RNA
<213> Artificial Sequence

<400> 121
uuaaaaggga caguauucu 19

<210> 122
<211> 21
<212> RNA
<213> Artificial Sequence

<400> 122
agaauacugu cccuuuuaag c 21

<210> 123
<211> 21
<212> RNA
<213> Artificial Sequence

<400> 123
gcuuaaaagg gacaguauuc u 21

<210> 124
<211> 23
<212> RNA
<213> Artificial Sequence

<400> 124
agaauacugu cccuuuuaag caa 23

<210> 125
<211> 19
<212> RNA
<213> Artificial Sequence

<400> 125
uuaaaaggga caguauucu 19

<210> 126
<211> 21
<212> RNA
<213> Artificial Sequence

<400> 126
agaauacugu cccuuuuaag c 21

<210> 127
<211> 21
<212> RNA
<213> Artificial Sequence

<400> 127
gcuuaaaagg gacaguauuc u 21

<210> 128
<211> 23
<212> RNA
<213> Artificial Sequence

<400> 128
agaauacugu cccuuuuaag caa 23

<210> 129
<211> 19
<212> RNA
<213> Artificial Sequence

<400> 129
uuaaaaggga caguauucu 19

<210> 130
<211> 21
<212> RNA
<213> Artificial Sequence

<400> 130
agaauacugu cccuuuuaag c 21

<210> 131
<211> 21
<212> RNA
<213> Artificial Sequence

<400> 131
gcuuaaaagg gacaguauuc u 21

<210> 132
<211> 23
<212> RNA
<213> Artificial Sequence

<400> 132
agaauacugu cccuuuuaag caa 23

<210> 133
<211> 19
<212> RNA
<213> Artificial Sequence

<400> 133
acaguauucu cagugcucu 19

<210> 134
<211> 21
<212> RNA
<213> Artificial Sequence

<400> 134
agagcacuga gaauacuguc c 21

<210> 135
<211> 21
<212> RNA
<213> Artificial Sequence

<400> 135
ggacaguauu cucagugcuc u 21

<210> 136
<211> 23
<212> RNA
<213> Artificial Sequence

<400> 136
agagcacuga gaauacuguc ccu 23

<210> 137
<211> 19
<212> RNA
<213> Artificial Sequence

<400> 137

acaguauucu cagugcucu 19

<210> 138
<211> 21
<212> RNA
<213> Artificial Sequence

<400> 138
agagcacuga gaauacuguc c 21

<210> 139
<211> 21
<212> RNA
<213> Artificial Sequence

<400> 139
ggacaguauu cucagugcuc u 21

<210> 140
<211> 23
<212> RNA
<213> Artificial Sequence

<400> 140
agagcacuga gaauacuguc ccu 23

<210> 141
<211> 19
<212> RNA
<213> Artificial Sequence

<400> 141
acaguauucu cagugcucu 19

<210> 142
<211> 21
<212> RNA
<213> Artificial Sequence

<400> 142
agagcacuga gaauacuguc c 21

<210> 143
<211> 21
<212> RNA
<213> Artificial Sequence

<400> 143
ggacaguauu cucagugcuc u 21

<210> 144
<211> 23
<212> RNA
<213> Artificial Sequence

<400> 144
agagcacuga gaauacuguc ccu 23

<210> 145
<211> 19
<212> RNA
<213> Artificial Sequence

<400> 145
uauucucagu gcucuccua 19

<210> 146
<211> 21
<212> RNA
<213> Artificial Sequence

<400> 146
uaggagagca cugagaauac u 21

<210> 147
<211> 21
<212> RNA
<213> Artificial Sequence

<400> 147
aguauucuca gugcucuccu a 21

<210> 148
<211> 23
<212> RNA
<213> Artificial Sequence

<400> 148
uaggagagca cugagaauac ugu 23

<210> 149
<211> 19
<212> RNA
<213> Artificial Sequence

<400> 149
uauucucagu gcucuccua 19

<210> 150
<211> 21
<212> RNA
<213> Artificial Sequence

<400> 150
uaggagagca cugagaauac u 21

<210> 151
<211> 21
<212> RNA
<213> Artificial Sequence

<400> 151
aguauucuca gugcucuccu a 21

<210> 152
<211> 23
<212> RNA
<213> Artificial Sequence

<400> 152
uaggagagca cugagaauac ugu 23

<210> 153
<211> 19
<212> RNA
<213> Artificial Sequence

<400> 153
uauucucagu gcucuccua 19

<210> 154
<211> 21
<212> RNA
<213> Artificial Sequence

<400> 154
uaggagagca cugagaauac u 21

<210> 155
<211> 21
<212> RNA
<213> Artificial Sequence

<400> 155
aguauucuca gugcucuccu a 21

<210> 156
<211> 23
<212> RNA
<213> Artificial Sequence

<400> 156
uaggagagca cugagaauac ugu 23

<210> 157
<211> 19
<212> RNA
<213> Artificial Sequence

<400> 157
aguauucuca gugcucucc 19

<210> 158
<211> 21
<212> RNA
<213> Artificial Sequence

<400> 158
ggagagcacu gagaauacug u 21

<210> 159
<211> 21
<212> RNA
<213> Artificial Sequence

<400> 159
acaguauucu cagugcucuc c 21

<210> 160
<211> 23
<212> RNA
<213> Artificial Sequence

<400> 160
ggagagcacu gagaauacug ucc 23

<210> 161
<211> 19
<212> RNA

<213> Artificial Sequence

<400> 161
aguauucuca gugcucucc 19

<210> 162
<211> 21
<212> RNA
<213> Artificial Sequence

<400> 162
ggagagcacu gagaauacug u 21

<210> 163
<211> 21
<212> RNA
<213> Artificial Sequence

<400> 163
acaguauucu cagugcucuc c 21

<210> 164
<211> 23
<212> RNA
<213> Artificial Sequence

<400> 164
ggagagcacu gagaauacug ucc 23

<210> 165
<211> 19
<212> RNA
<213> Artificial Sequence

<400> 165
aguauucuca gugcucucc 19

<210> 166
<211> 21
<212> RNA
<213> Artificial Sequence

<400> 166
ggagagcacu gagaauacug u 21

<210> 167
<211> 19
<212> RNA
<213> Artificial Sequence

<400> 167
aguauucuca gugcucucc 19

<210> 168
<211> 23
<212> RNA
<213> Artificial Sequence

<400> 168
ggagagcacu gagaauacug ucc 23

<210> 169
<211> 19

<212> RNA
<213> Artificial Sequence

<400> 169
ggacaguauu cucagugcu 19

<210> 170
<211> 21
<212> RNA
<213> Artificial Sequence

<400> 170
agcacugaga auacuguccc u 21

<210> 171
<211> 21
<212> RNA
<213> Artificial Sequence

<400> 171
agggacagua uucucagugc u 21

<210> 172
<211> 23
<212> RNA
<213> Artificial Sequence

<400> 172
agcacugaga auacuguccc uuu 23

<210> 173
<211> 19
<212> RNA
<213> Artificial Sequence

<400> 173
ggacaguauu cucagugcu 19

<210> 174
<211> 21
<212> RNA
<213> Artificial Sequence

<400> 174
agcacugaga auacuguccc u 21

<210> 175
<211> 21
<212> RNA
<213> Artificial Sequence

<400> 175
agggacagua uucucagugc u 21

<210> 176
<211> 23
<212> RNA
<213> Artificial Sequence

<400> 176
Agcacugaga auacuguccc uuu 23

<210> 177

<211> 19
<212> RNA
<213> Artificial Sequence

<400> 177
ggacaguauu cucagugcu 19

<210> 178
<211> 21
<212> RNA
<213> Artificial Sequence

<400> 178
agcacugaga auacuguccc u 21

<210> 179
<211> 21
<212> RNA
<213> Artificial Sequence

<400> 179
agggacagua uucucagugc u 21

<210> 180
<211> 23
<212> RNA
<213> Artificial Sequence

<400> 180
agcacugaga auacuguccc uuu 23

<210> 181
<211> 19
<212> RNA
<213> Artificial Sequence

<400> 181
uuaaaaggga caguauucu 19

<210> 182
<211> 21
<212> RNA
<213> Artificial Sequence

<400> 182
agaauacugu cccuuuuaag c 21

<210> 183
<211> 21
<212> RNA
<213> Artificial Sequence

<400> 183
gcuuaaaagg gacaguauuc u 21

<210> 184
<211> 23
<212> RNA
<213> Artificial Sequence

<400> 184
agaauacugu cccuuuuaag caa 23

<210> 185
<211> 19
<212> RNA
<213> Artificial Sequence

<400> 185
uuaaaaggga caguauucu 19

<210> 186
<211> 21
<212> RNA
<213> Artificial Sequence

<400> 186
agaauacugu cccuuuuaag c 21

<210> 187
<211> 21
<212> RNA
<213> Artificial Sequence

<400> 187
gcuuaaaagg gacaguauuc u 21

<210> 188
<211> 23
<212> RNA
<213> Artificial Sequence

<400> 188
agaauacugu cccuuuuaag caa 23

<210> 189
<211> 19
<212> RNA
<213> Artificial Sequence

<400> 189
uuaaaaggga caguauucu 19

<210> 190
<211> 21
<212> RNA
<213> Artificial Sequence

<400> 190
agaauacugu cccuuuuaag c 21

<210> 191
<211> 21
<212> RNA
<213> Artificial Sequence

<400> 191
gcuuaaaagg gacaguauuc u 21

<210> 192
<211> 23
<212> RNA
<213> Artificial Sequence

<400> 192
agaauacugu cccuuuuaag caa 23

<210> 193
<211> 19
<212> RNA
<213> Artificial Sequence

<400> 193
uuaaaaggga caguauucu 19

<210> 194
<211> 21
<212> RNA
<213> Artificial Sequence

<400> 194
agaauacugu cccuuuuaag c 21

<210> 195
<211> 21
<212> RNA
<213> Artificial Sequence

<400> 195
gcuuaaaagg gacaguauuc u 21

<210> 196
<211> 23
<212> RNA
<213> Artificial Sequence

<400> 196
agaauacugu cccuuuuaag caa 23

<210> 197
<211> 19
<212> RNA
<213> Artificial Sequence

<400> 197
uuaaaaggga caguauucu 19

<210> 198
<211> 21
<212> RNA
<213> Artificial Sequence

<400> 198
agaauacugu cccuuuuaag c 21

<210> 199
<211> 21
<212> RNA
<213> Artificial Sequence

<400> 199
gcuuaaaagg gacaguauuc u 21

<210> 200
<211> 23
<212> RNA
<213> Artificial Sequence

<400> 200

agaauacugu cccuuuuaag caa 23

<210> 201
<211> 19
<212> RNA
<213> Artificial Sequence

<400> 201
uuaaaaggga caguauucu 19

<210> 202
<211> 21
<212> RNA
<213> Artificial Sequence

<400> 202
agaauacugu cccuuuuaag c 21

<210> 203
<211> 21
<212> RNA
<213> Artificial Sequence

<400> 203
gcuuaaaagg gacaguauuc u 21

<210> 204
<211> 23
<212> RNA
<213> Artificial Sequence

<400> 204
agaauacugu cccuuuuaag caa 23

<210> 205
<211> 19
<212> RNA
<213> Artificial Sequence

<400> 205
acaguauucu cagugcucu 19

<210> 206
<211> 21
<212> RNA
<213> Artificial Sequence

<400> 206
agagcacuga gaauacuguc c 21

<210> 207
<211> 21
<212> RNA
<213> Artificial Sequence

<400> 207
ggacaguauu cucagugcuc u 21

<210> 208
<211> 23
<212> RNA
<213> Artificial Sequence

<400> 208
agagcacuga gaauacuguc ccu 23

<210> 209
<211> 19
<212> RNA
<213> Artificial Sequence

<400> 209
acaguauucu cagugcucu 19

<210> 210
<211> 21
<212> RNA
<213> Artificial Sequence

<400> 210
agagcacuga gaauacuguc c 21

<210> 211
<211> 21
<212> RNA
<213> Artificial Sequence

<400> 211
ggacaguauu cucagugcuc u 21

<210> 212
<211> 23
<212> RNA
<213> Artificial Sequence

<400> 212
agagcacuga gaauacuguc ccu 23

<210> 213
<211> 19
<212> RNA
<213> Artificial Sequence

<400> 213
acaguauucu cagugcucu 19

<210> 214
<211> 21
<212> RNA
<213> Artificial Sequence

<400> 214
agagcacuga gaauacuguc c 21

<210> 215
<211> 21
<212> RNA
<213> Artificial Sequence

<400> 215
ggacaguauu cucagugcuc u 21

<210> 216
<211> 23
<212> RNA
<213> Artificial Sequence

<400> 216
agagcacuga gaauacuguc ccu 23

<210> 217
<211> 19
<212> RNA
<213> Artificial Sequence

<400> 217
acaguauucu cagugcucu 19

<210> 218
<211> 21
<212> RNA
<213> Artificial Sequence

<400> 218
agagcacuga gaauacuguc c 21

<210> 219
<211> 21
<212> RNA
<213> Artificial Sequence

<400> 219
ggacaguauu cucagugcuc u 21

<210> 220
<211> 23
<212> RNA
<213> Artificial Sequence

<400> 220
agagcacuga gaauacuguc ccu 23

<210> 221
<211> 19
<212> RNA
<213> Artificial Sequence

<400> 221
acaguauucu cagugcucu 19

<210> 222
<211> 21
<212> RNA
<213> Artificial Sequence

<400> 222
agagcacuga gaauacuguc c 21

<210> 223
<211> 21
<212> RNA
<213> Artificial Sequence

<400> 223
ggacaguauu cucagugcuc u 21

<210> 224
<211> 23
<212> RNA

<213> Artificial Sequence

<400> 224
agagcacuga gaauacuguc ccu 23

<210> 225
<211> 19
<212> RNA
<213> Artificial Sequence

<400> 225
acaguauucu cagugcucu 19

<210> 226
<211> 21
<212> RNA
<213> Artificial Sequence

<400> 226
agagcacuga gaauacuguc c 21

<210> 227
<211> 21
<212> RNA
<213> Artificial Sequence

<400> 227
ggacaguauu cucagugcuc u 21

<210> 228
<211> 23
<212> RNA
<213> Artificial Sequence

<400> 228
agagcacuga gaauacuguc ccu 23

<210> 229
<211> 19
<212> RNA
<213> Artificial Sequence

<400> 229
uauucucagu gcucuccua 19

<210> 230
<211> 21
<212> RNA
<213> Artificial Sequence

<400> 230
uaggagagca cugagaauac u 21

<210> 231
<211> 21
<212> RNA
<213> Artificial Sequence

<400> 231
aguauucuca gugcucuccu a 21

<210> 232
<211> 23

<212> RNA
<213> Artificial Sequence

<400> 232
uaggagagca cugagaauac ugu 23

<210> 233
<211> 19
<212> RNA
<213> Artificial Sequence

<400> 233
uauucucagu gcucuccua 19

<210> 234
<211> 21
<212> RNA
<213> Artificial Sequence

<400> 234
uaggagagca cugagaauac u 21

<210> 235
<211> 21
<212> RNA
<213> Artificial Sequence

<400> 235
aguauucuca gugcucuccu a 21

<210> 236
<211> 23
<212> RNA
<213> Artificial Sequence

<400> 236
uaggagagca cugagaauac ugu 23

<210> 237
<211> 19
<212> RNA
<213> Artificial Sequence

<400> 237
uauucucagu gcucuccua 19

<210> 238
<211> 21
<212> RNA
<213> Artificial Sequence

<400> 238
uaggagagca cugagaauac u 21

<210> 239
<211> 21
<212> RNA
<213> Artificial Sequence

<400> 239
aguauucuca gugcucuccu a 21

<210> 240

<211> 23
<212> RNA
<213> Artificial Sequence

<400> 240
uaggagagca cugagaauac ugu 23

<210> 241
<211> 19
<212> RNA
<213> Artificial Sequence

<400> 241
uauucucagu gcucuccua 19

<210> 242
<211> 21
<212> RNA
<213> Artificial Sequence

<400> 242
uaggagagca cugagaauac u 21

<210> 243
<211> 21
<212> RNA
<213> Artificial Sequence

<400> 243
aguauucuca gugcucuccu a 21

<210> 244
<211> 23
<212> RNA
<213> Artificial Sequence

<400> 244
uaggagagca cugagaauac ugu 23

<210> 245
<211> 19
<212> RNA
<213> Artificial Sequence

<400> 245
uauucucagu gcucuccua 19

<210> 246
<211> 21
<212> RNA
<213> Artificial Sequence

<400> 246
uaggagagca cugagaauac u 21

<210> 247
<211> 21
<212> RNA
<213> Artificial Sequence

<400> 247
aguauucuca gugcucuccu a 21

<210> 248
<211> 23
<212> RNA
<213> Artificial Sequence

<400> 248
uaggagagca cugagaauac ugu 23

<210> 249
<211> 19
<212> RNA
<213> Artificial Sequence

<400> 249
uauucucagu gcucuccua 19

<210> 250
<211> 21
<212> RNA
<213> Artificial Sequence

<400> 250
uaggagagca cugagaauac u 21

<210> 251
<211> 21
<212> RNA
<213> Artificial Sequence

<400> 251
aguauucuca gugcucuccu a 21

<210> 252
<211> 23
<212> RNA
<213> Artificial Sequence

<400> 252
uaggagagca cugagaauac ugu 23

<210> 253
<211> 19
<212> RNA
<213> Artificial Sequence

<400> 253
aguauucuca gugcucucc 19

<210> 254
<211> 21
<212> RNA
<213> Artificial Sequence

<400> 254
ggagagcacu gagaauacug u 21

<210> 255
<211> 21
<212> RNA
<213> Artificial Sequence

<400> 255
acaguauucu cagugcucuc c 21

<210> 256
<211> 23
<212> RNA
<213> Artificial Sequence

<400> 256
ggagagcacu gagaauacug ucc 23

<210> 257
<211> 19
<212> RNA
<213> Artificial Sequence

<400> 257
aguauucuca gugcucucc 19

<210> 258
<211> 21
<212> RNA
<213> Artificial Sequence

<400> 258
ggagagcacu gagaauacug u 21

<210> 259
<211> 21
<212> RNA
<213> Artificial Sequence

<400> 259
acaguauucu cagugcucuc c 21

<210> 260
<211> 23
<212> RNA
<213> Artificial Sequence

<400> 260
ggagagcacu gagaauacug ucc 23

<210> 261
<211> 19
<212> RNA
<213> Artificial Sequence

<400> 261
aguauucuca gugcucucc 19

<210> 262
<211> 21
<212> RNA
<213> Artificial Sequence

<400> 262
ggagagcacu gagaauacug u 21

<210> 263
<211> 21
<212> RNA
<213> Artificial Sequence

<400> 263

acaguauucu cagugcucuc c 21

<210> 264
<211> 23
<212> RNA
<213> Artificial Sequence

<400> 264
ggagagcacu gagaauacug ucc 23

<210> 265
<211> 19
<212> RNA
<213> Artificial Sequence

<400> 265
aguauucuca gugcucucc 19

<210> 266
<211> 21
<212> RNA
<213> Artificial Sequence

<400> 266
ggagagcacu gagaauacug u 21

<210> 267
<211> 21
<212> RNA
<213> Artificial Sequence

<400> 267
acaguauucu cagugcucuc c 21

<210> 268
<211> 23
<212> RNA
<213> Artificial Sequence

<400> 268
ggagagcacu gagaauacug ucc 23

<210> 269
<211> 19
<212> RNA
<213> Artificial Sequence

<400> 269
aguauucuca gugcucucc 19

<210> 270
<211> 21
<212> RNA
<213> Artificial Sequence

<400> 270
ggagagcacu gagaauacug u 21

<210> 271
<211> 21
<212> RNA
<213> Artificial Sequence

<400> 271
acaguauucu cagugcucuc c 21

<210> 272
<211> 23
<212> RNA
<213> Artificial Sequence

<400> 272
ggagagcacu gagaauacug ucc 23

<210> 273
<211> 21
<212> RNA
<213> Artificial Sequence

<400> 273
acaguauucu cagugcucuc c 21

<210> 274
<211> 23
<212> RNA
<213> Artificial Sequence

<400> 274
ggagagcacu gagaauacug ucc 23

<210> 275
<211> 21
<212> RNA
<213> Artificial Sequence

<400> 275
acaguauucu cagugcucuc c 21

<210> 276
<211> 23
<212> RNA
<213> Artificial Sequence

<400> 276
ggagagcacu gagaauacug ucc 23

<210> 277
<211> 19
<212> RNA
<213> Artificial Sequence

<400> 277
ggacaguauu cucagugcu 19

<210> 278
<211> 21
<212> RNA
<213> Artificial Sequence

<400> 278
agcacugaga auacuguccc u 21

<210> 279
<211> 21
<212> RNA
<213> Artificial Sequence

<400> 279
agggacagua uucucagugc u 21

<210> 280
<211> 23
<212> RNA
<213> Artificial Sequence

<400> 280
agcacugaga auacuguccc uuu 23

<210> 281
<211> 19
<212> RNA
<213> Artificial Sequence

<400> 281
ggacaguauu cucagugcu 19

<210> 282
<211> 21
<212> RNA
<213> Artificial Sequence

<400> 282
agcacugaga auacuguccc u 21

<210> 283
<211> 21
<212> RNA
<213> Artificial Sequence

<400> 283
agggacagua uucucagugc u 21

<210> 284
<211> 23
<212> RNA
<213> Artificial Sequence

<400> 284
agcacugaga auacuguccc uuu 23

<210> 285
<211> 19
<212> RNA
<213> Artificial Sequence

<400> 285
ggacaguauu cucagugcu 19

<210> 286
<211> 21
<212> RNA
<213> Artificial Sequence

<400> 286
agcacugaga auacuguccc u 21

<210> 287
<211> 21
<212> RNA

<213> Artificial Sequence

<400> 287
agggacagua uucucagugc u 21

<210> 288
<211> 23
<212> RNA
<213> Artificial Sequence

<400> 288
agcacugaga auacuguccc uuu 23

<210> 289
<211> 19
<212> RNA
<213> Artificial Sequence

<400> 289
ggacaguauu cucagugcu 19

<210> 290
<211> 21
<212> RNA
<213> Artificial Sequence

<400> 290
agcacugaga auacuguccc u 21

<210> 291
<211> 21
<212> RNA
<213> Artificial Sequence

<400> 291
agggacagua uucucagugc u 21

<210> 292
<211> 23
<212> RNA
<213> Artificial Sequence

<400> 292
agcacugaga auacuguccc uuu 23

<210> 293
<211> 19
<212> RNA
<213> Artificial Sequence

<400> 293
ggacaguauu cucagugcu 19

<210> 294
<211> 21
<212> RNA
<213> Artificial Sequence

<400> 294
agcacugaga auacuguccc u 21

<210> 295
<211> 21

<212> RNA
<213> Artificial Sequence

<400> 295
agggacagua uucucagugc u 21

<210> 296
<211> 23
<212> RNA
<213> Artificial Sequence

<400> 296
agcacugaga auacuguccc uuu 23

<210> 297
<211> 19
<212> RNA
<213> Artificial Sequence

<400> 297
ggacaguauu cucagugcu 19

<210> 298
<211> 21
<212> RNA
<213> Artificial Sequence

<400> 298
agcacugaga auacuguccc u 21

<210> 299
<211> 21
<212> RNA
<213> Artificial Sequence

<400> 299
agggacagua uucucagugc u 21

<210> 300
<211> 23
<212> RNA
<213> Artificial Sequence

<400> 300
agcacugaga auacuguccc uuu 23

<210> 301
<211> 19
<212> RNA
<213> Artificial Sequence

<400> 301
uuaaaaggga caguauuca 19

<210> 302
<211> 21
<212> RNA
<213> Artificial Sequence

<400> 302
ugaauacugu cccuuuuaag c 21

<210> 303

<211> 19
<212> RNA
<213> Artificial Sequence

<400> 303
ggacaguauu cucagugca 19

<210> 304
<211> 21
<212> RNA
<213> Artificial Sequence

<400> 304
ugcacugaga auacuguccc u 21

<210> 305
<211> 19
<212> RNA
<213> Artificial Sequence

<400> 305
acaguauucu cagugcuca 19

<210> 306
<211> 21
<212> RNA
<213> Artificial Sequence

<400> 306
ugagcacuga gaauacuguc c 21

<210> 307
<211> 19
<212> RNA
<213> Artificial Sequence

<400> 307
aguauucuca gugcucuca 19

<210> 308
<211> 21
<212> RNA
<213> Artificial Sequence

<400> 308
ugagagcacu gagaauacug u 21

<210> 309
<211> 19
<212> RNA
<213> Artificial Sequence

<400> 309
uauucucagu gcucuccua 19

<210> 310
<211> 21
<212> RNA
<213> Artificial Sequence

<400> 310
uaggagagca cugagaauac u 21

<210> 311
<211> 19
<212> RNA
<213> Artificial Sequence

<400> 311
aguauucuca gugcucuca 19

<210> 312
<211> 21
<212> RNA
<213> Artificial Sequence

<400> 312
ugagagcacu gagaauacug u 21

<210> 313
<211> 19
<212> RNA
<213> Artificial Sequence

<400> 313
aguauucuca gugcucuca 19

<210> 314
<211> 21
<212> RNA
<213> Artificial Sequence

<400> 314
ugagagcacu gagaauacug u 21

<210> 315
<211> 21
<212> RNA
<213> Artificial Sequence

<400> 315
gcuuaaaagg gacaguauuc a 21

<210> 316
<211> 23
<212> RNA
<213> Artificial Sequence

<400> 316
ugaauacugu cccuuuuaag caa 23

<210> 317
<211> 21
<212> RNA
<213> Artificial Sequence

<400> 317
gcuuaaaagg gacaguauuc a 21

<210> 318
<211> 23
<212> RNA
<213> Artificial Sequence

<400> 318
ugaauacugu cccuuuuaag caa 23

<210> 319
<211> 20
<212> DNA
<213> Artificial Sequence

<400> 319
gtgaccgatg gcttcagttc 20

<210> 320
<211> 20
<212> DNA
<213> Artificial Sequence

<400> 320
atggataggc aggtggactt 20

<210> 321
<211> 19
<212> DNA
<213> Artificial Sequence

<400> 321
ggtcggagtc aacggattt 19

<210> 322
<211> 19
<212> DNA
<213> Artificial Sequence

<400> 322
ccagcatcgc cccacttga 19

<210> 323
<211> 20
<212> DNA
<213> Artificial Sequence

<400> 323
gtgaccgatg gcttcagttc 20

<210> 324
<211> 20
<212> DNA
<213> Artificial Sequence

<400> 324
atggataggc aggtggactt 20

<210> 325
<211> 24
<212> DNA
<213> Artificial Sequence

<400> 325
agcttctttg cagctccttc gttg 24

<210> 326
<211> 24
<212> DNA
<213> Artificial Sequence

<400> 326

ttctgaccca ttcccaccat caca 24

<210> 327
<211> 19
<212> RNA
<213> Artificial Sequence

<400> 327
uuaaaaggga caguauuca 19

<210> 328
<211> 21
<212> RNA
<213> Artificial Sequence

<400> 328
ugaauacugu cccuuuuaag c 21

<210> 329
<211> 21
<212> RNA
<213> Artificial Sequence

<400> 329
gcuuaaaagg gacaguauuc a 21

<210> 330
<211> 23
<212> RNA
<213> Artificial Sequence

<400> 330
ugaauacugu cccuuuuaag caa 23

<210> 331
<211> 19
<212> RNA
<213> Artificial Sequence

<400> 331
uuaaaaggga caguauuca 19

<210> 332
<211> 21
<212> RNA
<213> Artificial Sequence

<400> 332
ugaauacugu cccuuuuaag c 21

<210> 333
<211> 21
<212> RNA
<213> Artificial Sequence

<400> 333
gcuuaaaagg gacaguauuc a 21

<210> 334
<211> 23
<212> RNA
<213> Artificial Sequence

<400> 334
ugaauacugu cccuuuuaag caa 23

<210> 335
<211> 19
<212> RNA
<213> Artificial Sequence

<400> 335
uuaaaaggga caguauuca 19

<210> 336
<211> 21
<212> RNA
<213> Artificial Sequence

<400> 336
ugaauacugu cccuuuuaag c 21

<210> 337
<211> 21
<212> RNA
<213> Artificial Sequence

<400> 337
gcuuaaaagg gacaguauuc a 21

<210> 338
<211> 23
<212> RNA
<213> Artificial Sequence

<400> 338
ugaauacugu cccuuuuaag caa 23

<210> 339
<211> 19
<212> RNA
<213> Artificial Sequence

<400> 339
uuaaaaggga caguauuca 19

<210> 340
<211> 21
<212> RNA
<213> Artificial Sequence

<400> 340
ugaauacugu cccuuuuaag c 21

<210> 341
<211> 21
<212> RNA
<213> Artificial Sequence

<400> 341
gcuuaaaagg gacaguauuc a 21

<210> 342
<211> 23
<212> RNA

<213> Artificial Sequence

<400> 342
ugaauacugu cccuuuuaag caa 23

<210> 343
<211> 19
<212> RNA
<213> Artificial Sequence

<400> 343
uuaaaaggga caguauuca 19

<210> 344
<211> 21
<212> RNA
<213> Artificial Sequence

<400> 344
ugaauacugu cccuuuuaag c 21

<210> 345
<211> 21
<212> RNA
<213> Artificial Sequence

<400> 345
gcuuaaaagg gacaguauuc a 21

<210> 346
<211> 23
<212> RNA
<213> Artificial Sequence

<400> 346
ugaauacugu cccuuuuaag caa 23

<210> 347
<211> 19
<212> RNA
<213> Artificial Sequence

<400> 347
uuaaaaggga caguauuca 19

<210> 348
<211> 21
<212> RNA
<213> Artificial Sequence

<400> 348
ugaauacugu cccuuuuaag c 21

<210> 349
<211> 21
<212> RNA
<213> Artificial Sequence

<400> 349
gcuuaaaagg gacaguauuc a 21

<210> 350
<211> 23

<212> RNA
<213> Artificial Sequence

<400> 350
ugaauacugu cccuuuuaag caa 23

<210> 351
<211> 19
<212> RNA
<213> Artificial Sequence

<400> 351
acaguauucu cagugcuca 19

<210> 352
<211> 21
<212> RNA
<213> Artificial Sequence

<400> 352
ugagcacuga gaauacuguc c 21

<210> 353
<211> 21
<212> RNA
<213> Artificial Sequence

<400> 353
ggacaguauu cucagugcuc a 21

<210> 354
<211> 23
<212> RNA
<213> Artificial Sequence

<400> 354
ugagcacuga gaauacuguc ccu 23

<210> 355
<211> 19
<212> RNA
<213> Artificial Sequence

<400> 355
acaguauucu cagugcuca 19

<210> 356
<211> 21
<212> RNA
<213> Artificial Sequence

<400> 356
ugagcacuga gaauacuguc c 21

<210> 357
<211> 21
<212> RNA
<213> Artificial Sequence

<400> 357
ggacaguauu cucagugcuc a 21

<210> 358

<211> 23
<212> RNA
<213> Artificial Sequence

<400> 358
ugagcacuga gaauacuguc ccu 23

<210> 359
<211> 19
<212> RNA
<213> Artificial Sequence

<400> 359
acaguauucu cagugcuca 19

<210> 360
<211> 21
<212> RNA
<213> Artificial Sequence

<400> 360
ugagcacuga gaauacuguc c 21

<210> 361
<211> 21
<212> RNA
<213> Artificial Sequence

<400> 361
ggacaguauu cucagugcuc a 21

<210> 362
<211> 23
<212> RNA
<213> Artificial Sequence

<400> 362
ugagcacuga gaauacuguc ccu 23

<210> 363
<211> 19
<212> RNA
<213> Artificial Sequence

<400> 363
acaguauucu cagugcuca 19

<210> 364
<211> 21
<212> RNA
<213> Artificial Sequence

<400> 364
ugagcacuga gaauacuguc c 21

<210> 365
<211> 21
<212> RNA
<213> Artificial Sequence

<400> 365
ggacaguauu cucagugcuc a 21

<210> 366
<211> 23
<212> RNA
<213> Artificial Sequence

<400> 366
ugagcacuga gaauacuguc ccu 23

<210> 367
<211> 19
<212> RNA
<213> Artificial Sequence

<400> 367
acaguauucu cagugcuca 19

<210> 368
<211> 21
<212> RNA
<213> Artificial Sequence

<400> 368
ugagcacuga gaauacuguc c 21

<210> 369
<211> 21
<212> RNA
<213> Artificial Sequence

<400> 369
ggacaguauu cucagugcuc a 21

<210> 370
<211> 23
<212> RNA
<213> Artificial Sequence

<400> 370
ugagcacuga gaauacuguc ccu 23

<210> 371
<211> 19
<212> RNA
<213> Artificial Sequence

<400> 371
acaguauucu cagugcuca 19

<210> 372
<211> 21
<212> RNA
<213> Artificial Sequence

<400> 372
ugagcacuga gaauacuguc c 21

<210> 373
<211> 21
<212> RNA
<213> Artificial Sequence

<400> 373
ggacaguauu cucagugcuc a 21

<210> 374
<211> 23
<212> RNA
<213> Artificial Sequence

<400> 374
ugagcacuga gaauacuguc ccu 23

<210> 375
<211> 19
<212> RNA
<213> Artificial Sequence

<400> 375
aguauucuca gugcucuca 19

<210> 376
<211> 21
<212> RNA
<213> Artificial Sequence

<400> 376
ugagagcacu gagaauacug u 21

<210> 377
<211> 21
<212> RNA
<213> Artificial Sequence

<400> 377
acaguauucu cagugcucuc a 21

<210> 378
<211> 23
<212> RNA
<213> Artificial Sequence

<400> 378
ugagagcacu gagaauacug ucc 23

<210> 379
<211> 19
<212> RNA
<213> Artificial Sequence

<400> 379
aguauucuca gugcucuca 19

<210> 380
<211> 21
<212> RNA
<213> Artificial Sequence

<400> 380
ugagagcacu gagaauacug u 21

<210> 381
<211> 21
<212> RNA
<213> Artificial Sequence

<400> 381

acaguauucu cagugcucuc a 21

<210> 382
<211> 23
<212> RNA
<213> Artificial Sequence

<400> 382
ugagagcacu gagaauacug ucc 23

<210> 383
<211> 19
<212> RNA
<213> Artificial Sequence

<400> 383
aguauucuca gugcucuca 19

<210> 384
<211> 21
<212> RNA
<213> Artificial Sequence

<400> 384
ugagagcacu gagaauacug u 21

<210> 385
<211> 21
<212> RNA
<213> Artificial Sequence

<400> 385
acaguauucu cagugcucuc a 21

<210> 386
<211> 23
<212> RNA
<213> Artificial Sequence

<400> 386
ugagagcacu gagaauacug ucc 23

<210> 387
<211> 19
<212> RNA
<213> Artificial Sequence

<400> 387
aguauucuca gugcucuca 19

<210> 388
<211> 21
<212> RNA
<213> Artificial Sequence

<400> 388
ugagagcacu gagaauacug u 21

<210> 389
<211> 21
<212> RNA
<213> Artificial Sequence

<400> 389
acaguauucu cagugcucuc a 21

<210> 390
<211> 23
<212> RNA
<213> Artificial Sequence

<400> 390
ugagagcacu gagaauacug ucc 23

<210> 391
<211> 19
<212> RNA
<213> Artificial Sequence

<400> 391
aguauucuca gugcucuca 19

<210> 392
<211> 21
<212> RNA
<213> Artificial Sequence

<400> 392
ugagagcacu gagaauacug u 21

<210> 393
<211> 21
<212> RNA
<213> Artificial Sequence

<400> 393
acaguauucu cagugcucuc a 21

<210> 394
<211> 23
<212> RNA
<213> Artificial Sequence

<400> 394
ugagagcacu gagaauacug ucc 23

<210> 395
<211> 19
<212> RNA
<213> Artificial Sequence

<400> 395
aguauucuca gugcucuca 19

<210> 396
<211> 21
<212> RNA
<213> Artificial Sequence

<400> 396
ugagagcacu gagaauacug u 21

<210> 397
<211> 21
<212> RNA
<213> Artificial Sequence

<400> 397
acaguauucu cagugcucuc a 21

<210> 398
<211> 23
<212> RNA
<213> Artificial Sequence

<400> 398
ugagagcacu gagaauacug ucc 23

<210> 399
<211> 19
<212> RNA
<213> Artificial Sequence

<400> 399
ggacaguauu cucagugca 19

<210> 400
<211> 21
<212> RNA
<213> Artificial Sequence

<400> 400
ugcacugaga auacuguccc u 21

<210> 401
<211> 21
<212> RNA
<213> Artificial Sequence

<400> 401
agggacagua uucucagugc a 21

<210> 402
<211> 23
<212> RNA
<213> Artificial Sequence

<400> 402
ugcacugaga auacuguccc uuu 23

<210> 403
<211> 19
<212> RNA
<213> Artificial Sequence

<400> 403
ggacaguauu cucagugca 19

<210> 404
<211> 21
<212> RNA
<213> Artificial Sequence

<400> 404
ugcacugaga auacuguccc u 21

<210> 405
<211> 21
<212> RNA

<213> Artificial Sequence

<400> 405
agggacagua uucucagugc a 21

<210> 406
<211> 23
<212> RNA
<213> Artificial Sequence

<400> 406
ugcacugaga auacuguccc uuu 23

<210> 407
<211> 19
<212> RNA
<213> Artificial Sequence

<400> 407
ggacaguauu cucagugca 19

<210> 408
<211> 21
<212> RNA
<213> Artificial Sequence

<400> 408
ugcacugaga auacuguccc u 21

<210> 409
<211> 21
<212> RNA
<213> Artificial Sequence

<400> 409
agggacagua uucucagugc a 21

<210> 410
<211> 23
<212> RNA
<213> Artificial Sequence

<400> 410
ugcacugaga auacuguccc uuu 23

<210> 411
<211> 19
<212> RNA
<213> Artificial Sequence

<400> 411
ggacaguauu cucagugca 19

<210> 412
<211> 21
<212> RNA
<213> Artificial Sequence

<400> 412
ugcacugaga auacuguccc u 21

<210> 413
<211> 21

<212> RNA
<213> Artificial Sequence

<400> 413
agggacagua uucucagugc a 21

<210> 414
<211> 23
<212> RNA
<213> Artificial Sequence

<400> 414
ugcacugaga auacuguccc uuu 23

<210> 415
<211> 19
<212> RNA
<213> Artificial Sequence

<400> 415
ggacaguauu cucagugca 19

<210> 416
<211> 21
<212> RNA
<213> Artificial Sequence

<400> 416
ugcacugaga auacuguccc u 21

<210> 417
<211> 21
<212> RNA
<213> Artificial Sequence

<400> 417
agggacagua uucucagugc a 21

<210> 418
<211> 23
<212> RNA
<213> Artificial Sequence

<400> 418
ugcacugaga auacuguccc uuu 23

<210> 419
<211> 19
<212> RNA
<213> Artificial Sequence

<400> 419
ggacaguauu cucagugca 19

<210> 420
<211> 21
<212> RNA
<213> Artificial Sequence

<400> 420
ugcacugaga auacuguccc u 21

<210> 421

<211> 21
<212> RNA
<213> Artificial Sequence

<400> 421
agggacagua uucucagugc a 21

<210> 422
<211> 23
<212> RNA
<213> Artificial Sequence

<400> 422
ugcacugaga auacuguccc uuu 23

<210> 423
<211> 19
<212> RNA
<213> Artificial Sequence

<400> 423
uuaaaaggga caguauucu 19

<210> 424
<211> 21
<212> RNA
<213> Artificial Sequence

<400> 424
agaauacugu cccuuuuaag c 21

<210> 425
<211> 19
<212> RNA
<213> Artificial Sequence

<400> 425
ggacaguauu cucagugcu 19

<210> 426
<211> 21
<212> RNA
<213> Artificial Sequence

<400> 426
agcacugaga auacuguccc u 21

<210> 427
<211> 19
<212> RNA
<213> Artificial Sequence

<400> 427
acaguauucu cagugcucu 19

<210> 428
<211> 21
<212> RNA
<213> Artificial Sequence

<400> 428
agagcacuga gaauacuguc c 21

<210> 429
<211> 19
<212> RNA
<213> Artificial Sequence

<400> 429
uauucucagu gcucuccua 19

<210> 430
<211> 21
<212> RNA
<213> Artificial Sequence

<400> 430
uaggagagca cugagaauac u 21